# EUROPEAN PATENT APPLICATION

(11) **EP 4 803 517 A1**
(43) Date of publication of application: **09.09.2026**
(21) Application number: 24884973.9
(22) Date of filing: 01.11.2024
(51) Int. Cl.: C07D 413/04, C07D 307/93, C07D 491/048, A61K 31/343, A61P 35/00

(54) **PROTEIN TRANSLATION INHIBITOR**

(30) Priority: 03.11.2023 CN 202311459664; 16.01.2024 CN 202410063442; 22.10.2024 CN 202411479439
(71) Applicant: Duality Biologics (Shanghai) Co., Ltd., Shanghai 201204 (CN)
(72) Inventor: LI, Bing, Suzhou, Jiangsu 215000 (CN); LIN, Shengchao, Suzhou, Jiangsu 215000 (CN); ZHANG, Yu, Suzhou, Jiangsu 215000 (CN); HUA, Haiqing, Suzhou, Jiangsu 215000 (CN); ZHU, Zhongyuan, Suzhou, Jiangsu 215000 (CN)
(74) Representative: J A Kemp LLP
(86) International application number: PCT/CN2024/129371
(87) International publication number: WO 2025/092976

(57) **Abstract**

The present invention relates to the field of pharmaceutical chemistry, and in particular to a protein translation inhibitor as represented by formula (II) and a use thereof in treatment and/or prevention of diseases.

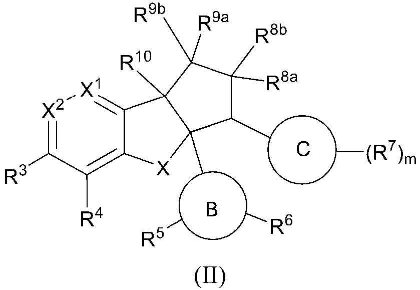

## Description

The present application is based on and claims priority to Chinese Patent Application No. CN202311459664.X filed on Nov. 3, 2023, Chinese Patent Application No. CN202410063442.4 filed on Jan. 16, 2024, and Chinese Patent Application No. CN202411479439.7 filed on Oct. 22, 2024, the disclosure of each of which is incorporated herein by reference in its entirety.

### TECHNICAL FIELD

The present disclosure pertains to the field of pharmaceutical chemistry, and particularly relates to a protein translation inhibitor and use thereof in the treatment and/or prevention of diseases.

### BACKGROUND

Abnormal protein translation is a common feature of malignant tumors, manifested as the up-regulation of oncoproteins, growth factors, and signal transduction proteins associated with proliferation, survival, and metastasis. The expression of oncogenic drivers is regulated by the eukaryotic translation initiation factor 4F (eIF4F) complex. eIF4F mediates the recruitment of ribosomes to mRNA, initiating the translation process from mRNA to protein.

The eIF4F complex consists of three subunits: the mRNA 5' cap-binding protein eIF4E, the scaffold protein eIF4G, and the RNA helicase eIF4A. Natural products have been reported to inhibit eIF4A-mediated translation and exhibit anti-proliferative and anti-tumor phenotypes both *in vivo* and *in vitro.* Taking Rocaglamide as an example, it has been demonstrated to bind to and stabilize the non-translating RNA/eIF4A complex, blocking ribosomal scanning, thereby inhibiting the translation of target mRNA and regulating the expression of related oncogenic factors.

Currently, there is a need to develop more protein translation inhibitors to meet the clinical demands of tumor diseases.

### SUMMARY

In a first aspect of the present disclosure, provided is a compound represented by formula II or a racemic mixture, an enantiomer, a diastereoisomer, a pharmaceutically acceptable salt, or an isotopically labeled compound thereof, or a mixture of the aforementioned forms: 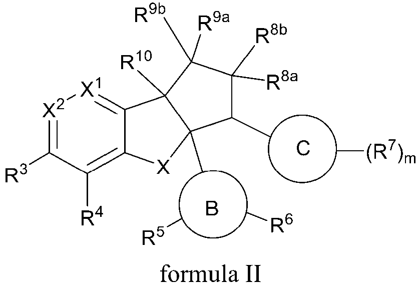 wherein
X¹ is selected from N and C(R¹), wherein R¹ is selected from hydrogen, halogen, cyano, -OH, - (C₁-C₆)alkyl, and -O-(C₁-C₆)alkyl;
X² is selected from N and C(R²), wherein R² is selected from hydrogen, halogen, cyano, -OH, - (C₁-C₆)alkyl, and -O-(C₁-C₆)alkyl;
R³ is selected from -(C₃-C₆)cycloalkyl, -(3- to 8-membered)heterocyclyl, -(C₆-C₁₀)aryl, and -(5-to 12-membered)heteroaryl, wherein the -(C₃-C₆)cycloalkyl, -(3- to 8-membered)heterocyclyl, - (C₆-C₁₀)aryl, and -(5- to 12-membered)heteroaryl are optionally substituted with one or more R³¹; each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, -(C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, - (C₆-C₁₀)aryl, -(5- to 12-membered)heteroaryl, -S(=O)₂-NH₂, -S(=O)₂-NH(C₁-C₆)alkyl, -S(=O)₂-N[(C₁-C₆)alkyl]₂, -NH-C(=O)H, -NH-C(=O)-(C₁-C₆)alkyl, -N[(C₁-C₆)alkyl]-C(=O)-(C₁-C₆)alkyl, N[(C₁-C₆)alkyl]-C(=O)H, -NH-S(=O)₂-(C₁-C₆)alkyl, -N[(C₁-C₆)alkyl]-S(=O)₂-(C₁-C₆)alkyl, - P(=O)[(C₁-C₆)alkyl]₂, -P(=O)[(C₁-C₆)alkyl]-NH₂, -P(=O)[(C₁-C₆)alkyl]-NH(C₁-C₆)alkyl, and - P(=O)[(C₁-C₆)alkyl]-N[(C₁-C₆)alkyl]₂, wherein the -(C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], -(C₁-C₆)alkylene-N[(C₁-C₆)alkyl]₂, - (C₆-C₁₀)aryl, -(5- to 12-membered)heteroaryl, -(C₁-C₆)alkylene-O(C₁-C₆)alkyl, and oxo;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4- to 10-membered)heterocyclyl, -(C₆-C₁₀)aryl, -O-(C₆-C₁₀)aryl, -(5-to 12-membered)heteroaryl, and -O-(5- to 12-membered)heteroaryl;
X is selected from O, S, NH, N[(C₁-C₆)alkyl], N[C(=O)-R^{c}], C(R^{a})(R^{b}), C(=O), C[=C(R^{a})R^{b}], S(=O), and S(=O)₂;
R^{a} and R^{b} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4-to 10-membered)heterocyclyl, -(C₆-C₁₀)aryl, -O-(C₆-C₁₀)aryl, -(5- to 12-membered)heteroaryl, and -O-(5- to 12-membered)heteroaryl;
ring B is selected from -(C₆-C₁₀)aryl and -(5- to 12-membered)heteroaryl;
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, oxo, -OH, -O-(C₁-C₄)alkyl, -NH₂, -NH(C₁-C₄)alkyl, and N[(C₁-C₄)alkyl]₂; or
R⁵ and R⁶, together with the carbon atoms to which they are attached, form 5-membered heterocyclyl;
ring C is selected from -(C₆-C₁₀)aryl and -(5- to 12-membered)heteroaryl;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, and -NHC(=O)-(C₁-C₆)alkyl, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, oxo, -OH, -O-(C₁-C₄)alkyl, -NH₂, -NH(C₁-C₄)alkyl, and -N[(C₁-C₄)alkyl]₂;
m is 1, 2, 3, 4, 5, 6, 7, 8, or 9;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -[(C₁-C₈)alkylene]N(R)C(=O)R, -[(C₁-C₈)alkylene]N(R)C(=O)N(R)R, -C(=O)R, -C(=O)N(R)R, -C(=O)[(C₁-C₈)alkylene]N(R)R, - C(=O)OR, -C(=S)N(R)R, -SR, -S(=O)R, -S(=O)₂R, -S(=O)₂N(R)R, -N(R)C(=O)R, - N(R)C(=O)N(R)R, -P(=O)(OR)(OR), -(C₆-C₁₀)aryl, -(5- to 12-membered)heteroaryl, -(C₃-C₆)cycloalkyl, and -(4- to 10-membered)heterocyclyl;
each R is independently selected from hydrogen, -OH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -(C₁-C₄)alkylene-O-(C₁-C₆)alkyl, -O-(C₁-C₆)haloalkyl, -S-(C₁-C₆)alkyl, -S(=O)₂-(C₁-C₆)alkyl, -(C₃-C₆)cycloalkyl, -(4- to 10-membered)heterocyclyl, -O-(C₃-C₆)cycloalkyl, -O-(4- to 10-membered)heterocyclyl, -C(=O)-H, - C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(C₃-C₆)cycloalkyl, -[(C₁-C₆)alkylene]-(4- to 10-membered)heterocyclyl, -(C₆-C₁₀)aryl, -[(C₁-C₆)alkylene]-(C₆-C₁₀)aryl, and -(5- to 12-membered)heteroaryl, wherein the -(C₁-C₆)alkylene-, -(C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, - NH₂, -NH(C₁-C₄)alkyl, N[(C₁-C₄)alkyl]₂, and (C₁-C₄)alkyl;
or R^{8a} and R^{8b} are combined to form oxo, -(C₂-C₆)alkenyl, -(C₃-C₆)cycloalkyl, or -(4- to 10-membered)heterocyclyl;
or R^{9a} and R^{9b} are combined to form oxo, -(C₂-C₆)alkenyl, -(C₃-C₆)cycloalkyl, or -(4- to 10-membered)heterocyclyl;
or R^{8a} and R^{9a}, together with the carbon atoms to which they are attached, form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, - O-(C₁-C₄)alkyl, -NH₂, -NH(C₁-C₄)alkyl, -N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
or R^{8b} and R^{9b}, together with the carbon atoms to which they are attached, form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, - O-(C₁-C₄)alkyl, -NH₂, -NH(C₁-C₄)alkyl, -N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
R¹⁰ is selected from hydrogen, cyano, -OH, and -NH₂; or R¹⁰ and R^{9a}, together with the atoms to which they are attached, form (5- to 6-membered)heterocyclyl, wherein the (5- to 6-membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

In the first aspect of the present disclosure, also provided is a compound represented by formula II or a racemic mixture, an enantiomer, a diastereoisomer, a pharmaceutically acceptable salt, or an isotopically labeled compound thereof, or a mixture of the aforementioned forms: 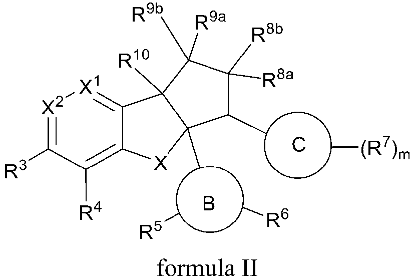 wherein
X¹ is selected from N and C(R¹), wherein R¹ is selected from hydrogen, halogen, cyano, -OH, - (C₁-C₆)alkyl, and -O-(C₁-C₆)alkyl;
X² is selected from N and C(R²), wherein R² is selected from hydrogen, halogen, cyano, -OH, - (C₁-C₆)alkyl, and -O-(C₁-C₆)alkyl;
R³ is selected from -(C₃-C₆)cycloalkyl, -(3- to 8-membered)heterocyclyl, -(C₆-C₁₀)aryl, and -(5-to 12-membered)heteroaryl, and R³ is optionally substituted with one or more R³¹;
each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, -(C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, and -C(=O)-N[(C₁-C₆)alkyl]₂, wherein the -(C₁-C₆)alkyl, -(C₂-C₆)alkenyl, -(C₂-C₆)alkynyl, -(C₃-C₆)cycloalkyl, and - (4- to 10-membered)heterocyclyl are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], and -(C₁-C₆)alkylene-N[(C₁-C₆)alkyl]₂;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4- to 10-membered)heterocyclyl, -(C₆-C₁₀)aryl, -O-(C₆-C₁₀)aryl, -(5-to 12-membered)heteroaryl, and -O-(5- to 12-membered)heteroaryl;
X is selected from O, S, NH, N[(C₁-C₆)alkyl], N[C(=O)-R^{c}], C(R^{a})(R^{b}), C(=O), C[=C(R^{a})R^{b}], S(=O), and S(=O)₂;
R^{a} and R^{b} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4-to 10-membered)heterocyclyl, -(C₆-C₁₀)aryl, -O-(C₆-C₁₀)aryl, -(5- to 12-membered)heteroaryl, and -O-(5- to 12-membered)heteroaryl;
ring B is selected from -(C₆-C₁₀)aryl and -(5- to 12-membered)heteroaryl;
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, -OH, -O-(C₁-C₄)alkyl, -NH₂, -NH(C₁-C₄)alkyl, and N[(C₁-C₄)alkyl]₂; or
R⁵ and R⁶, together with the carbon atoms to which they are attached, form 5-membered heterocyclyl;
ring C is selected from -(C₆-C₁₀)aryl and -(5- to 12-membered)heteroaryl;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, and -C(=O)-N[(C₁-C₆)alkyl]₂, wherein the -(C₁-C₆)alkyl, - (C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, -OH, -O-(C₁-C₄)alkyl, -NH₂, -NH(C₁-C₄)alkyl, and -N[(C₁-C₄)alkyl]₂;
m is 1, 2, 3, 4, 5, 6, 7, 8, or 9;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -C(=O)R, -C(=O)N(R)R, -C(=O)[(C₁-C₈)alkylene]N(R)R, -C(=O)OR, -C(=S)N(R)R, -SR, -S(=O)R, -S(=O)₂R, -S(=O)₂N(R)R, - N(R)C(=O)R, -N(R)C(=O)N(R)R, -P(=O)(OR)(OR), -(C₆-C₁₀)aryl, -(5- to 12-membered)heteroaryl, -(C₃-C₆)cycloalkyl, and -(4- to 10-membered)heterocyclyl;
each R is independently selected from hydrogen, -OH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -(C₃-C₆)cycloalkyl, -(4- to 10-membered)heterocyclyl, -O-(C₃-C₆)cycloalkyl, -O-(4- to 10-membered)heterocyclyl, -C(=O)-H, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(4- to 10-membered)heterocyclyl, -(C₆-C₁₀)aryl, -[(C₁-C₆)alkylene]-(C₆-C₁₀)aryl, and -(5- to 12-membered)heteroaryl, wherein the -(C₁-C₆)alkylene-, - (C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, - NH₂, -NH(C₁-C₄)alkyl, and N[(C₁-C₄)alkyl]₂;
or R^{8a} and R^{8b}, and R^{9a} and R^{9b} are independently combined to form oxo, -(C₂-C₆)alkenyl, -(C₃-C₆)cycloalkyl, or -(4- to 10-membered)heterocyclyl;
or R^{8a} and R^{9a}, and R^{8b} and R^{9b}, together with the carbon atoms to which they are respectively attached, independently form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, -O-(C₁-C₄)alkyl, -NH₂, -NH(C₁-C₄)alkyl, - N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
R¹⁰ is selected from hydrogen, cyano, -OH, and -NH₂; or R¹⁰ and R^{9b}, together with the atoms to which they are attached, form (5- to 6-membered)heterocyclyl, wherein the (5- to 6-membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

In certain embodiments, the compound is selected from a compound represented by formula I: 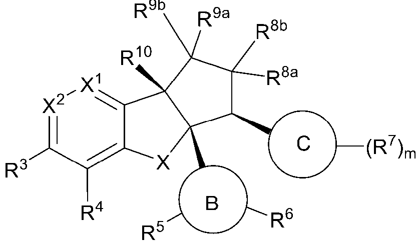 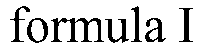 wherein
X¹ is selected from N and C(R¹), wherein R¹ is selected from hydrogen, halogen, cyano, -OH, - (C₁-C₆)alkyl, and -O-(C₁-C₆)alkyl;
X² is selected from N and C(R²), wherein R² is selected from hydrogen, halogen, cyano, -OH, - (C₁-C₆)alkyl, and -O-(C₁-C₆)alkyl;
R³ is selected from -(C₃-C₆)cycloalkyl, -(3- to 8-membered)heterocyclyl, -(C₆-C₁₀)aryl, and -(5-to 12-membered)heteroaryl, wherein the -(C₃-C₆)cycloalkyl, -(3- to 8-membered)heterocyclyl, - (C₆-C₁₀)aryl, and -(5- to 12-membered)heteroaryl are optionally substituted with one or more R³¹;
each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, -(C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, - (C₆-C₁₀)aryl, -(5- to 12-membered)heteroaryl, -S(=O)₂-NH₂, -S(=O)₂-NH(C₁-C₆)alkyl, -S(=O)₂-N[(C₁-C₆)alkyl]₂, -NH-C(=O)H, -NH-C(=O)-(C₁-C₆)alkyl, -N[(C₁-C₆)alkyl]-C(=O)-(C₁-C₆)alkyl, N[(C₁-C₆)alkyl]-C(=O)H, -NH-S(=O)₂-(C₁-C₆)alkyl, -N[(C₁-C₆)alkyl]-S(=O)₂-(C₁-C₆)alkyl, - P(=O)[(C₁-C₆)alkyl]₂, -P(=O)[(C₁-C₆)alkyl]-NH₂, -P(=O)[(C₁-C₆)alkyl]-NH(C₁-C₆)alkyl, and - P(=O)[(C₁-C₆)alkyl]-N[(C₁-C₆)alkyl]₂, wherein the -(C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], -(C₁-C₆)alkylene-N[(C₁-C₆)alkyl]₂, - (C₆-C₁₀)aryl, -(5- to 12-membered)heteroaryl, -(C₁-C₆)alkylene-O(C₁-C₆)alkyl, and oxo;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4- to 10-membered)heterocyclyl, -(C₆-C₁₀)aryl, -O-(C₆-C₁₀)aryl, -(5-to 12-membered)heteroaryl, and -O-(5- to 12-membered)heteroaryl;
X is selected from O, S, NH, N[(C₁-C₆)alkyl], N[C(=O)-R^{c}], C(R^{a})(R^{b}), C(=O), C[=C(R^{a})R^{b}], S(=O), and S(=O)₂;
R^{a} and R^{b} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4-to 10-membered)heterocyclyl, -(C₆-C₁₀)aryl, -O-(C₆-C₁₀)aryl, -(5- to 12-membered)heteroaryl, and -O-(5- to 12-membered)heteroaryl;
ring B is selected from -(C₆-C₁₀)aryl and -(5- to 12-membered)heteroaryl;
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, oxo, -OH, -O-(C₁-C₄)alkyl, -NH₂, -NH(C₁-C₄)alkyl, and N[(C₁-C₄)alkyl]₂; or
R⁵ and R⁶, together with the carbon atoms to which they are attached, form 5-membered heterocyclyl;
ring C is selected from -(C₆-C₁₀)aryl and -(5- to 12-membered)heteroaryl;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, and -NHC(=O)-(C₁-C₆)alkyl, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, oxo, -OH, -O-(C₁-C₄)alkyl, -NH₂, -NH(C₁-C₄)alkyl, and -N[(C₁-C₄)alkyl]₂;
m is 1, 2, 3, 4, 5, 6, 7, 8, or 9;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -[(C₁-C₈)alkylene]NHC(=O)R, -[(C₁-C₈)alkylene]N(R)C(=O)N(R)R, -C(=O)R, -C(=O)N(R)R, -C(=O)[(C₁-C₈)alkylene]N(R)R, - C(=O)OR, -C(=S)N(R)R, -SR, -S(=O)R, -S(=O)₂R, -S(=O)₂N(R)R, -N(R)C(=O)R, - N(R)C(=O)N(R)R, -P(=O)(OR)(OR), -(C₆-C₁₀)aryl, -(5- to 12-membered)heteroaryl, -(C₃-C₆)cycloalkyl, and -(4- to 10-membered)heterocyclyl;
each R is independently selected from hydrogen, -OH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -(C₁-C₄)alkylene-O-(C₁-C₆)alkyl, -O-(C₁-C₆)haloalkyl, -S-(C₁-C₆)alkyl, -S(=O)₂-(C₁-C₆)alkyl, -(C₃-C₆)cycloalkyl, -(4- to 10-membered)heterocyclyl, -O-(C₃-C₆)cycloalkyl, -O-(4- to 10-membered)heterocyclyl, -C(=O)-H, - C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(C₃-C₆)cycloalkyl, -[(C₁-C₆)alkylene]-(4- to 10-membered)heterocyclyl, -(C₆-C₁₀)aryl, -[(C₁-C₆)alkylene]-(C₆-C₁₀)aryl, and -(5- to 12-membered)heteroaryl, wherein the -(C₁-C₆)alkylene-, -(C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, - NH₂, -NH(C₁-C₄)alkyl, N[(C₁-C₄)alkyl]₂, and (C₁-C₄)alkyl;
or R^{8a} and R^{8b} are combined to form oxo, -(C₂-C₆)alkenyl, -(C₃-C₆)cycloalkyl, or -(4- to 10-membered)heterocyclyl;
or R^{9a} and R^{9b} are combined to form oxo, -(C₂-C₆)alkenyl, -(C₃-C₆)cycloalkyl, or -(4- to 10-membered)heterocyclyl;
or R^{8a} and R^{9a}, together with the carbon atoms to which they are attached, form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, - O-(C₁-C₄)alkyl, -NH₂, -NH(C₁-C₄)alkyl, -N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
or R^{8b} and R^{9b}, together with the carbon atoms to which they are attached, form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, - O-(C₁-C₄)alkyl, -NH₂, -NH(C₁-C₄)alkyl, -N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
R¹⁰ is selected from hydrogen, cyano, -OH, and -NH₂; or R¹⁰ and R^{9a}, together with the atoms to which they are attached, form (5- to 6-membered)heterocyclyl, wherein the (5- to 6-membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

In certain embodiments, the compound is selected from a compound represented by formula I: 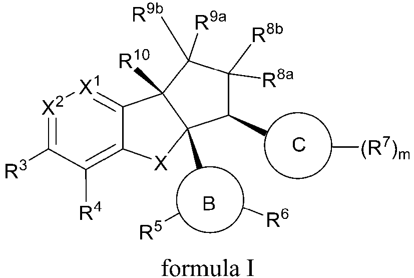 wherein
X¹ is selected from N and C(R¹), wherein R¹ is selected from hydrogen, halogen, cyano, -OH, - (C₁-C₆)alkyl, and -O-(C₁-C₆)alkyl;
X² is selected from N and C(R²), wherein R² is selected from hydrogen, halogen, cyano, -OH, - (C₁-C₆)alkyl, and -O-(C₁-C₆)alkyl;
R³ is selected from -(C₃-C₆)cycloalkyl, -(3- to 8-membered)heterocyclyl, -(C₆-C₁₀)aryl, and -(5-to 12-membered)heteroaryl, and R³ is optionally substituted with one or more R³¹;
each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, -(C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, and -C(=O)-N[(C₁-C₆)alkyl]₂, wherein the -(C₁-C₆)alkyl, -(C₂-C₆)alkenyl, -(C₂-C₆)alkynyl, -(C₃-C₆)cycloalkyl, and - (4- to 10-membered)heterocyclyl are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], and -(C₁-C₆)alkylene-N[(C₁-C₆)alkyl]₂;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4- to 10-membered)heterocyclyl, -(C₆-C₁₀)aryl, -O-(C₆-C₁₀)aryl, -(5-to 12-membered)heteroaryl, and -O-(5- to 12-membered)heteroaryl;
X is selected from O, S, NH, N[(C₁-C₆)alkyl], N[C(=O)-R^{c}], C(R^{a})(R^{b}), C(=O), C[=C(R^{a})R^{b}], S(=O), and S(=O)₂;
R^{a} and R^{b} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4-to 10-membered)heterocyclyl, -(C₆-C₁₀)aryl, -O-(C₆-C₁₀)aryl, -(5- to 12-membered)heteroaryl, and -O-(5- to 12-membered)heteroaryl;
ring B is selected from -(C₆-C₁₀)aryl and -(5- to 12-membered)heteroaryl;
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, -OH, -O-(C₁-C₄)alkyl, -NH₂, -NH(C₁-C₄)alkyl, and N[(C₁-C₄)alkyl]₂; or
R⁵ and R⁶, together with the carbon atoms to which they are attached, form 5-membered heterocyclyl;
ring C is selected from -(C₆-C₁₀)aryl and -(5- to 12-membered)heteroaryl;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, and -C(=O)-N[(C₁-C₆)alkyl]₂, wherein the -(C₁-C₆)alkyl, - (C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, -OH, -O-(C₁-C₄)alkyl, -NH₂, -NH(C₁-C₄)alkyl, and -N[(C₁-C₄)alkyl]₂;
m is 1, 2, 3, 4, 5, 6, 7, 8, or 9;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -C(=O)R, -C(=O)N(R)R, -C(=O)[(C₁-C₈)alkylene]N(R)R, -C(=O)OR, -C(=S)N(R)R, -SR, -S(=O)R, -S(=O)₂R, -S(=O)₂N(R)R, - N(R)C(=O)R, -N(R)C(=O)N(R)R, -P(=O)(OR)(OR), -(C₆-C₁₀)aryl, -(5- to 12-membered)heteroaryl, -(C₃-C₆)cycloalkyl, and -(4- to 10-membered)heterocyclyl;
each R is independently selected from hydrogen, -OH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -(C₃-C₆)cycloalkyl, -(4- to 10-membered)heterocyclyl, -O-(C₃-C₆)cycloalkyl, -O-(4- to 10-membered)heterocyclyl, -C(=O)-H, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(4- to 10-membered)heterocyclyl, -(C₆-C₁₀)aryl, -[(C₁-C₆)alkylene]-(C₆-C₁₀)aryl, and -(5- to 12-membered)heteroaryl, wherein the -(C₁-C₆)alkylene-, - (C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, - NH₂, -NH(C₁-C₄)alkyl, and N[(C₁-C₄)alkyl]₂;
or R^{8a} and R^{8b}, and R^{9a} and R^{9b} are independently combined to form oxo, -(C₂-C₆)alkenyl, -(C₃-C₆)cycloalkyl, or -(4- to 10-membered)heterocyclyl;
or R^{8a} and R^{9a}, and R^{8b} and R^{9b}, together with the carbon atoms to which they are respectively attached, independently form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, -O-(C₁-C₄)alkyl, -NH₂, -NH(C₁-C₄)alkyl, - N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
R¹⁰ is selected from hydrogen, cyano, -OH, and -NH₂; or R¹⁰ and R^{9b}, together with the atoms to which they are attached, form (5- to 6-membered)heterocyclyl, wherein the (5- to 6-membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

In certain embodiments, the compound is selected from a compound represented by formula I: 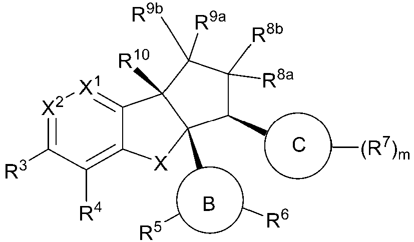 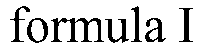 wherein
X¹ is selected from N and C(R¹), wherein R¹ is selected from hydrogen, halogen, cyano, -OH, - (C₁-C₆)alkyl, and -O-(C₁-C₆)alkyl;
X² is selected from N and C(R²), wherein R² is selected from hydrogen, halogen, cyano, -OH, - (C₁-C₆)alkyl, and -O-(C₁-C₆)alkyl;
R³ is selected from -(C₃-C₆)cycloalkyl, -(4- to 8-membered)heterocyclyl, -(C₆-C₁₀)aryl, and -(5-to 12-membered)heteroaryl, and R³ is optionally substituted with one or more R³¹;
each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, -(C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, and -C(=O)-N[(C₁-C₆)alkyl]₂, wherein the -(C₁-C₆)alkyl, -(C₂-C₆)alkenyl, -(C₂-C₆)alkynyl, -(C₃-C₆)cycloalkyl, and - (4- to 10-membered)heterocyclyl are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], and -(C₁-C₆)alkylene-N[(C₁-C₆)alkyl]₂;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4- to 10-membered)heterocyclyl, -(C₆-C₁₀)aryl, -O-(C₆-C₁₀)aryl, -(5-to 12-membered)heteroaryl, and -O-(5- to 12-membered)heteroaryl;
X is selected from O, S, NH, N[(C₁-C₆)alkyl], N[C(=O)-R^{c}], C(R^{a})(R^{b}), C(=O), C[=C(R^{a})R^{b}], S(=O), and S(=O)₂;
R^{a} and R^{b} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4-to 10-membered)heterocyclyl, -(C₆-C₁₀)aryl, -O-(C₆-C₁₀)aryl, -(5- to 12-membered)heteroaryl, and -O-(5- to 12-membered)heteroaryl;
ring B is selected from -(C₆-C₁₀)aryl and -(5- to 12-membered)heteroaryl;
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, -OH, -O-(C₁-C₄)alkyl, -NH₂, -NH(C₁-C₄)alkyl, and N[(C₁-C₄)alkyl]₂; or R⁵ and R⁶, together with the carbon atoms to which they are attached, form 5-membered heterocyclyl;
ring C is selected from -(C₆-C₁₀)aryl and -(5- to 12-membered)heteroaryl;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, and -C(=O)-N[(C₁-C₆)alkyl]₂, wherein the -(C₁-C₆)alkyl, - (C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, -OH, -O-(C₁-C₄)alkyl, -NH₂, -NH(C₁-C₄)alkyl, and -N[(C₁-C₄)alkyl]₂;
m is 1, 2, 3, 4, 5, 6, 7, 8, or 9;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -C(=O)R, -C(=O)N(R)R, -C(=O)[(C₁-C₈)alkylene]N(R)R, -C(=O)OR, -C(=S)N(R)R, -SR, -S(=O)R, -S(=O)₂R, -S(=O)₂N(R)R, - N(R)C(=O)R, -N(R)C(=O)N(R)R, -P(=O)(OR)(OR), -(C₆-C₁₀)aryl, -(5- to 12-membered)heteroaryl, -(C₃-C₆)cycloalkyl, and -(4- to 10-membered)heterocyclyl;
each R is independently selected from hydrogen, -OH, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -(C₃-C₆)cycloalkyl, -(4- to 10-membered)heterocyclyl, -O-(C₃-C₆)cycloalkyl, -O-(4- to 10-membered)heterocyclyl, -C(=O)-H, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(4- to 10-membered)heterocyclyl, -(C₆-C₁₀)aryl, -[(C₁-C₆)alkylene]-(C₆-C₁₀)aryl, and -(5- to 12-membered)heteroaryl, wherein the -(C₁-C₆)alkylene-, - (C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, - NH₂, -NH(C₁-C₄)alkyl, and N[(C₁-C₄)alkyl]₂;
or R^{8a} and R^{8b}, and R^{9a} and R^{9b} are independently combined to form oxo, -(C₂-C₆)alkenyl, -(C₃-C₆)cycloalkyl, or -(4- to 10-membered)heterocyclyl;
or R^{8a} and R^{9a}, and R^{8b} and R^{9b}, together with the carbon atoms to which they are respectively attached, independently form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, -O-(C₁-C₄)alkyl, -NH₂, -NH(C₁-C₄)alkyl, - N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
R¹⁰ is selected from hydrogen, cyano, -OH, and -NH₂; or R¹⁰ and R^{9b}, together with the atoms to which they are attached, form (5- to 6-membered)heterocyclyl, wherein the (5- to 6-membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

In certain embodiments, the -N(R^{c})R^{c} is -NHR^{c}.

In certain embodiments, the -N(R)R is -NHR.

In certain embodiments, the -[(C₁-C₈)alkylene]N(R)R is -[(C₁-C₈)alkylene]NHR.

In certain embodiments, the -C(=O)N(R)R is -C(=O)NHR.

In certain embodiments, the -C(=O)[(C₁-C₈)alkylene]N(R)R is -C(=O)[(C₁-C₈)alkylene]NHR.

In certain embodiments, the -C(=S)N(R)R is -C(=S)NHR.

In certain embodiments, the -S(=O)₂N(R)R is -S(=O)₂NHR.

In certain embodiments, the -N(R)C(=O)R is -NHC(=O)R.

In certain embodiments, the -N(R)C(=O)N(R)R is -NHC(=O)N(R)R, -N(R)C(=O)NHR-, or NHC(=O)NHR.

In certain embodiments, the -P(=O)(OR)(OR) is -P(=O)(OH)(OR).

In certain embodiments, R¹ is selected from hydrogen, halogen, cyano, -OH, -(C₁-C₄)alkyl, and - O-(C₁-C₄)alkyl.

In certain embodiments, R¹ is selected from hydrogen, fluorine, chlorine, cyano, -OH, methyl, ethyl, propyl, -O-methyl, -O-ethyl, and -O-propyl.

In certain embodiments, R¹ is selected from hydrogen and -O-methyl.

In certain embodiments, R¹ is -O-methyl, and X¹ is selected from N and C(OCH₃).

In certain embodiments, X¹ is selected from C(R¹), and R1 is as defined in any one of the embodiments of the present disclosure.

In certain embodiments, X¹ is C(OCH₃).

In certain embodiments, X¹ is N.

In certain embodiments, R² is selected from hydrogen, halogen, cyano, -OH, -(C₁-C₄)alkyl, and - O-(C₁-C₄)alkyl.

In certain embodiments, R² is selected from hydrogen, fluorine, chlorine, cyano, -OH, methyl, ethyl, propyl, -O-methyl, -O-ethyl, and -O-propyl.

In certain embodiments, R² is hydrogen, and X² is selected from N and CH.

In certain embodiments, X² is selected from C(R²).

In certain embodiments, X² is selected from CH.

In certain embodiments, R³¹ is selected from oxo, hydrogen, halogen, cyano, nitro, -OH, -SH, - NH₂, -(C₁-C₄)alkyl, -(C₁-C₄)alkylene-OH, -(C₁-C₄)alkylene-N[(C₁-C₄)alkyl]₂, -(C₂-C₄)alkenyl, - (C₂-C₄)alkynyl, -(C₁-C₄)haloalkyl, -O-(C₁-C₄)alkyl, -O-(C₁-C₄)haloalkyl, -S-(C₁-C₄)alkyl, - NH(C₁-C₄)alkyl, -N[(C₁-C₄)alkyl]₂, -(C₃-C₆)cycloalkyl, -(4- to 8-membered)heterocyclyl, -O-(C₃-C₆)cycloalkyl, -O-(4- to 8-membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, and -C(=O)-N[(C₁-C₄)alkyl]₂.

In certain embodiments, R³¹ is selected from oxo, hydrogen, halogen, cyano, nitro, -OH, -SH, - NH₂, methyl, ethyl, propyl, -methylene-OH, -ethylene-OH, -propylene-OH, -methylene-N(methyl)₂, -ethylene-N(methyl)₂, -propylene-N(methyl)₂, vinyl, propenyl, ethynyl, propynyl, halomethyl, haloethyl, halopropyl, -O-methyl, -O-ethyl, -O-propyl, -O-halomethyl, -O-haloethyl, -O-halopropyl, -S-methyl, -S-ethyl, -S-propyl, -NH-methyl, -NH-ethyl, -NH-propyl, -N(methyl)₂, -N(methyl)-ethyl, -N(methyl)-propyl, -N(ethyl)₂, -N(ethyl)-propyl, cyclopropyl, cyclopentyl, cyclohexyl, oxetanyl, tetrahydrofuranyl, tetrahydropyranyl, 1,4-dioxanyl, azetidinyl, pyrrolidinyl, piperidinyl, piperazinyl, homopiperidinyl, homopiperazinyl, morpholinyl, -O-cyclopropyl, -O-cyclopentyl, -O-cyclohexyl, -O-tetrahydrofuranyl, -O-tetrahydropyranyl, -O-1,4-dioxanyl, -O-pyrrolidinyl, -O-piperidinyl, -O-piperazinyl, -O-morpholinyl, -C(=O)-methyl, -C(=O)-ethyl, - C(=O)-propyl, -C(=O)-cyclopropyl, -C(=O)-cyclopentyl, -C(=O)-cyclohexyl, -C(=O)-O-methyl, - C(=O)-O-ethyl, -C(=O)-O-propyl, -C(=O)-O-cyclopropyl, -C(=O)-O-cyclopentyl, -C(=O)-O-cyclohexyl, -C(=O)-NH₂, -C(=O)-NH-methyl, -C(=O)-NH-ethyl, -C(=O)-NH-propyl, -C(=O)-N(methyl)₂, -C(=O)-N(methyl)-ethyl, and -C(=O)-N(ethyl)₂.

In certain embodiments, R³¹ is selected from oxo, hydrogen, fluorine, chlorine, cyano, -OH, -SH, -NH₂, methyl, halomethyl, -O-methyl, -S-methyl, -NH-methyl, -CH₂OH, -CH₂CH₂N(CH₃)₂, - C(=O)-NH₂, and cyclopropyl.

In certain embodiments, R³¹ is selected from oxo, hydrogen, fluorine, cyano, -NH₂, -CH₂OH, - CH₂NH₂, methyl, trifluoromethyl, and -O-methyl.

In certain embodiments, R³¹ is selected from oxo, hydrogen, -NH₂, -CH₂OH, and methyl.

In certain embodiments, R³¹ is selected from hydrogen, fluorine, chlorine, cyano, -OH, -SH, -NH₂, -(C₁-C₄)alkyl, -O-(C₁-C₄)alkyl, -NH-(C₁-C₄)alkyl, -(C₁-C₄)alkylene-OH, -(C₁-C₄)haloalkyl, -(C₁-C₄)alkylene-NH₂, -(C₁-C₄)alkylene-NH(C₁-C₄)alkyl, and -(C₁-C₄)alkylene-N[(C₁-C₄)alkyl]₂.

In certain embodiments, R³¹ is selected from hydrogen, fluorine, chlorine, cyano, -OH, -SH, -NH₂, -(C₁-C₄)alkyl, -(C₁-C₄)alkylene-OH, -(C₁-C₄)haloalkyl, -(C₁-C₄)alkylene-NH₂, -(C₁-C₄)alkylene-NH(C₁-C₄)alkyl, and -(C₁-C₄)alkylene-N[(C₁-C₄)alkyl]₂.

In certain embodiments, R³¹ is selected from hydrogen, fluorine, chlorine, cyano, -OH, -SH, -NH₂, -(C₁-C₄)alkyl, -(C₁-C₄)haloalkyl, -O-(C₁-C₄)alkyl, and -(C₁-C₄)alkylene-OH.

In certain embodiments, R³¹ is selected from hydrogen, fluorine, chlorine, cyano, -OH, -SH, -NH₂, -(C₁-C₄)alkyl, and -(C₁-C₄)alkylene-OH.

In certain embodiments, R³¹ is selected from hydrogen, fluorine, chlorine, cyano, -OH, -SH, -NH₂, methyl, halomethyl, -O-methyl, -CH₂OH, -CH₂NH₂, and -CH₂CH₂N(CH₃)₂.

In certain embodiments, R³¹ is selected from hydrogen, fluorine, chlorine, cyano, -OH, -SH, -NH₂, methyl, halomethyl, -CH₂OH, and -CH₂CH₂N(CH₃)₂.

In certain embodiments, R³¹ is selected from hydrogen, fluorine, chlorine, cyano, -OH, -SH, and - NH₂.

In certain embodiments, R³¹ is selected from hydrogen, fluorine, chlorine, cyano, -OH, -SH, -NH₂, -(C₁-C₄)alkyl, -O-(C₁-C₄)alkyl, -(C₁-C₄)alkylene-OH, and -(C₁-C₄)haloalkyl.

In certain embodiments, R³¹ is selected from hydrogen, fluorine, cyano, -NH₂, methyl, trifluoromethyl, -O-methyl, and -CH₂OH.

In certain embodiments, R³ is selected from -(C₃-C₆)cycloalkyl, -(3- to 6-membered)heterocyclyl, -(C₆-C₁₀)aryl, and -(5- to 12-membered)heteroaryl, wherein the -(C₃-C₆)cycloalkyl, -(3- to 6-membered)heterocyclyl, -(C₆-C₁₀)aryl, and -(5- to 12-membered)heteroaryl are optionally substituted with one or more R³¹, and R³¹ is as defined in any one of the embodiments of the present disclosure.

In certain embodiments, R³ is selected from -(3- to 6-membered)heterocyclyl, -(C₆-C₁₀)aryl, and - (5- to 12-membered)heteroaryl, wherein the -(3- to 6-membered)heterocyclyl, -(C₆-C₁₀)aryl, and - (5- to 12-membered)heteroaryl are optionally substituted with one or more R³¹, and R³¹ is as defined in any one of the embodiments of the present disclosure.

In certain embodiments, R³ is selected from -(C₆-C₁₀)aryl and -(5- to 12-membered)heteroaryl, wherein the -(C₆-C₁₀)aryl and -(5- to 12-membered)heteroaryl are optionally substituted with one or more R³¹, and R³¹ is as defined in any one of the embodiments of the present disclosure.

In certain embodiments, R³ is selected from phenyl and -(5- to 10-membered)heteroaryl, wherein the phenyl and -(5- to 10-membered)heteroaryl are optionally substituted with one or more R³¹, and R³¹ is as defined in any one of the embodiments of the present disclosure.

In certain embodiments, R³ is selected from phenyl and -(5- to 9-membered)heteroaryl, wherein the phenyl and -(5- to 9-membered)heteroaryl are optionally substituted with one or more R³¹, and R³¹ is as defined in any one of the embodiments of the present disclosure.

In certain embodiments, R³ is selected from -(5- to 6-membered)heteroaryl, wherein the -(5- to 6-membered)heteroaryl is optionally substituted with 1, 2, 3, or 4 R³¹, and R³¹ is as defined in any one of the embodiments of the present disclosure.

In certain embodiments, R³ is selected from oxazolyl, thiazolyl, pyrazolyl, isoxazolyl, isothiazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl, wherein the oxazolyl, thiazolyl, pyrazolyl, isoxazolyl, isothiazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl are optionally substituted with 1, 2, or 3 R³¹, and R³¹ is as defined in any one of the embodiments of the present disclosure.

In certain embodiments, R³ is selected from oxazolyl, thiazolyl, pyrazolyl, isoxazolyl, isothiazolyl, imidazolyl, triazolyl, tetrazolyl, pyridinyl, pyrazinyl, pyridazinyl, and pyrimidinyl, wherein the oxazolyl, thiazolyl, pyrazolyl, isoxazolyl, isothiazolyl, imidazolyl, triazolyl, tetrazolyl, pyridinyl, pyrazinyl, pyridazinyl, and pyrimidinyl are optionally substituted with 1, 2, or 3 R³¹, and R³¹ is as defined in any one of the embodiments of the present disclosure.

In certain embodiments, R³ is selected from 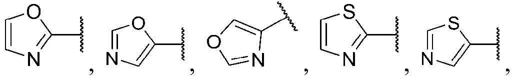 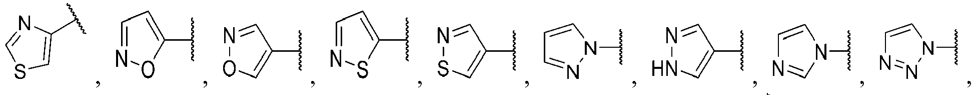 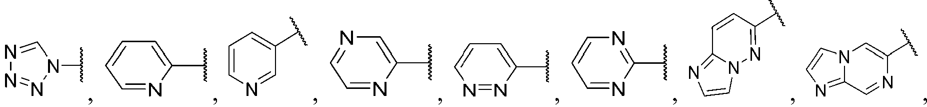 and 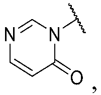 wherein the 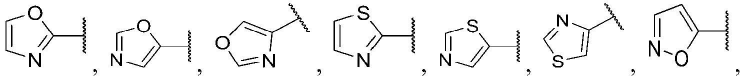 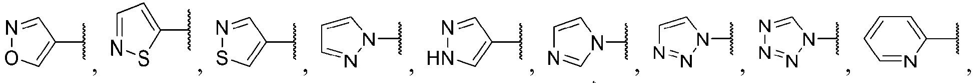 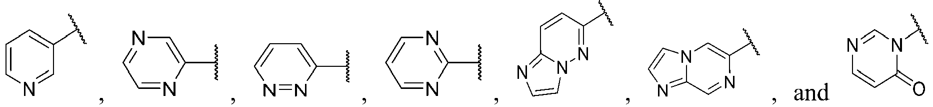 are optionally substituted with 1, 2, or 3 R³¹, and R³¹ is as defined in any one of the embodiments of the present disclosure.

In certain embodiments, R³ is selected from 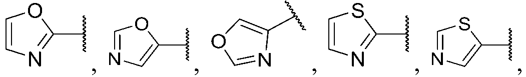 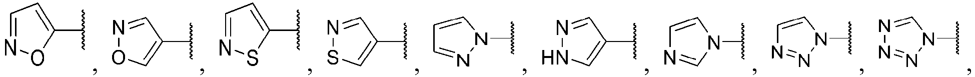 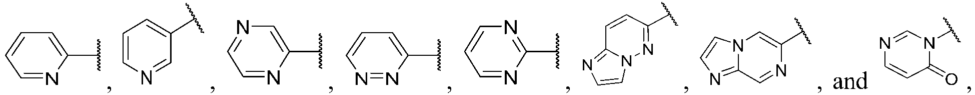 wherein the 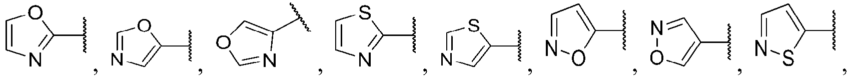 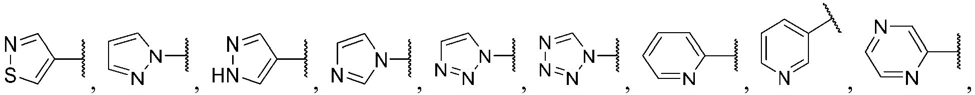 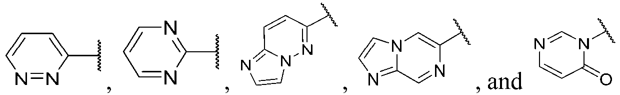 are optionally substituted with 1, 2, or 3 R³¹, and R³¹ is as defined in any one of the embodiments of the present disclosure.

In certain embodiments, R³ is selected from 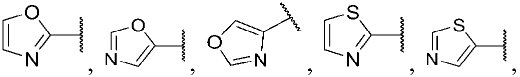 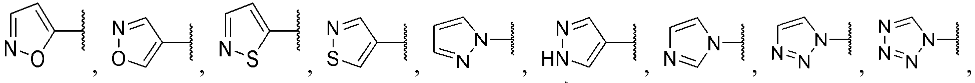 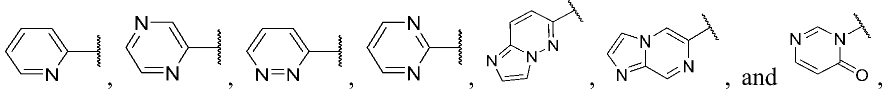 wherein the 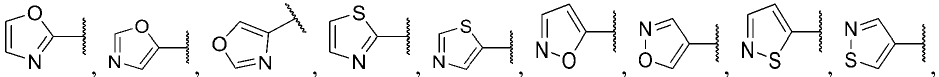 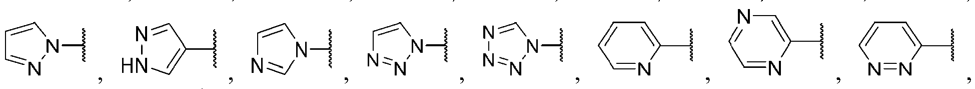 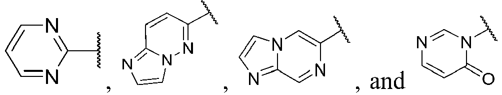 are optionally substituted with 1, 2, or 3 R³¹, and R³¹ is as defined in any one of the embodiments of the present disclosure.

In certain embodiments, R³ is selected from 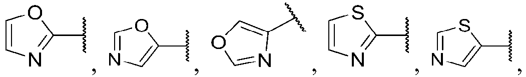 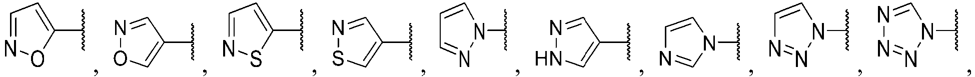 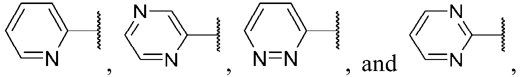 wherein the 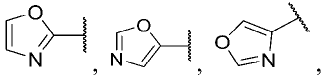 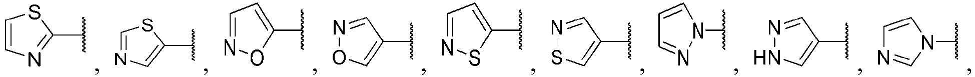 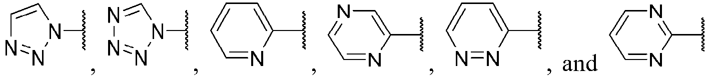 are optionally substituted with 1, 2, or 3 R³¹, and R³¹ is as defined in any one of the embodiments of the present disclosure.

In certain embodiments, R³ is selected from

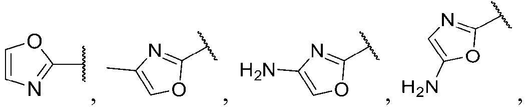

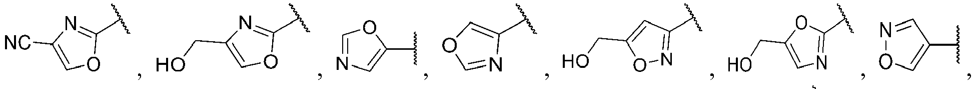

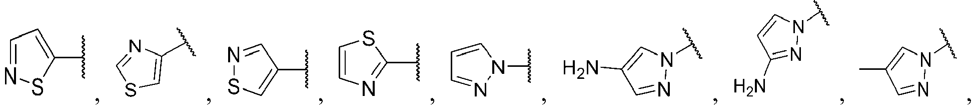

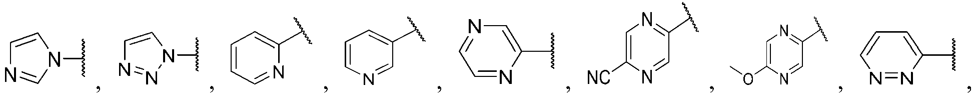

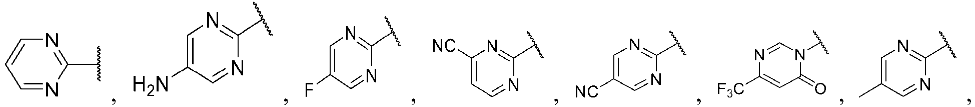

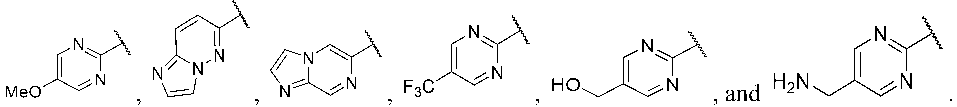

In certain embodiments, R³ is selected from

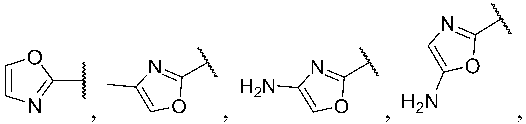

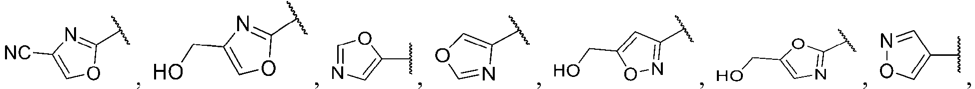

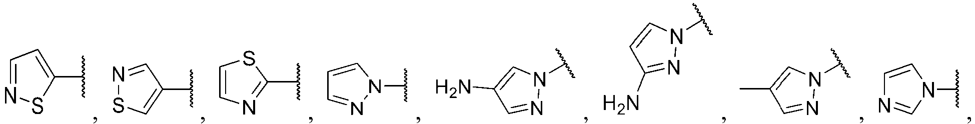

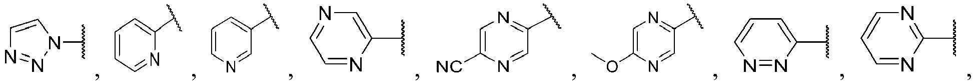

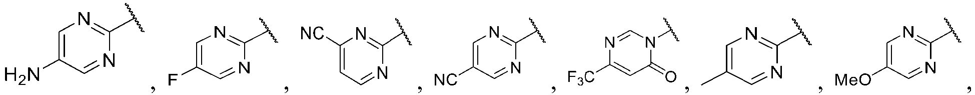

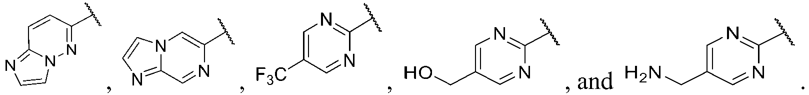

In certain embodiments, R³ is selected from

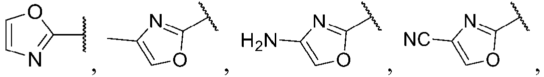

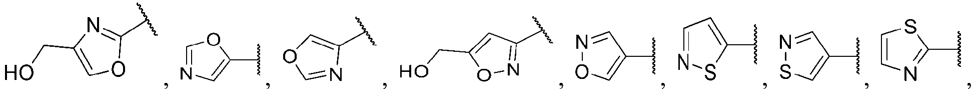

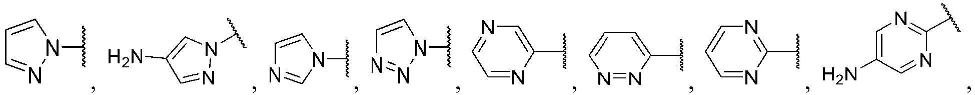

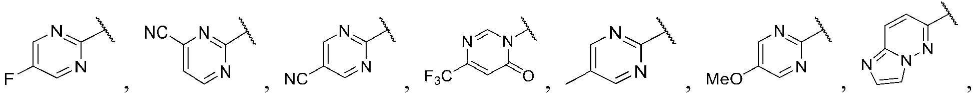

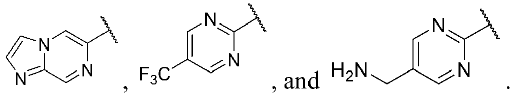

In certain embodiments, R³ is selected from

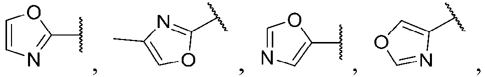

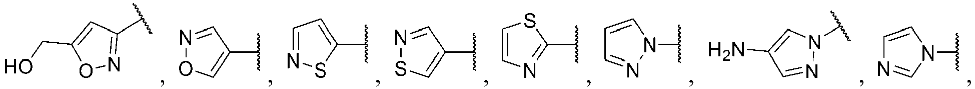

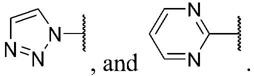

In certain embodiments, R³ is selected from oxazolyl, pyrazolyl, pyridinyl, pyrimidinyl, pyrazinyl, pyridazinyl, pyridazinoimidazolyl, and pyrazinoimidazolyl, wherein the oxazolyl, pyrazolyl, pyridinyl, pyrimidinyl, pyrazinyl, pyridazinyl, pyridazinoimidazolyl, and pyrazinoimidazolyl are optionally substituted with 1, 2, or 3 R³¹, and R³¹ is as defined in any one of the embodiments of the present disclosure.

In certain embodiments, R³ is selected from 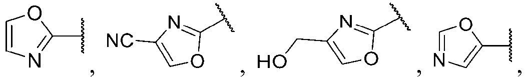 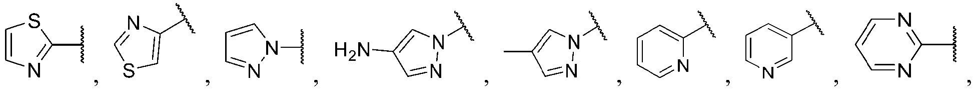 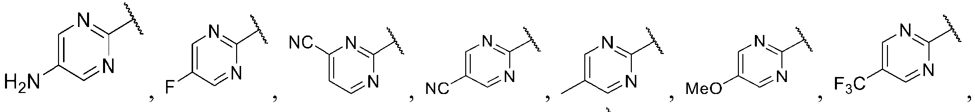 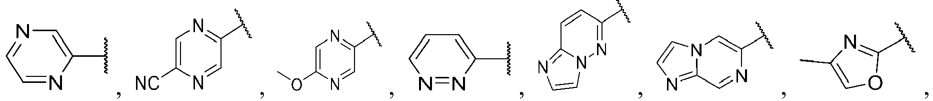 and

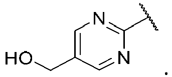

In certain embodiments, R³ is selected from 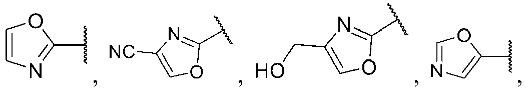 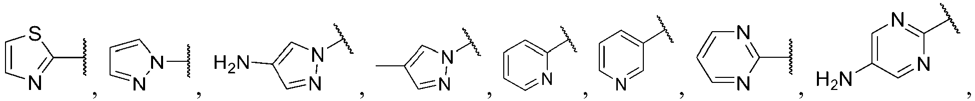 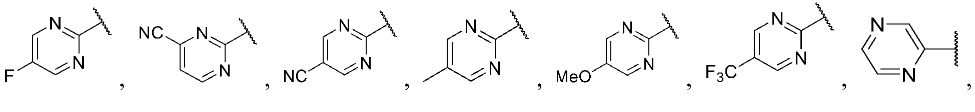 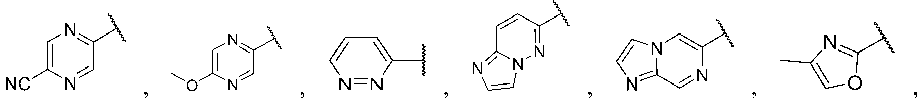 and

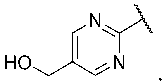

In certain embodiments, R³ is selected from oxazolyl, pyrazolyl, pyrimidinyl, and pyrazinyl, wherein the oxazolyl, pyrazolyl, pyrimidinyl, and pyrazinyl are optionally substituted with 1, 2, or 3 R³¹ (as defined in any one of the embodiments of the present disclosure), for example, substituted with fluorine, cyano, amino, methyl, trifluoromethyl, -methylene-OH, and -O-methyl.

In certain embodiments, R³ is selected from oxazolyl, pyrazolyl, and pyrimidinyl, wherein the oxazolyl, pyrazolyl, and pyrimidinyl are optionally substituted with 1, 2, or 3 R³¹, for example, substituted with methyl.

In certain embodiments, R³ is selected from 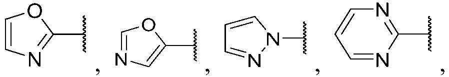 and 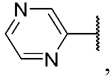 wherein the 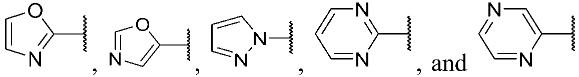 are optionally substituted with 1, 2, or 3 R³¹ (as defined in any one of the embodiments of the present disclosure), for example, substituted with fluorine, cyano, amino, methyl, trifluoromethyl, -methylene-OH, and -O-methyl.

In certain embodiments, R³ is selected from 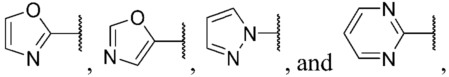 wherein the 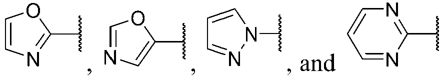 are optionally substituted with 1, 2, or 3 R³¹, for example, substituted with methyl.

In certain embodiments, R³ is selected from

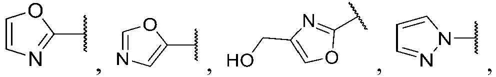

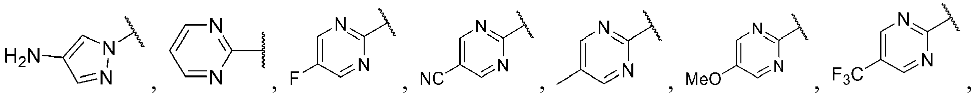

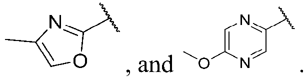

In certain embodiments, R³ is selected from 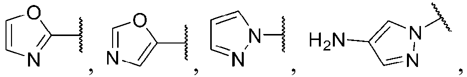 and

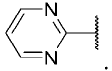

In certain embodiments, R³ is selected from oxazolyl, wherein the oxazolyl is optionally substituted with 1, 2, or 3 R³¹, for example, optionally substituted with 1, 2, or 3 methyl.

In certain embodiments, R³ is selected from

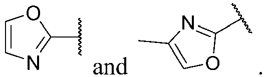

In certain embodiments, R³ is selected from -(C₃-C₆)cycloalkyl and -(4- to 6-membered)heterocyclyl, wherein the -(C₃-C₆)cycloalkyl and -(4- to 6-membered)heterocyclyl are optionally substituted with one or more R³¹, and R³¹ is as defined in any one of the embodiments of the present disclosure.

In certain embodiments, R³ is selected from cyclopropyl, oxetanyl, azetidinyl, tetrahydrofuranyl, pyrrolidinyl, tetrahydropyranyl, piperidinyl, piperazinyl, and morpholinyl, wherein the aforementioned groups are optionally substituted with one or more R³¹, and R³¹ is as defined in any one of the embodiments of the present disclosure.

In certain embodiments, R³ is selected from

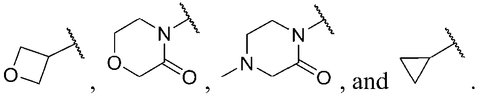

In certain embodiments, R³ is selected from -(3- to 6-membered)heterocyclyl, wherein the -(3- to 6-membered)heterocyclyl is optionally substituted with one or more R³¹, and R³¹ is as defined in any one of the embodiments of the present disclosure.

In certain embodiments, R³ is selected from oxetanyl, azetidinyl, tetrahydrofuranyl, pyrrolidinyl, tetrahydropyranyl, piperidinyl, piperazinyl, and morpholinyl, wherein the aforementioned groups are optionally substituted with one or more R³¹, and R³¹ is as defined in any one of the embodiments of the present disclosure.

In certain embodiments, R³ is

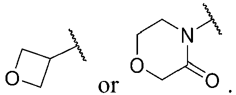

In certain embodiments, R³ is

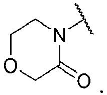

In certain embodiments, R⁴ is selected from hydrogen, halogen, cyano, -OH, -NH₂, -(C₁-C₄)alkyl, -(C₁-C₄)haloalkyl, -O-(C₁-C₄)alkyl, -O-(C₁-C₄)haloalkyl, -(C₃-C₆)cycloalkyl, -O-(C₃-C₆)cycloalkyl, -(4- to 8-membered)heterocyclyl, -O-(4- to 8-membered)heterocyclyl, -(C₆-C₁₀)aryl, -O-(C₆-C₁₀)aryl, -(5- to 10-membered)heteroaryl, and -O-(5- to 10-membered)heteroaryl.

In certain embodiments, R⁴ is selected from hydrogen, halogen, cyano, -OH, -NH₂, methyl, ethyl, propyl, halomethyl, haloethyl, halopropyl, -O-methyl, -O-ethyl, -O-propyl, -O-halomethyl, -O-haloethyl, -O-halopropyl, cyclopropyl, cyclopentyl, cyclohexyl, -O-cyclopropyl, -O-cyclopentyl, -O-cyclohexyl, oxetanyl, tetrahydrofuranyl, tetrahydropyranyl, 1,4-dioxanyl, azetidinyl, pyrrolidinyl, piperidinyl, piperazinyl, homopiperidinyl, homopiperazinyl, morpholinyl, -O-tetrahydrofuranyl, -O-tetrahydropyranyl, -O-1,4-dioxanyl, -O-pyrrolidinyl, -O-piperidinyl, -O-piperazinyl, -O-morpholinyl, phenyl, naphthyl, -O-phenyl, -O-naphthyl, oxazolyl, thiazolyl, pyrazolyl, imidazolyl, triazolyl, tetrazolyl, pyridinyl, pyrimidinyl, indolyl, quinolinyl, -O-oxazolyl, -O-thiazolyl, -O-pyrazolyl, -O-imidazolyl, -O-triazolyl, -O-tetrazolyl, -O-pyridinyl, -O-pyrimidinyl, -O-indolyl, and -O-quinolinyl.

In certain embodiments, R⁴ is selected from hydrogen, fluorine, chlorine, cyano, -OH, -NH₂, methyl, halomethyl, -O-methyl, -NH-methyl, and cyclopropyl.

In certain embodiments, R⁴ is selected from hydrogen, fluorine, chlorine, cyano, -OH, and -NH₂. In certain embodiments, R⁴ is hydrogen.

In certain embodiments, R^{c} is selected from hydrogen, -(C₁-C₄)alkyl, -(C₁-C₄)haloalkyl, -O-(C₁-C₄)alkyl, -O-(C₁-C₄)haloalkyl, -(C₃-C₆)cycloalkyl, -O-(C₃-C₆)cycloalkyl, -(4- to 8-membered)heterocyclyl, -O-(4- to 8-membered)heterocyclyl, -(C₆-C₁₀)aryl, -O-(C₆-C₁₀)aryl, -(5-to 10-membered)heteroaryl, and -O-(5- to 10-membered)heteroaryl.

In certain embodiments, R^{c} is selected from hydrogen, methyl, ethyl, propyl, halomethyl, haloethyl, halopropyl, -O-methyl, -O-ethyl, -O-propyl, -O-halomethyl, -O-haloethyl, -O-halopropyl, cyclopropyl, cyclopentyl, cyclohexyl, -O-cyclopropyl, -O-cyclopentyl, -O-cyclohexyl, oxetanyl, tetrahydrofuranyl, tetrahydropyranyl, 1,4-dioxanyl, azetidinyl, pyrrolidinyl, piperidinyl, piperazinyl, homopiperidinyl, homopiperazinyl, morpholinyl, -O-tetrahydrofuranyl, -O-tetrahydropyranyl, -O-1,4-dioxanyl, -O-pyrrolidinyl, -O-piperidinyl, -O-piperazinyl, -O-morpholinyl, phenyl, naphthyl, -O-phenyl, -O-naphthyl, oxazolyl, thiazolyl, pyrazolyl, imidazolyl, triazolyl, tetrazolyl, pyridinyl, pyrimidinyl, indolyl, quinolinyl, -O-oxazolyl, -O-thiazolyl, -O-pyrazolyl, -O-imidazolyl, -O-triazolyl, -O-tetrazolyl, -O-pyridinyl, -O-pyrimidinyl, -O-indolyl, and -O-quinolinyl.

In certain embodiments, R^{c} is selected from hydrogen, methyl, ethyl, halomethyl, -O-methyl, -NH-methyl, and cyclopropyl.

R^{a} and R^{b} are each independently selected from hydrogen, halogen, cyano, -OR^{c}, -SR^{c}, -N(R^{c})R^{c}, -(C₁-C₄)alkyl, and -(C₁-C₄)haloalkyl, wherein R^{c} is as defined in any one of the embodiments of the present disclosure.

In certain embodiments, R^{a} and R^{d} are each independently selected from hydrogen, halogen, cyano, -OR^{c}, -SR^{c}, -N(R^{c})R^{c}, methyl, ethyl, propyl, halomethyl, haloethyl, and halopropyl, wherein R^{c} is as defined in any one of the embodiments of the present disclosure.

In certain embodiments, R^{a} and R^{d} are each independently selected from hydrogen, fluorine, chlorine, cyano, -OH, -O-methyl, -SH, -S-methyl, -NH₂, -N(methyl)₂, -N(methyl)-ethyl, - N(ethyl)₂, methyl, ethyl, propyl, halomethyl, haloethyl, and halopropyl.

X is selected from O, S, NH, N[(C₁-C₄)alkyl], N[C(=O)-R^{c}], C(R^{a})(R^{b}), C(=O), C[=C(R^{a})R^{b}], S(=O), and S(=O)₂, wherein R^{a}, R^{b}, and R^{c} are as defined in any one of the embodiments of the present disclosure.

In certain embodiments, X is selected from O, S, NH, N(CH₃), N(CH₂CH₃), N[C(=O)-CH₃], CH₂, C(CH₃)₂, C(CH₃)(CH₂CH₃), C(CH₂CH₃)₂, C(=O), C(=CH₂), C(=CF₂), C(=CHCF₃), S(=O), and S(=O)₂.

In certain embodiments, X is selected from O, S, and NH.

In certain embodiments, X is selected from O and S.

In certain embodiments, X is O.

In certain embodiments, ring B is selected from -(C₆-C₁₀)aryl and 5- to 10-membered heteroaryl.

In certain embodiments, ring B is selected from phenyl, naphthyl, oxazolyl, thiazolyl, pyrazolyl, imidazolyl, triazolyl, tetrazolyl, pyridinyl, pyrimidinyl, benzofuranyl, benzothienyl, indolyl, quinolinyl, and isoquinolinyl.

In certain embodiments, ring B is phenyl.

In certain embodiments, the structural unit

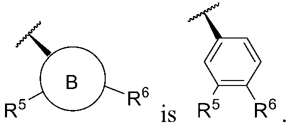

In certain embodiments, R⁵ and R⁶ are each independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4- to 8-membered)heterocyclyl, -O-(C₃-C₆)cycloalkyl, -O-(4- to 8-membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, -C(=O)-N[(C₁-C₄)alkyl]₂, - C(=O)-H, and -C(=N-OH)-H, wherein the -(C₁-C₄)alkyl, -(C₂-C₄)alkenyl, -(C₂-C₄)alkynyl, -(C₃-C₆)cycloalkyl, and -(4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, and - N(ethyl)-propyl.

In certain embodiments, R⁵ and R⁶ are each independently selected from hydrogen, fluorine, chlorine, bromine, cyano, nitro, -OH, -SH, -NH₂, methyl, ethyl, propyl, vinyl, propenyl, ethynyl, propynyl, halomethyl, haloethyl, halopropyl, -O-methyl, -O-ethyl, -O-propyl, -O-halomethyl, -O-haloethyl, -O-halopropyl, -S-methyl, -S-ethyl, -S-propyl, -NH-methyl, -NH-ethyl, -NH-propyl, - N(methyl)₂, -N(methyl)-ethyl, -N(methyl)-propyl, -N(ethyl)₂, -N(ethyl)-propyl, cyclopropyl, cyclopentyl, cyclohexyl, oxetanyl, tetrahydrofuranyl, tetrahydropyranyl, 1,4-dioxanyl, azetidinyl, pyrrolidinyl, piperidinyl, piperazinyl, homopiperidinyl, homopiperazinyl, morpholinyl, -O-cyclopropyl, -O-cyclopentyl, -O-cyclohexyl, -O-tetrahydrofuranyl, -O-tetrahydropyranyl, -O-1,4-dioxanyl, -O-pyrrolidinyl, -O-piperidinyl, -O-piperazinyl, -O-morpholinyl, -C(=O)-methyl, - C(=O)-ethyl, -C(=O)-propyl, -C(=O)-cyclopropyl, -C(=O)-cyclopentyl, -C(=O)-cyclohexyl, - C(=O)-O-methyl, -C(=O)-O-ethyl, -C(=O)-O-propyl, -C(=O)-O-cyclopropyl, -C(=O)-O-cyclopentyl, -C(=O)-O-cyclohexyl, -C(=O)-NH₂, -C(=O)-NH-methyl, -C(=O)-NH-ethyl, -C(=O)-NH-propyl, -C(=O)-N(methyl)₂, -C(=O)-N(methyl)-ethyl, -C(=O)-N(ethyl)₂, -C(=O)-H, and - C(=N-OH)-H.

In certain embodiments, R⁵ and R⁶ are each independently selected from hydrogen, fluorine, chlorine, bromine, cyano, -OH, -NH₂, methyl, ethyl, -O-methyl, -NH-methyl, -N(methyl)₂, - N(methyl)-ethyl, cyclopropyl, azetidinyl, -O-cyclopropyl, -C(=O)-methyl, -C(=O)-cyclopropyl, - C(=O)-H, and -C(=N-OH)-H.

In certain embodiments, R⁵ and R⁶ are each independently selected from hydrogen, fluorine, chlorine, bromine, cyano, -OH, -NH₂, -(C₁-C₄)alkyl, -(C₁-C₄)alkyl-OH, -(C₁-C₄)alkyl-NH₂, -(C₁-C₄)haloalkyl, -O-(C₁-C₄)alkyl, -O-(C₁-C₄)haloalkyl, and -(C₃-C₆)cycloalkyl.

In certain embodiments, R⁵ and R⁶ are each independently selected from hydrogen, fluorine, chlorine, bromine, cyano, -OH, -NH₂, -(C₁-C₄)alkyl, -(C₁-C₄)haloalkyl, -O-(C₁-C₄)alkyl, -O-(C₁-C₄)haloalkyl, and -(C₃-C₆)cycloalkyl.

In certain embodiments, R⁵ is selected from hydrogen, fluorine, chlorine, bromine, cyano, -OH, - NH₂, and -CH₂NH₂.

In certain embodiments, R⁵ is selected from hydrogen, fluorine, chlorine, bromine, cyano, -OH, and -NH₂.

In certain embodiments, R⁵ is selected from hydrogen and -NH₂.

In certain embodiments, R⁵ is hydrogen.

In certain embodiments, R⁶ is selected from hydrogen, fluorine, chlorine, bromine, cyano, -OH, - NH₂, -O-methyl, and cyclopropyl.

In certain embodiments, R⁶ is selected from cyano, bromine, -NH₂, -O-methyl, and cyclopropyl. In certain embodiments, R⁶ is selected from hydrogen, fluorine, chlorine, bromine, cyano, -OH, - NH₂, and -O-methyl.

In certain embodiments, R⁶ is selected from cyano, -O-methyl, and cyclopropyl.

In certain embodiments, R⁶ is selected from hydrogen, cyano, and -O-methyl.

In certain embodiments, R⁶ is selected from cyano and -O-methyl.

In certain embodiments, R⁵ and R⁶, together with the atoms to which they are attached, form 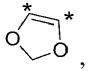 wherein * indicates the position of the attached atom.

In certain embodiments, ring C is selected from -(C₆-C₁₀)aryl and -(5- to 10-membered)heteroaryl.

In certain embodiments, ring C is selected from phenyl, naphthyl, oxazolyl, thiazolyl, pyrazolyl, imidazolyl, triazolyl, tetrazolyl, pyridinyl, pyrimidinyl, benzofuranyl, benzothienyl, indolyl, quinolinyl, and isoquinolinyl.

In certain embodiments, ring C is phenyl.

In certain embodiments, the structural unit 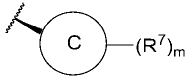 is

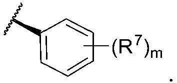

In certain embodiments, the structural unit 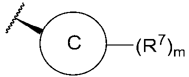 is

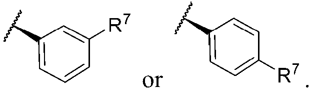

In certain embodiments, the structural unit 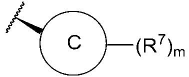 is

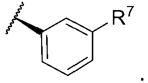

In certain embodiments, each R⁷ is independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4- to 8-membered)heterocyclyl, -O-(C₃-C₆)cycloalkyl, -O-(4- to 8-membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, -C(=O)-N[(C₁-C₄)alkyl]₂, and -NHC(=O)-(C₁-C₄)alkyl, wherein the -(C₁-C₄)alkyl, -(C₂-C₄)alkenyl, -(C₂-C₄)alkynyl, -(C₃-C₆)cycloalkyl, and -(4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, and - N(ethyl)-propyl.

In certain embodiments, each R⁷ is independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4- to 8-membered)heterocyclyl, -O-(C₃-C₆)cycloalkyl, -O-(4- to 8-membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, and -C(=O)-N[(C₁-C₄)alkyl]₂, wherein the -(C₁-C₄)alkyl, -(C₂-C₄)alkenyl, -(C₂-C₄)alkynyl, -(C₃-C₆)cycloalkyl, and - (4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, and -N(ethyl)-propyl.

In certain embodiments, each R⁷ is independently selected from hydrogen, halogen, cyano, nitro, -OH, -SH, -NH₂, methyl, ethyl, propyl, -methylene-OH, -ethylene-OH, -propylene-OH, - methylene-NH₂, -ethylene-NH₂, -propylene-NH₂, vinyl, propenyl, ethynyl, propynyl, halomethyl, haloethyl, halopropyl, -O-methyl, -O-ethyl, -O-propyl, -O-halomethyl, -O-haloethyl, -O-halopropyl, -S-methyl, -S-ethyl, -S-propyl, -NH-methyl, -NH-ethyl, -NH-propyl, -N(methyl)₂, - N(methyl)-ethyl, -N(methyl)-propyl, -N(ethyl)₂, -N(ethyl)-propyl, cyclopropyl, cyclopentyl, cyclohexyl, oxetanyl, tetrahydrofuranyl, tetrahydropyranyl, 1,4-dioxanyl, azetidinyl, pyrrolidinyl, piperidinyl, piperazinyl, homopiperidinyl, homopiperazinyl, morpholinyl, -O-cyclopropyl, -O-cyclopentyl, -O-cyclohexyl, -O-tetrahydrofuranyl, -O-tetrahydropyranyl, -O-1,4-dioxanyl, -O-pyrrolidinyl, -O-piperidinyl, -O-piperazinyl, -O-morpholinyl, -C(=O)-methyl, -C(=O)-ethyl, - C(=O)-propyl, -C(=O)-cyclopropyl, -C(=O)-cyclopentyl, -C(=O)-cyclohexyl, -C(=O)-O-methyl, - C(=O)-O-ethyl, -C(=O)-O-propyl, -C(=O)-O-cyclopropyl, -C(=O)-O-cyclopentyl, -C(=O)-O-cyclohexyl, -C(=O)-NH₂, -C(=O)-NH-methyl, -C(=O)-NH-ethyl, -C(=O)-NH-propyl, -C(=O)-N(methyl)₂, -C(=O)-N(methyl)-ethyl, -C(=O)-N(ethyl)₂, -C(=O)-H, -C(=N-OH)-H, -NHC(=O)-methylene-NH₂, and -NHC(=O)-ethylene-NH₂.

In certain embodiments, each R⁷ is independently selected from hydrogen, halogen, cyano, nitro, -OH, -SH, -NH₂, methyl, ethyl, propyl, -methylene-OH, -ethylene-OH, -propylene-OH, - methylene-NH₂, -ethylene-NH₂, -propylene-NH₂, vinyl, propenyl, ethynyl, propynyl, halomethyl, haloethyl, halopropyl, -O-methyl, -O-ethyl, -O-propyl, -O-halomethyl, -O-haloethyl, -O-halopropyl, -S-methyl, -S-ethyl, -S-propyl, -NH-methyl, -NH-ethyl, -NH-propyl, -N(methyl)₂, - N(methyl)-ethyl, -N(methyl)-propyl, -N(ethyl)₂, -N(ethyl)-propyl, cyclopropyl, cyclopentyl, cyclohexyl, oxetanyl, tetrahydrofuranyl, tetrahydropyranyl, 1,4-dioxanyl, azetidinyl, pyrrolidinyl, piperidinyl, piperazinyl, homopiperidinyl, homopiperazinyl, morpholinyl, -O-cyclopropyl, -O-cyclopentyl, -O-cyclohexyl, -O-tetrahydrofuranyl, -O-tetrahydropyranyl, -O-1,4-dioxanyl, -O-pyrrolidinyl, -O-piperidinyl, -O-piperazinyl, -O-morpholinyl, -C(=O)-methyl, -C(=O)-ethyl, - C(=O)-propyl, -C(=O)-cyclopropyl, -C(=O)-cyclopentyl, -C(=O)-cyclohexyl, -C(=O)-O-methyl, - C(=O)-O-ethyl, -C(=O)-O-propyl, -C(=O)-O-cyclopropyl, -C(=O)-O-cyclopentyl, -C(=O)-O-cyclohexyl, -C(=O)-NH₂, -C(=O)-NH-methyl, -C(=O)-NH-ethyl, -C(=O)-NH-propyl, -C(=O)-N(methyl)₂, -C(=O)-N(methyl)-ethyl, -C(=O)-N(ethyl)₂, -C(=O)-H, and -C(=N-OH)-H.

In certain embodiments, each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, -(C₁-C₄)alkylene-NH₂, and -NHC(=O)-(C₁-C₄)alkylene-NH₂.

In certain embodiments, each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂.

In certain embodiments, each R⁷ is independently selected from hydrogen, fluorine, chlorine, bromine, -OH, -NH₂, -methylene-OH, -ethylene-OH, -propylene-OH, -methylene-NH₂, -ethylene-NH₂, -propylene-NH₂, -NHC(=O)-methylene-NH₂, and -NHC(=O)-ethylene-NH₂.

In certain embodiments, each R⁷ is independently selected from hydrogen, fluorine, chlorine, bromine, cyano, -OH, -NH₂, -methylene-OH, -ethylene-OH, -propylene-OH, -methylene-NH₂, - ethylene-NH₂, and -propylene-NH₂.

In certain embodiments, each R⁷ is independently selected from hydrogen, fluorine, -OH, -NH₂, - NHC(=O)CH₂NH₂, -CH₂OH, and -CH₂NH₂.

In certain embodiments, each R⁷ is independently selected from hydrogen, fluorine, -OH, -NH₂, - CH₂OH, and -CH₂NH₂.

In certain embodiments, each R⁷ is independently selected from hydrogen, fluorine, chlorine, bromine, -OH, and -NH₂.

In certain embodiments, each R⁷ is independently selected from hydrogen, -OH, and -NH₂.

In certain embodiments, each R⁷ is independently selected from hydrogen and -NH₂.

In certain embodiments, m is 1, 2, 3, 4, or 5.

In certain embodiments, m is 1, 2, or 3.

In certain embodiments, m is 1.

In certain embodiments, each R is independently selected from hydrogen, -OH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -(C₁-C₄)alkylene-O-(C₁-C₄)alkyl, -O-(C₁-C₄)haloalkyl, -S-(C₁-C₄)alkyl, -S(=O)₂-(C₁-C₄)alkyl, -(C₃-C₆)cycloalkyl, -(4- to 8-membered)heterocyclyl, -O-(C₃-C₆)cycloalkyl, -O-(4- to 8-membered)heterocyclyl, - C(=O)-H, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, -C(=O)-N[(C₁-C₄)alkyl]₂, -[(C₁-C₄)alkylene]-(C₃-C₆)cycloalkyl, -[(C₁-C₄)alkylene]-(4- to 8-membered)heterocyclyl, -(C₆-C₁₀)aryl, -[(C₁-C₄)alkylene]-(C₆-C₁₀)aryl, and -(5- to 10-membered)heteroaryl, wherein the -(C₁-C₄)alkylene-, -(C₁-C₄)alkyl, -(C₂-C₄)alkenyl, -(C₂-C₄)alkynyl, -(C₃-C₆)cycloalkyl, -(4- to 8-membered)heterocyclyl, -(C₆-C₁₀)aryl, and -(5- to 10-membered)heteroaryl are optionally substituted with 1, 2, 3, 4, 5, 6, or 7 groups selected from hydrogen, halogen, -OH, -O-(C₁-C₄)alkyl, -NH₂, -NH(C₁-C₄)alkyl, N[(C₁-C₄)alkyl]₂, and (C₁-C₄)alkyl.

In certain embodiments, each R is independently selected from hydrogen, -OH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -(C₃-C₆)cycloalkyl, -(4- to 8-membered)heterocyclyl, -O-(C₃-C₆)cycloalkyl, -O-(4- to 8-membered)heterocyclyl, -C(=O)-H, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, -C(=O)-N[(C₁-C₄)alkyl]₂, -[(C₁-C₄)alkylene]-(4- to 8-membered)heterocyclyl, - (C₆-C₁₀)aryl, -[(C₁-C₄)alkylene]-(C₆-C₁₀)aryl, and -(5- to 10-membered)heteroaryl, wherein the - (C₁-C₄)alkylene-, -(C₁-C₄)alkyl, -(C₂-C₄)alkenyl, -(C₂-C₄)alkynyl, -(C₃-C₆)cycloalkyl, -(4- to 8-membered)heterocyclyl, -(C₆-C₁₀)aryl, and -(5- to 10-membered)heteroaryl are optionally substituted with 1, 2, 3, 4, 5, 6, or 7 groups selected from hydrogen, halogen, -OH, -O-(C₁-C₄)alkyl, -NH₂, -NH(C₁-C₄)alkyl, and N[(C₁-C₄)alkyl]₂.

In certain embodiments, each R is independently selected from hydrogen, -OH, -NH₂, -(C₁-C₄)alkyl, -(C₁-C₄)haloalkyl, -O-(C₁-C₄)alkyl, -(C₁-C₄)alkylene-O-(C₁-C₆)alkyl, -O-(C₁-C₄)haloalkyl, -S(=O)₂-(C₁-C₄)alkyl, -(C₃-C₆)cycloalkyl, -(4- to 8-membered)heterocyclyl, - C(=O)-H, -C(=O)-(C₁-C₄)alkyl, -C(=O)-(C₃-C₆)cycloalkyl, -[(C₁-C₄)alkylene]-(C₃-C₆)cycloalkyl, and -[(C₁-C₄)alkylene]-(4- to 8-membered)heterocyclyl, wherein the -(C₁-C₄)alkylene-, -(C₁-C₄)alkyl, -(C₃-C₆)cycloalkyl, and -(4- to 8-membered)heterocyclyl are optionally substituted with 1, 2, 3, 4, or 5 groups selected from hydrogen, halogen, -OH, -O-methyl, -O-ethyl, -O-propyl, - NH₂, -NH-methyl, -NH-ethyl, -NH-propyl, -N(methyl)₂, -N(methyl)-ethyl, -N(methyl)-propyl, - N(ethyl)₂, -N(ethyl)-propyl, methyl, and ethyl.

In certain embodiments, each R is independently selected from hydrogen, -OH, -NH₂, -(C₁-C₄)alkyl, -(C₁-C₄)haloalkyl, -O-(C₁-C₄)alkyl, -O-(C₁-C₄)haloalkyl, -(C₃-C₆)cycloalkyl, -(4- to 8-membered)heterocyclyl, -C(=O)-H, -C(=O)-(C₁-C₄)alkyl, -C(=O)-(C₃-C₆)cycloalkyl, and -[(C₁-C₄)alkylene]-(4- to 8-membered)heterocyclyl, wherein the -(C₁-C₄)alkylene-, -(C₁-C₄)alkyl, -(C₃-C₆)cycloalkyl, and -(4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, and - N(ethyl)-propyl.

In certain embodiments, each R is independently selected from hydrogen, -OH, -NH₂, methyl, ethyl, propyl, halomethyl, haloethyl, halopropyl, -methylene-O-methyl, -methylene-O-ethyl, - ethylene-O-methyl, -ethylene-O-ethyl, -O-methyl, -O-ethyl, -O-propyl, -O-halomethyl, -O-haloethyl, -O-halopropyl, -C(=O)-H, -C(=O)-methyl, -C(=O)-ethyl, -C(=O)-propyl, -S(=O)₂-methyl, -S(=O)₂-ethyl, -S(=O)₂-propyl, cyclopropyl, cyclobutyl, cyclopentyl, cyclohexyl, oxetanyl, tetrahydrofuranyl, tetrahydropyranyl, azetidinyl, pyrrolidinyl, piperidinyl, piperazinyl, morpholinyl, -methylene-cyclobutyl, -methylene-oxetanyl, and -methylene-azetidinyl, wherein the methyl, ethyl, propyl, cyclopropyl, cyclobutyl, cyclopentyl, cyclohexyl, oxetanyl, tetrahydrofuranyl, tetrahydropyranyl, azetidinyl, pyrrolidinyl, piperidinyl, piperazinyl, and morpholinyl are optionally substituted with 1, 2, or 3 groups selected from -OH, -OCH₃, -NH₂, NHCH₃, N(CH₃)₂, and -CH₃.

In certain embodiments, each R is independently selected from hydrogen, -OH, -NH₂, methyl, ethyl, propyl, halomethyl, haloethyl, halopropyl, -O-methyl, -O-ethyl, -O-propyl, -O-halomethyl, -O-haloethyl, -O-halopropyl, -C(=O)-H, -C(=O)-methyl, -C(=O)-ethyl, -C(=O)-propyl, oxetanyl, tetrahydrofuranyl, tetrahydropyranyl, azetidinyl, pyrrolidinyl, piperidinyl, piperazinyl, and morpholinyl, wherein the methyl, ethyl, propyl, oxetanyl, tetrahydrofuranyl, tetrahydropyranyl, azetidinyl, pyrrolidinyl, piperidinyl, piperazinyl, and morpholinyl are optionally substituted with 1, 2, or 3 groups selected from -OH and -NH₂.

In certain embodiments, each R is independently selected from hydrogen, -OH, -NH₂, -CH₃, - CH₂CH₃, -CH₂OH, -CH₂CH₂OH, -CH₂OCH₃, -CH₂NH₂, -CH₂CH₂NH₂, -CH₂CH₂NHCH₃, - CH₂N(CH₃)₂, -CH(N(CH₃)₂)CH₂OH, -CH₂OCH₂CH₂OH, -OCH₃, -C(=O)-H, -C(=O)CH₃, - C(=O)CH₂OH, -S(=O)₂CH₃, cyclobutyl-OH, oxetanyl, azetidinyl, azetidinyl-OH, pyrrolidinyl, piperazinyl, azetidinyl-CH₃, piperazinyl-CH₃, and -CH₂-azetidinyl.

In certain embodiments, each R is independently selected from hydrogen, -OH, -NH₂, -CH₃, - CH₂OH, -CH₂CH₂OH, -CH₂NH₂, -CH₂CH₂NH₂, -OCH₃, -C(=O)-H, -C(=O)CH₂OH, oxetanyl, and azetidinyl-OH.

In certain embodiments, each R is independently selected from hydrogen, -OH, -(C₁-C₄)alkyl, - (C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -(C₃-C₆)cycloalkyl, -(4- to 8-membered)heterocyclyl, -O-(C₃-C₆)cycloalkyl, -O-(4- to 8-membered)heterocyclyl, -C(=O)-H, -C(=O)-(C₁-C₄)alkyl, -C(=O)-(C₃-C₆)cycloalkyl, *-C(=O)-O-*(C₁-C₄)alkyl, -C(=O)-O-(C₃-C₆)cycloalkyl, -C(=O)-NH₂, -C(=O)-NH(C₁-C₄)alkyl, -C(=O)-N[(C₁-C₄)alkyl]₂, -[(C₁-C₄)alkylene]-(4- to 8-membered)heterocyclyl, -(C₆-C₁₀)aryl, -[(C₁-C₄)alkylene]-(C₆-C₁₀)aryl, and -(5- to 10-membered)heteroaryl, wherein the -(C₁-C₄)alkylene-, - (C₁-C₄)alkyl, -(C₂-C₄)alkenyl, -(C₂-C₄)alkynyl, -(C₃-C₆)cycloalkyl, -(4- to 8-membered)heterocyclyl, -(C₆-C₁₀)aryl, and -(5- to 10-membered)heteroaryl are optionally substituted with 1, 2, 3, 4, 5, 6, or 7 groups selected from hydrogen, halogen, -OH, -O-(C₁-C₄)alkyl, -NH₂, -NH(C₁-C₄)alkyl, and N[(C₁-C₄)alkyl]₂.

In certain embodiments, each R is independently selected from hydrogen, -OH, -(C₁-C₄)alkyl, - (C₁-C₄)haloalkyl, -O-(C₁-C₄)alkyl, -O-(C₁-C₄)haloalkyl, -(C₃-C₆)cycloalkyl, -(4- to 8-membered)heterocyclyl, -C(=O)-H, -C(=O)-(C₁-C₄)alkyl, -C(=O)-(C₃-C₆)cycloalkyl, and -[(C₁-C₄)alkylene]-(4- to 8-membered)heterocyclyl, wherein the -(C₁-C₄)alkylene-, -(C₁-C₄)alkyl, -(C₃-C₆)cycloalkyl, and -(4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, and - N(ethyl)-propyl.

In certain embodiments, each R is independently selected from hydrogen, -OH, methyl, ethyl, propyl, halomethyl, haloethyl, halopropyl, -O-methyl, -O-ethyl, -O-propyl, -O-halomethyl, -O-haloethyl, -O-halopropyl, -C(=O)-H, -C(=O)-methyl, -C(=O)-ethyl, -C(=O)-propyl, oxetanyl, tetrahydrofuranyl, tetrahydropyranyl, azetidinyl, pyrrolidinyl, piperidinyl, piperazinyl, and morpholinyl, wherein the methyl, ethyl, propyl, oxetanyl, tetrahydrofuranyl, tetrahydropyranyl, azetidinyl, pyrrolidinyl, piperidinyl, piperazinyl, and morpholinyl are optionally substituted with 1, 2, or 3 groups selected from -OH and -NH₂.

In certain embodiments, each R is independently selected from hydrogen, -OH, -CH₃, -CH₂OH, - CH₂CH₂OH, -CH₂CH₂NH₂, -OCH₃, -C(=O)-H, -C(=O)CH₂OH, oxetanyl, azetidinyl, and azetidinyl-OH.

In certain embodiments, each R is independently selected from hydrogen, -OH, -CH₃, -CH₂OH, - CH₂CH₂OH, -CH₂CH₂NH₂, -OCH₃, -C(=O)-H, -C(=O)CH₂OH, oxetanyl, and azetidinyl-OH.

In certain embodiments, each R is independently selected from hydrogen, -OH, -NH₂, -(C₁-C₄)alkyl, -O-(C₁-C₄)alkyl, -C(=O)-(C₁-C₄)alkyl, -S(=O)₂(C₁-C₄)alkyl, -(C₃-C₆)cycloalkyl, -(4- to 8-membered)heterocyclyl, -methylene-(C₃-C₆)cycloalkyl, and -methylene-(4- to 8-membered)heterocyclyl, wherein the -(C₁-C₄)alkyl, -(C₃-C₆)cycloalkyl, and -(4- to 8-membered)heterocyclyl are each optionally substituted with 1, 2, 3, 4, or 5 groups selected from hydrogen, halogen, -OH, -NH₂, -NH(C₁-C₄)alkyl, -N[(C₁-C₄)alkyl]₂, -O-(C₁-C₄)alkyl, and -(C₁-C₄)alkyl.

In certain embodiments, each R is independently selected from hydrogen, -OH, -NH₂, -(C₁-C₄)alkyl, -O-(C₁-C₄)alkyl, and -(4- to 8-membered)heterocyclyl, wherein the -(C₁-C₄)alkyl and - (4- to 8-membered)heterocyclyl are optionally substituted with 1, 2, 3, 4, or 5 groups selected from hydrogen, halogen, -OH, -NH₂, -NH(C₁-C₄)alkyl, -N[(C₁-C₄)alkyl]₂, and -O-(C₁-C₄)alkyl.

In certain embodiments, each R is independently selected from hydrogen, -OH, -(C₁-C₄)alkyl, and -O-(C₁-C₄)alkyl, wherein the aforementioned groups are each independently optionally substituted with 1, 2, or 3 groups selected from hydrogen, halogen, -OH, -NH₂, -NH-methyl, and -N(methyl)₂. In certain embodiments, each R is independently selected from hydrogen, -OH, methyl, ethyl, propyl, -O-methyl, -O-ethyl, -O-propyl, -methylene-O-ethyl, -C(=O)-methyl, -S(=O)₂-methyl, cyclobutyl, oxetanyl, azetidinyl, pyrrolidinyl, piperazinyl, -methylene-cyclobutyl, -methylene-oxetanyl, and -methylene-azetidinyl, wherein the aforementioned groups are each independently optionally substituted with 1, 2, or 3 groups selected from -OH, -NH₂, OCH₃, NHCH₃, N(CH₃)₂, and CH₃.

In certain embodiments, each R is independently selected from methyl, ethyl, propyl, -O-methyl, -O-ethyl, -O-propyl, oxetanyl, and azetidinyl, wherein the aforementioned groups are each independently optionally substituted with 1, 2, or 3 groups selected from -OH and -NH₂.

In certain embodiments, R^{8a}, R^{8b}, R^{9a}, and R^{9b} are each independently selected from hydrogen, halogen, cyano, -(C₁-C₄)alkyl, -(C₁-C₄)haloalkyl, -(C₂-C₄)alkenyl, -(C₂-C₄)alkynyl, -OR, -N(R)R, -[(C₁-C₆)alkylene]R, -[(C₁-C₆)alkylene]OR, -[(C₁-C₆)alkylene]N(R)R, -[(C₁-C₆)alkylene]N(R)C(=O)R, -[(C₁-C₆)alkylene]N(R)C(=O)N(R)R, -C(=O)R, -C(=O)N(R)R, - C(=O)[(C₁-C₆)alkylene]N(R)R, -C(=O)OR, -C(=S)N(R)R, -SR, -S(=O)R, -S(=O)₂R, - S(=O)₂N(R)R, -N(R)C(=O)R, -N(R)C(=O)N(R)R, -P(=O)(OR)(OR), -(C₆-C₁₀)aryl, -(5- to 10-membered)heteroaryl, -(C₃-C₆)cycloalkyl, and -(4- to 8-membered)heterocyclyl, wherein R is as defined in any one of the embodiments of the present disclosure.

R^{8a}, R^{8b}, R^{9a}, and R^{9b} are each independently selected from hydrogen, halogen, cyano, -(C₁-C₄)alkyl, -(C₁-C₄)haloalkyl, -(C₂-C₄)alkenyl, -(C₂-C₄)alkynyl, -OR, -N(R)R, -[(C₁-C₆)alkylene]R, -[(C₁-C₆)alkylene]OR, -[(C₁-C₆)alkylene]N(R)R, -C(=O)R, -C(=O)N(R)R, -C(=O)[(C₁-C₆)alkylene]N(R)R, -C(=O)OR, -C(=S)N(R)R, -SR, -S(=O)R, -S(=O)₂R, -S(=O)₂N(R)R, - N(R)C(=O)R, -N(R)C(=O)N(R)R, -P(=O)(OR)(OR), -(C₆-C₁₀)aryl, -(5- to 10-membered)heteroaryl, -(C₃-C₆)cycloalkyl, and -(4- to 8-membered)heterocyclyl, wherein R is as defined in any one of the embodiments of the present disclosure.

In certain embodiments, R^{8a}, R^{8b}, R^{9a}, and R^{9b} are each independently selected from hydrogen, cyano, -OR, -N(R)R, -[(C₁-C₄)alkylene]R, -[(C₁-C₄)alkylene]OR, -[(C₁-C₄)alkylene]N(R)R, - [(C₁-C₄)alkylene]N(R)C(=O)R, -[(C₁-C₄)alkylene]N(R)C(=O)N(R)R, -C(=O)R, -C(=O)N(R)R, - C(=O)[(C₁-C₄)alkylene]N(R)R, -C(=O)OR, -C(=S)N(R)R, -S(=O)R, -S(=O)₂R, -S(=O)₂N(R)R, - N(R)C(=O)R, and -N(R)C(=O)N(R)R, wherein R is as defined in any one of the embodiments of the present disclosure.

In certain embodiments, R^{8a}, R^{8b}, R^{9a}, and R^{9b} are each independently selected from hydrogen, - OR, -N(R)R, -[(C₁-C₄)alkylene]R, -[(C₁-C₄)alkylene]OR, -[(C₁-C₄)alkylene]N(R)R, -C(=O)R, - C(=O)N(R)R, -C(=O)[(C₁-C₄)alkylene]N(R)R, -C(=O)OR, -C(=S)N(R)R, -S(=O)R, -S(=O)₂R, - S(=O)₂N(R)R, -N(R)C(=O)R, and -N(R)C(=O)N(R)R, wherein R is as defined in the present disclosure.

In certain embodiments, R^{8a} and R^{8b} are each independently selected from hydrogen, -methylene-R, -ethylene-R, -propylene-R, -methylene-OR, -ethylene-OR, -propylene-OR, -methylene-N(R)R, -ethylene-N(R)R, -propylene-N(R)R, -C(=O)R, -C(=O)N(R)R, and -C(=O)OR, wherein R is as defined in any one of the embodiments of the present disclosure.

In certain embodiments, R^{8a} and R^{8b} are each independently selected from hydrogen, -methylene-R, -methylene-OR, -methylene-N(R)R, -C(=O)R, -C(=O)OR, and -C(=O)N(R)R, wherein R is as defined in the present disclosure; for example, each R is independently selected from hydrogen, - OH, -NH₂, -(C₁-C₄)alkyl, -O-(C₁-C₄)alkyl, -C(=O)-(C₁-C₄)alkyl, -S(=O)₂(C₁-C₄)alkyl, and -(4- to 8-membered)heterocyclyl, wherein the -(C₁-C₄)alkyl and -(4- to 8-membered)heterocyclyl are each optionally substituted with 1, 2, 3, 4, or 5 groups selected from hydrogen, halogen, -OH, - NH₂, -NHCH₃, and CH₃.

In certain embodiments, R^{8a} and R^{8b} are each independently selected from hydrogen, -methylene-R, -methylene-OR, -methylene-N(R)R, -C(=O)R, -C(=O)OR, and -C(=O)N(R)R, wherein R is as defined in any one of the embodiments of the present disclosure.

In certain embodiments, R^{8a} is hydrogen.

In certain embodiments, R^{8a} is

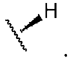

In certain embodiments, R^{8b} is selected from hydrogen,

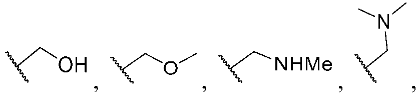

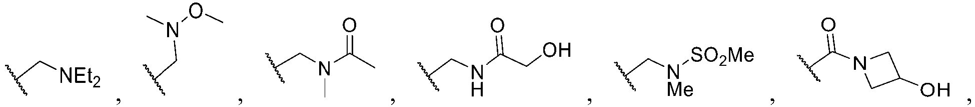

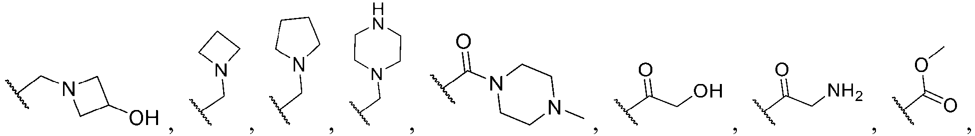

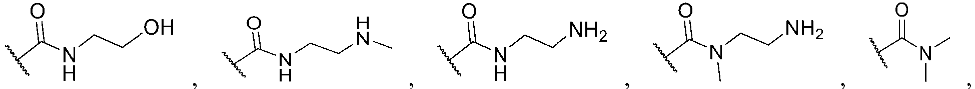

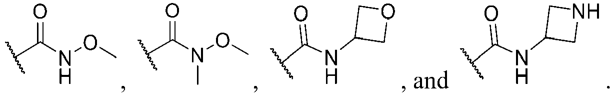

In certain embodiments, R^{8b} is selected from hydrogen, 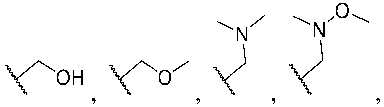 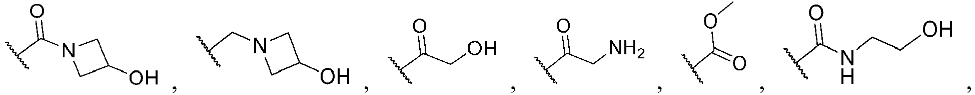 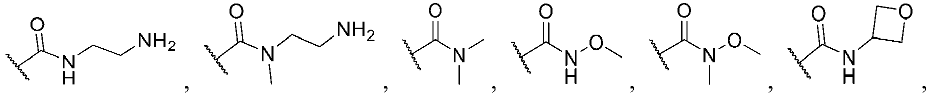 and

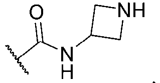

In certain embodiments, R^{8b} is selected from hydrogen,

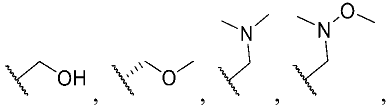

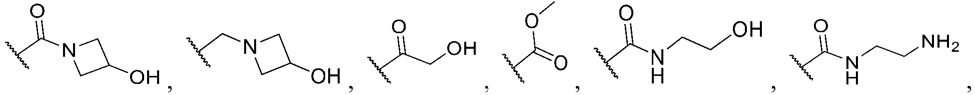

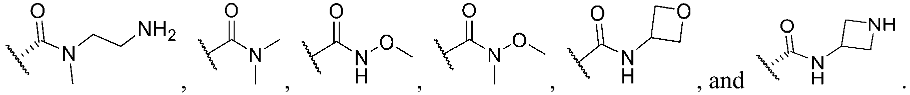

In certain embodiments, R^{8b} is selected from hydrogen,

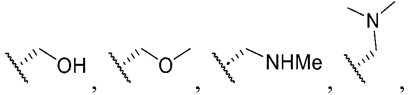

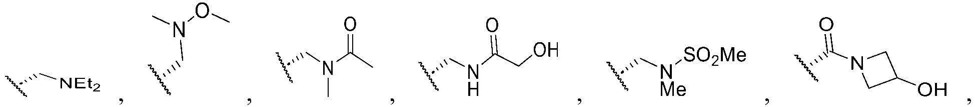

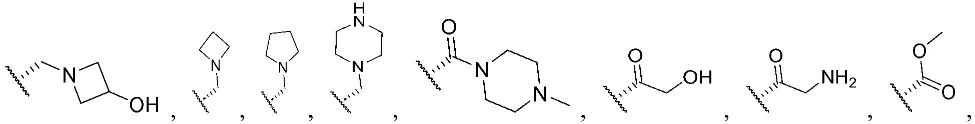

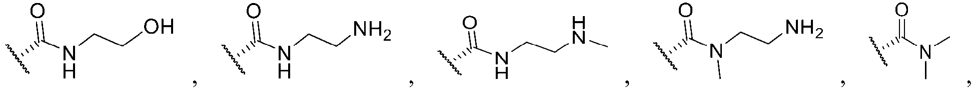

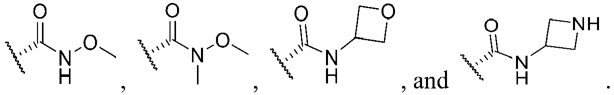

In certain embodiments, R^{8b} is selected from hydrogen,

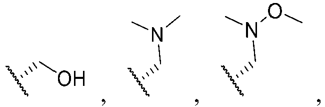

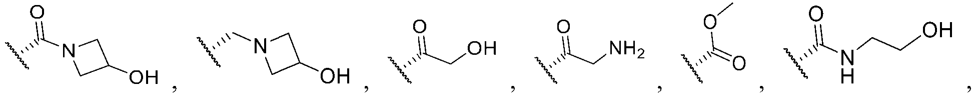

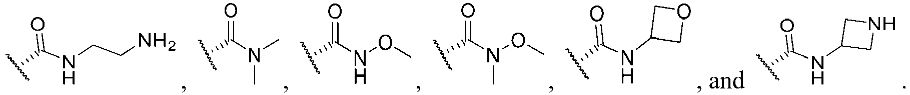

In certain embodiments, R^{8b} is selected from hydrogen,

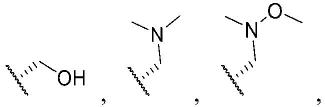

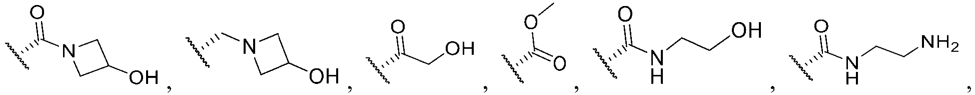

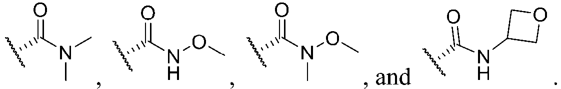

In certain embodiments, R^{8b} is selected from hydrogen, -methylene-R, -methylene-N(R)R, - C(=O)OR, -C(=O)N(R)R, and -C(=O)R, wherein R is as defined in any one of the embodiments of the present disclosure; for example, each R is independently selected from hydrogen, -OH, methyl, ethyl, -O-methyl, -C(=O)-methyl, -S(=O)₂-methyl, azetidinyl, pyrrolidinyl, and piperazinyl, wherein the aforementioned groups are each independently optionally substituted with 1, 2, or 3 groups selected from -OH and -NH₂.

In certain embodiments, R^{8b} is selected from -methylene-R, -C(=O)OR, -C(=O)N(R)R, and - C(=O)R, wherein R is as defined in any one of the embodiments of the present disclosure.

In certain embodiments, R^{8b} is selected from hydrogen,

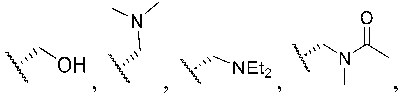

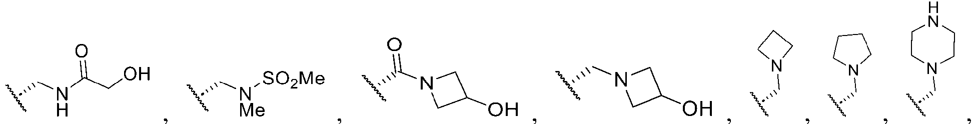

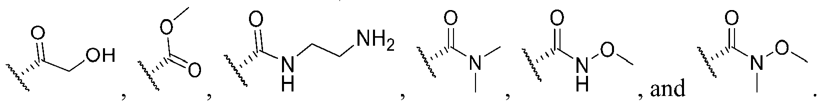

In certain embodiments, R^{8b} is selected from

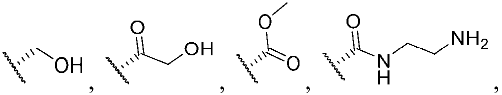

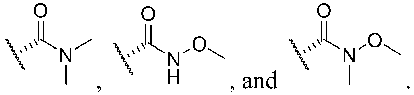

In certain embodiments, R^{8b} is selected from hydrogen,

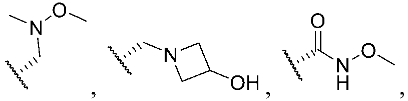

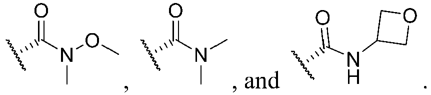

In certain embodiments, R^{8b} is selected from hydrogen,

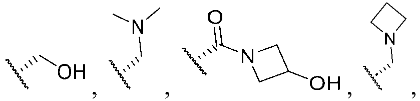

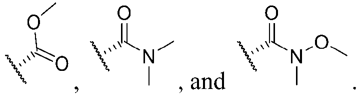

In certain embodiments, R^{8b} is selected from hydrogen,

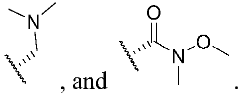

In certain embodiments, R^{9a} and R^{9b} are each independently selected from hydrogen, -CN, -OR, - NHR, -N(R)R, -NHC(=O)R, -methylene-R, -ethylene-R, -propylene-R, -methylene-N(R)R, - ethylene-N(R)R, -propylene-N(R)R, -methylene-NHC(=O)R, and -methylene-NHC(=O)NHR, wherein R is as defined in any one of the embodiments of the present disclosure.

In certain embodiments, R^{9a} and R^{9b} are each independently selected from hydrogen, -OR, -NHR, -methylene-R, -ethylene-R, -propylene-R, -methylene-N(R)R, -ethylene-N(R)R, -propylene-N(R)R, and -NHC(=O)R, wherein R is as defined in any one of the embodiments of the present disclosure.

In certain embodiments, R^{9a} and R^{9b} are each independently selected from hydrogen, -CN, -OR, - N(R)R, -NHC(=O)R, -methylene-N(R)R, -methylene-NHC(=O)R, and -methylene-NHC(=O)NHR, wherein R is as defined in any one of the embodiments of the present disclosure; for example, each R is independently selected from hydrogen, -OH, -(C₁-C₄)alkyl, -(C₁-C₄)alkylene-O-(C₁-C₄)alkyl, -S(=O)₂(C₁-C₄)alkyl, -(C₃-C₆)cycloalkyl, -(4- to 8-membered)heterocyclyl, -methylene-(C₃-C₆)cycloalkyl, and -methylene-(4- to 8-membered)heterocyclyl, wherein the -(C₁-C₄)alkyl, -(C₃-C₆)cycloalkyl, and -(4- to 8-membered)heterocyclyl are each optionally substituted with 1, 2, 3, 4, or 5 groups selected from hydrogen, halogen, -OH, -NH₂, -NH(C₁-C₄)alkyl, -N[(C₁-C₄)alkyl]₂, -O-(C₁-C₄)alkyl, and -(C₁-C₄)alkyl.

In certain embodiments, R^{9a} and R^{9b} are each independently selected from hydrogen, -OR, -N(R)R, -methylene-N(R)R, and -NHC(=O)R, wherein R is as defined in any one of the embodiments of the present disclosure.

In certain embodiments, R^{9a} is selected from hydrogen, -CN, -N(R)R, -NHC(=O)R, -methylene-N(R)R, -methylene-NHC(=O)R, and -methylene-NHC(=O)NHR, wherein R is as defined in any one of the embodiments of the present disclosure; for example, each R is independently selected from hydrogen, -OH, methyl, ethyl, -methylene-O-ethyl, -S(=O)₂-methyl, cyclobutyl, azetidinyl, -methylene-cyclobutyl, and -methylene-azetidinyl, wherein the aforementioned groups are each independently optionally substituted with 1, 2, or 3 groups selected from -OH, OCH₃, NHCH₃, N(CH₃)₂, and CH₃.

**In** certain embodiments, R^{9a} is selected from hydrogen and -methylene-N(R)R, wherein R is as defined in any one of the embodiments of the present disclosure.

**In** certain embodiments, R^{9a} is selected from hydrogen, -CN, -NHCH₃, -N(CH₃)₂, -NHC(=O)H, - NHC(=O)CH₂OH, -NHC(=O)CH₂OCH₃, -NHC(=O)CH₂NHCH₃, -NHC(=O)CH₂N(CH₃)₂, - CH₂NH₂, -CH₂NHCH₃, -CH₂N(CH₃)₂, -CH₂N(CH₂CH₃)₂, -CH₂N(CH₃)(CH₂CH₃), - CH₂NHC(=O)CH₂OH, -CH₂NHC(=O)CH₂OCH₃, -CH₂NHC(=O)CH₂NHCH₃, - CH₂NHC(=O)CH₂N(CH₃)₂, -CH₂NHC(=O)H, -CH₂NHC(=O)CH₃, -CH₂NHC(=O)NHOH, - CH₂NHC(=O)NHCH₂CH₂0H, -CH₂NHC(=O)CH(N(CH₃)₂)CH₂OH, -CH₂NHC(=O)CH₂NHCH₃, -CH₂NHC(=O)CH₂N(CH₃)₂, -CH₂NHC(=O)CH₂N(CH₂CH₃)₂, -CH₂NHC(=O)CH₂OCH₂CH₂OH, -CH₂NHS(=O)₂CH₃, -CH₂NHC(=O)cyclobutyl-OH, -CH₂NHC(=O)-azetidinyl-CH₃, and - CH₂NHC(=O)CH₂-azetidinyl.

**In** certain embodiments, R^{9a} is selected from hydrogen, -CH₂NH₂, -CH₂NHCH₃, -NHC(=O)H, - NHC(=O)CH₂OH, and -CH₂NHC(=O)CH₂OH.

**In** certain embodiments, R^{9a} is selected from hydrogen, -CH₂NH₂, -NHC(=O)H, - NHC(=O)CH₂OH, and -CH₂NHC(=O)CH₂OH.

In certain embodiments, R^{9a} is selected from hydrogen, 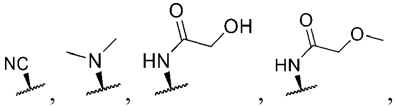 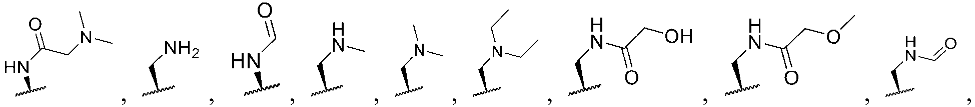 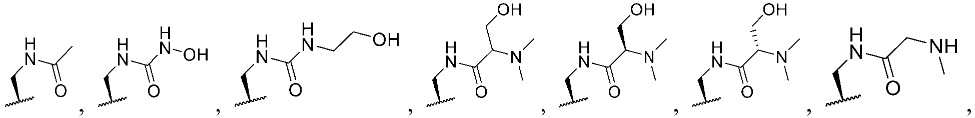 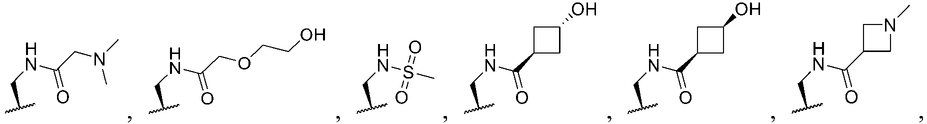 and

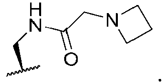

In certain embodiments, R^{9a} is selected from hydrogen,

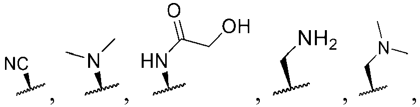

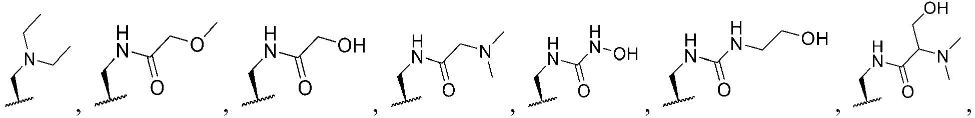

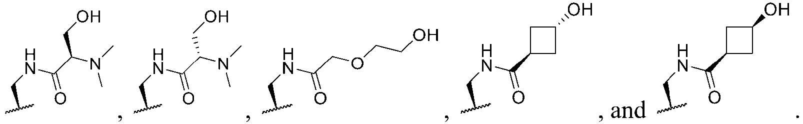

In certain embodiments, R^{9a} is selected from hydrogen,

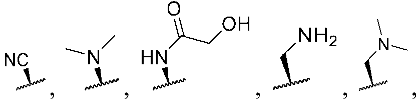

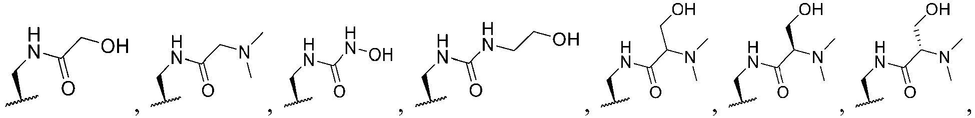

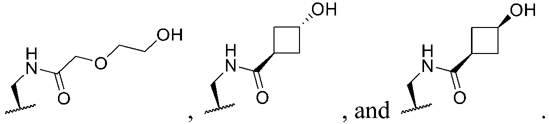

In certain embodiments, R^{9a} is selected from hydrogen, 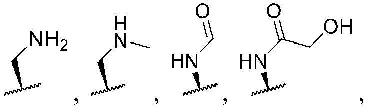 and

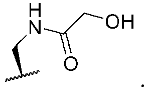

In certain embodiments, R^{9a} is selected from hydrogen, 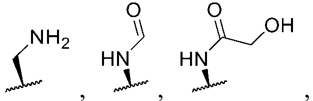 and

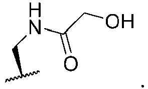

In certain embodiments, R^{9a} is selected from hydrogen,

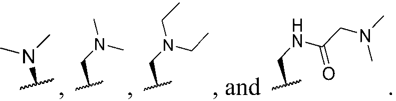

In certain embodiments, R^{9a} is selected from hydrogen,

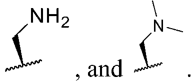

In certain embodiments, R^{9a} is selected from hydrogen and

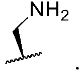

In certain embodiments, R^{9a} is hydrogen.

In certain embodiments, R^{9a} is

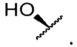

In certain embodiments, R^{9b} is -OH.

In certain embodiments, R^{9b} is selected from hydrogen and

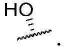

In certain embodiments, R^{9b} is

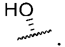

In certain embodiments, R^{9b} is selected from hydrogen,

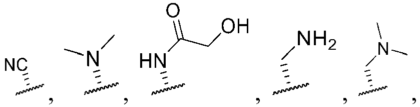

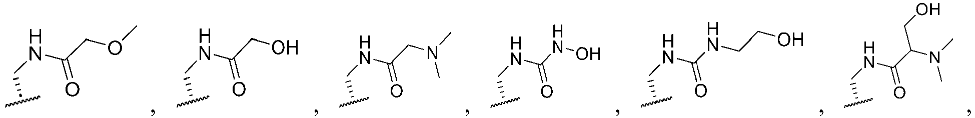

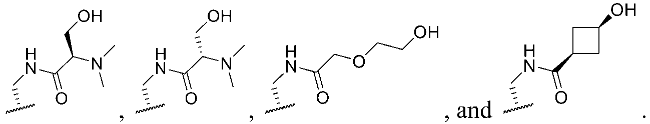

In certain embodiments, R^{8a} and R^{8b}, and R^{9a} and R^{9b} are independently combined to form oxo, - (C₂-C₄)alkenyl, -(C₃-C₆)cycloalkyl, or -(4- to 8-membered)heterocyclyl.

In certain embodiments, R^{8a} and R^{8b} are combined to form oxo, -(C₂-C₄)alkenyl, -(C₃-C₆)cycloalkyl, or -(4- to 8-membered)heterocyclyl.

In certain embodiments, R^{9a} and R^{9b} are combined to form oxo, -(C₂-C₄)alkenyl, -(C₃-C₆)cycloalkyl, or -(4- to 8-membered)heterocyclyl.

In certain embodiments, R^{8a} and R^{8b}, and R^{9a} and R^{9b} are independently combined to form oxo, vinyl, propenyl, cyclopropyl, cyclobutyl, cyclopentyl, oxetanyl, tetrahydrofuranyl, tetrahydropyranyl, azetidinyl, pyrrolidinyl, piperidinyl, or piperazinyl.

In certain embodiments, R^{8a} and R^{8b} are combined to form oxo, vinyl, propenyl, cyclopropyl, cyclobutyl, cyclopentyl, oxetanyl, tetrahydrofuranyl, tetrahydropyranyl, azetidinyl, pyrrolidinyl, piperidinyl, or piperazinyl.

In certain embodiments, R^{9a} and R^{9b} are combined to form oxo, vinyl, propenyl, cyclopropyl, cyclobutyl, cyclopentyl, oxetanyl, tetrahydrofuranyl, tetrahydropyranyl, azetidinyl, pyrrolidinyl, piperidinyl, or piperazinyl.

In certain embodiments, R^{8a} and R^{9a}, and R^{8b} and R^{9b}, together with the carbon atoms to which they are respectively attached, independently form (C₃-C₆)cycloalkyl, (4- to 8-membered)heterocyclyl, or (5- to 9-membered)heteroaryl, wherein the aforementioned group is optionally substituted with 1, 2, 3, 4, 5, 6, or 7 groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl. In certain embodiments, R^{8a} and R^{9a}, together with the carbon atoms to which they are attached, form (C₃-C₆)cycloalkyl, (4- to 8-membered)heterocyclyl, or (5- to 9-membered)heteroaryl, wherein the aforementioned group is optionally substituted with 1, 2, 3, 4, 5, 6, or 7 groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl.

In certain embodiments, R^{8b} and R^{9b}, together with the carbon atoms to which they are attached, form (C₃-C₆)cycloalkyl, (4- to 8-membered)heterocyclyl, or (5- to 9-membered)heteroaryl, wherein the aforementioned group is optionally substituted with 1, 2, 3, 4, 5, 6, or 7 groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl.

In certain embodiments, R^{8a} and R^{9a}, and R^{8b} and R^{9b}, together with the carbon atoms to which they are respectively attached, independently form (4- to 8-membered)heterocyclyl or (5- to 9-membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl.

In certain embodiments, R^{8a} and R^{9a}, together with the carbon atoms to which they are attached, form (4- to 8-membered)heterocyclyl or (5- to 9-membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, - N(methyl)-ethyl, -N(ethyl)₂, cyclopropyl, cyclobutyl, cyclopentyl, phenyl, and naphthyl.

In certain embodiments, R^{8b} and R^{9b}, together with the carbon atoms to which they are attached, form (4- to 8-membered)heterocyclyl or (5- to 9-membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, - N(methyl)-ethyl, -N(ethyl)₂, cyclopropyl, cyclobutyl, cyclopentyl, phenyl, and naphthyl.

In certain embodiments, R^{8a} and R^{9a}, and R^{8b} and R^{9b}, together with the carbon atoms to which they are respectively attached, independently form oxetanyl, tetrahydrofuranyl, tetrahydropyranyl, azetidinyl, pyrrolidinyl, piperidinyl, piperazinyl, pyridinonyl, pyrimidinonyl, oxazolyl, thiazolyl, pyrazolyl, imidazolyl, triazolyl, tetrazolyl, pyridinyl, pyrimidinyl, benzothienyl, or indolyl, wherein the aforementioned group is optionally substituted with 1, 2, or 3 groups selected from hydrogen, fluorine, chlorine, -OH, -O-methyl, -O-ethyl, -NH₂, -NH-methyl, -NH-ethyl, - N(methyl)₂, -N(methyl)-ethyl, -N(ethyl)₂, cyclopropyl, cyclobutyl, cyclopentyl, phenyl, and naphthyl.

In certain embodiments, R^{8a} and R^{9a}, together with the carbon atoms to which they are attached, form oxetanyl, tetrahydrofuranyl, tetrahydropyranyl, azetidinyl, pyrrolidinyl, piperidinyl, piperazinyl, pyridinonyl, pyrimidinonyl, oxazolyl, thiazolyl, pyrazolyl, imidazolyl, triazolyl, tetrazolyl, pyridinyl, pyrimidinyl, benzothienyl, or indolyl, wherein the aforementioned group is optionally substituted with 1, 2, or 3 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl.

In certain embodiments, R^{8b} and R^{9b}, together with the carbon atoms to which they are attached, form oxetanyl, tetrahydrofuranyl, tetrahydropyranyl, azetidinyl, pyrrolidinyl, piperidinyl, piperazinyl, pyridinonyl, pyrimidinonyl, oxazolyl, thiazolyl, pyrazolyl, imidazolyl, triazolyl, tetrazolyl, pyridinyl, pyrimidinyl, benzothienyl, or indolyl, wherein the aforementioned group is optionally substituted with 1, 2, or 3 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl.

In certain embodiments, R^{8a} and R^{9a} are absent, and R^{8b} and R^{9b}, together with the atoms to which they are attached, form 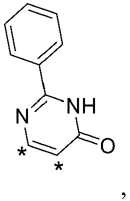 wherein * indicates the position of the attached atom.

In certain embodiments, R¹⁰ is selected from -OH; or R¹⁰ and R^{9b}, together with the atoms to which they are attached, form 5-membered nitrogen-containing heterocyclyl, wherein the 5-membered nitrogen-containing heterocyclyl is optionally substituted with 1, 2, 3, 4, or 5 groups selected from hydrogen, halogen, -OH, methyl, ethyl, -O-methyl, -O-ethyl, -NH₂, -NH-methyl, -NH-ethyl, - N(methyl)₂, -N(methyl)-ethyl, and -N(ethyl)₂.

In certain embodiments, R¹⁰ is -OH; or R¹⁰ and R^{9b}, together with the atoms to which they are attached, form imidazolinyl, wherein the imidazolinyl is optionally substituted with 1, 2, or 3 groups selected from halogen, -OH, methyl, -NH₂, -NH-methyl, and -N(methyl)₂.

In certain embodiments, R¹⁰ is -OH; or R¹⁰ and R^{9b}, together with the atoms to which they are attached, form 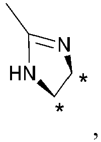 wherein * indicates the position of the attached atom.

In certain embodiments, R¹⁰ is selected from -OH; or R¹⁰ and R^{9a}, together with the atoms to which they are attached, form 5-membered nitrogen-containing heterocyclyl, wherein the 5-membered nitrogen-containing heterocyclyl is optionally substituted with 1, 2, 3, 4, or 5 groups selected from hydrogen, halogen, -OH, methyl, ethyl, -O-methyl, -O-ethyl, -NH₂, -NH-methyl, -NH-ethyl, - N(methyl)₂, -N(methyl)-ethyl, and -N(ethyl)₂.

In certain embodiments, R¹⁰ is -OH; or R¹⁰ and R^{9a}, together with the atoms to which they are attached, form imidazolinyl, wherein the imidazolinyl is optionally substituted with 1, 2, or 3 groups selected from halogen, -OH, methyl, -NH₂, -NH-methyl, and -N(methyl)₂.

In certain embodiments, R¹⁰ is -OH; or R¹⁰ and R^{9a}, together with the atoms to which they are attached, form 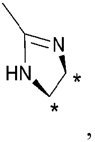 wherein * indicates the position of the attached atom.

In certain embodiments, R¹⁰ is -OH.

In certain embodiments, R¹⁰ is

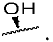

In certain embodiments, in the compound having the structure represented by formula I:
X¹ is selected from N and C(R¹), wherein R¹ is selected from hydrogen, halogen, cyano, -OH, - (C₁-C₄)alkyl, and -O-(C₁-C₄)alkyl;
X² is C(R²), wherein R² is selected from hydrogen, halogen, cyano, -OH, -(C₁-C₄)alkyl, and -O-(C₁-C₄)alkyl;
R³¹ is selected from oxo, hydrogen, fluorine, chlorine, cyano, -OH, -SH, -NH₂, -(C₁-C₄)alkyl, -O-(C₁-C₄)alkyl, -NH-(C₁-C₄)alkyl, -(C₁-C₄)alkylene-OH, -(C₁-C₄)haloalkyl, -(C₁-C₄)alkylene-NH₂, -(C₁-C₄)alkylene-NH(C₁-C₄)alkyl, and -(C₁-C₄)alkylene-N[(C₁-C₄)alkyl]₂;
R³ is selected from -(C₃-C₆)cycloalkyl, -(3- to 6-membered)heterocyclyl, and -(5- to 9-membered)heteroaryl, and R³ is optionally substituted with 1, 2, 3, or 4 R³¹;
R⁴ is selected from hydrogen, fluorine, chlorine, cyano, -OH, -NH₂, methyl, halomethyl, -O-methyl, -NH-methyl, and cyclopropyl;
X is selected from O, S, NH, and N[(C₁-C₄)alkyl];
ring B is phenyl;
R⁵and R⁶ are each independently selected from hydrogen, fluorine, chlorine, bromine, cyano, - OH, -NH₂, -(C₁-C₄)alkyl, -(C₁-C₄)alkyl-OH, -(C₁-C₄)alkyl-NH₂, -(C₁-C₄)haloalkyl, -O-(C₁-C₄)alkyl, -O-(C₁-C₄)haloalkyl, and -(C₃-C₆)cycloalkyl; or R⁵ and R⁶, together with the atoms to which they are attached, form 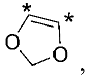 wherein * indicates the position of the attached atom;
ring C is phenyl;
each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂;
m is 1, 2, or 3;
each R is independently selected from hydrogen, -OH, NH₂, -(C₁-C₄)alkyl, -(C₁-C₄)haloalkyl, -O-(C₁-C₄)alkyl, -O-(C₁-C₄)haloalkyl, -(C₃-C₆)cycloalkyl, -(4- to 8-membered)heterocyclyl, -C(=O)-H, -C(=O)-(C₁-C₄)alkyl, -C(=O)-(C₃-C₆)cycloalkyl, and -[(C₁-C₄)alkylene]-(4- to 8-membered)heterocyclyl, wherein the -(C₁-C₄)alkylene-, -(C₁-C₄)alkyl, -(C₃-C₆)cycloalkyl, and - (4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, -N(ethyl)-propyl, methyl, and ethyl;
R^{8a} is 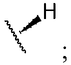
R^{8b} is selected from hydrogen, -methylene-R, -ethylene-R, -propylene-R, -methylene-OR, - ethylene-OR, -propylene-OR, -methylene-N(R)R, -ethylene-N(R)R, -propylene-N(R)R, -C(=O)R, -C(=O)N(R)R, and -C(=O)OR;
R^{9a} is selected from hydrogen, -OR, -N(R)R, -methylene-N(R)R, -methylene-NHC(=O)R, and - NHC(=O)R;
R^{9b} is selected from hydrogen and 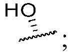
R¹⁰ is

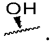

In certain embodiments, in the compound having the structure represented by formula I:
X¹ is selected from N and C(R¹), wherein R¹ is selected from hydrogen, halogen, cyano, -OH, - (C₁-C₄)alkyl, and -O-(C₁-C₄)alkyl;
X² is C(R²), wherein R² is selected from hydrogen, halogen, cyano, -OH, -(C₁-C₄)alkyl, and -O-(C₁-C₄)alkyl;
R³¹ is selected from oxo, hydrogen, fluorine, chlorine, cyano, -OH, -SH, -NH₂, -(C₁-C₄)alkyl, -O-(C₁-C₄)alkyl, -NH-(C₁-C₄)alkyl, -(C₁-C₄)alkylene-OH, -(C₁-C₄)haloalkyl, -(C₁-C₄)alkylene-NH₂, -(C₁-C₄)alkylene-NH(C₁-C₄)alkyl, and -(C₁-C₄)alkylene-N[(C₁-C₄)alkyl]₂;
R³ is selected from -(C₃-C₆)cycloalkyl, -(3- to 6-membered)heterocyclyl, and -(5- to 9-membered)heteroaryl, and R³ is optionally substituted with 1, 2, 3, or 4 R³¹;
R⁴ is selected from hydrogen, fluorine, chlorine, cyano, -OH, -NH₂, methyl, halomethyl, -O-methyl, -NH-methyl, and cyclopropyl;
X is selected from O, S, NH, and N[(C₁-C₄)alkyl];
ring B is phenyl;
R⁵ and R⁶ are each independently selected from hydrogen, fluorine, chlorine, bromine, cyano, - OH, -NH₂, -(C₁-C₄)alkyl, -(C₁-C₄)alkyl-OH, -(C₁-C₄)alkyl-NH₂, -(C₁-C₄)haloalkyl, -O-(C₁-C₄)alkyl, -O-(C₁-C₄)haloalkyl, and -(C₃-C₆)cycloalkyl; or R⁵ and R⁶, together with the atoms to which they are attached, form 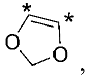 wherein * indicates the position of the attached atom;
ring C is phenyl;
each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂;
m is 1, 2, or 3;
each R is independently selected from hydrogen, -OH, NH₂, -(C₁-C₄)alkyl, -(C₁-C₄)haloalkyl, -O-(C₁-C₄)alkyl, -O-(C₁-C₄)haloalkyl, -(C₃-C₆)cycloalkyl, -(4- to 8-membered)heterocyclyl, -C(=O)-H, -C(=O)-(C₁-C₄)alkyl, -C(=O)-(C₃-C₆)cycloalkyl, and -[(C₁-C₄)alkylene]-(4- to 8-membered)heterocyclyl, wherein the -(C₁-C₄)alkylene-, -(C₁-C₄)alkyl, -(C₃-C₆)cycloalkyl, and - (4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, and -N(ethyl)-propyl;
R^{8a} is 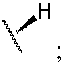
R^{8b} is selected from hydrogen, -methylene-R, -ethylene-R, -propylene-R, -methylene-OR, - ethylene-OR, -propylene-OR, -methylene-N(R)R, -ethylene-N(R)R, -propylene-N(R)R, -C(=O)R, -C(=O)N(R)R, and -C(=O)OR;
R^{9a} is selected from hydrogen, -OR, -N(R)R, -methylene-N(R)R, and -NHC(=O)R;
R^{9b} is selected from hydrogen and 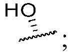
R¹⁰ is

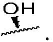

In certain embodiments, in the compound having the structure represented by formula I:
X¹ is selected from N and C(R¹), wherein R¹ is selected from hydrogen, halogen, cyano, -OH, - (C₁-C₄)alkyl, and -O-(C₁-C₄)alkyl;
X² is selected from N and C(R²), wherein R² is selected from hydrogen, halogen, cyano, -OH, - (C₁-C₄)alkyl, and -O(C₁-C₄)alkyl;
R³¹ is selected from hydrogen, fluorine, chlorine, cyano, -OH, -SH, -NH₂, -(C₁-C₄)alkyl, -(C₁-C₄)alkylene-OH, -(C₁-C₄)haloalkyl, -(C₁-C₄)alkylene-NH₂, -(C₁-C₄)alkylene-NH(C₁-C₄)alkyl, and -(C₁-C₄)alkylene-N[(C₁-C₄)alkyl]₂;
R³ is selected from -(5- to 6-membered)heteroaryl, and R³ is optionally substituted with 1, 2, 3, or 4 R³¹;
R⁴ is selected from hydrogen, fluorine, chlorine, cyano, -OH, -NH₂, methyl, halomethyl, -O-methyl, -NH-methyl, and cyclopropyl;
X is selected from O, S, NH, and N[(C₁-C₄)alkyl];
ring B is phenyl;
R⁵ and R⁶ are each independently selected from hydrogen, fluorine, chlorine, bromine, cyano, - OH, -NH₂, -(C₁-C₄)alkyl, -(C₁-C₄)haloalkyl, -O-(C₁-C₄)alkyl, -O-(C₁-C₄)haloalkyl, and -(C₃-C₆)cycloalkyl; or R⁵ and R⁶, together with the atoms to which they are attached, form 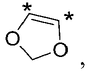 wherein * indicates the position of the attached atom;
ring C is phenyl;
each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂;
m is 1, 2, or 3;
each R is independently selected from hydrogen, -OH, -(C₁-C₄)alkyl, -(C₁-C₄)haloalkyl, -O-(C₁-C₄)alkyl, -O-(C₁-C₄)haloalkyl, -(C₃-C₆)cycloalkyl, -(4- to 8-membered)heterocyclyl, -C(=O)-H, - C(=O)-(C₁-C₄)alkyl, -C(=O)-(C₃-C₆)cycloalkyl, and -[(C₁-C₄)alkylene]-(4- to 8-membered)heterocyclyl, wherein the -(C₁-C₄)alkylene-, -(C₁-C₄)alkyl, -(C₃-C₆)cycloalkyl, and - (4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, and -N(ethyl)-propyl;
R^{8a} is 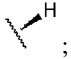
R^{8b} is selected from hydrogen, -methylene-R, -ethylene-R, -propylene-R, -methylene-OR, - ethylene-OR, -propylene-OR, -methylene-N(R)R, -ethylene-N(R)R, -propylene-N(R)R, -C(=O)R, -C(=O)N(R)R, and -C(=O)OR;
R^{9a} is selected from hydrogen, -OR, -N(R)R, -methylene-N(R)R, and -NHC(=O)R;
R^{9b} is selected from hydrogen and 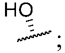
R¹⁰ is

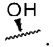

In certain embodiments, in the compound having the structure represented by formula I:
X¹ is selected from N and C(R¹), wherein R¹ is selected from hydrogen, fluorine, chlorine, cyano, -OH, methyl, ethyl, propyl, -O-methyl, -O-ethyl, and -O-propyl;
X² is selected from C(R²), wherein R² is selected from hydrogen, fluorine, chlorine, cyano, -OH, methyl, ethyl, propyl, -O-methyl, -O-ethyl, and -O-propyl;
R³¹ is selected from hydrogen, fluorine, chlorine, cyano, -OH, -SH, -NH₂, methyl, halomethyl, - O-methyl, -CH₂OH, -CH₂NH₂, and -CH₂CH₂N(CH₃)₂;
R³ is selected from cyclopropyl, oxetanyl, piperazinyl, piperazinonyl, morpholinyl, morpholinonyl, oxazolyl, thiazolyl, pyrazolyl, isoxazolyl, isothiazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl, and R³ is optionally substituted with 1, 2, or 3 R³¹;
R⁴ is selected from hydrogen, fluorine, chlorine, cyano, -OH, and -NH₂;
X is selected from O and S;
the structural unit 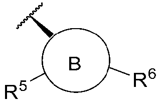 is 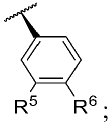
R⁵ is selected from hydrogen, -OH, -NH₂, and -CH₂NH₂;
R⁶ is selected from hydrogen, fluorine, chlorine, bromine, cyano, -OH, -NH₂, cyclopropyl, and - O-methyl;
or R⁵ and R⁶, together with the atoms to which they are attached, form 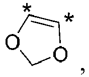 wherein * indicates the position of the attached atom;
the structural unit 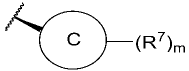 is 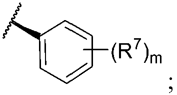
each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂;
m is 1, 2, or 3;
R^{8a} is 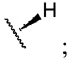
R^{8b} is selected from hydrogen, -methylene-R, -methylene-N(R)R, -C(=O)OR, -C(=O)N(R)R, and -C(=O)R, wherein R is as defined in any one of the embodiments of the present disclosure; for example, each R is independently selected from hydrogen, -OH, methyl, ethyl, -O-methyl, -C(=O)-methyl, -S(=O)₂-methyl, azetidinyl, pyrrolidinyl, and piperazinyl, wherein the aforementioned groups are each independently optionally substituted with 1, 2, or 3 groups selected from -OH and -NH₂;
R^{9a} is selected from hydrogen, -CN, -N(R)R, -NHC(=O)R, -methylene-N(R)R, -methylene-NHC(=O)R, and -methylene-NHC(=O)NHR, wherein R is as defined in any one of the embodiments of the present disclosure; for example, each R is independently selected from hydrogen, -OH, methyl, ethyl, -methylene-O-ethyl, -S(=O)₂-methyl, cyclobutyl, azetidinyl, - methylene-cyclobutyl, and -methylene-azetidinyl, wherein the aforementioned groups are each independently optionally substituted with 1, 2, or 3 groups selected from -OH, OCH₃, NHCH₃, N(CH₃)₂, and CH₃;
R^{9b} is selected from hydrogen and 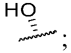
R¹⁰ is

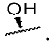

In certain embodiments, in the compound having the structure represented by formula I:
X¹ is selected from N and C(R¹), wherein R¹ is selected from hydrogen, fluorine, chlorine, cyano, -OH, methyl, ethyl, propyl, -O-methyl, -O-ethyl, and -O-propyl;
X² is selected from C(R²), wherein R² is selected from hydrogen, fluorine, chlorine, cyano, -OH, methyl, ethyl, propyl, -O-methyl, -O-ethyl, and -O-propyl;
R³¹ is selected from hydrogen, fluorine, chlorine, cyano, -OH, -SH, -NH₂, methyl, halomethyl, - O-methyl, -CH₂OH, and -CH₂CH₂N(CH₃)₂;
R³ is selected from cyclopropyl, oxetanyl, piperazinyl, piperazinonyl, morpholinyl, morpholinonyl, oxazolyl, thiazolyl, pyrazolyl, isoxazolyl, isothiazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl, and R³ is optionally substituted with 1, 2, or 3 R³¹;
R⁴ is selected from hydrogen, fluorine, chlorine, cyano, -OH, and -NH₂;
X is selected from O and S;
the structural unit 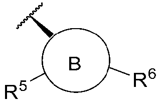 is 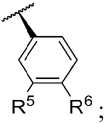
R⁵ is selected from hydrogen, -OH, -NH₂, and -CH₂NH₂;
R⁶ is selected from hydrogen, fluorine, chlorine, bromine, cyano, -OH, -NH₂, and -O-methyl;
or R⁵ and R⁶, together with the atoms to which they are attached, form 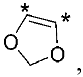 wherein * indicates the position of the attached atom;
the structural unit 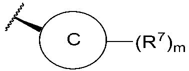 is 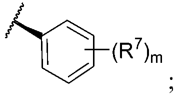
each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂;
m is 1, 2, or 3;
each R is independently selected from hydrogen, -OH, -(C₁-C₄)alkyl, -O-(C₁-C₄)alkyl, -C(=O)-H, -C(=O)CH₂OH, oxetanyl, and azetidinyl-OH, wherein the aforementioned groups are each independently optionally substituted with 1, 2, or 3 groups selected from hydrogen, halogen, -OH, -NH₂, -NH-methyl, and -N(methyl)₂;
R^{8a} is 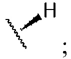
R^{8b} is selected from H, -methylene-R, -methylene-N(R)R, -C(=O)OR, -C(=O)N(R)R, and - C(=O)R;
R^{9a} is selected from hydrogen, -methylene-N(R)R, and -NHC(=O)R;
R^{9b} is selected from hydrogen and 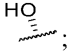
R¹⁰ is

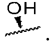

In certain embodiments, in the compound having the structure represented by formula I:
X¹ is selected from N and C(R¹), wherein R¹ is selected from hydrogen, fluorine, chlorine, cyano, -OH, methyl, ethyl, propyl, -O-methyl, -O-ethyl, and -O-propyl;
X² is selected from N and C(R²), wherein R² is selected from hydrogen, fluorine, chlorine, cyano, -OH, methyl, ethyl, propyl, -O-methyl, -O-ethyl, and -O-propyl;
R³¹ is selected from hydrogen, fluorine, chlorine, cyano, -OH, -SH, -NH₂, methyl, halomethyl, - CH₂OH, and -CH₂CH₂N(CH₃)₂;
R³ is selected from oxazolyl, pyrazolyl, and pyrimidinyl, and R³ is optionally substituted with 1, 2, or 3 R³¹;
R⁴ is selected from hydrogen, fluorine, chlorine, cyano, -OH, and -NH₂;
X is selected from O;
the structural unit 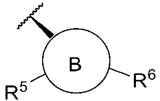 is 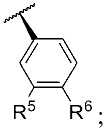
R⁵ is selected from hydrogen and -NH₂;
R⁶ is selected from hydrogen, fluorine, chlorine, bromine, cyano, -OH, -NH₂, and -O-methyl;
the structural unit 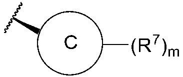 is 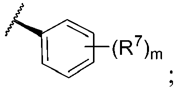
each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂;
m is 1, 2, or 3;
each R is independently selected from hydrogen, -OH, -(C₁-C₄)alkyl, and -O-(C₁-C₄)alkyl, wherein the aforementioned groups are each independently optionally substituted with 1, 2, or 3 groups selected from hydrogen, halogen, -OH, -NH₂, -NH-methyl, and -N(methyl)₂;
R^{8a} is 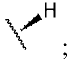
R^{8b} is selected from -methylene-R, -C(=O)OR, -C(=O)N(R)R, and -C(=O)R;
R^{9a} is selected from hydrogen and -methylene-N(R)R;
R^{9b} is selected from hydrogen and 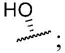
R¹⁰ is

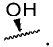

In certain embodiments, in the compound having the structure represented by formula I:
X¹ is C(R¹);
R¹ is selected from hydrogen, fluorine, chlorine, cyano, -OH, methyl, ethyl, propyl, -O-methyl, - O-ethyl, and -O-propyl;
X² is selected from CH;
R³¹ is selected from hydrogen, fluorine, chlorine, cyano, -OH, -SH, -NH₂, -CH₂OH, -CH₂NH₂, methyl, trifluoromethyl, and -O-methyl;
R³ is selected from 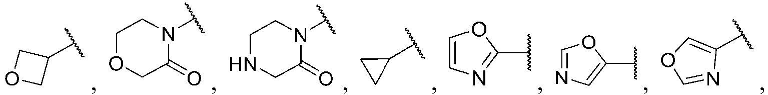 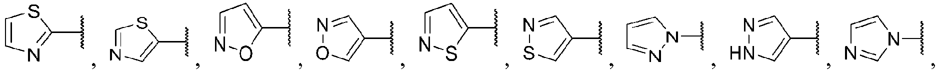 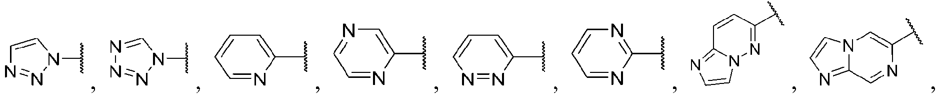
and 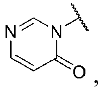 and R³ is optionally substituted with 1, 2, or 3 R³¹;
R⁴ is hydrogen;
X is O;
the structural unit 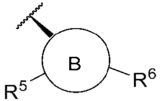 is 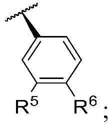
R⁵ is selected from hydrogen, -OH, -NH₂, and -CH₂NH₂;
R⁶ is selected from hydrogen, fluorine, chlorine, bromine, cyano, -OH, -NH₂, cyclopropyl, and - O-methyl;
or R⁵ and R⁶, together with the atoms to which they are attached, form 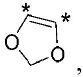 wherein * indicates the position of the attached atom;
the structural unit 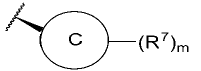 is 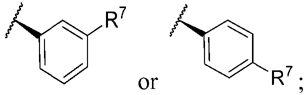
each R⁷ is independently selected from hydrogen, fluorine, chlorine, bromine, -OH, and -NH₂;
R^{8a} is 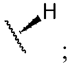
R^{8b} is selected from hydrogen, 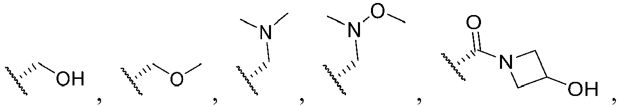 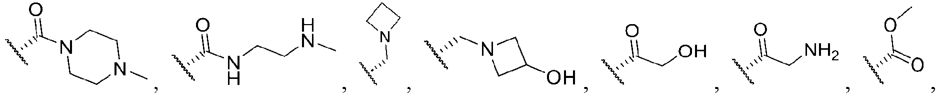 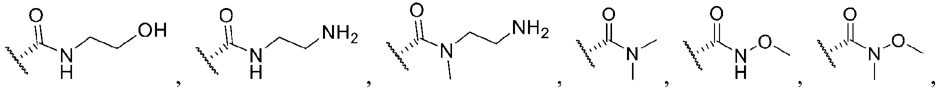 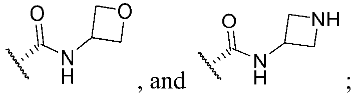
R^{9a} is selected from hydrogen, 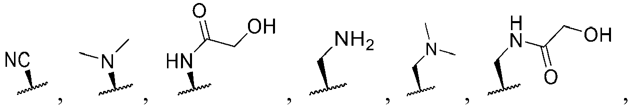 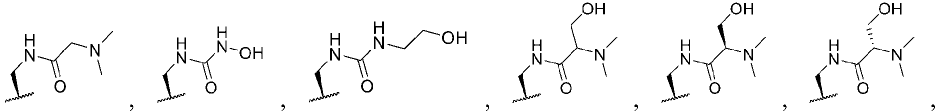 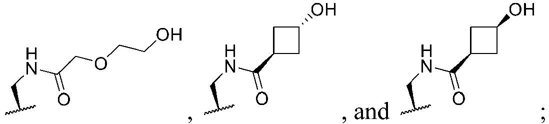
R^{9b} is selected from hydrogen and 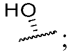
R¹⁰ is

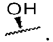

In certain embodiments, in the compound having the structure represented by formula I:
X¹ is C(R¹);
R¹ is selected from hydrogen, fluorine, chlorine, cyano, -OH, methyl, ethyl, propyl, -O-methyl, - O-ethyl, and -O-propyl;
X² is selected from CH;
R³¹ is selected from hydrogen, fluorine, chlorine, cyano, -OH, -SH, -NH₂, -CH₂OH, methyl, trifluoromethyl, and -O-methyl;
R³ is selected from 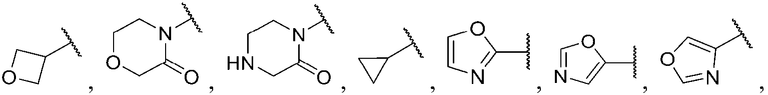 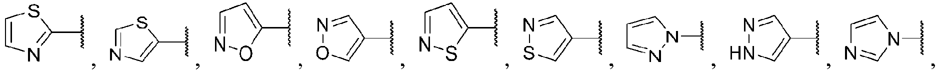 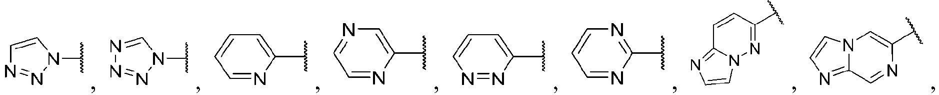 and 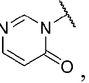 and R³ is optionally substituted with 1, 2, or 3 R³¹;
R⁴ is hydrogen;
X is O;
the structural unit 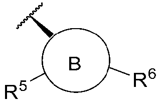 is 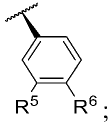
R⁵ is selected from hydrogen, -OH, -NH₂, and -CH₂NH₂;
R⁶ is selected from hydrogen, fluorine, chlorine, bromine, cyano, -OH, -NH₂, and -O-methyl;
or R⁵ and R⁶, together with the atoms to which they are attached, form 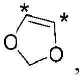 wherein * indicates the position of the attached atom;
the structural unit 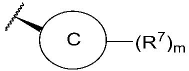 is 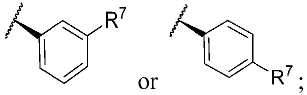
each R⁷ is independently selected from hydrogen, fluorine, chlorine, bromine, -OH, and -NH₂;
R^{8a} is 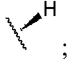
R^{8b} is selected from hydrogen, 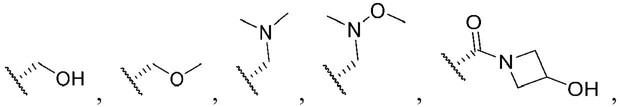 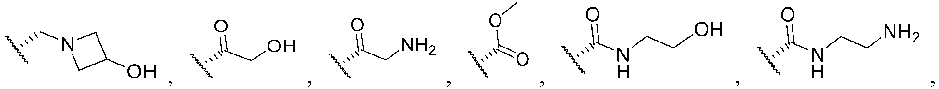 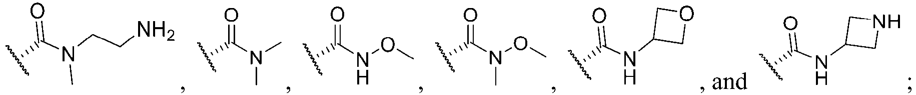
R^{9a} is selected from hydrogen, 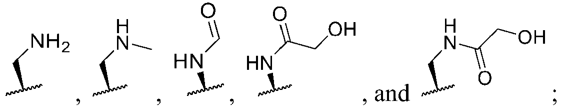
R^{9b} is selected from hydrogen and 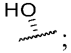
R¹⁰ is

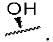

In certain embodiments, in the compound having the structure represented by formula I:
X¹ is C(R¹);
R¹ is selected from hydrogen, fluorine, chlorine, cyano, -OH, methyl, ethyl, propyl, -O-methyl, - O-ethyl, and -O-propyl;
X² is selected from CH;
R³¹ is selected from hydrogen, fluorine, chlorine, cyano, -OH, -SH, and -NH₂;
R³ is selected from 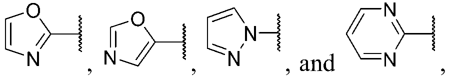 and R³ is optionally substituted with 1, 2, or 3 R³¹;
R⁴ is hydrogen;
X is O;
the structural unit 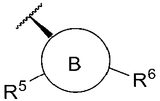 is 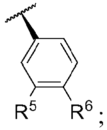
R⁵ is selected from hydrogen;
R⁶ is selected from hydrogen, fluorine, chlorine, bromine, cyano, -OH, -NH₂, and -O-methyl;
the structural unit 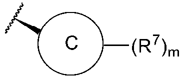 is 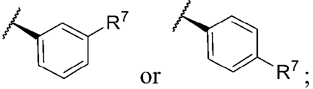
each R⁷ is independently selected from hydrogen, fluorine, chlorine, bromine, -OH, and -NH₂;
R^{8a} is 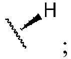
R^{8b} is selected from 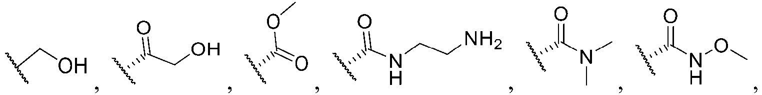 and 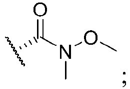
R^{9a} is selected from hydrogen and 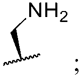
R^{9b} is selected from hydrogen and 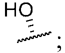
R¹⁰ is

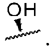

In certain embodiments, the racemic mixture is a racemate.

In certain embodiments, the compound is selected from a compound represented by formula I-1: 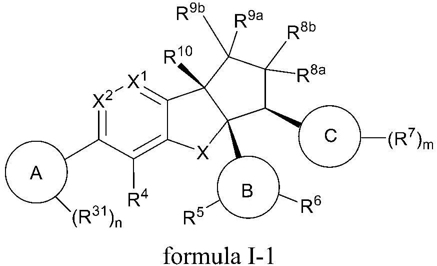 wherein
X, X¹, X², ring B, ring C, R³¹, R⁴, R⁵, R⁶, R⁷, R^{8a}, R^{8b}, R^{9a}, R^{9b}, R¹⁰, and m are as defined in any one of the embodiments of the present disclosure;
n is 0, 1, 2, 3, 4, or 5;
ring A is selected from 5- to 10-membered heteroaryl.

In certain embodiments, the structural fragment 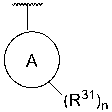 is as defined for R³, and R³ is as defined in any one of the embodiments of the present disclosure.

In certain embodiments, ring A is 5- to 9-membered heteroaryl.

In certain embodiments, ring A is 5- to 6-membered heteroaryl.

In certain embodiments, ring A is selected from oxazolyl, thiazolyl, pyrazolyl, imidazolyl, triazolyl, tetrazolyl, pyridinyl, and pyrimidinyl.

In certain embodiments, ring A is selected from

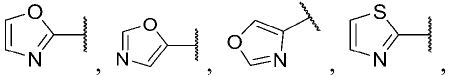

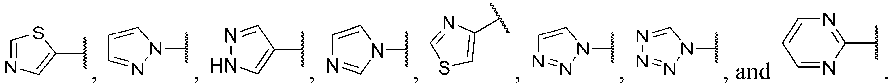

In certain embodiments, ring A is selected from

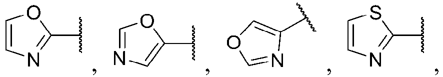

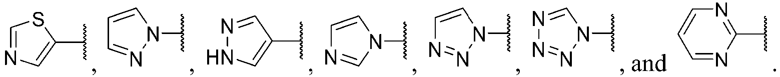

In certain embodiments, ring A is selected from oxazolyl and pyrimidinyl.

In certain embodiments, ring A is selected from

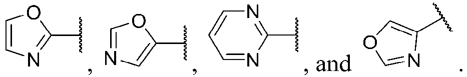

In certain embodiments, the compound is selected from a compound represented by formula I-1-A: 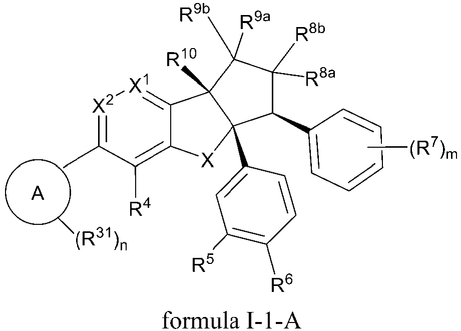 wherein
X, X¹, X², R³¹, R⁴, R⁵, R⁶, R⁷, R^{8a}, R^{8b}, R^{9a}, R^{9b}, R¹⁰, and m are as defined in any one of the embodiments of the present disclosure, and ring A and n are as defined in any one of the embodiments of the present disclosure.

In certain embodiments, the structural unit 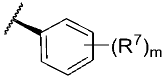 is

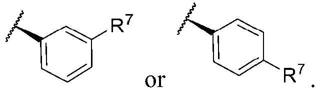

In certain embodiments, the structural unit 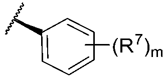 is

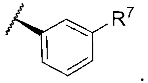

In certain embodiments, the compound is selected from a compound represented by formula I-1-B: 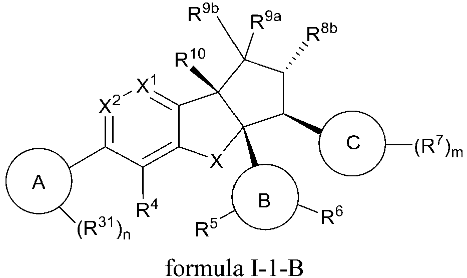 wherein
X, X¹, X², ring B, ring C, R³¹, R⁴, R⁵, R⁶, R⁷, R^{8b}, R^{9a}, R^{9b}, R¹⁰, and m are as defined in any one of the embodiments of the present disclosure, and ring A and n are as defined in any one of the embodiments of the present disclosure.

In certain embodiments, the compound is selected from a compound represented by formula I-1-AB-1: 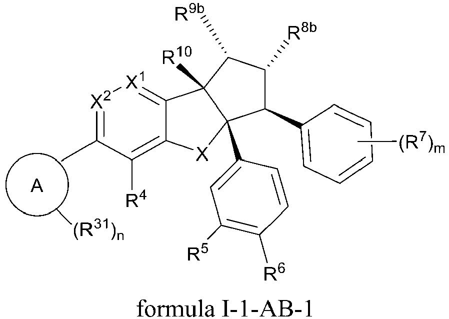 wherein
X, X¹, X², R³¹, R⁴, R⁵, R⁶, R⁷, R^{8b}, R^{9b}, R¹⁰, and m are as defined in any one of the embodiments of the present disclosure, and ring A and n are as defined in any one of the embodiments of the present disclosure.

In certain embodiments, the compound is selected from a compound represented by formula I-1-AB-1A or formula I-1-AB-1B: 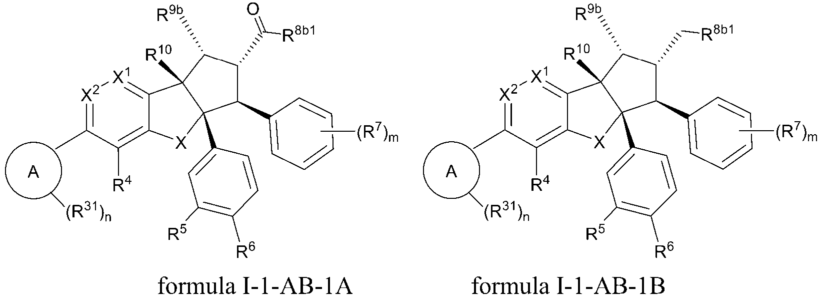 wherein
X, X¹, X², R³¹, R⁴, R⁵, R⁶, R⁷, R^{9b}, R¹⁰, and m are as defined in any one of the embodiments of the present disclosure, ring A and n are as defined in any one of the embodiments of the present disclosure, and R^{8b1} is selected from R and N(R)R, wherein R is as defined in any one of the embodiments of the present disclosure.

In certain embodiments, the compound is selected from a compound represented by formula I-1-AB-1C or formula I-1-AB-1D: 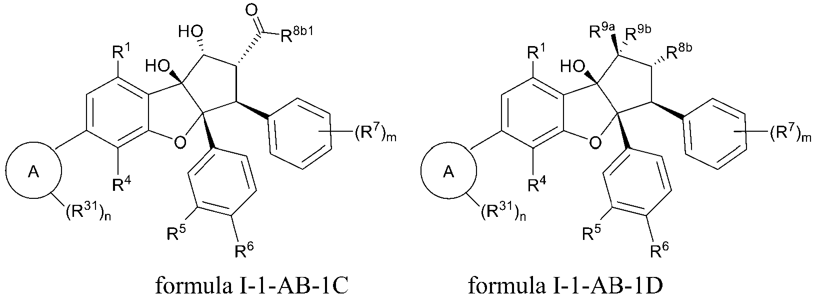 wherein
R³¹, R¹, R⁴, R⁵, R⁶, R⁷, R^{8b}, R^{9b}, R^{9a}, and m are as defined in any one of the embodiments of the present disclosure, ring A and n are as defined in any one of the embodiments of the present disclosure, and R^{8b1} is selected from R and N(R)R, wherein R is as defined in any one of the embodiments of the present disclosure.

In certain embodiments, the compound is selected from a compound represented by formula I-1-AB-1D:
wherein
R¹ is selected from hydrogen, halogen, cyano, -OH, -(C₁-C₄)alkyl, and -O-(C₁-C₄)alkyl, preferably selected from hydrogen, fluorine, chlorine, cyano, -OH, methyl, ethyl, propyl, -O-methyl, -O-ethyl, and -O-propyl;
ring A is selected from oxazolyl, pyrazolyl, and pyrimidinyl, preferably selected from 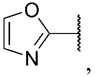 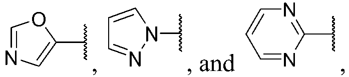 and ring A is optionally substituted with 1, 2, or 3 R³¹;
each R³¹ is independently selected from hydrogen, fluorine, chlorine, cyano, -OH, -SH, -NH₂, - (C₁-C₄)alkyl, -O-(C₁-C₄)alkyl, -(C₁-C₄)alkylene-OH, -(C₁-C₄)alkylene-NH₂, and -(C₁-C₄)haloalkyl, preferably selected from hydrogen, fluorine, chlorine, cyano, -OH, -SH, -NH₂, - CH₂OH, -CH₂NH₂, -CH₃, -CF₃, and -OCH₃;
or the structural fragment 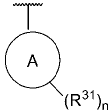 is selected from 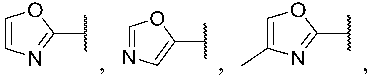 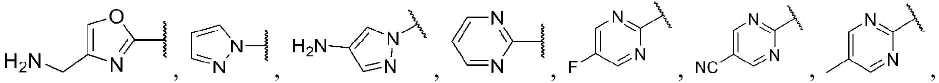 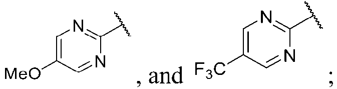
R⁴ is hydrogen;
R⁵ is hydrogen or -NH₂;
R⁶ is selected from hydrogen, fluorine, chlorine, bromine, cyano, -OH, -NH₂, -(C₁-C₄)alkyl, -(C₁-C₄)alkyl-OH, -(C₁-C₄)alkyl-NH₂, -(C₁-C₄)haloalkyl, -O-(C₁-C₄)alkyl, -O-(C₁-C₄)haloalkyl, and - (C₃-C₆)cycloalkyl, preferably selected from hydrogen, fluorine, chlorine, bromine, cyano, -OH, - NH₂, cyclopropyl, and -O-methyl;
each R⁷ is independently selected from hydrogen, fluorine, chlorine, bromine, -OH, and -NH₂; m is 0, 1, 2, or 3;
R^{8b} is selected from hydrogen, -methylene-R, -methylene-OR, -methylene-N(R)R, -C(=O)R, - C(=O)OR, and -C(=O)N(R)R, wherein R is as defined in any one of the embodiments of the present disclosure; for example, each R is independently selected from hydrogen, -OH, -NH₂, - (C₁-C₄)alkyl, -O-(C₁-C₄)alkyl, -C(=O)-(C₁-C₄)alkyl, -S(=O)₂(C₁-C₄)alkyl, and -(4- to 8-membered)heterocyclyl, wherein the -(C₁-C₄)alkyl and -(4- to 8-membered)heterocyclyl are each optionally substituted with 1, 2, 3, 4, or 5 groups selected from hydrogen, halogen, -OH, -NH₂, - NHCH₃, and CH₃; R^{8b} is preferably selected from hydrogen, 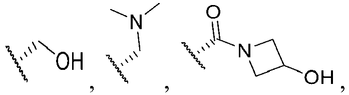 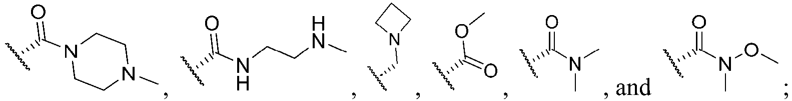
R^{9a} is selected from hydrogen, -CN, -N(R)R, -NHC(=O)R, -methylene-N(R)R, -methylene-NHC(=O)R, and -methylene-NHC(=O)NHR, wherein R is as defined in any one of the embodiments of the present disclosure; for example, each R is independently selected from hydrogen, -OH, methyl, ethyl, -methylene-O-ethyl, -S(=O)₂-methyl, cyclobutyl, azetidinyl, - methylene-cyclobutyl, and -methylene-azetidinyl, wherein the aforementioned groups are each independently optionally substituted with 1, 2, or 3 groups selected from -OH, OCH₃, NHCH₃, N(CH₃)₂, and CH₃; R^{9a} is preferably selected from hydrogen, 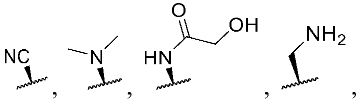 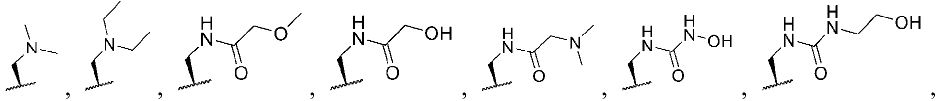 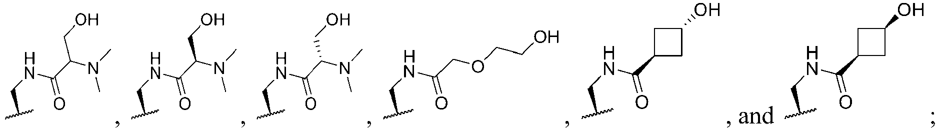
R^{9b} is selected from hydrogen and

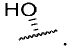

In certain embodiments, the compound is selected from

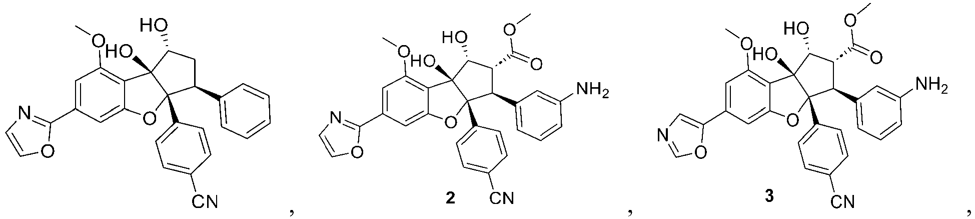

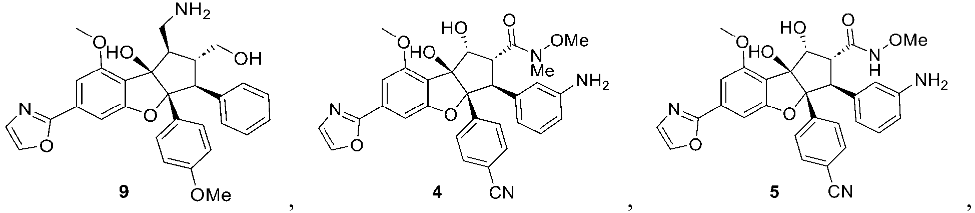

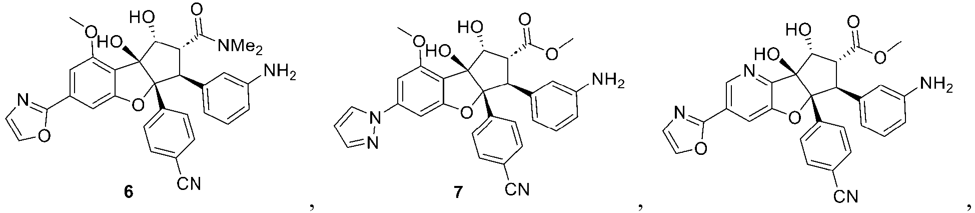

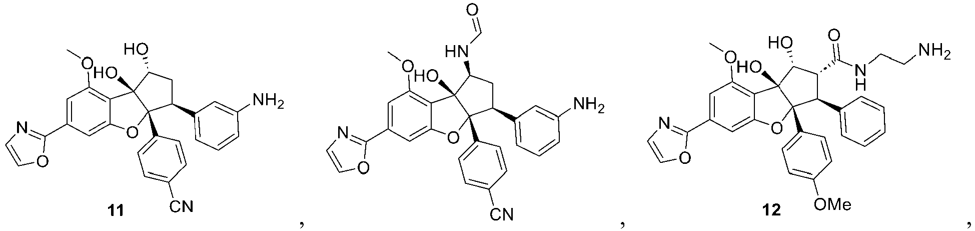

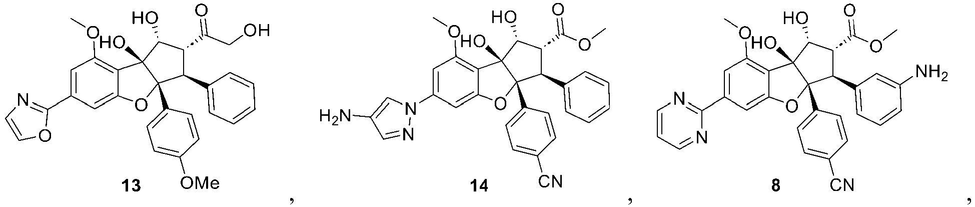

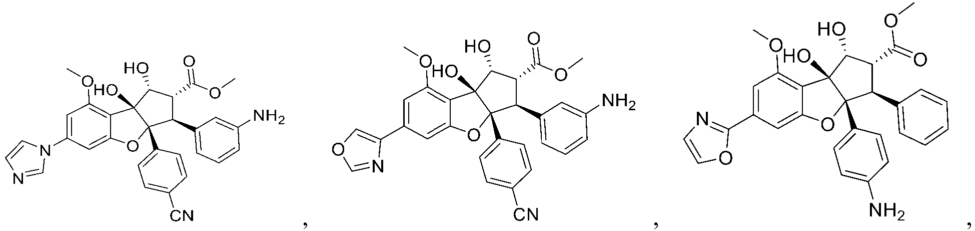

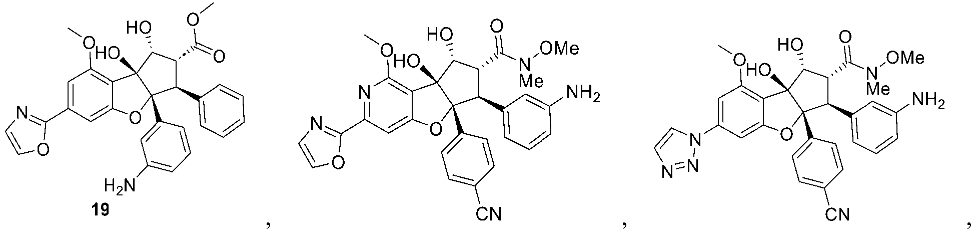

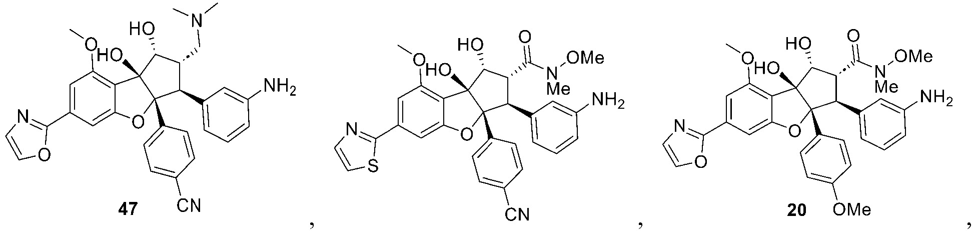

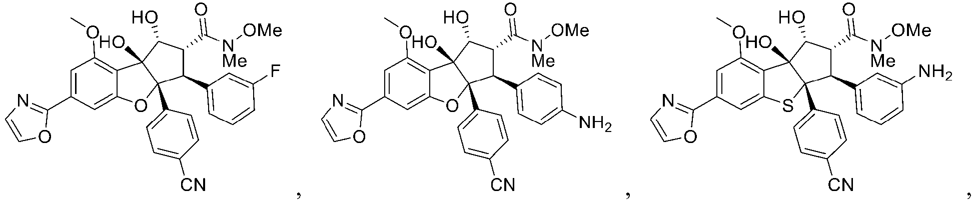

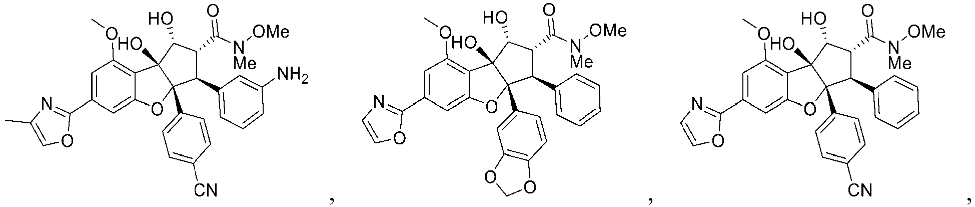

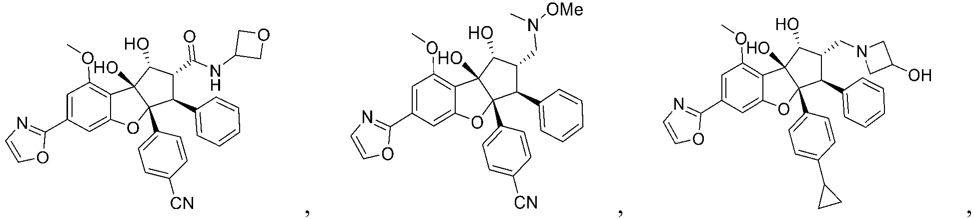

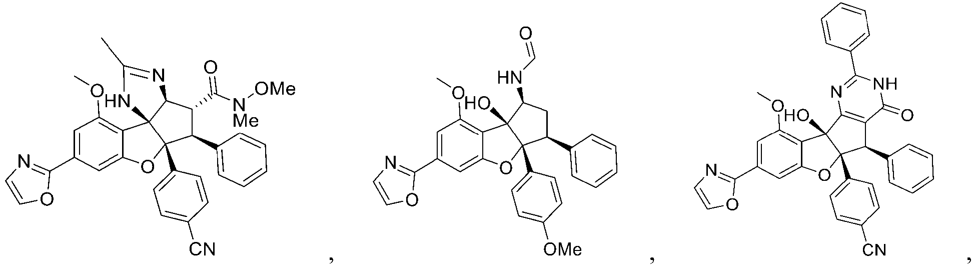

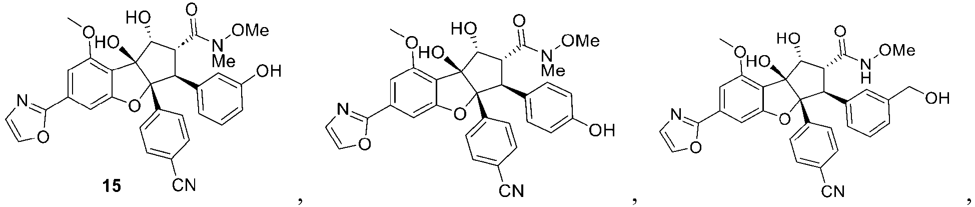

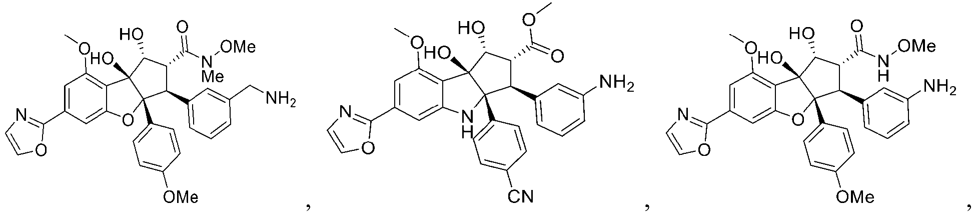

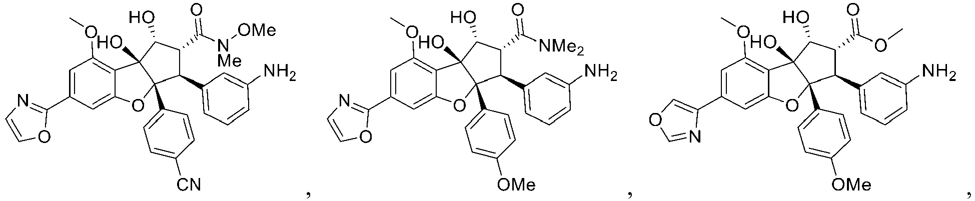

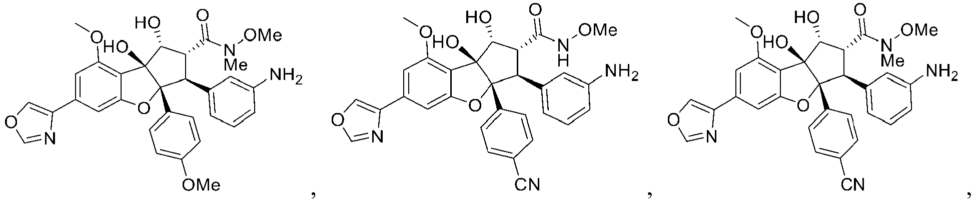

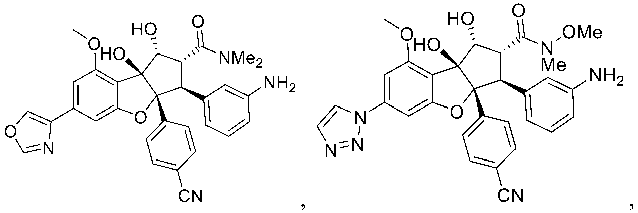

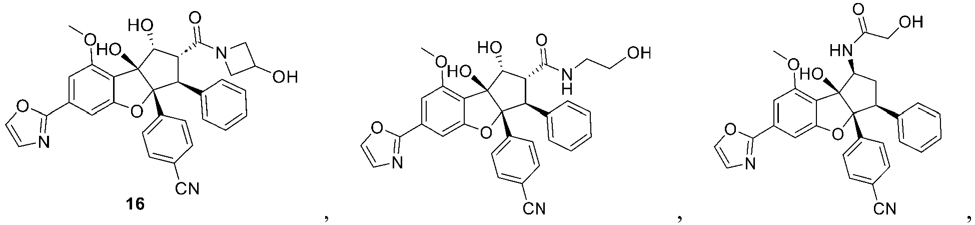

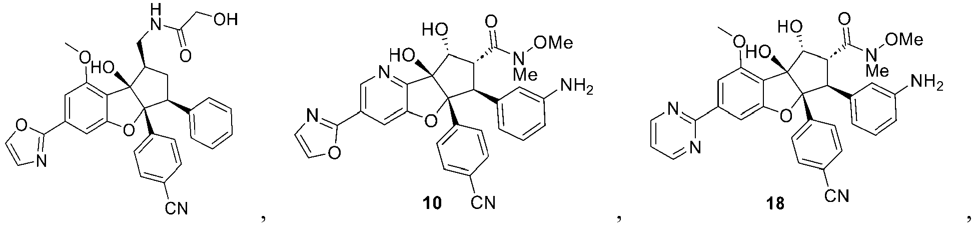

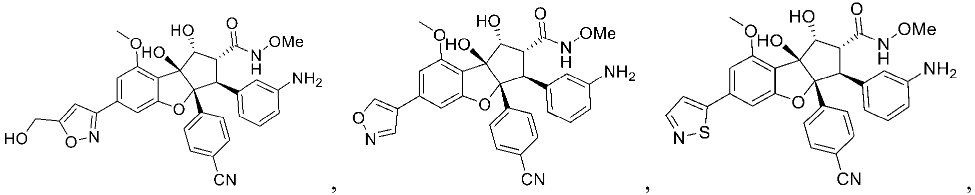

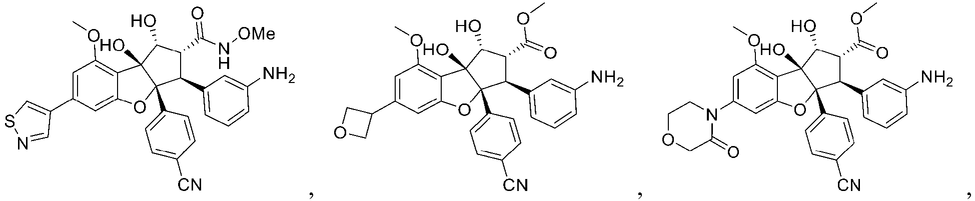

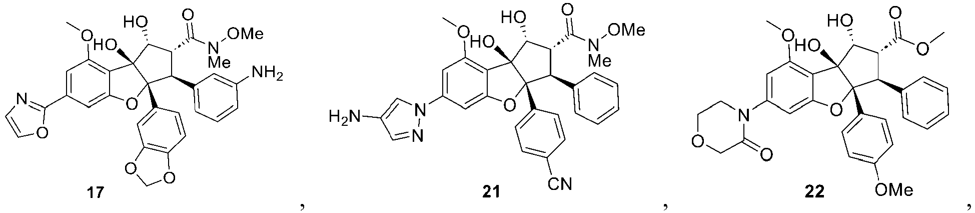

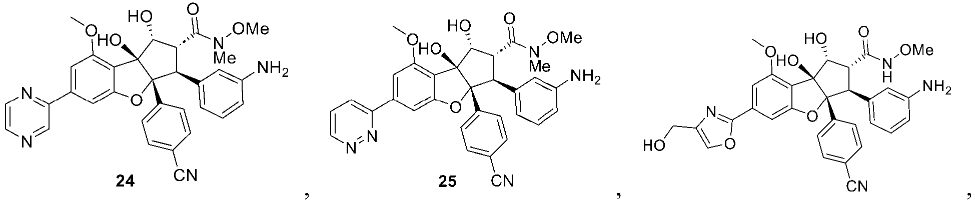

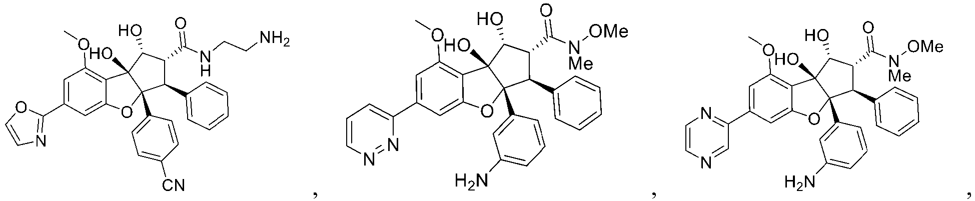

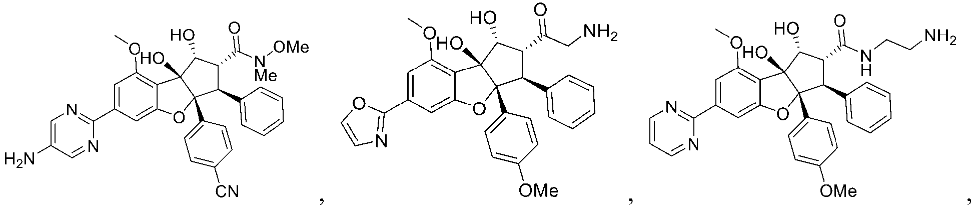

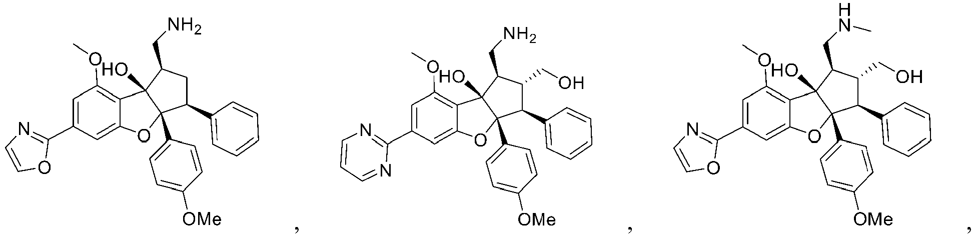

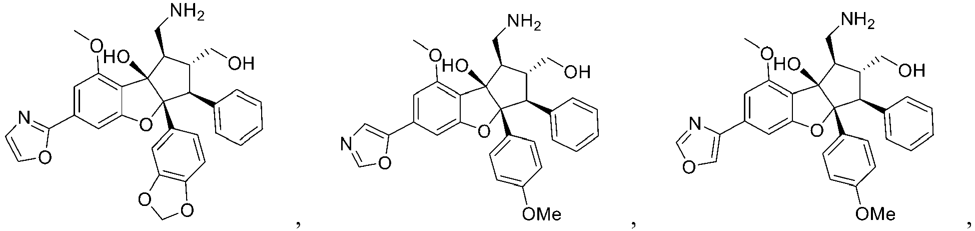

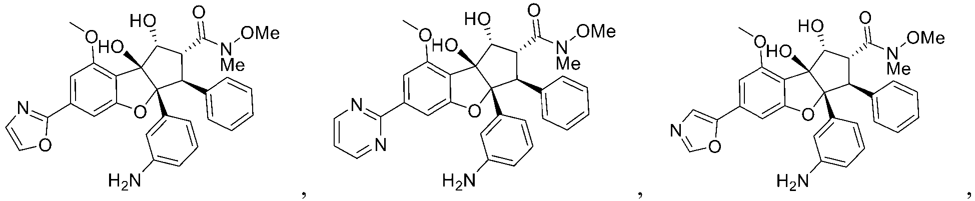

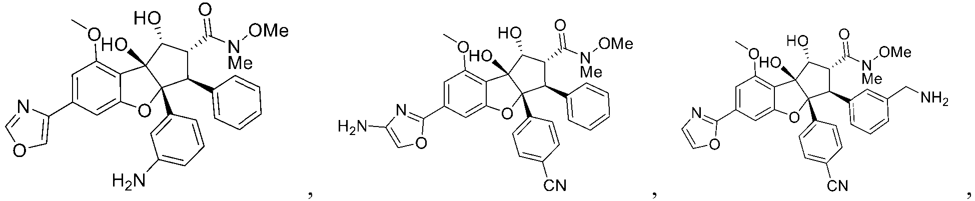

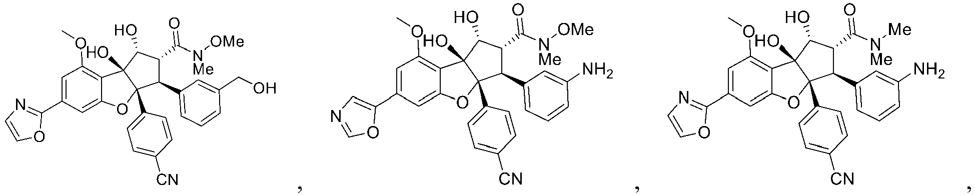

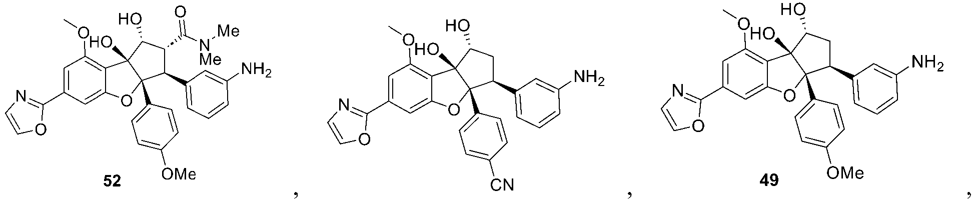

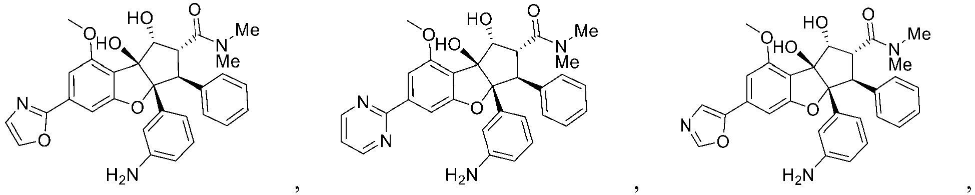

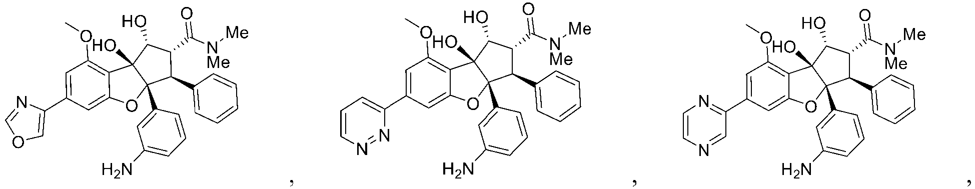

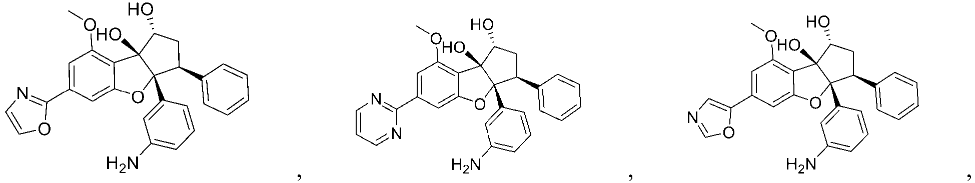

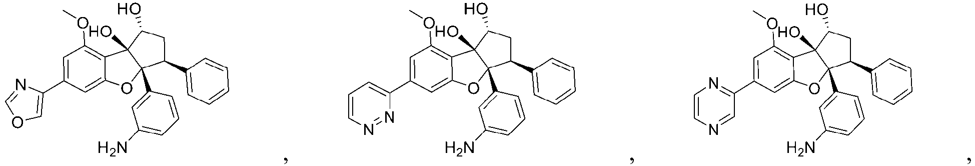

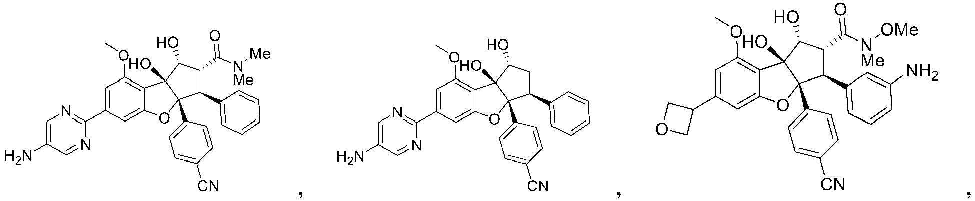

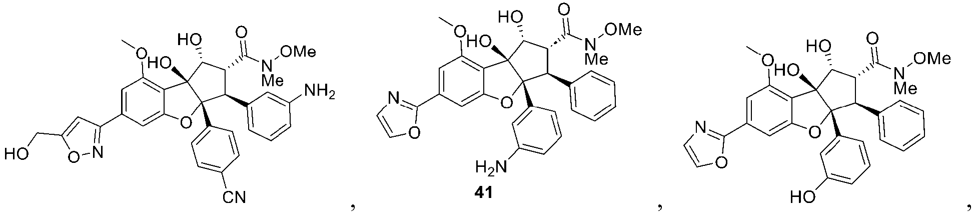

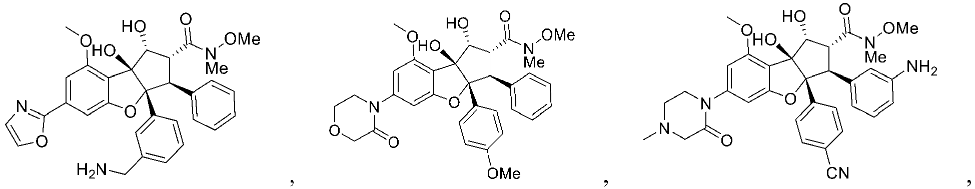

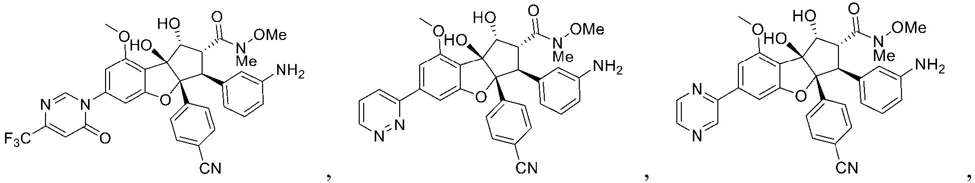

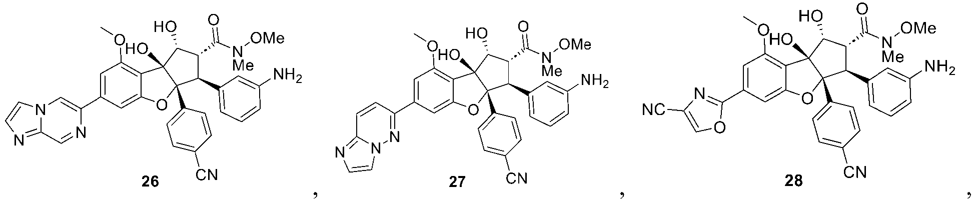

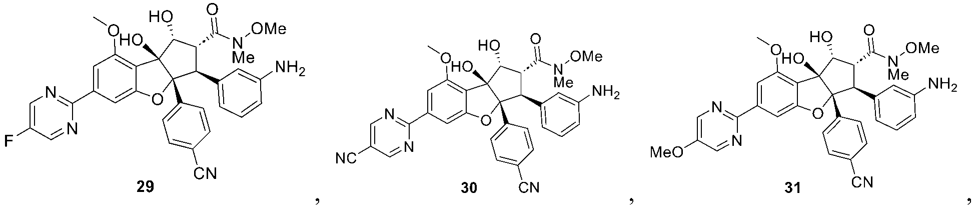

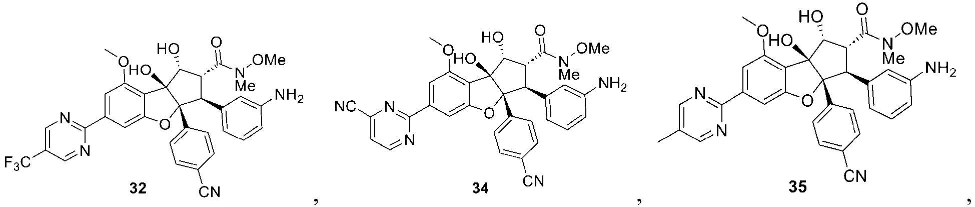

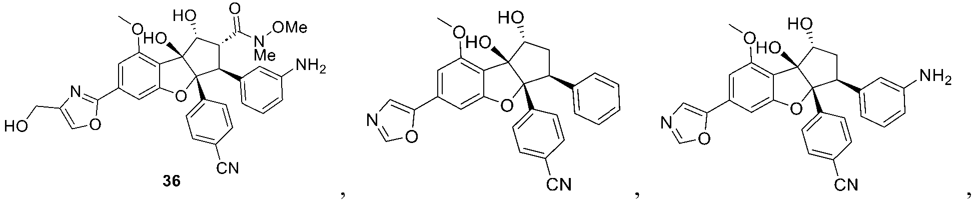

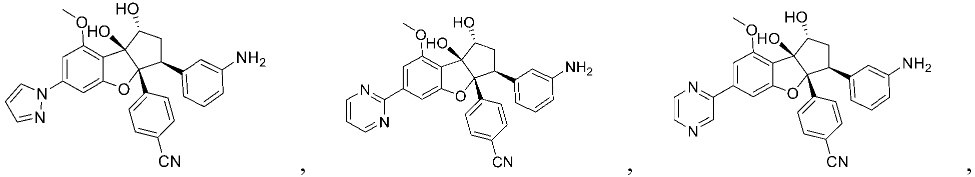

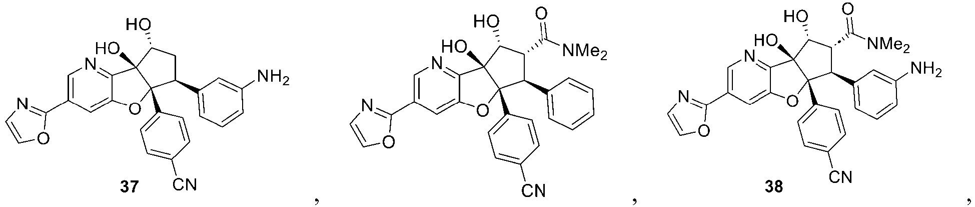

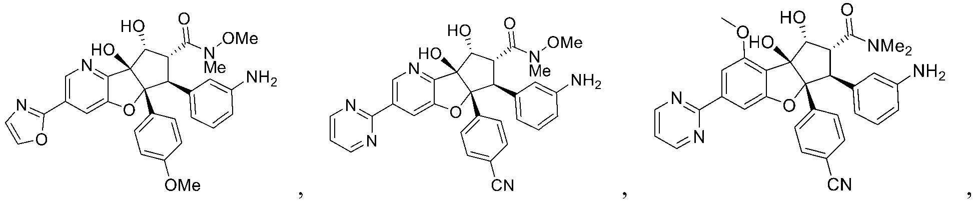

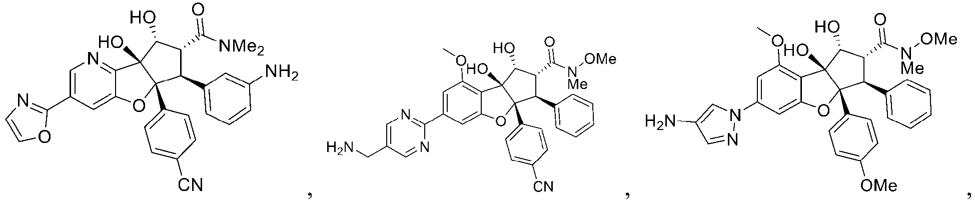

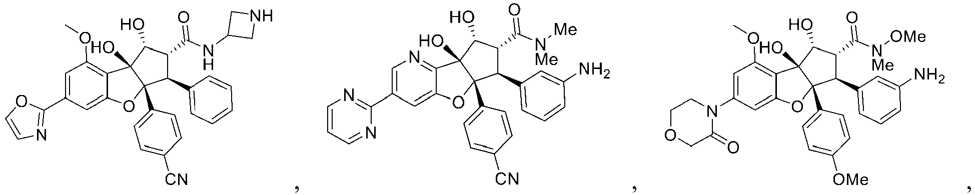

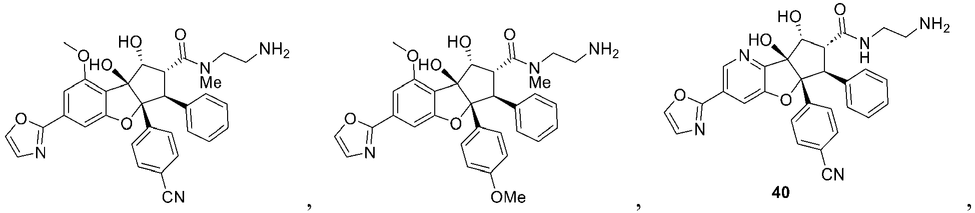

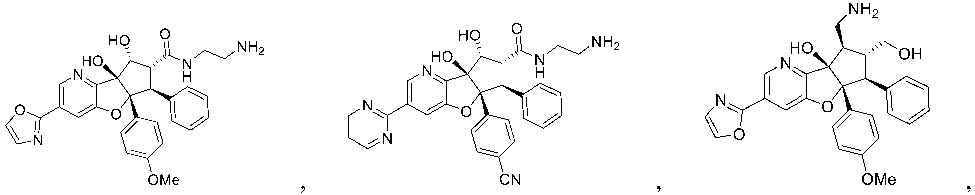

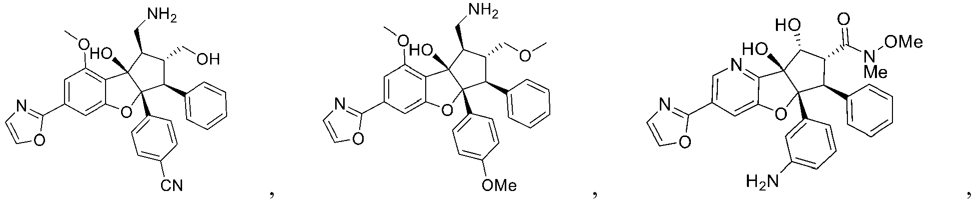

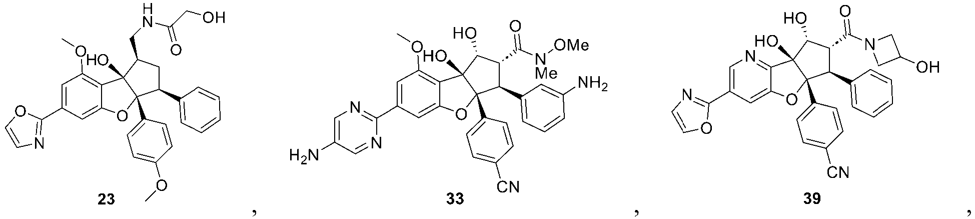

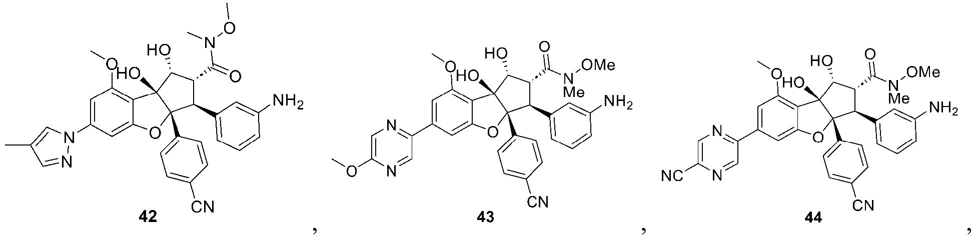

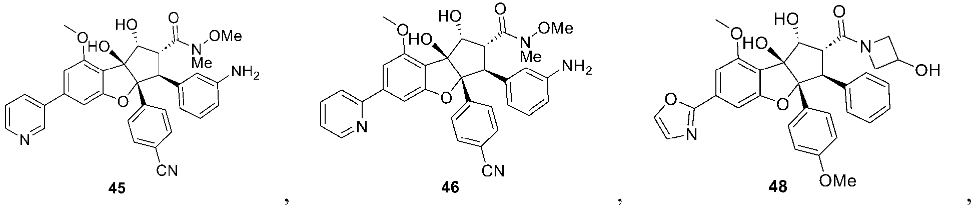

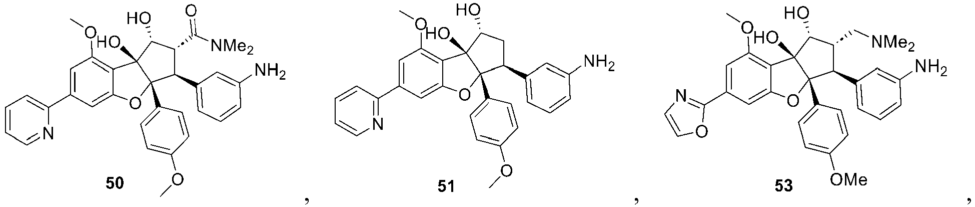

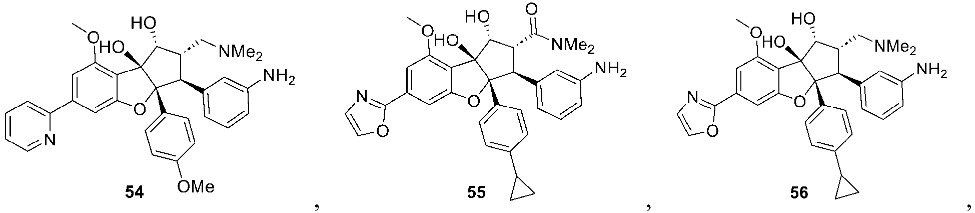

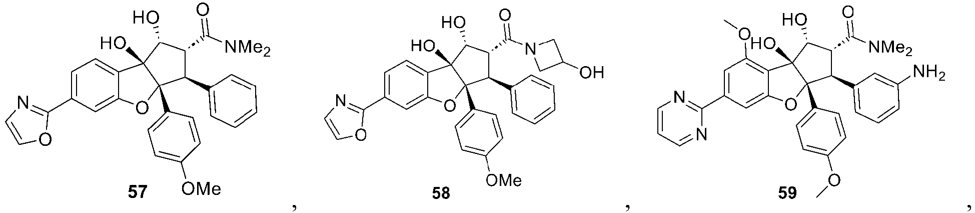

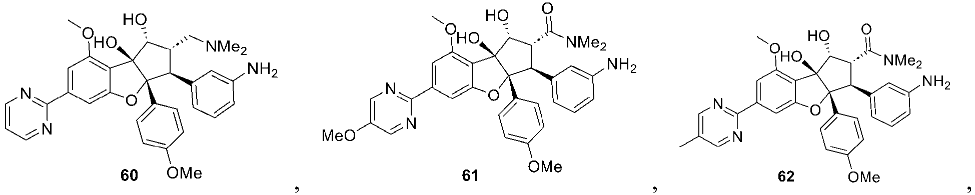

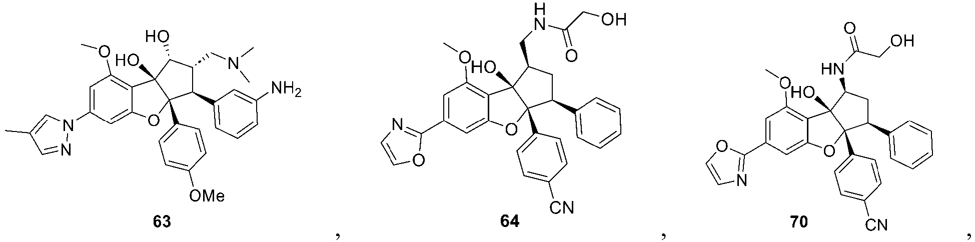

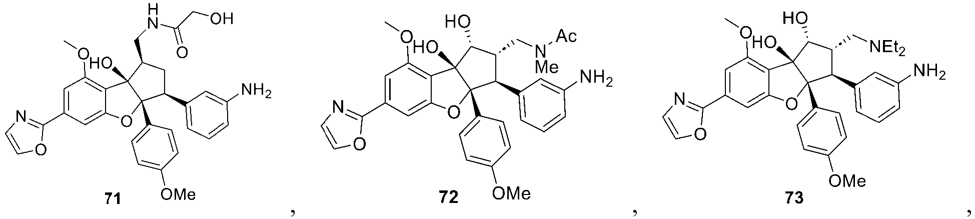

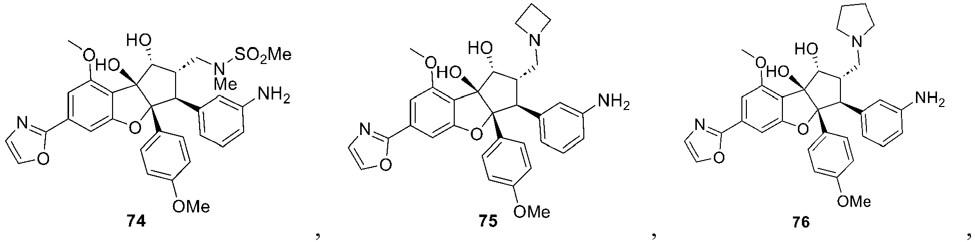

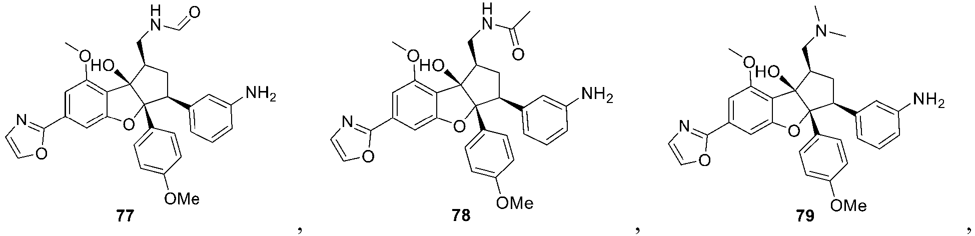

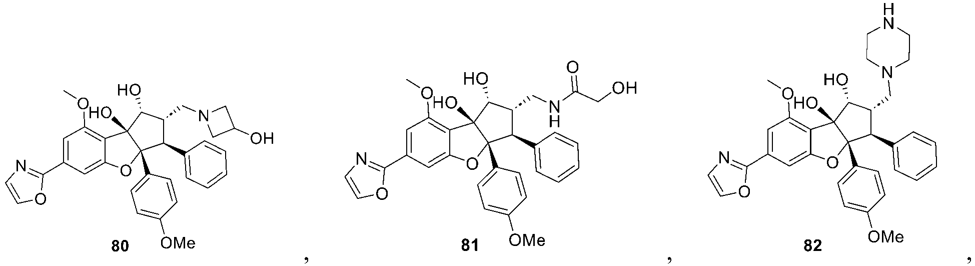

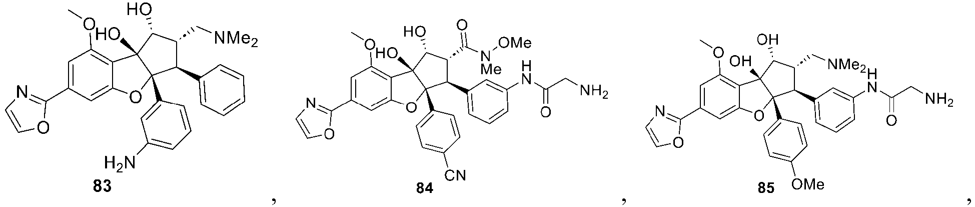

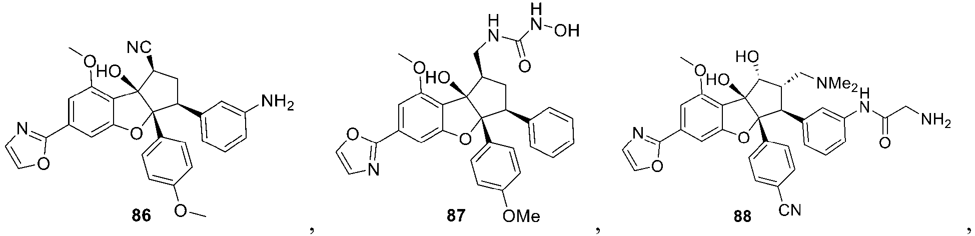

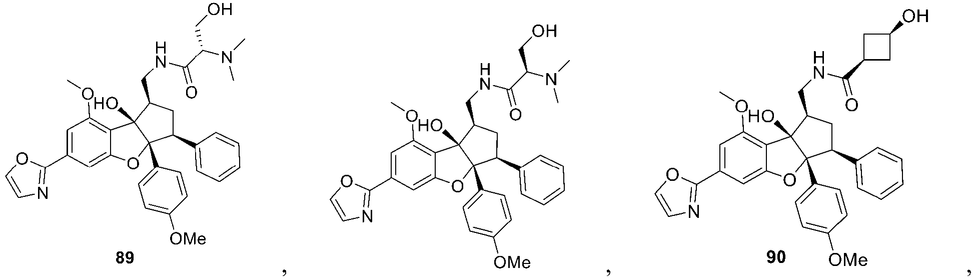

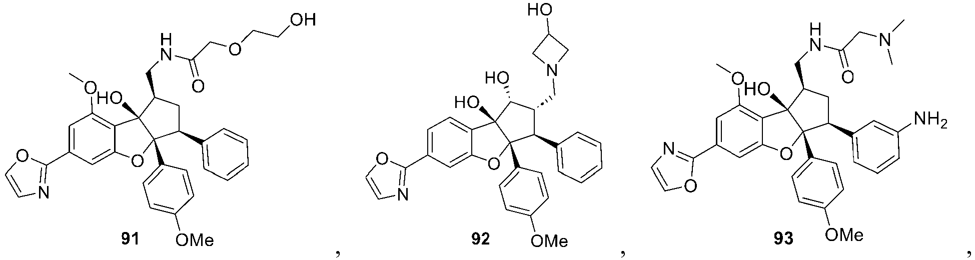

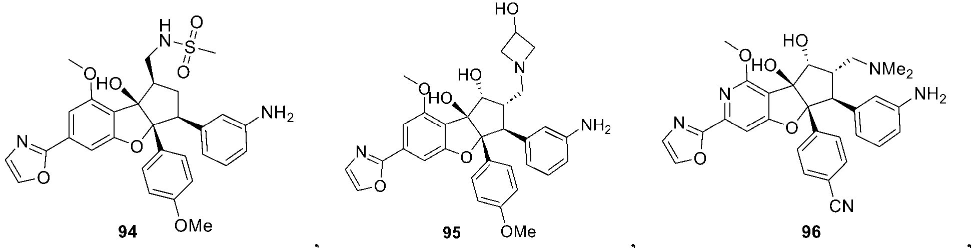

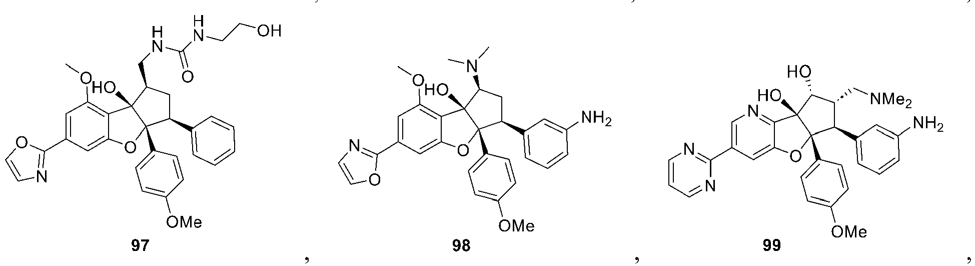

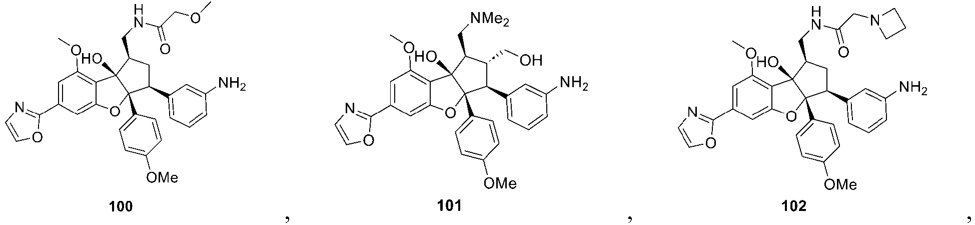

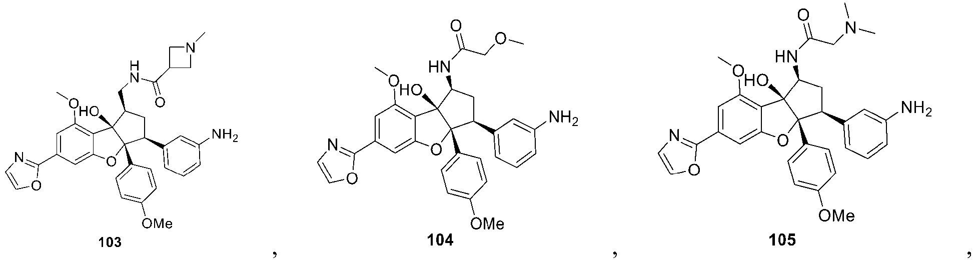

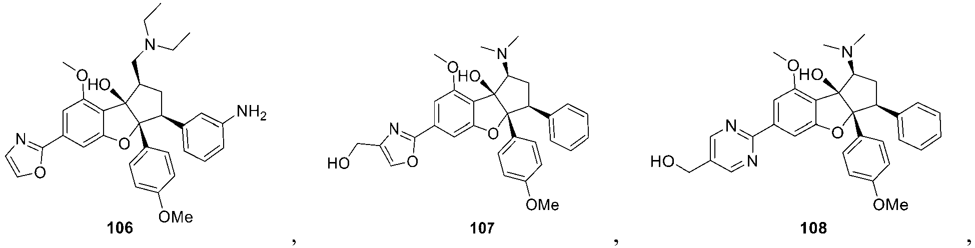

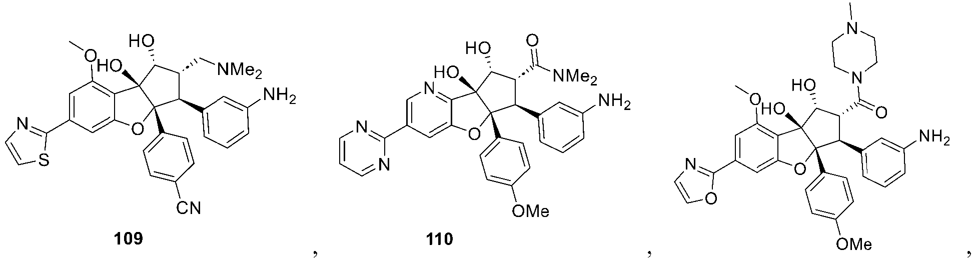

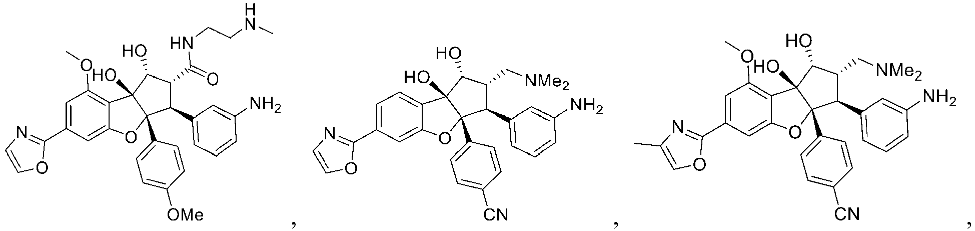

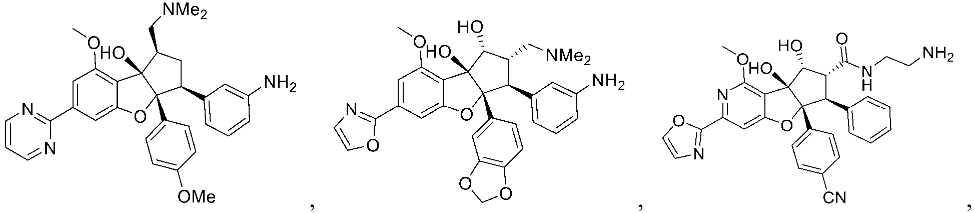

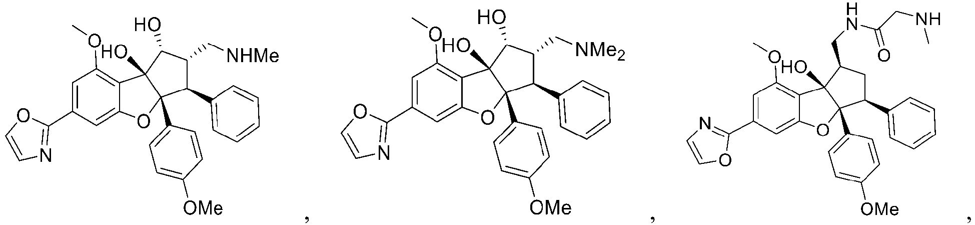

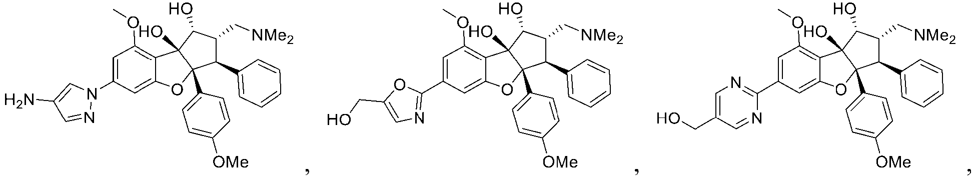

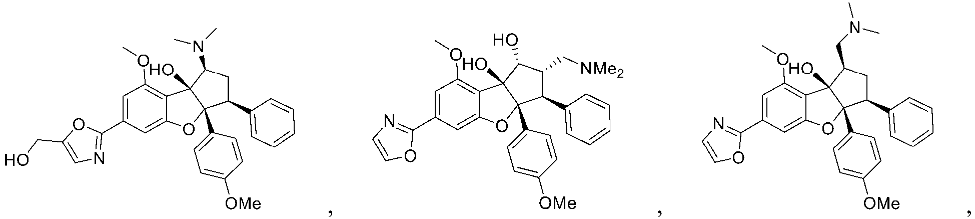

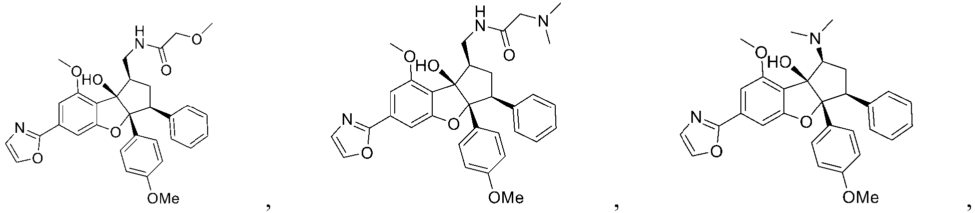

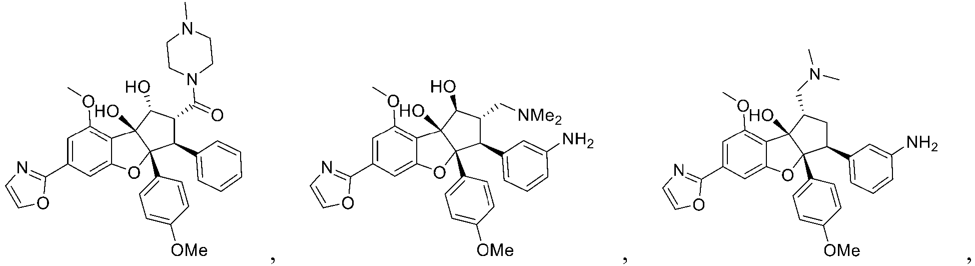

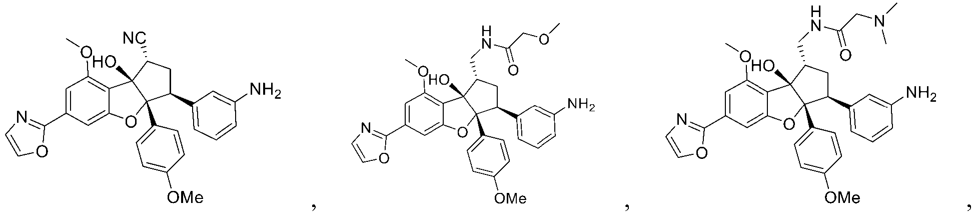

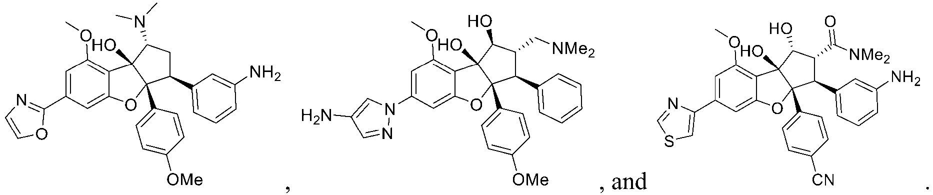

In certain embodiments, the compound is a racemate of the compound represented by formula I, formula I-1, formula I-1-A, formula I-1-B, formula I-1-AB-1, formula I-1-AB-1A, formula I-1-AB-1B, formula I-1-AB-1C, or formula I-1-AB-1D.

In certain embodiments, the compound is a mixture of the compound represented by formula I, formula I-1, formula I-1-A, formula I-1-B, formula I-1-AB-1, formula I-1-AB-1A, formula I-1-AB-1B, formula I-1-AB-1C, or formula I-1-AB-1D with an enantiomer thereof.

In certain embodiments, in the mixture of the compound represented by formula I, formula I-1, formula I-1-A, formula I-1-B, formula I-1-AB-1, formula I-1-AB-1A, formula I-1-AB-1B, formula I-1-AB-1C, or formula I-1-AB-1D with the enantiomer thereof, the content of the compound represented by formula I, formula I-1, formula I-1-A, formula I-1-B, formula I-1-AB-1, formula I-1-AB-1A, formula I-1-AB-1B, formula I-1-AB-1C, or formula I-1-AB-1D is ≥ 60%, or ≥ 70%, or ≥ 80%, or ≥ 85%, or ≥ 90%, or ≥ 91%, or ≥ 92%, or ≥ 93%, or ≥ 94%, or ≥ 95%, or ≥ 96%, or ≥ 97%, or ≥ 98%, or ≥ 99%.

In a second aspect of the present disclosure, provided is a pharmaceutical composition, comprising at least one of the compounds or the racemic mixtures, the enantiomers, the diastereoisomers, the pharmaceutically acceptable salts, or the isotopically labeled compounds thereof, or the mixtures of the aforementioned forms according to the first aspect of the present disclosure, and one or more pharmaceutically acceptable carriers and/or excipients. In certain embodiments, the compound or the racemic mixture, the enantiomer, the diastereoisomer, the pharmaceutically acceptable salt, or the isotopically labeled compound thereof, or the mixture of the aforementioned forms is present in the pharmaceutical composition in an effective amount, preferably in a therapeutically effective amount, or in a prophylactically effective amount.

In a third aspect of the present disclosure, provided is use of the compound or the racemic mixture, the enantiomer, the diastereoisomer, the pharmaceutically acceptable salt, or the isotopically labeled compound thereof, or the mixture of the aforementioned forms according to the first aspect of the present disclosure, or the pharmaceutical composition according to the second aspect of the present disclosure in the preparation of a medicament for treating and/or preventing a disease or disorder or reducing the severity of the disease or disorder, wherein the disease or disorder is a tumor or cancer.

In the third aspect of the present disclosure, also provided is the compound or the racemic mixture, the enantiomer, the diastereoisomer, the pharmaceutically acceptable salt, or the isotopically labeled compound thereof, or the mixture of the aforementioned forms according to the first aspect of the present disclosure, or the pharmaceutical composition according to the second aspect of the present disclosure for use in treating and/or preventing a disease or disorder or reducing the severity of the disease or disorder, wherein the disease or disorder is a tumor or cancer.

In the third aspect of the present disclosure, further provided is a method for treating and/or preventing a disease or disorder or reducing the severity of the disease or disorder, comprising administering to an individual in need an effective amount of the compound or the racemic mixture, the enantiomer, the diastereoisomer, the pharmaceutically acceptable salt, or the isotopically labeled compound thereof, or the mixture of the aforementioned forms according to the first aspect of the present disclosure, or the pharmaceutical composition according to the second aspect of the present disclosure, wherein the disease or disorder is a tumor or cancer.

In certain embodiments, the tumor or cancer is selected from leukemia, malignant lymphoma, multiple myeloma, gastric cancer, colorectal cancer, pancreatic cancer, colon cancer, breast cancer, ovarian cancer, liver cancer, and lung cancer.

In certain embodiments, the tumor or cancer is selected from leukemia, malignant lymphoma, multiple myeloma, gastric cancer, colorectal cancer, breast cancer, ovarian cancer, liver cancer, and lung cancer.

In certain embodiments, the tumor or cancer is selected from gastric cancer, colorectal cancer, breast cancer, ovarian cancer, and liver cancer.

In certain embodiments, the colorectal cancer is selected from colorectal adenocarcinoma and colon cancer.

In certain embodiments, the breast cancer is selected from ductal breast cancer.

### Definitions of terms

The various terms and phrases used in the present application have general meanings well known to those skilled in the art. Nevertheless, the present application still intends to provide a more detailed illustration and explanation of these terms and phrases herein. In the event of any discrepancy between the terms and phrases mentioned and their well-known meanings, the meanings expressed in the present disclosure shall prevail.

As used in the present application, the term "pharmaceutically acceptable salt" refers to a salt of the compound of the present disclosure that is pharmaceutically acceptable and possesses the desired pharmacological activity of the parent compound. Such salts include acid addition salts formed with inorganic or organic acids; or salts formed by the replacement of acidic protons present on the parent compound by metal ions; or coordination compounds formed with organic bases.

As used in the present application, the term "isotopically labeled compound" refers to a compound in which one or more atoms are replaced by an atom having the same atomic number but an atomic mass or mass number different from the atomic mass or mass number predominant in nature. Examples of isotopes suitable for incorporation into the compounds of the present disclosure include, but are not limited to, hydrogen isotopes such as ²H and ³H; carbon isotopes such as ¹¹C, ¹³C, and ¹⁴C; chlorine isotopes such as ³⁶Cl; fluorine isotopes such as ¹⁸F; iodine isotopes such as ¹²³I and ¹²⁵I; nitrogen isotopes such as ¹³N and ¹⁵N; oxygen isotopes such as ¹⁵O, ¹⁷O, and ¹⁸O; and sulfur isotopes such as ³⁵S.

As used in the present application, the term "racemic mixture" encompasses "racemate", and the term "racemate" refers to an equimolar mixture of an optically active chiral molecule and an enantiomer thereof; the term "enantiomer" refers to one of a pair of molecular entities that are mirror images of each other and are non-superimposable, and a mixture of enantiomers can be separated under, for example, chiral resolution conditions; the term "diastereoisomer" refers to a stereoisomer that is not related to mirror images; diastereoisomers are characterized by some differences in physicochemical properties, and a mixture of diastereoisomers can be separated under, for example, chromatographic or crystallization conditions.

As used in the present application, "optionally substituted with..." means that the group may be unsubstituted or substituted with a substituent. For example, -(C₁-C₆)alkyl optionally substituted with halogen means that the -(C₁-C₆)alkyl may be unsubstituted or substituted with halogen, resulting in haloalkyl. It should be understood that when a group consists of multiple moieties, if one of the moieties can be substituted, then that moiety in the group can also be substituted. For example, the expression "a group is selected from -(C₁-C₆)alkyl, -O-(C₁-C₆)alkyl, and -NH(C₁-C₆)alkyl, wherein the -(C₁-C₆)alkyl is optionally substituted with halogen" means that the -(C₁-C₆)alkyl, the (C₁-C₆)alkyl in the -O-(C₁-C₆)alkyl, and the (C₁-C₆)alkyl in the -NH(C₁-C₆)alkyl are all optionally substituted with halogen. For another example, "R is selected from methyl, -O-methyl, -NH-methyl, -S-methyl, -C(=O)-methyl, and -C(=O)NH-methyl, wherein the methyl is optionally substituted with 1 hydroxyl or amino" means that the methyl, as well as the methyl in the -O-methyl, -NH-methyl, -S-methyl, -C(=O)-methyl, and -C(=O)NH-methyl, are all optionally substituted with 1 hydroxyl or amino.

As used in the present application, the term "halogen" refers to fluorine, chlorine, bromine, or iodine.

As used in the present application, the term "alkyl" refers to a linear or branched monovalent saturated hydrocarbon group. For example, (C₁-C₆)alkyl refers to alkyl having 1 to 6 carbon atoms, such as 1, 2, 3, 4, 5, or 6 carbon atoms; (C₁-C₄)alkyl refers to alkyl having 1 to 4 carbon atoms, such as 1, 2, 3, or 4 carbon atoms. Non-limiting examples of alkyl include, but are not limited to, methyl, ethyl, propyl, propyl, butyl, etc.

As used in the present application, the term "alkylene" refers to a linear or branched divalent saturated hydrocarbon group. For example, (C₁-C₈)alkylene refers to alkylene having 1 to 8 carbon atoms, such as 1, 2, 3, 4, 5, 6, 7, or 8 carbon atoms; (C₁-C₄)alkylene refers to alkylene having 1 to 4 carbon atoms, such as 1, 2, 3, or 4 carbon atoms; (C₁-C₈)alkylene includes (C₁-C₇)alkylene, (C₁-C₆)alkylene, (C₁-C₅)alkylene, (C₁-C₄)alkylene, etc. Non-limiting examples of alkylene include, but are not limited to, methylene, ethylene, propylene, butylene, etc.

As used in the present application, the term "halo" means that the modified group is substituted with one or more halogens, for example, substituted with 1, 2, 3, 4, 5, or 6 halogens. For example, "(C₁-C₆)haloalkyl" refers to (C₁-C₆)alkyl as defined above substituted with one or more halogens; non-limiting examples include, but are not limited to, CF₃, CHF₂, CF₂CF₃, or the like.

As used in the present application, the term "alkenyl" refers to a linear or branched unsaturated hydrocarbon group containing at least one double bond. (C₂-C₆)alkenyl refers to alkenyl having 2 to 6 (e.g., 2, 3, 4, 5, or 6) carbon atoms, including (C₂-C₅)alkenyl, (C₂-C₄)alkenyl, (C₂-C₃)alkenyl, etc.; non-limiting examples include, but are not limited to, -CH=CH₂, -CH=CH-CH=CH₂, - CH=C(CH)₃-CH₃, or the like.

As used in the present application, the term "alkynyl" refers to a linear or branched unsaturated hydrocarbon group containing at least one triple bond. For example, (C₂-C₆)alkynyl refers to alkynyl having 2 to 6 (e.g., 2, 3, 4, 5, or 6) carbon atoms, including (C₂-C₅)alkynyl, (C₂-C₄)alkynyl, (C₂-C₃)alkynyl, etc.; non-limiting examples include, but are not limited to, ethynyl, propynyl, or the like.

As used in the present application, the term "cycloalkyl" refers to a monovalent saturated hydrocarbon group consisting of carbon atoms. For example, (C₃-C₆)cycloalkyl refers to cycloalkyl consisting of 3 to 6 (e.g., 3, 4, 5, or 6) carbon atoms. The cycloalkyl includes monocyclic, bicyclic, or polycyclic systems, including spiro, fused, or bridged rings. Non-limiting examples include, but are not limited to, cyclobutyl, cyclopentyl, cyclohexyl, or the like.

As used in the present application, the term "heterocyclyl" refers to a saturated or partially unsaturated monovalent cyclic group consisting of ring atoms, wherein 1, 2, 3, or 4 ring atoms are heteroatoms and the remainder are carbon atoms; preferably, the heteroatoms are selected from N, O, or S, wherein the nitrogen atom is optionally quaternized, and the nitrogen and sulfur heteroatoms may be optionally oxidized; preferably, the carbon atoms are optionally substituted with =O. For example, (4- to 10-membered)heterocyclyl refers to heterocyclyl consisting of 4, 5, 6, 7, 8, 9, or 10 ring atoms, and (4- to 10-membered)heterocyclyl includes (4- to 9-membered)heterocyclyl, (4- to 8-membered)heterocyclyl, (4- to 7-membered)heterocyclyl, (4- to 6-membered)heterocyclyl, 5-membered heterocyclyl, 6-membered heterocyclyl, etc. The heterocyclyl includes monocyclic, bicyclic, or polycyclic systems, including spiro, fused, or bridged rings. Non-limiting examples include, but are not limited to, oxetanyl, azetidinyl, pyrrolidinyl, piperidinyl, piperazinyl, morpholinyl, imidazolinyl, or the like.

As used in the present application, the term "aryl" refers to an unsaturated carbocyclic group with a conjugated π-electron system. For example, (C₆-C₁₀)aryl consists of 6 to 10 (e.g., 6, 7, 8, 9, or 10) carbon atoms. Non-limiting examples include, but are not limited to, phenyl, etc.

As used in the present application, the term "heteroaryl" refers to an unsaturated group with a conjugated π-electron system consisting of ring atoms, wherein 1, 2, 3, or 4 ring atoms are heteroatoms and the remainder are carbon atoms; preferably, the heteroatoms are selected from N, O, or S, wherein the nitrogen atom is optionally quaternized, and the nitrogen and sulfur heteroatoms may be optionally oxidized. For example, (5- to 12-membered)heteroaryl consists of 5 to 12 (e.g., 5, 6, 7, 8, 9, 10, 11, or 12) ring atoms, including 5- to 10-membered heteroaryl, 5- to 9-membered heteroaryl, 6- to 9-membered heteroaryl, 5- to 6-membered heteroaryl, etc. The heteroaryl includes monocyclic and polycyclic systems; non-limiting examples include, but are not limited to, imidazolyl, pyridinyl, or the like.

As used in the present application, the term "pharmaceutically acceptable carrier and/or excipient" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995). Pharmaceutically acceptable carriers and/or excipients include, but are not limited to: pH adjusters, surfactants, ionic strength enhancers, diluents, agents for maintaining osmotic pressure, agents for delaying absorption, preservatives, and stabilizers. For example, pH adjusters include, but are not limited to, phosphate buffers. Surfactants include, but are not limited to, cationic, anionic, or non-ionic surfactants, such as Tween-80. Ionic strength enhancers include, but are not limited to, sodium chloride. Preservatives include, but are not limited to, various antibacterial and antifungal agents, such as parabens, chlorobutanol, phenol, sorbic acid, etc. Agents for maintaining osmotic pressure include, but are not limited to, sugars, NaCl, and analogs thereof. Agents for delaying absorption include, but are not limited to, monostearate and gelatin. Diluents include, but are not limited to, water, aqueous buffers (e.g., buffered saline), alcohols and polyols (e.g., glycerol), etc. Preservatives include, but are not limited to, various antibacterial and antifungal agents, such as thimerosal, 2-phenoxyethanol, parabens, chlorobutanol, phenol, sorbic acid, etc. Stabilizers have the meaning generally understood by those skilled in the art, and they are capable of stabilizing the desired activity of the active ingredient in a drug, and include, but are not limited to, sodium glutamate, gelatin, SPGA, sugars (e.g., sorbitol, mannitol, starch, sucrose, lactose, dextran, or glucose), amino acids (e.g., glutamic acid or glycine), proteins (e.g., dried whey, albumin, or casein) or degradation products thereof (e.g., lactalbumin hydrolysate), etc.

As used in the present application, the term "treat", "treating", or "treatment" refers to the intent to alleviate, relieve, ameliorate, or eliminate a targeted disease state or condition. A subject is considered successfully "treated" if, after receiving a therapeutic amount of the ligand-drug conjugate or the racemic mixture, the enantiomer, the diastereoisomer, or the pharmaceutically acceptable salt thereof, or the mixture of the aforementioned forms according to the method described herein, the subject exhibits an observable and/or detectable reduction or improvement in one or more indications and symptoms. It should also be understood that the treatment of the disease state or disorder includes not only complete cure, but also the achievement of some biologically or medically relevant outcomes even if a complete cure is not attained.

As used in the present application, the term "prevent", "preventing", or "prevention" refers to the intent to avoid, reduce, block, or delay the occurrence of a disease or disease-related symptom that has not yet appeared prior to administration of the relevant drug. "Prevention" does not necessarily require completely blocking the onset of a disease or disease-related symptom. For example, if the administration of the relevant drug can reduce the subject's risk of developing a specific disease or disease-related symptom, or mitigate the severity of a later-appearing related symptom, it can also be considered as "preventing" the onset or development of the disease.

As used in the present application, the DMSO solvent used for dissolving compounds in nuclear magnetic resonance structure identification is deuterated DMSO, i.e., DMSO-*d₆*.

Beneficial effects:
The compounds provided in the present disclosure have inhibitory effects on protein translation, and can significantly inhibit cell proliferation *in vitro* and suppress tumor growth *in vivo.*

In an assay for inhibition of *in vitro* proliferation of tumor cells, the small molecule compounds of the present application exhibit significant inhibitory activity against the proliferation of BT474, DLD-1, NCI-N87, NUGC-4, SNU-423, MKN-45, SK-OV-3, Ls174T, and HCC1954.

The small molecule compounds of the present application have significant tumor inhibitory effects in a BALB/c nude mouse subcutaneous xenograft tumor model, such as an NCI-H716, BT474, DLD-1, NCI-N87, NUGC-4, SNU-423, MKN-45, SK-OV-3, Ls174T, or HCC1954 xenograft tumor model.

The small molecule compounds of the present application have significant tumor inhibitory effects in both *in vivo* and *in vitro* hematological tumor models, such as NCI-H929 and MM.1R hematological tumor models.

Furthermore, the compounds provided in the present disclosure have excellent pharmacokinetic properties.

### DETAILED DESCRIPTION

Embodiments of the present disclosure will be described in detail below with reference to examples, but those skilled in the art will appreciate that the following examples are provided solely to illustrate the present disclosure and should not be construed as limiting the scope of the present disclosure. Procedures without specified conditions in the examples were conducted under conventional conditions or conditions recommended by the manufacturers. All reagents or instruments without specified manufacturers used herein are conventional products that are commercially available.

**Table 1**

| Abbreviation | Meaning | Abbreviation | Meaning |
|---|---|---|---|
| DMF | N,N-Dimethylformamide | HOBT | 1-Hydroxybenzotriazole |
| DIEA | Diisopropylethylamine | LC-MS | Liquid chromatography-mass spectrometry |
| HPLC | High-performance liquid chromatography | TLC | Thin-layer chromatography |
| HATU | O-(7-Azabenzotriazol-1-yl)-N,N,N',N'-tetramethyluronium hexafluorophosphate | Na₂SO₄ | Anhydrous sodium sulfate |
| EA or EtOAc | Ethyl acetate | DMSO | Dimethyl sulfoxide |
| DIPEA | N,N-Diisopropylethylamine | Pd(dba)₃ | Tris(dibenzylideneacetone)dipal ladium |
| Boc₂O | Di-tert-butyl dicarbonate | THF | Tetrahydrofuran |
| MeCN | Acetonitrile | NaBH(OAc)₃ | Sodium triacetoxyborohydride |
| Pd/C | Palladium on carbon | NaOMe | Sodium methoxide |
| Cs₂CO₃ | Cesium carbonate | Pd(PPh₃)₄ | Tetrakis(triphenylphosphine)pal ladium |
| AcOH | Acetic acid | Zn(CN)₂ | Zinc cyanide |
| DPPF | 1,1'-Bis(diphenylphosphino)ferrocene | LiOH.H₂O | Lithium hydroxide monohydrate |
| NMP | N-Methyl-2-pyrrolidone | Pd(OH)₂/C | Palladium hydroxide on carbon |
| STAB | Sodium triacetoxyborohydride | PPh₃ | Triphenylphosphine |
| Pd(OAc)₂ | Palladium acetate | Pyridine.HF | Pyridine hydrofluoride |
| DIAD | Diisopropyl azodicarboxylate | FA | Formic acid |
| EDCI | 1-Ethyl-(3-dimethylaminopropyl)carbodiimide hydrochloride | TEA | Triethylamine |
| DMA | Dimethylamine | CHCl₃ | Trichloromethane, chloroform |
| TFE | Trifluoroethanol | t-BuXPhos Pd G3 | Methanesulfonato(2-di-tert-butylphosphino-2',4',6'-triisopropyl-1,1'-biphenyl)(2'-amino-1,1'-biphenyl-2-yl)palladium(II) |
| PE | Petroleum ether | DMAc | N,N-Dimethylacetamide |
| TIPS | Triisopropylsilyl | TMP | 2,4,6-Trimethylpyridine |
| HOAT | N-Hydroxy-7-azabenzotriazole | Pin₂B₂ | Bis(pinacolato)diboron |
| BH₃·DMS | Dimethyl sulfide borane | Cy₃P | Tricyclohexylphosphine |
| DIBAL-H | Diisobutylaluminum hydride | DMAP | 4-Dimethylaminopyridine |
| MsCl | Methanesulfonyl chloride | Na(AcO)₃BH | Sodium triacetoxyborohydride |

### General chiral column resolution method

The racemate compound of the present disclosure was dissolved in methanol and resolved on a preparative chiral chromatography column (see the table below) to give the product compound.

| | |
|---|---|
| Instrument | SFC-150 Mgm (Waters) or equivalent instrument |
| Preparative chiral chromatography columns | Daicel IC (25 × 250 mm, 10 µm), Daicel OJ (25 × 250 mm, 10 µm), Daicel AD (25 × 250 mm, 10 µm), or chromatography columns of other brands |
| Column temperature | 30 °C |
| Mobile phases | Mobile phase A: CO₂ |
| | Mobile phase B: MeOH or EtOH, with 0.2%-0.5% NH₃ (7 M in MeOH) added |
| Flow rate | 100 mL/min |
| Detection wavelength | 214 nm |

### Example 1

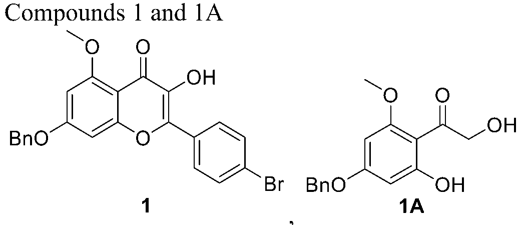

Compounds 1 and 1A were synthesized according to the literature: Liu, Tao; Nair, Somarajan J.; Lescarbeau, André; Belani, Jitendra; Peluso, Stéphane; Conley, James et al. (2012): Synthetic silvestrol analogues as potent and selective protein synthesis inhibitors. In Journal of medicinal chemistry 55 (20), pp. 8859-8878. DOI: 10.1021/jm3011542. See Supporting information, page 7, compound S2.

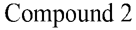

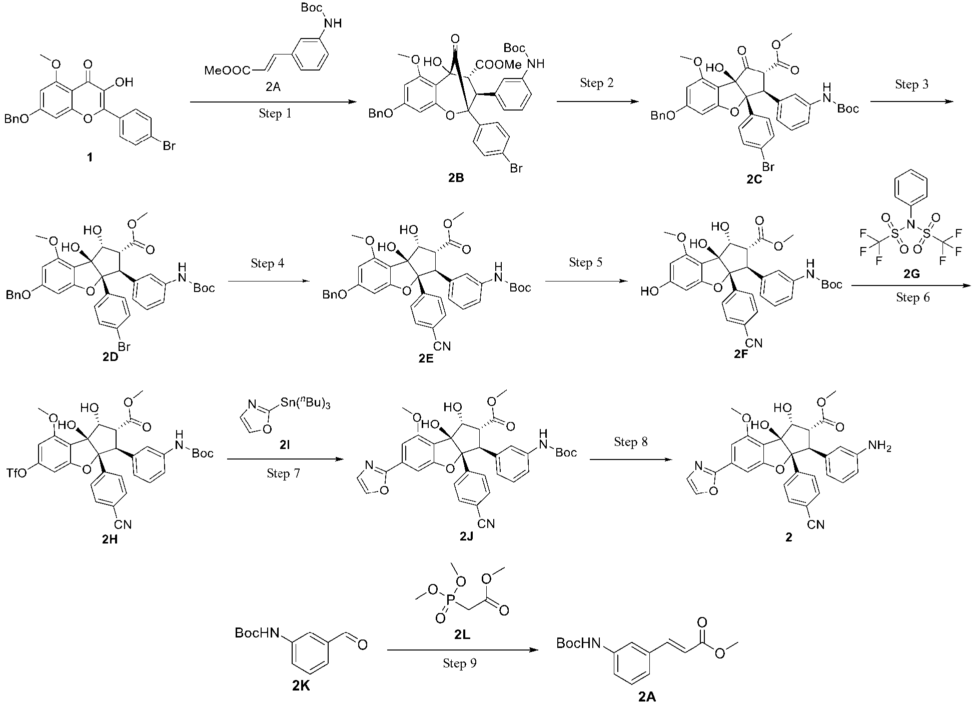

Step 1: Compound 1 (750 mg, 1.66 mmol) and compound 2A (2752 mg, 9.93 mmol) were dissolved in CHCl₃/TFE (7/3, 15 mL). Then, the reaction mixture was injected at a rate of 15 mL/h via a 20 mL syringe using a syringe pump into a coil wound around a jacketed glass cylinder equipped with a 250 W ultraviolet lamp. The temperature of the external circulation cold trap was adjusted to maintain the internal reaction temperature at 0-5 °C, and the UV lamp was turned on simultaneously. The reaction mixture was irradiated for 1 h, concentrated under reduced pressure to remove the solvent, and then separated by column chromatography (EA/PE = 1/3) to remove excess cinnamate, thus giving a racemate of the desired compound 2B (900 mg, yield: 75%) as a yellow oily crude product. LCMS [M+H+18]⁺ = 748.5.

Step 2: The racemate of compound 2B (5.2 g, 7.96 mmol) was mixed with MeOH (50 mL), and NaOMe (1.1 g, 19.91 mmol) was added at 0 °C. The mixture was stirred at 65 °C for 1.5 h. The solution was concentrated, sequentially washed with H₂O, an aqueous NH₄Cl solution, and saturated brine, dried over Na₂SO₄, concentrated under reduced pressure, and purified on a silica gel column (0.2% FA, EA/PE = 1/3) to give a racemate of compound 2C (3.8 g, yield: 65%). LCMS [M+H-18]⁺ = 714.4.

Step 3: The racemate of compound 2C (3.8 g, 5.21 mmol) was mixed with MeCN/CHCl₃ (1/1, 100 mL/100 mL), and NaBH(OAc)₃ (5.5 g, 26.03 mmol) and AcOH (3.1 g, 52.05 mmol) were added at 0 °C. The mixture was stirred at room temperature for 2 h. The reaction solution was extracted with EA. The organic phase was sequentially washed with water and saturated brine, dried over anhydrous Na₂SO₄, and purified on a silica gel column (PE:EA = 1/1) to give a racemate of compound 2D (1.9 g, yield: 50%). LCMS [M+H]⁺ = 716.5.

Step 4: The racemate of compound 2D (1.3 g, 1.78 mmol), Pd₂(dba)₃ (325.0 mg, 0.36 mmol), DPPF (393.6 mg, 0.71 mmol), and Zn(CN)₂ (519.5 mg, 4.44 mmol) in a solution of NMP (20 mL) was heated to 150 °C and maintained at this temperature for 2 h. The solution was diluted with water, extracted with EA, sequentially washed with water and brine, dried over anhydrous Na₂SO₄, and purified on a silica gel column (EA/PE = 1/1) to give a racemate of compound 2E (1.1 g, yield: 91%).

¹H NMR (400 MHz, CDCl₃) δ 7.48 - 7.40 (m, 6H), 7.37 (dt, J = 12.4, 5.3 Hz, 3H), 6.96 (t, J = 7.9 Hz, 1H), 6.85 (s, 1H), 6.51 (d, J = 7.7 Hz, 1H), 6.45 (s, 1H), 6.36 (d, J = 1.9 Hz, 1H), 6.22 (d, J = 1.9 Hz, 1H), 5.09 (d, J = 1.7 Hz, 2H), 5.01 (d, J = 6.4 Hz, 1H), 4.35 (d, J = 14.2 Hz, 1H), 3.95 - 3.81 (m, 4H), 3.66 (d, J = 3.8 Hz, 3H), 1.53 - 1.47 (m, 9H). LCMS [M+H-18]⁺ = 605.4.

Step 5: Pd(OH)₂/C (10%, 250 mg) was added to a solution of the racemate of compound 2E (500.0 mg, 0.74 mol) in EA/MeOH/THF/DCM (20:2:2:0.2, 40 mL). The mixture was purged with hydrogen three times and then stirred at room temperature for 16 h. The suspension was filtered, and the filter cake was washed with MeOH/DCM (1/10). The filtrate was concentrated and purified on a silica gel column (DCM/MeOH = 10/1) to give a racemate of compound 2F (300 mg, yield: 69%).

¹H NMR (400 MHz, CDCl₃) δ 7.42 (d, J = 8.6 Hz, 2H), 7.33 (d, J = 8.6 Hz, 2H), 6.98 (dd, J = 16.1, 8.2 Hz, 1H), 6.87 (s, 1H), 6.51 (d, J = 7.7 Hz, 1H), 6.33 (s, 1H), 6.24 (d, J = 1.8 Hz, 1H), 6.12 (d, J = 1.7 Hz, 1H), 4.98 (d, J = 6.3 Hz, 1H), 4.35 (d, J = 14.2 Hz, 1H), 3.90 (dd, J = 14.1, 6.4 Hz, 1H), 3.83 (d, J = 15.4 Hz, 3H), 3.67 (d, J = 4.5 Hz, 3H), 1.50 (d, J = 4.8 Hz, 9H). LCMS [M+H]⁺ = 515.4.

Step 6: The racemate of compound 2F (306.0 mg, 0.52 mmol) and K₂CO₃ (143.6 mg, 1.04 mmol) were stirred in DMF (10 mL). Compound 2G (185.8 mg, 0.52 mmol) was added. The reaction solution was stirred at 60 °C for 20 min, then diluted with water, extracted with EA, sequentially washed with water and brine, dried over anhydrous Na₂SO₄, and purified on a silica gel column (EA/PE = 1/1) to give a racemate of compound 2H (300 mg, yield: 80%).

¹H NMR (400 MHz, CDCl₃) δ 7.41 (d, J = 8.4 Hz, 2H), 7.33 (d, J = 8.5 Hz, 2H), 7.00 - 6.92 (m, 2H), 6.69 (d, J = 1.7 Hz, 1H), 6.53 (d, J = 7.6 Hz, 1H), 6.45 (d, J = 1.7 Hz, 1H), 6.39 (s, 1H), 4.99 (d, J = 5.9 Hz, 1H), 4.39 (d, J = 14.2 Hz, 1H), 3.99 - 3.91 (m, 4H), 3.68 (s, 3H), 1.50 (s, 9H). LCMS [M-H]⁺ = 719.2.

Step 7: A solution of the racemate of compound 2H (30 mg, 0.042 mmol) was added to DMF (3 mL), and then compound 2I (60 mg, 0.167 mmol), Pd(PPh₃)₄ (4.8 mg, 0.0042 mmol), and CuI (1.6 mg, 0.0083 mmol) were added under a nitrogen atmosphere. The mixture was stirred at 100 °C for 1 h. The suspension was filtered and then purified on a silica gel column (DCM/MeOH = 10/1) to give a racemate of compound 2J (15 mg, yield: 56%). LCMS [M+H]⁺ = 640.3.

Step 8: THF/6 M HCl (1/1, 4 mL) was added to a solution of the racemate of compound 2J (15 mg, 0.02 mmol), and the mixture was stirred at room temperature for 1 h. The solution was concentrated and purified by silica gel chromatography (DCM/MeOH) to give a racemate of 2 (4.5 mg, yield: 35%) as a white solid, which was further resolved on a chiral column to give compound 2.

LCMS [M+H]⁺ = 540.1.

¹H NMR (400 MHz, DMSO) δ 8.25 (d, *J =* 0.7 Hz, 1H), 7.50 (d, *J =* 8.6 Hz, 2H), 7.41 (d, *J =* 0.7 Hz, 1H), 7.31 (d, *J =* 8.6 Hz, 2H), 7.19 (d, *J =* 1.1 Hz, 1H), 7.14 (d, *J =* 1.0 Hz, 1H), 6.67 (t, *J =* 7.7 Hz, 1H), 6.26 (s, 1H), 6.19 - 6.10 (m, 2H), 5.63 (s, 1H), 5.45 *(d, J=* 5.4 Hz, 1H), 4.83 (s, 2H), 4.66 (t, *J =* 5.1 Hz, 1H), 4.27 (d, *J =* 14.0 Hz, 1H), 3.97 (dd, *J =* 13.9, 4.8 Hz, 1H), 3.83 (s, 3H), 3.58 (s, 3H).

Step 9: 60% NaH (21.87 g, 546.88 mmol) was added to THF (2.5 L) at 0 °C, and the mixture was stirred at 0 °C for 1 h. Then, compound 2L (99.59 g, 546.88 mmol) was added dropwise, and the mixture was stirred at 0 °C for 2 h. A solution of compound 2K (100.00 g, 451.97 mmol) in THF (300 mL) was added dropwise.The mixture was stirred at room temperature for 3 h. Then, 10% HCl was added, and the mixture was extracted with ethyl acetate. The organic phases were combined, dried over Na₂SO₄, filtered, and concentrated to give a crude product, which was then slurried with MTBE to give 2A (120 g, yield: 95.73%) as a pale yellow solid.

¹H NMR (400 MHz, DMSO) δ 9.47 (s, 1H), 7.74 (s, 1H), 7.58 (d, J = 16.0 Hz, 1H), 7.49 (d, *J =* 7.2 Hz, 1H), 7.36 - 7.28 (m, 2H), 6.49 (d, *J =* 16.0 Hz, 1H), 3.73 (s, 3H), 1.49 (s, 9H).

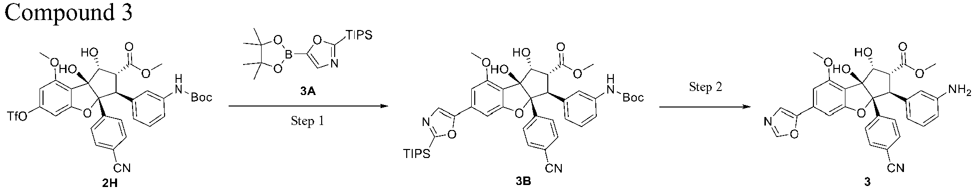

Step 1: Na₂CO₃ (48.8 mg, 0.14 mL), Pd(PPh₃)₄ (4.88 mg, 0.01 mmol), and compound 3A (28.3 mg, 0.08 mmol) were added to a solution of the racemate of compound 2H (50 mg, 0.07 mmol) in dioxane/water (2 mL/0.5 mL), and the mixture was allowed to react at 80 °C for 2 h under a nitrogen atmosphere, then filtered, and concentrated under reduced pressure. The crude product was purified by flash column chromatography (DCM/EA = 1/1) to give a racemate of compound 3B (40 mg, yield: 76%) as a light yellow solid. LCMS: [M+H]⁺ = 796.4.

Step 2: The racemate of compound 3B (20 mg, 0.025 mmol) was dissolved in 6 M HCl (1 mL)/THF (0.5 mL), and the solution was stirred for 3 h. The reaction solution was purified by preparative HPLC (ACN/H₂O from 0 to 100%) to give a racemate of 3 (6 mg, yield: 45%) as a white solid, which was further resolved on a chiral column to give compound 3.

LCMS: [M+H]⁺ = 540.2.

¹H NMR (400 MHz, DMSO) δ 8.46 (s, 1H), 7.79 (s, 1H), 7.51 (d, *J* = 8.4 Hz, 2H), 7.32 (d, *J* = 8.4 Hz, 2H), 7.03 - 6.89 (m, 3H), 6.68 (s, 3H), 4.68 (d, *J =* 4.8 Hz, 1H), 4.34 (d, *J =* 13.6 Hz, 1H), 4.09 (dd, *J =* 14.0, 4.8 Hz, 1H), 3.83 (s, 3H), 3.59 (s, 3H).

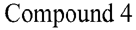

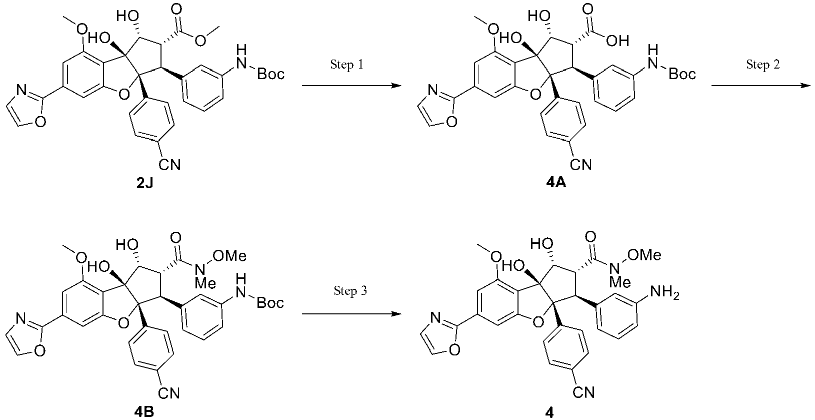

Step 1: A solution of the racemate of compound 2J (150 mg, 0.23 mmol) and LiOH (25 mg, 0.59 mmol) in THF/H₂O/MeOH (10 mL) was stirred at room temperature for 3 h, then adjusted to pH = 5 with 3 M hydrochloric acid, and concentrated and purified on a silica gel column (DCM/MeOH) to give a racemate of 4A (130 mg, yield: 88%) as a white solid. LCMS [M+1]⁺ = 626.1.

Step 2: HATU (61 mg, 0.16 mmol) and DIEA (31 mg, 0.24 mmol) were added to a solution of the racemate of compound 4A (50 mg, 0.08 mmol) in DMF (3 mL), and N,O-dimethylhydroxylamine (16 mg, 0.16 mmol) was added under a N₂ atmosphere. The mixture was stirred at room temperature for 3 h and then concentrated and purified on silica gel (DCM/MeOH) to give a racemate of 4B (20 mg, yield: 38%) as a white solid. LCMS [M+1]⁺ = 669.2.

Step 3: The racemate of compound 4B (12 mg, 0.02 mmol) was dissolved in a solution of THF/6 M HCl (1/1, 4 mL). The resulting solution was stirred at room temperature for 1 h and then concentrated and purified on silica gel (DCM/MeOH) to give a racemate of 4 (2.2 mg, yield: 22%) as a white solid, which was further resolved on a chiral column to give 4 as a white solid. LCMS [M+H]⁺ = 569.1.

1H NMR (400 MHz, DMSO) δ 8.25 (s, 1H), 7.53 (d, J = 8.5 Hz, 2H), 7.41 (s, 1H), 7.31 (d, J = 8.5 Hz, 2H), 7.20 (d, J = 0.9 Hz, 1H), 7.15 (s, 1H), 6.66 (t, J = 7.7 Hz, 1H), 6.21 (s, 1H), 6.16 (d, J = 7.9 Hz, 1H), 6.06 (d, J = 7.5 Hz, 1H), 5.61 (s, 1H), 5.07 (d, J = 4.8 Hz, 1H), 4.83 (s, 2H), 4.78 (t, J = 4.8 Hz, 1H), 4.32 (d, J = 13.9 Hz, 1H), 4.16 (s, 1H), 3.90 (s, 3H), 3.84 (s, 3H), 3.09 (s, 3H).

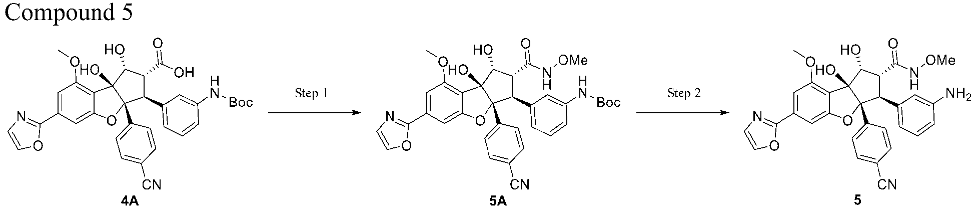

Step 1: HATU (36.4 mg, 0.1 mmol) and DIEA (18.6 mg, 0.14 mmol) were mixed with a solution of the racemate of compound 4A (30 mg, 0.05 mmol) in DMF (3 mL) under N₂, and then O-methylhydroxylamine (8 mg, 0.10 mmol) was added. The mixture was stirred at room temperature for 3 h, then concentrated, and purified by silica gel chromatography (DCM/MeOH) to give a racemate of 5A (20 mg, yield: 65%) as a white solid. LCMS [M+1]⁺ = 655.2.

Step 2: The racemate of compound 5A (12 mg, 0.03 mmol) was dissolved in a solution of THF/6 M HCl (1/1, 4 mL). The resulting solution was stirred at room temperature for 1 h and then concentrated and purified by silica gel chromatography (DCM/MeOH) to give a racemate of 5 (2.6 mg, yield: 31%) as a white solid, which was further resolved on a chiral column to give compound 5.

LCMS [M+H]⁺ = 555.1.

¹H NMR (400 MHz, DMSO) δ 11.27 (s, 1H), 8.26 (s, 1H), 7.54 (d, J = 8.5 Hz, 2H), 7.42 (s, 1H), 7.30 (d, J = 8.3 Hz, 2H), 7.20 (s, 1H), 7.16 (s, 1H), 7.11 (t, J = 7.5 Hz, 1H), 6.87 (d, J = 18.2 Hz, 3H), 5.65 (s, 1H), 5.27 (s, 1H), 4.60 (d, J = 4.4 Hz, 1H), 4.46 (d, J = 13.9 Hz, 1H), 3.84 (s, 3H), 3.74 (dd, J = 14.1, 4.1 Hz, 1H), 3.54 (s, 3H).

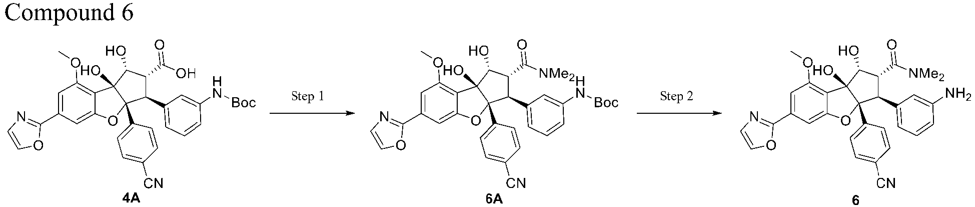

Step 1: The racemate of compound 4A (30.0 mg, 0.05 mmol), HATU (36.5 mg, 0.10 mmol), and DIEA (18.6 mg, 0.14 mmol) were stirred in DMF (3 mL) for 5 min. Dimethylamine hydrochloride (7.8 mg, 0.10 mmol) was added to the mixture, and the resulting mixture was stirred for 10 min. The solution was extracted with EA twice. The organic layer was washed with saturated brine, dried over Na₂SO₄, concentrated under reduced pressure, and purified on a silica gel column (DCM/MeOH = 10/1) to give a racemate of compound 6A (16.0 mg, yield: 51%). LCMS [M+H]⁺ = 653.2.

Step 2: The racemate of compound 6A (16.0 mg, 0.02 mmol) was mixed with THF (0.5 mL), and 6 M HCl (0.5 mL) was added at 0 °C. The mixture was stirred at room temperature for 4 h. The reaction solution was concentrated and purified by preparative high-performance liquid chromatography to give a racemate of 6 (5.9 mg, yield: 46%) as a white solid, which was further resolved on a chiral column to give compound 6.

LCMS [M+H]⁺ = 553.2.

¹H NMR (400 MHz, DMSO) δ 8.25 (s, 1H), 7.51 (d, J = 8.4 Hz, 2H), 7.41 (s, 1H), 7.35 (d, J = 8.4 Hz, 2H), 7.18 (d, J = 19.6 Hz, 2H), 6.73 (d, J = 7.4 Hz, 1H), 6.40 - 6.17 (m, 3H), 5.62 (s, 1H), 4.98 (s, 1H), 4.73 (s, 1H), 4.39 (d, J = 13.4 Hz, 1H), 4.15 (dd, J = 13.3, 4.9 Hz, 1H), 3.84 (s, 3H), 3.29 (s, 3H), 2.79 (s, 3H).

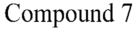

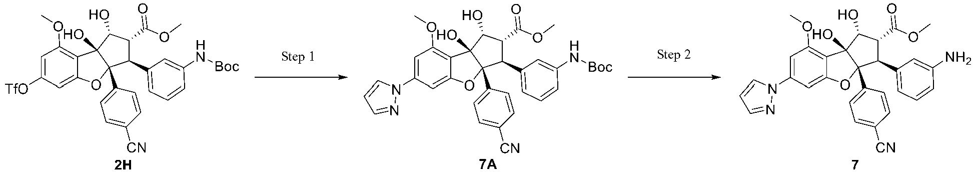

Step 1: The racemate of compound 2H (50 mg, 2.2 mmol), pyrazole (3.7 g, 13.3 mmol), tBuXPhos-Pd-G3 (4.8 mg, 0.01 mmol), tBuXPhos (12.78 mg, 0.03 mmol), and Cs₂CO₃ (67 mg, 0.21 mmol) were dissolved in 1,4-dioxane (2 mL). The reaction mixture was stirred at 100 °C for 16 h under a nitrogen atmosphere. After the reaction was completed, the reaction solution was quenched with water and extracted with EA, followed by liquid separation. The organic phase was concentrated and then purified on a silica gel column (DCM/EA = 1/1) to give a racemate of 7A (10 mg, yield: 20%) as a pale yellow solid. LCMS [M+H]⁺ = 639.2.

Step 2: The racemate of compound 7A (10 mg, 0.016 mmol) was dissolved in THF (0.5 mL), and 6 M HCl (0.5 mL) was added. The mixture was stirred at room temperature for 4 h. The reaction solution was purified by flash column chromatography (ACN-H₂O = 0-100%) to give a racemate of 7 (4 mg, yield: 47%) as a white solid, which was further resolved on a chiral column to give compound 7.

LCMS [M+H]⁺ = 539.1.

1H NMR (400 MHz, DMSO) δ 8.58 (d, J = 2.4 Hz, 1H), 7.75 (d, J = 1.6 Hz, 1H), 7.51 (d, J = 8.4 Hz, 2H), 7.31 (d, J = 8.4 Hz, 2H), 7.13 (d, J = 1.6 Hz, 1H), 7.05 (d, J = 1.6 Hz, 1H), 6.68 (t, J = 7.6 Hz, 1H), 6.58 - 6.54 (m, 1H), 6.23 (s, 1H), 6.14 (m, 2H), 5.54 (s, 1H), 5.39 (d, J *=* 5.2 Hz, 1H), 4.82 (s, 2H), 4.64 (t, J = 5.2 Hz, 1H), 4.26 (d, J = 14.0 Hz, 1H), 3.95 (m, 1H), 3.82 (s, 3H), 3.58 (s, 3H).

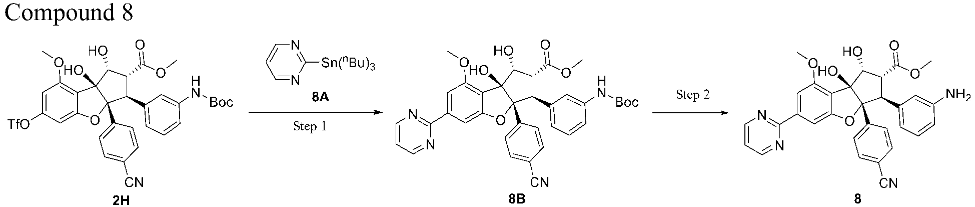

Step 1: Pd(PPh₃)₄ (9 mg, 0.0075 mmol) and CuI (28.5 mg, 0.015 mmol) were added to a solution of the racemate of compound 2H (54 mg, 0.075 mmol) and compound 8A (215 mg, 0.6 mmol) in DMF (5 mL). Then, the mixture was purged with nitrogen for 2 min and allowed to react under microwave at 120 °C for 2 h. The reaction solution was concentrated, and the resulting crude product was purified on a preparative chromatography column to give a racemate of 8B (20 mg, 40.9%) as a white solid.

LCMS: [M+H-C₄H₈]⁺ = 595.2.

Step 2: An aqueous HCl solution (0.5 mL, 6 M) was added to a solution of the racemate of compound 8B (20 mg, 0.0308 mmol) in THF (1 mL). The reaction solution was allowed to react at room temperature for 3 h and then concentrated, and the resulting crude product was purified by preparative HPLC to give a racemate of 8 (9 mg, yield: 53.1%) as a white solid, which was further resolved on a chiral column to give compound 8.

LCMS: [M+H]⁺ = 551.2.

¹H NMR (400 MHz, DMSO) δ 9.53 (s, 2H), 8.94 (d, J = 4.9 Hz, 2H), 7.64 (d, J = 2.8 Hz, 2H), 7.55 - 7.43 (m, 3H), 7.36 (d, J = 8.6 Hz, 2H), 7.14 (t, J = 7.6 Hz, 1H), 7.06 - 6.82 (m, 3H), 5.66 (d, J = 58.4 Hz, 2H), 4.74 (d, J = 4.6 Hz, 1H), 4.41 (d, J = 13.8 Hz, 1H), 4.18 (d, J = 4.8 Hz, 1H), 3.86 (s, 3H), 3.60 (s, 3H).

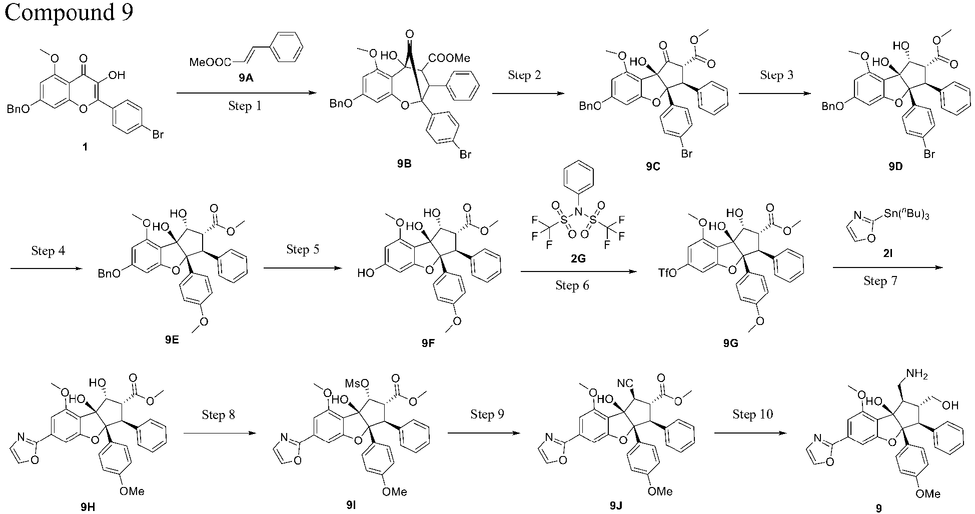

Step 1: Compound 1 (1.00 g, 2.21 mmol) and compound 9A (3.58 g, 22.08 mmol) were dissolved in a solution of TFE/trichloromethane (CHCl₃/TFE = 9/1, 18 mL). Then, the resulting solution was drawn into a 20 mL syringe mounted on a syringe pump, and the syringe pump was connected to an FEP tube. The FEP tube was sequentially wound around two quartz column sleeves, with a 250 W ultraviolet lamp tube in each quartz column sleeve. The temperature of the two column sleeves was cooled to -5 °C using a low-temperature circulation pump, and a room-temperature water bath was applied externally to the entire lamp-column sleeve system. The ultraviolet lamps were stably activated, and then the syringe pump was started to inject the reaction solution into the FEP tube at 30 mL/h. The reaction solution was then subjected to stable irradiation for 1 h. After the reaction was completed, the reaction solution was concentrated and separated by column chromatography (ethyl acetate/petroleum ether = 3/1) to remove excess cinnamate starting material, thus giving a crude product. The crude product was diluted with ethyl acetate, allowed to react at 65 °C for 1 h, and then concentrated again to give a racemate of the target compound 9B (0.96 g, yield: 71%) as a yellow oily crude product.

LCMS [M+H]⁺ = 615.5.

Step 2: The racemate of compound 9B (1.4 g, 2.27 mmol) was dissolved in methanol (100 mL), and then NaOMe (0.3 g, 5.7 mmol) was added at 0 °C. The reaction solution was allowed to react at 65 °C for 1 h, concentrated, and then separated by column chromatography (0.5% formic acid dissolved in ethyl acetate/petroleum ether (1/3)) to give a racemate of the target product 9C (0.96 g, yield: 68%) as a yellow oil.

LCMS [M+H]⁺ = 615.1.

Step 3: The racemate of compound 9C (0.60 g, 0.977 mmol) and AcOH (1.06 g, 17.586 mmol) were dissolved in MeCN (40 mL) and DCM (40 mL). Then, the solution was cooled to 0 °C and placed under a N₂ atmosphere, and STAB (1.04 g, 4.89 mmol) was added in portions. The mixture was stirred at room temperature for 1 h. The reaction solution was quenched with water. An aqueous NaHCO₃ solution was added, and the mixture was extracted with DCM twice. The organic layer was dried, concentrated under reduced pressure, and purified on a silica gel column (PE:EA) to give a racemate of 9D (0.32 g, yield: 53%) as a white solid.

LCMS: [M+H]⁺ = 617.2.

Step 4: The racemate of compound 9D (2.00 g, 3.24 mmol), Cs₂CO₃ (1.58 g, 4.86 mmol), and MeOH (0.21 g, 6.48 mmol) were dissolved in toluene (30 mL), and then tBuXPhos Pd G3 (51 mg, 0.06 mmol) was added. The mixture was heated to 85 °C and allowed to react for 3 h. The reaction solution was concentrated, diluted with a solution of MeOH/DCM (MeOH/DCM = 1/1), and filtered. The filtrate was concentrated and then separated by column chromatography (EA/PE = 60%) to give a racemate of the target product 9E (810 mg, yield: 44%) as a brown oil.

LCMS: [M-H]⁻ = 567.0.

Step 5: The racemate of compound 9E (300 mg, 0.53 mmol) and Pd/C (10%, 150 mg) were dissolved in MeOH (15 mL), and then the solution was allowed to react at room temperature for 2 h under a H₂ atmosphere. The reaction solution was filtered, and the filtrate was concentrated and then separated by column chromatography to give a racemate of the target product 9F (270 mg, crude product, yield not calculated) as a white solid.

LCMS: [M+H]⁺ = 479.4.

Step 6: The racemate of compound 9F (360 mg, 0.75 mmol), compound 2G (268 mg, 0.75 mmol), and K₂CO₃ (207 mg, 1.5 mmol) were added to 5 mL of DMF, and the mixture was stirred at 60 °C for 1.5 h, then cooled to room temperature, and extracted with ethyl acetate and water. The organic phase was separated, dried over anhydrous Na₂SO₄, filtered, and then concentrated. The crude product was purified on a silica gel column (EA/DCM from 0 to 50%) to give a racemate of 9G (360 mg, yield: 78%) as a yellow solid.

LCMS: [M-H]⁻ = 609.2.

Step 7: The racemate of compound 9G (340 mg, 0.56 mmol) was dissolved in DMF (5 mL), and compound 2I (1600 mg, 4.46 mmol), Pd(PPh₃)₄ (65 mg, 0.06 mmol), and CuI (21 mg, 0.11 mmol) were added. The mixture was allowed to react at 110 °C for 16 h under a nitrogen atmosphere. The reaction solution was cooled to room temperature and directly purified on a silica gel column (PE/EA = 1/1) to give a racemate of 9H (180 mg, yield: 61%) as a white solid.

LCMS [M+H]⁺ = 530.0.

Step 8: MsCl (171 mL, 1.5 mmol) was added to a mixture of the racemate of compound 9H (80 mg, 0.15 mmol) and pyridine (0.5 mL) at 0 °C, and the resulting mixture was allowed to react at 0 °C for 1 h, then diluted with ethyl acetate (5 mL), and washed with 1 M HCl (5 mL), followed by liquid separation. The organic phase was purified on a silica gel column (DCM/EA = 1/1) to give a racemate of 9I (90 mg, yield: 97%) as a white solid.

LCMS [M+H]⁺ = 608.1.

Step 9: The racemate of compound 9I (90 mg, 0.15 mmol) was dissolved in DMF (0.5 mL), and NaCN (36 mg, 0.75 mmol) was added. The mixture was stirred at room temperature for 16 h and then extracted with ethyl acetate. The organic phase was separated, dried over anhydrous Na₂SO₄, filtered, and then concentrated. The crude product was purified on a silica gel column (DCM/EA = 1/1) to give a racemate of 9J (70 mg, yield: 88%) as a white solid.

LCMS [M+H]⁺ = 539.1.

Step 10: The racemate of compound 9J (10 mg, 0.016 mmol) was dissolved in THF (0.5 mL), and a 2 M solution of lithium aluminum hydride in THF (0.08 mL) was added at 0 °C. The mixture was slowly warmed to room temperature and stirred for 1 h. The reaction solution was quenched with Na₂SO₄·10H₂O, and the resulting mixture was filtered. The filtrate was purified on a medium-pressure preparative column (ACN/H₂O = 0%-100%, containing 0.1% formic acid) to give a racemate of 9 (7 mg, yield: 70%) as a white solid, which was further resolved on a chiral column to give compound 9.

LCMS [M+H]⁺ = 515.1.

¹H NMR (400 MHz, DMSO) δ 8.26 (s, 1H), 7.42 (s, 1H), 7.22 (d, J = 4.8 Hz, 2H), 7.13 (d, J = 8.4 Hz, 2H), 7.09 - 7.04 (m, 3H), 6.81 - 6.76 (m, 2H), 6.71 (d, J = 8.8 Hz, 2H), 3.97 (s, 3H), 3.66 (s, 3H), 3.23 (d, J = 13.6 Hz, 5H), 3.13 (m, 1H), 2.93 (s, 1H).

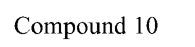

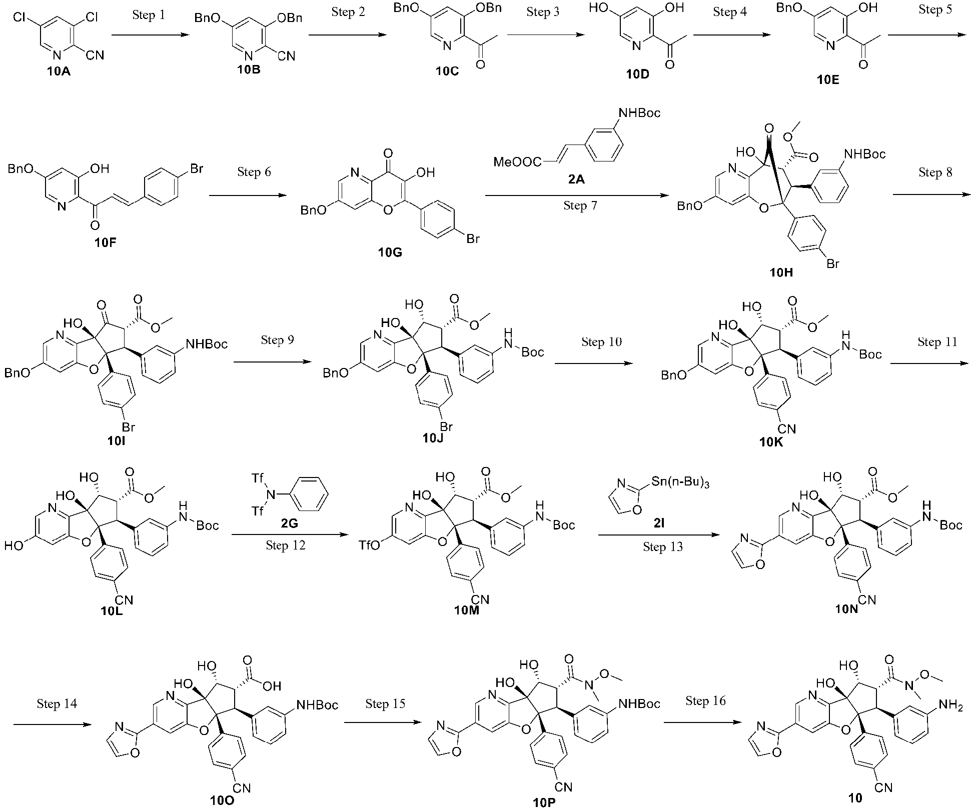

Step 1: NaH (9.06 g, 234.6 mmol) was slowly added to a solution of 10A (12.11 g, 70 mmol) in THF (120 mL) at 0 °C. The mixture was stirred at room temperature for 0.5 h. Then, BnOH (16.2 g, 150 mmol) was added, and the mixture was stirred at room temperature for 3 h, slowly quenched with water at 0 °C, and extracted with EtOAc. The organic phase was separated, dried over anhydrous Na₂SO₄, filtered, and concentrated under reduced pressure to give compound 10B (19.6 g, yield: 89%). LCMS [M+H]⁺ = 317.2.

Step 2: A MeMgBr solution (124.9 mL, 124.9 mmol) was slowly added dropwise to a solution of 10B (13.16 g, 41.6 mmol) in THF (150 mL) at -30 °C. The mixture was stirred at room temperature for 4.3 h, then quenched with 6 N HCl, and stirred for another 2 h. The organic phase was washed with 1 N NaOH and water, separated, dried over anhydrous Na₂SO₄, filtered, and concentrated under reduced pressure. The crude product was purified by silica gel chromatography to give compound 10C (7.12 g, yield: 46%). LCMS [M+H]⁺ = 334.2.

Step 3: Compound 10C (8.5 g, 25.5 mmol) was dissolved in EA (60 mL), and Pd(OH)₂ (850 mg) was added. The mixture was stirred at room temperature for 18 h under a hydrogen atmosphere and then concentrated to give 10D (3.9 g, yield: 100%) as a white solid. LCMS [M+H] ⁺ = 154.2. Step 4: BnBr (4.36 g, 25.5 mmol) was added dropwise to a solution of compound 10D (3.9 g, 25.5 mmol) and K₂CO₃ (9.85 g, 71.4 mmol) in acetone (40 mL), and the mixture was stirred at room temperature for 48 h, acidified with acetic acid, diluted with water, and extracted with EA. The organic layer was concentrated and washed with petroleum ether to give 10E (4.80 g, yield: 77%) as a yellowish-white solid. LCMS [M+H]⁺ = 244.1.

Step 5: NaOH (2.368 g, 59.2 mmol) and 4-bromobenzaldehyde (3.65 g, 19.73 mol) were added to a solution of compound 10E (4800 mg, 19.73 mmol) in MeOH (60 mL). The reaction solution was stirred at 70 °C for 18 h, then cooled, acidified with AcOH, and extracted with dichloromethane. The organic layer was washed with water and brine, dried, and concentrated. The crude product was slurried with PE/EA to give 10F (5.0 g, yield: 60%) as a white solid. LCMS [M+H]⁺ = 410.2. Step 6: NaOH (785 mg, 19.6 mmol, 7.0 eq.) and H₂O₂ (12.5 mL) were added to a solution of compound 10F (1150 mg, 2.8 mmol, 1.0 eq.) in MeOH/H₂O (130 mL/65 mL). The reaction solution was stirred at 50 °C for 3 h. The mixture was cooled and filtered. The solid was washed with a small amount of dichloromethane and acidified with AcOH, and extraction was performed with DCM. The organic layer was washed with water and brine, dried, and concentrated to give 10G (670 mg, yield: 57%) as a white solid. LCMS [M+H]⁺ = 424.0.

¹H NMR (400 MHz, DMSO) δ 9.99 (s, 1H), 8.56 (d, *J =* 2.4 Hz, 1H), 8.18 (d, *J =* 8.8 Hz, 2H), 7.94 (d, *J =* 2.4 Hz, 1H), 7.80 (d, *J =* 8.8 Hz, 2H), 7.60 - 7.37 (m, 5H), 5.34 (s, 2H).

Step 7: Compound 10G (2.5 g, 5.9 mmol) and compound 2A (9.8 g, 35.38 mmol) were dissolved in CHCl₃/TFE (7/3, 20 mL). The reaction mixture was injected at a rate of 12 mL/h via a syringe using a syringe pump into a coil wound around a jacketed glass cylinder equipped with a 250 W ultraviolet lamp. The temperature of the external circulation cold trap was adjusted to maintain the internal reaction temperature at 0-5 °C, and the UV lamp was turned on simultaneously. The reaction mixture was irradiated for 1 h and then concentrated under reduced pressure to remove the solvent. The residue was purified by silica gel column chromatography (EtOAc/hexane) to remove excess cinnamate, thus giving a racemate of 10H (3.6 g, yield: 75%) as a light yellow oily product. LCMS [M+H]⁺ = 700.9.

Step 8: The racemate of compound 10H (2.5 g, 3.56 mmol) was dissolved in methanol (60 mL), and then NaOMe (481 mg, 8.91 mmol) was added at 0 °C. Subsequently, the reaction solution was stirred at 65 °C for 1.5 h. After the reaction was completed, the reaction solution was concentrated, extracted with EA, washed with water, an NH₄Cl solution, and saturated brine, then dried over anhydrous sodium sulfate, concentrated under reduced pressure, and finally purified on a silica gel column (0.2% FA, EA/PE = 1/3) to give a racemate of compound 10I (3.0 g, crude product). LCMS [M+H]⁺ = 701.0.

Step 9: The racemate of compound 10I (3.0 g, 4.28 mmol) was dissolved in a mixed solvent of ACN/CHCl₃ (1/1, 40 mL/40 mL), and Na(AcO)₃BH (4.533 g, 21.38 mmol) and AcOH (2.566 g, 47.0 mmol) were added at 0 °C. Then, the reaction solution was stirred at room temperature for 2 h. After the reaction was completed, the reaction solution was concentrated, extracted with EA, washed with water and saturated brine, then dried over anhydrous sodium sulfate, concentrated under reduced pressure, and finally purified on a silica gel column (0.2% FA, EA/PE = 3/2) to give a racemate of compound 10J (500 mg). LCMS [M+H]⁺ = 703.0.

¹H NMR (400 MHz, CDCl₃) δ 8.06 (s, 1H), 7.86 (d, J = 1.9 Hz, 1H), 7.43 (d, J = 4.4 Hz, 4H), 7.40 (dd, J = 8.2, 5.0 Hz, 2H), 7.28 (s, 2H), 7.15 (d, J = 8.5 Hz, 1H), 7.10 (d, J = 2.0 Hz, 1H), 7.06 (d, J = 8.7 Hz, 2H), 7.00 (t, J = 7.9 Hz, 1H), 6.90 (s, 1H), 6.57 (d, J = 7.6 Hz, 1H), 6.35 (s, 1H), 5.14 (s, 2H), 5.06 (d, J = 6.0 Hz, 1H), 4.41 (d, J = 13.9 Hz, 1H), 3.95 (dd, J = 14.0, 6.0 Hz, 1H), 3.67 (s, 3H), 1.51 (s, 9H).

Step 10: Pd₂(dba)₃ (128 mg, 0.14 mmol) was added to a solution of the racemate of compound 10J (500 mg, 0.71 mmol), DPPF (155 mg, 0.28 mmol), and Zn(CN)₂ (208 mg, 1.80 mmol) in NMP (25 mL). The reaction solution was heated to 150 °C and allowed to react for 1.2 h under a N₂ atmosphere, then diluted with water, and extracted with EA. The organic layer was washed with water and brine, dried, concentrated, and purified on a silica gel column (EA/PE = 1/1) to give a racemate of 10K (300 mg, yield: 65%) as a yellow solid. LCMS: [M+H]⁺ = 650.0.

Step 11: Pd(OH)₂/C (10%, 0.1 g) was added to a solution of the racemate of compound 10K (300 mg, 0.46 mol) in EA/MeOH/THF/DCM (20:2:2:0.2, 7 mL). The mixture was stirred at room temperature for 18 h under a hydrogen atmosphere and then filtered to remove the solid, and the filter cake was washed with MeOH/DCM (1/10). The filtrates were combined and concentrated to give a racemate of 10 L (0.22 g, yield: 87.0%) as a white solid. LCMS: [M+H]⁺ = 560.2.

Step 12: K₂CO₃ (116 mg, 0.78 mmol) was added to a solution of the racemate of compound 10L (220 mg, 0.39 mmol) and compound 2G (150 mg, 0.39 mmol) in DMF (3 mL). The mixture was stirred at 60 °C for 1 h, then quenched with water, and extracted with EtOAc. The organic phase was separated, dried over anhydrous Na₂SO₄, filtered, and concentrated. The residue was purified by column chromatography (EA-PE/DCM = 0-50%) to give a racemate of 10 M (221 mg, yield: 82%) as a yellow solid. LCMS [M+H-C₄H₈]⁺ = 636.1.

Step 13: The racemate of compound 10M (70 mg, 0.1 mmol), compound 2I (280 mg, 0.8 mmol), Pd(PPh₃)₄ (12 mg, 0.01 mmol), and CuI (4 mg, 0.02 mmol) were added to DMF (3 mL), and then the mixture was stirred under microwave at 135 °C for 5 h under a N₂ atmosphere. Water was added, and the mixture was extracted with EA. The organic layer was washed with water and saturated brine, then dried, and concentrated. The crude product was dissolved in DCM, and Boc₂O (2 eq.), DIPEA (3 eq.), and DMAP (0.2 eq.) were added. The mixture was stirred at 40 °C for 18 h, then concentrated, and purified by flash chromatography (DCM/EA = 1: 1, DCM:MeOH = 20:1) to give a racemate of 10N (60 mg, yield: 98%) as a white solid. LCMS [M+H]⁺ = 611.2.

Step 14: H₂O (2 mL), MeOH (2 mL), and LiOH (12.6 mg, 0.30 mmol) were added to a solution of the racemate of compound 10N (60 mg, 0.1 mmol) in THF (2 mL), and then the mixture was stirred at room temperature for 5 h. The reaction solution was purified by reversed-phase chromatography (MeCH/HCO₂H/H₂O) to give a racemate of 10O (30 mg, yield: 51%) as a white solid. LCMS [M+H]⁺ = 597.2.

Step 15: The racemate of compound 10O (30 mg, 0.048 mmol), N,O-dimethylhydroxylamine (9 mg, 0.096 mmol), HATU (18 mg, 0.048 mmol), and TMP (24 mg, 0.192 mmol) were added to DMF (1 mL), and then the solution was stirred at 50 °C for 18 h. The reaction solution was purified by reversed-phase column chromatography (ACN/H₂O = 0%-100%) to give a racemate of 10P (10 mg, yield: 33%) as a white solid. LCMS: [M+H]⁺ = 640.3.

Step 16: 6 M HCl (0.5 mL) was added dropwise to a solution of the racemate of compound 10P (10 mg, 0.016 mmol) in THF (0.5 mL), and then the solution was stirred at room temperature for 4 h. The reaction solution was separated by preparative reversed-phase column chromatography (ACN/H₂O = 5%-95%, +0.1% NH₄HCO₃) to give a racemate of 10 (1.1 mg, yield: 10%) as a white solid, which was further resolved on a chiral column to give compound 10.LCMS: [M+H]⁺ = 540.2.

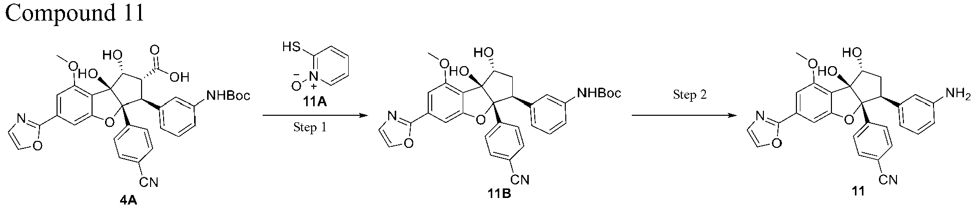

Step 1: A solution of the racemate of compound 4A (10 mg, 0.02 mmol), compound 11A (12.7 mg, 0.1 mmol), tert-dodecyl mercaptan (38.4 mg, 0.2 mmol), TMP (0.4 mg, 0.04 mmol), and Zinc meso-tetraphenylporphyrin (1.3 mg, 0.001 mmol) in DMF (1 mL) was transferred to a microwave tube. The microwave tube was irradiated under a 25 W red light lamp, with the temperature maintained at 80 °C. EDCI (36.6 mg, 0.2 mmol, dissolved in 1 mL of DMF) was added dropwise to the reaction system. The reaction solution was continuously stirred at 80 °C under 25 W red light lamp irradiation for 30 min. The reaction solutions from three parallel reactions were combined and then purified by reversed-phase column chromatography (H₂O:ACN = 0-100%) to give a racemate of 11B (9 mg, yield: 33%) as a white solid. LCMS: [M+H-C₄H₈]⁺ = 526.2.

Step 2: 6 M HCl (1 mL) was added to a solution of the racemate of compound 11B (25 mg, 0.04 mmol) in THF (1 mL). The reaction solution was stirred at room temperature overnight, concentrated under reduced pressure, and then purified by reversed-phase column chromatography (H₂O:ACN = 0-100%) to give a racemate of 11 (9 mg, yield: 40%) as a white solid, which was further resolved on a chiral column to give compound 11. LCMS [M+H]⁺ = 482.0.

¹H NMR (400 MHz, DMSO) δ 8.25 (s, 1H), 7.53 (d, *J =* 8.5 Hz, 2H), 7.41 (s, 1H), 7.30 (d, *J* = 8.5 Hz, 2H), 7.16 (d, *J =* 12.3 Hz, 2H), 6.73 (t, *J =* 7.7 Hz, 1H), 6.35 (s, 1H), 6.22 (t, *J =* 7.7 Hz, 2H), 5.34 (s, 1H), 4.90 - 4.73 (m, 3H), 4.47 (s, 1H), 4.03 (dd, *J =* 14.3, 6.1 Hz, 1H), 3.85 (s, 3H), 2.78 - 2.60 (m, 1H), 2.08 (dd, *J* = 12.8, 6.1 Hz, 1H).

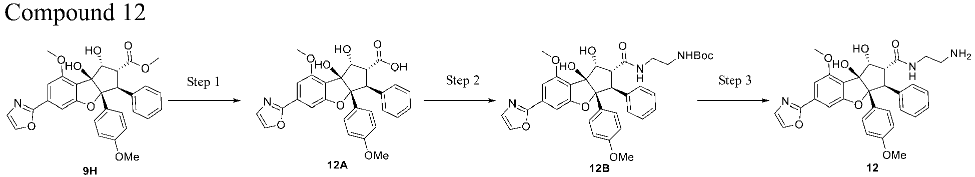

Step 1: The racemate of compound 9H (42.3 mg, 0.08 mmol) and LiOH (16.8 mg, 0.4 mmol) were dissolved in MeOH/H₂O (5 mL/1 mL), and the solution was stirred at room temperature for 12 h, adjusted to pH = 2-3 with a 1 M aqueous hydrochloric acid solution, and extracted with ethyl acetate (20 mL). The organic phase was dried over sodium sulfate and concentrated to give a racemate of 12A (35 mg, yield: 84.6%) as a white solid. LCMS [M+H]⁺ = 516.2.

Step 2: The racemate of compound 12A (31 mg, 0.06 mmol) was mixed with Boc-ethylenediamine (19 mg, 0.12 mmol), 2,4,6-trimethylpyridine (22 mg, 0.18 mmol), and HATU (34 mg, 0.09 mmol) in DMF (3 mL), and the mixture was stirred at room temperature for 12 h. Then, 50 mL of water was added to the reaction solution, and the mixture was extracted with ethyl acetate, washed with saturated brine, dried, and concentrated. The crude product was purified on a silica gel column (PE/EA = 1/2) to give a racemate of 12B (33 mg, yield: 83.7%) as a white solid. LCMS [M+H]⁺ = 658.2.

Step 3: An aqueous HCl solution (1 mL, 6 M) was added to a solution of the racemate of compound 12B (33 mg, 0.05 mmol) in THF (3 mL), and the reaction solution was allowed to react at room temperature for 12 h and then concentrated. The resulting crude product was purified on a preparative chromatography column to give a racemate of 12 (20 mg, yield: 71.4%) as a white solid, which was further resolved on a chiral column to give compound 12.

LCMS [M+H]⁺ = 558.2.

¹H NMR (400 MHz, DMSO) δ 8.45 (s, 2H), 8.24 (d, *J =* 0.6 Hz, 1H), 7.40 (d, *J =* 0.6 Hz, 1H), 7.19 (d, *J* = 1.0 Hz, 1H), 7.14 (d, *J=* 0.9 Hz, 1H), 7.10 - 7.01 (m, 4H), 6.97 (dd, *J=* 10.1, 7.4 Hz, 3H), 6.59 (d, *J =* 9.0 Hz, 2H), 5.32 (s, 1H), 4.65 (d, *J =* 4.8 Hz, 1H), 4.30 (d, *J =* 14.1 Hz, 1H), 3.90 - 3.86 (m, 1H), 3.84 (s, 3H), 3.59 (s, 3H), 3.14 (s, 2H), 2.65 (s, 2H).

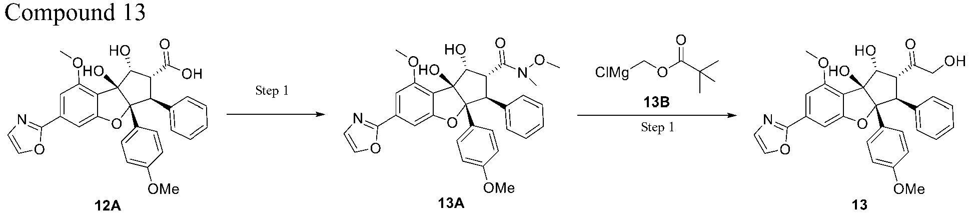

Step 1: The racemate of compound 12A (350 mg, 0.68 mmol) was dissolved in DMF (2 mL), and then N,O-dimethylhydroxylamine hydrochloride (132.5 mg, 1.36 mmol), HATU (310 mg, 0.82 mmol), and DIEA (263.2 mg, 2.04 mmol) were sequentially added. The reaction solution was stirred at room temperature for 2 h, then directly concentrated to dryness by rotary evaporation, and purified by reversed-phase chromatography (acetonitrile/water = 5%-95%, 214 nm, 20 min) to give a racemate of 13A (300 mg, yield: 79%) as a white solid. LCMS [M+Na]⁺ = 581.2.

Step 2: Iodomethyl pivalate (2000 mg, 8.26 mmol) was dissolved in a solution of anhydrous tetrahydrofuran (15 mL), and isopropylmagnesium chloride (2.0 M, 8.26 mL, 16.52 mmol) was added at -70 °C. The reaction mixture was stirred at -70 °C for 2 h to give a solution of compound 13B. The racemate of compound 13A (100 mg, 0.18 mmol) was dissolved in 1 mL of anhydrous tetrahydrofuran, and the solution of compound 13B (5 mL) was added at -70 °C. The reaction mixture was stirred at room temperature overnight, then diluted with water (15 mL), extracted with dichloromethane (15 mL × 2), washed with brine (15 mL × 2), dried over anhydrous sodium sulfate, filtered, and concentrated. The crude product was purified on a preparative chromatography column to give a racemate of 13 (5.5 mg, yield: 5.8%) as a white solid, which was further resolved on a chiral column to give compound 13. LCMS [M+H]⁺ = 530.0.

¹H NMR (400 MHz, DMSO-*d*₆) δ 8.25 (d, *J =* 0.8 Hz, 1H), 7.43-7.41 (m, 3H), 7.37-7.35 (m, 2H), 7.31-7.27 (m, 2H), 7.22 - 7.24 (m, 1H), 7.17 (d, *J =* 1.2 Hz, 1H), 7.12 (d, *J =* 1.2 Hz, 1H), 6.93-6.91 (m, 2H), 5.40 (s, 1H), 4.83 (d, *J* = 4.8 Hz, 1H), 4.40-4.36 (m, 1H), 3.80 (s, 3H), 3.76-3.73 (m, 4H), 3.10 (t, *J =* 12.0 Hz, 2H).

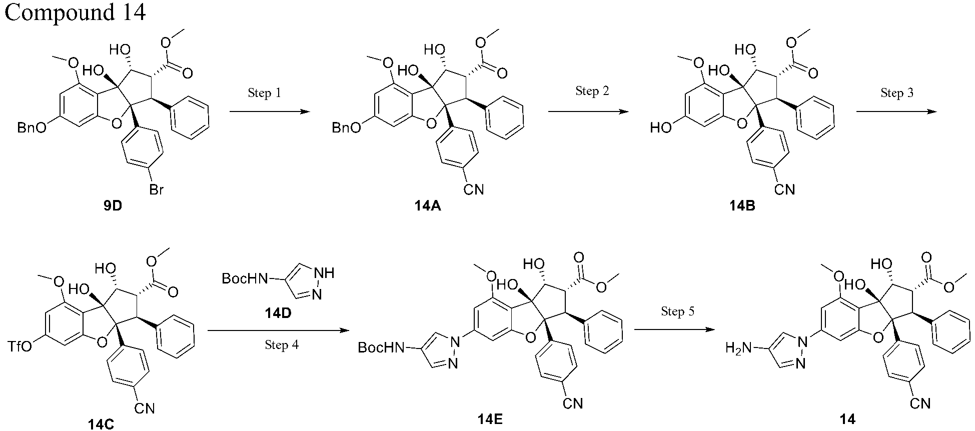

Step 1: A solution of the racemate of compound 9D (2.30 g, 3.7 mmol), Zn(CN)₂ (1.09 g, 9.3 mmol), Pd(dba)₃ (0.68 g, 0.75 mmol), and DPPF (0.83 g, 1.49 mmol) in NMP (160 mL) was stirred at 150 °C for 4 h.The mixture was extracted with H₂O and EA. The organic layers were combined and washed with brine (2 × 10 mL). The mixture was purified by flash column chromatography (PE/EA = 1/1) to give a racemate of 14A (1.9 g, yield: 90%) as a yellow solid.

LCMS: [M+H]⁺ = 564.2.

Step 2: Pd(OH)₂/C (10%, 1.4 g) was added to a solution of the racemate of compound 14A (2.8 g, 4.97 mmol) in EA/MeOH/THF/DCM (EA/MeOH/THF/DCM = 20:2:0.2:0.2, 120 mL) under a N₂ atmosphere. The suspension was stirred at room temperature for 4 h under a H₂ atmosphere. The reaction solution was filtered, and the filter cake was washed with a solution of MeOH/DCM (MeOH/DCM = 1/10). The filtrates were combined, concentrated, and purified by flash column chromatography (DCM/MeOH = 10/1) to give a racemate of 14B (1.6 g, yield: 68%) as a yellow solid.

LCMS: [M-H]⁺ = 474.2.

Step 3: At 0 °C, trifluoromethanesulfonic anhydride (1.1 g, 4.06 mmol, 1.2 eq.) was slowly added dropwise to a solution of the racemate of compound 14B (1.6 g, 3.38 mmol) and diisopropylethylamine (0.7 g, 5.75 mmol) in 150 mL of DCM, and the mixture was stirred for 45 min and then washed with 25 mL of saturated sodium bicarbonate. The organic layer was collected, and then the aqueous layer was extracted with DCM (20 mL). The organic layers were combined, sequentially washed with 10% citric acid (20 mL) and water (20 mL), and then dried. The filtrate was concentrated under reduced pressure and purified by flash column chromatography (DCM/EA = 10/1) to give a racemate of compound 14C (1.7 g, yield: 83%) in the form of a white powder.

LCMS: [M+H]⁺ = 606.2.

Step 4: The racemate of compound 14C (50 mg, 2.2 mmol), compound 14D (30.26 mg, 0.16 mmmol), tBuXPhos-Pd-G3 (4.8 mg, 0.01 mmol), tBuXPhos (12.78 mg, 0.03 mmol), and Cs₂CO₃ (67 mg, 0.21 mmol) were dissolved in 1,4-dioxane (2 mL). The reaction mixture was stirred at 100 °C for 16 h under a nitrogen atmosphere. After the reaction was completed, the reaction solution was extracted with EA and concentrated to give a racemate of compound 14E (50 mg, crude product, yield not calculated). LCMS [M+H]⁺ = 639.2.

Step 5: The racemate of compound 14E (50 mg) was dissolved in a solution of THF (0.5 mL), and 6 M HCl (0.5 mL) was added. The mixture was stirred at room temperature for 4 h. The reaction solution was purified by flash column chromatography (ACN-H₂O = 0-100%) to give a racemate of 14 (6 mg, yield: 12%) as a white solid, which was further resolved on a chiral column to give compound 14.

LCMS [M+H]⁺ = 539.2.

¹H NMR (400 MHz, DMSO) δ 7.78 (s, 1H), 7.47 (d, *J =* 8.6 Hz, 2H), 7.31 (d, *J =* 8.6 Hz, 2H), 7.26 (s, 1H), 7.09 - 7.03 (m, 2H), 7.02 - 6.94 (m, 4H), 6.92 (d, *J =* 1.6 Hz, 1H), 5.51 (s, 1H), 5.39 (d, *J =* 5.3 Hz, 1H), 4.67 (t, *J =* 5.1 Hz, 1H), 4.39 (d, *J =* 13.9 Hz, 1H), 4.18 (d, *J =* 18.8 Hz, 2H), 4.15 - 4.07 (m, 1H), 3.80 (s, 3H), 3.58 (s, 3H).

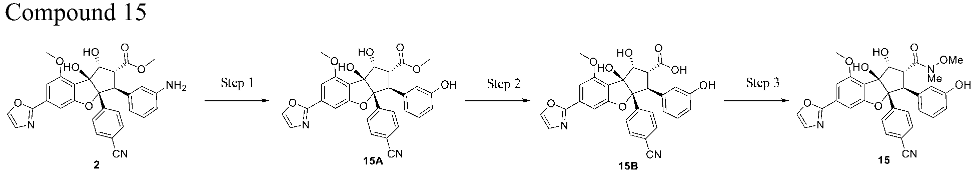

Step 1: HBF₄ (0.85 mL, 4.79 mmol) was added dropwise to a solution of the racemate of compound 2 (170 mg, 0.32 mmol) in ethanol (5 mL) at room temperature. After being stirred for 15 min, the reaction solution became clear. The solution was then cooled to 0 °C, and tert-butyl nitrite (65.0 mg, 0.63 mmol) was added. After 30 min, the reaction mixture was diluted with diethyl ether (8 mL) and filtered to give a solid, which was then washed with diethyl ether (5 mL) twice and dried. The solid was added to water (500 mL) containing copper nitrate (17.7 g, 94.5 mmol) and cuprous oxide (45.0 mg, 0.32 mmol), and the mixture was stirred at room temperature for 1 h. The aqueous solution was filtered to give a brown solid, which was then purified by flash column chromatography (ACN/H₂O) to give a racemate of 15A (20 mg, yield: 12.3%) as a yellow solid. LCMS [M+H]⁺ = 541.0.

Step 2: The racemate of compound 15A (20.0 mg, 0.04 mmol) and LiOH (34.8 mg, 0.40 mmo) were dissolved in THF/H₂O (5 mL/1 mL), and the solution was stirred at room temperature for 6 h. The reaction solution was purified by flash column chromatography (ACN/H₂O) to give 15B (17.0 mg, yield: 85.3%) as a white solid. LCMS [M+H] ⁺ = 527.0.

Step 3: Compound 15B (15.0 mg, 0.03 mmol), N,O-dimethylhydroxylamine hydrochloride (2.92 mg, 0.03 mmol), HATU (13.0 mg, 0.03 mmol), HOAT (2.25 mg, 0.03 mmol), and TMP (10.3 mg, 0.09 mmol) were added to DMF (1.0 mL). The mixture was stirred at room temperature for 16 h and purified by flash column chromatography (ACN/H₂O) to give a racemate of 15 (9.0 mg, yield: 55.4%) as a white solid, which was further resolved on a chiral column to give compound 15. LCMS [M+H]⁺ = 570.0.

¹H NMR: (400 MHz, CDCl3) δ 9.09 (s, 1H), 8.25 (s, 1H), 7.53 (d, *J =* 8.4 Hz, 2H), 7.41 (s, 1H), 7.30 (d, *J =* 8.4 Hz, 2H), 7.21 (s, 1H), 7.15 (s, 1H), 6.85 (s, 1H), 6.83 (d, J=8.4 Hz, 1H), 6.56 - 6.36 (m, 3H), 5.63 (s, 1H), 5.11 (d, *J=* 4.8 Hz, 1H), 4.79 (t, *J* = 4.8 Hz, 1H), 4.38 (d, *J* = 7.2 Hz, 1H), 4.18 - 4.13 (m, 1H), 3.90 (s, 3H), 3.84 (s, 3H), 3.09 (s, 3H).

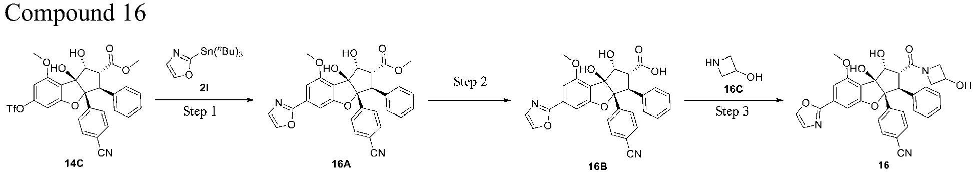

Step 1: Compound 2I (8.04 g, 22.46 mmol), Pd(PPh₃)₄ (763.35 mg, 0.6 mmol), and CuI (629.03 mg, 3.30 mmol) were added to a solution of the racemate of compound 14C (2.00 g, 3.30 mmol) in DMF (10 mL). The mixture was stirred under microwave at 115 °C for 2 h under a nitrogen atmosphere and then purified on a silica gel column (PE/EA = 1/1) to give a racemate of 16A (1.5 g, yield: 86%) as a white solid. LCMS [M+H]⁺ = 525.0.

Steps 2 and 3: The racemate of compound 16A (100.00 mg, 0.19 mmol) and LiOH (20.00 mg, 0.25 mmol) were dissolved in MeOH/H₂O (5 mL/1 mL), and the solution was stirred at room temperature for 12 h, adjusted to pH = 2-3 with a 1 M aqueous hydrochloric acid solution, and extracted with ethyl acetate (20 mL). The organic phase was dried over sodium sulfate and concentrated to give a racemate of 16B (crude product).

The racemate of 16B (crude product) was dissolved in DMF (3 mL), and HATU (144.50 mg, 0.38 mmol), compound 16C (27.78 mg, 0.38 mmol), and DIEA (73.67 mg, 0.57 mmol) were added. The mixture was stirred at room temperature for 12 h. 50 mL of water was added to the reaction solution, and the mixture was extracted with ethyl acetate, washed with saturated brine, dried, and concentrated. The crude product was purified on a silica gel column (PE/EA = 1/2) to give a racemate of 16 (20 mg, yield: 17%) as a white solid, which was further resolved on a chiral column to give compound 16.

LCMS [M+H]⁺ = 566.2.

¹H NMR (400 MHz, DMSO) δ 8.25 (s, 1H), 7.47 (d, *J* = 7.3 Hz, 2H), 7.41 (s, 1H), 7.32 (dd, *J =* 8.0, 5.0 Hz, 2H), 7.23 (s, 1H), 7.15 (s, 1H), 7.04 (t, *J* = 6.8 Hz, 2H), 6.96 (s, 3H), 5.77 (dd, *J =* 15.3, 5.4 Hz, 1H), 5.62 (d, *J =* 7.0 Hz, 1H), 5.06 (dd, *J =* 11.7, 5.0 Hz, 1H), 4.77 (t, *J =* 7.8 Hz, 1H), 4.67 (s, 1H), 4.62 - 4.50 (m, 2H), 4.47 (d, *J =* 13.8 Hz, 1H), 4.34 - 4.06 (m, 1H), 4.01 (dd, *J* = 16.6, 9.9 Hz, 1H), 3.93 (dd, *J =* 12.8, 6.8 Hz, 1H), 3.85 (d, *J =* 3.9 Hz, 3H), 3.59 (dd, *J =* 17.2, 7.4 Hz, 1H).

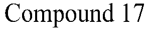

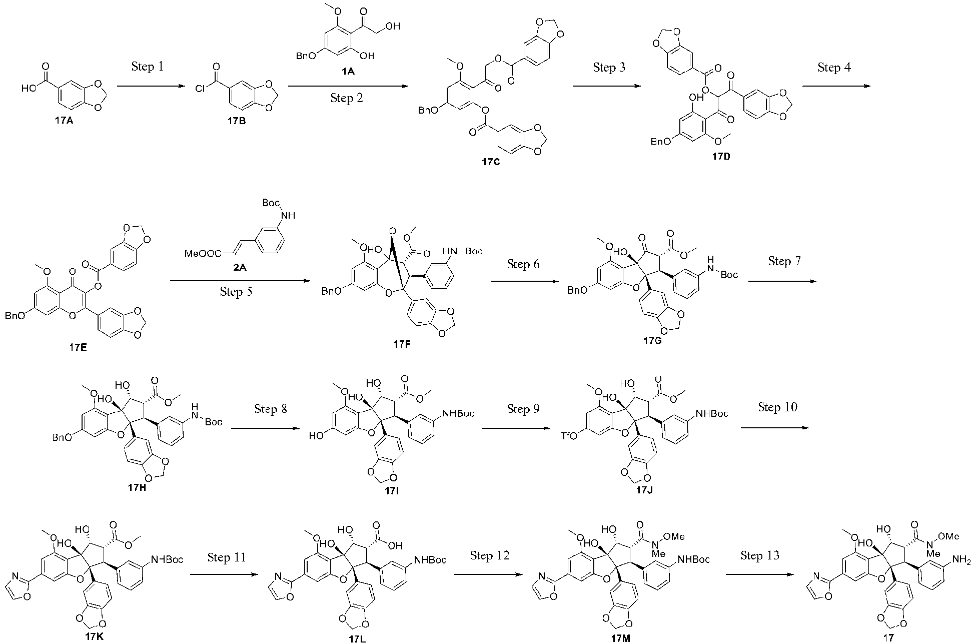

Step 1: Compound 17A (5.0 g, 30.10 mmol) was dissolved in chloroform (50 mL) under an ice-water bath, and N,N-dimethylformamide (0.5 mL) and thionyl chloride (5.37 g, 45.14 mmol) were sequentially added. The mixture was stirred at 75 °C for 12 h. The reaction mixture was concentrated to give 17B (6.0 g) as a yellow solid. LCMS [M+H]⁺ = 185.2.

Step 2: Compound 1A (4.0 g, 13.87 mmol) was dissolved in dichloromethane (30 mL) under an ice-water bath, and triethylamine (4.2 g, 41.61 mmol), DMAP (1.69 g, 13.8 mmol), and compound 17B (5.63 g, 30.52 mmol) were added. The mixture was stirred at room temperature for 1.5 h, then diluted with water (30 mL), and extracted with dichloromethane (30 mL × 2). The organic phase was washed with brine (30 mL × 2), dried over anhydrous sodium sulfate, filtered, concentrated, and purified by silica gel chromatography (PE/EA = 1/1) to give 17C (6.92 g, yield: 85%) as a white solid. LCMS [M+H]⁺ = 585.0.

Step 3: Compound 17C (3.5 g, 5.99 mmol) was dissolved in anhydrous tetrahydrofuran (30 mL), and a solution of LiHMDS in THF (1 M, 17.96 mL, 17.96 mmol) was added dropwise at -78 °C. The mixture was stirred at -78 °C for 1 h, then diluted with water (50 mL), extracted with ethyl acetate (50 mL × 2), washed with brine (50 mL × 2), dried over anhydrous sodium sulfate, filtered, concentrated, and then stirred with diethyl ether to give 17D (2.67 g, yield: 78%) as a white solid. LCMS [M+H]⁺ = 585.0.

Step 4: Compound 17D (900 mg, 1.54 mmol) was dissolved in AcOH (15 mL), and sodium acetate (316 mg, 3.85 mmol) was added. The reactants were stirred at 100 °C overnight. The reaction mixture was concentrated, extracted with dichloromethane (20 mL), washed with water (20 mL × 2) and brine (20 mL × 2), dried over anhydrous sodium sulfate, filtered, concentrated, and then stirred with diethyl ether to give 17E (800 mg, yield: 92%) as a yellow solid.

Step 5: Compound 17E (100 mg, 0.24 mmol) and compound 2A (397 mg, 1.44 mmol) were dissolved in CHCl₃/TFE (7/3, 5 mL). The reaction mixture was injected at a rate of 12 mL/h via a syringe using a syringe pump into a coil wound around a jacketed glass cylinder equipped with a 250 W ultraviolet lamp. The temperature of the external circulation cold trap was adjusted to maintain the internal reaction temperature at 0-5 °C, and the UV lamp was turned on simultaneously. The reaction mixture was irradiated for 1 h and concentrated under reduced pressure to remove the solvent. The residue was purified on a silica gel column (EtOAc/hexane) to remove excess compound 2A to give a racemate of 17F (190 mg, yield: 75%) as a pale yellow solid. LCMS [M+H] ⁺ = 696.1.

Step 6: The racemate of compound 17F (4.8 g, 6.91 mmol) was dissolved in MeOH (40 mL), and NaOMe (932.4 mg, 17.27 mmol) was added at 0 °C. The mixture was stirred at 65 °C for 1.5 h. The solution was concentrated, washed with H₂O, NH₄Cl (aq.), and brine, then dried over anhydrous Na₂SO₄, concentrated under reduced pressure, and purified on a silica gel column (0.2% FA, EA/PE = 1/3) to give a racemate of compound 17G (2.8 g, yield: 58%). LCMS [M+H-H₂O]⁺ = 677.9.

Step 7: The racemate of compound 17G (2.85 g, 4.10 mmol) was dissolved in ACN/CHCl₃ (1/1, 30 mL/30 mL), and Na(AcO)₃BH (4.3 g, 20.50 mmol) and AcOH (2.5 g, 41.01 mmol) were added at 0 °C. The mixture was stirred at room temperature for 2 h, concentrated, then extracted with EA, washed with water and brine, dried over anhydrous Na₂SO₄, and purified on a silica gel column (A: PE, B: 0.2% FA, EA) to give a racemate of compound 17H (1.6 g, yield: 56%). LCMS [M+H-H₂O]⁺ = 680.0.

Step 8: The racemate of compound 17H (400 mg, 0.57 mmol) was dissolved in MeOH (10 mL), and palladium hydroxide (40 mg) was added to the solution. The reactants were stirred at room temperature overnight under a hydrogen atmosphere. The reaction solution was filtered and washed with methanol (10 mL). The filtrate was concentrated to give a racemate of 17I (320 mg, yield: 91%) as a yellow solid. LCMS [M+H-H₂O]⁺ = 590.0.

Step 9: The racemate of compound 17I (320 mg, 0.53 mmol) was dissolved in DMF (5 mL), and 1,1,1-trifluoro-N-phenyl-N-((trifluoromethyl)sulfonyl)methanesulfonamide (189.3 mg, 0.53 mmol) and potassium carbonate (146.3 mg, 1.06 mmol) were added to the mixture. The reaction solution was stirred at 60 °C for 1 h and then purified by reversed-phase chromatography (acetonitrile/water = 5%-95%, 214 nm, 20 min) to give a racemate of 17J (320 mg, yield: 82%) as a white solid. LCMS [M+H]⁺ = 740.2.

Step 10: The racemate of compound 17J (50 mg, 0.07 mmol) was dissolved in DMF (1.5 mL), and 2-(tributylstannyl)oxazole (190 mg, 0.54 mmol), tetrakis(triphenylphosphine)palladium (8 mg, 0.007 mmol), and CuI (2.6 mg, 0.014 mmol) were sequentially added to the solution. The solution was stirred under microwave at 100 °C for 1.5 h. The reaction mixture was filtered, washed with MeOH (5 mL), and concentrated. The residue was purified by reversed-phase chromatography (acetonitrile/water = 5%-95%, 214 nm, 20 min) to give a racemate of 17K (30 mg, yield: 34%) as a yellow solid. LCMS [M+H]⁺ = 659.2.

Step 11: The racemate of compound 17K (35 mg, 0.05 mmol) was dissolved in tetrahydrofuran/water (3 mL/1 mL), and lithium hydroxide (4.20 mg, 0.1 mmol) was added to the solution. The reaction mixture was stirred at room temperature for 2 h, then concentrated, and purified by reversed-phase chromatography (acetonitrile/water = 5%-95%, 214 nm, 20 min) to give a racemate of 17L (30 mg, yield: 88%) as a yellow solid. LCMS [M+H]⁺ = 645.2.

Step 12: The racemate of compound 17L (30 mg, 0.05 mmol), N,O-dimethylhydroxylamine (10 mg, 0.1 mmol), HATU (22.8 mg, 0.06 mmol), and DIEA (19.35 mg, 0.15 mmol) were sequentially added to DMF (1.5 mL). The reaction mixture was stirred at room temperature for 2 h. The reaction solution was purified by reversed-phase chromatography (acetonitrile/water = 5%-95%, 214 nm, 20 min) to give a racemate of 17M (15 mg, yield: 47%) as a yellow solid. LCMS [M+H]⁺ = 688.2 Step 13: 6 N/HCl (0.3 mL) was added to a solution of the racemate of compound 17M (15 mg, 0.02 mmol) in tetrahydrofuran (0.5 mL). The mixture was stirred at room temperature for 12 h and then purified on a preparative chromatography column to give a racemate of 17 (3.8 mg, yield: 30%) as a white solid, which was further resolved on a chiral column to give compound 17.

LCMS [M+H]⁺ = 588.2.

¹H NMR (400 MHz, DMSO-*d*₆) δ 8.36 (s, 1H), 8.24 (s, 1H), 7.41 (s, 1H), 7.15 (d, J = 12.4 Hz, 2H), 6.72-6.60 (m, 4H), 6.23-6.18 (m, 3H), 6.04 (d, J = 7.6 Hz, 1H), 5.86 (d, J = 3.2 Hz, 2H), 5.37 (s, 1H), 4.92 (s, 1H), 4.83-4.73 (m, 3H), 4.16 (d, J = 14.0 Hz, 1H), 4.08 (brs, 1H), 3.88-3.85 (m, 6H), 3.06 (s, 3H).

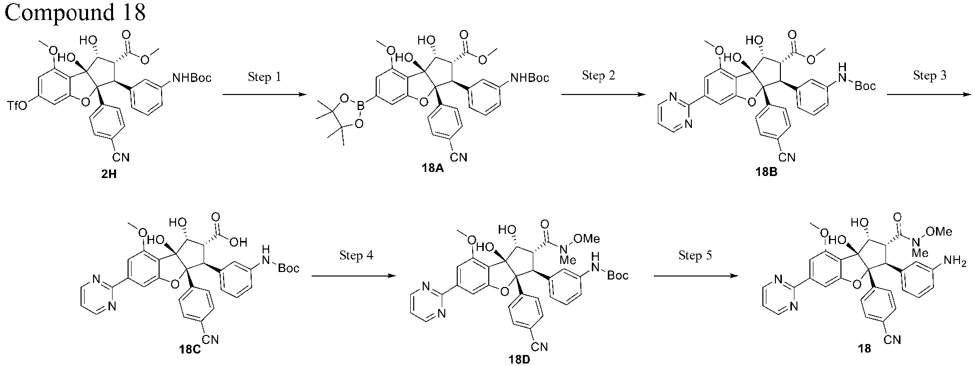

Step 1: Bis(pinacolato)diboron (35.2 mg, 0.14 mmol), potassium acetate (20.4 mg, 0.21 mmol), and Pd(dppf)Cl₂ (5.62 mg, 0.01 mmol) were added to a solution of the racemate of compound 2H (50 mg, 0.07 mmol) in dioxane (5 mL). The mixture was degassed under reduced pressure, purged with nitrogen 3 times, and then stirred at 85 °C for 10 h. The reaction solution was concentrated, and the residue was purified on a silica gel column (PE/EA = 5/1) to give a racemate of compound 18A (40.0 mg, yield: 83%) as a yellow solid. LC-MS: (M+H)⁺ = 699.3. ¹H NMR: (400 MHz, CDCl₃) δ 7.36 (dd, J = 8.4, 28.4 Hz, 4H), 7.22 (s, 1H), 7.14(d, J = 7.2 Hz,1H), 6.99 - 6.95 (m, 2H), 6.86 (s, 1H), 6.53 (d, J=7.6 Hz, 1H), 6.30 (s, 1H), 5.01 (d, J = 6.0 Hz, 1H), 4.36 (d, J = 14.4 Hz, 1H), 3.95 (s, 3H), 3.66 (s, 3H), 3.53 (s, 1H), 1.51 (s, 9H), 1.37 (s, 12H).

Step 2: 2-Bromopyrimidine (9.1 mg, 0.09 mmol), Na₂CO₃ (18.2 mg, 0.17 mmol), and Pd(dppf)Cl₂ (4.62 mg, 0.01 mmol) were added to a solution of the racemate of compound 18A (40 mg, 0.06 mmol) in dioxane/H₂O (5 mL/1 mL), and the mixture was heated by microwave to 100 °C and stirred for 1 h under a nitrogen atmosphere. The reaction solution was concentrated, and the residue was purified by flash column chromatography (ACN/H₂O) to give a racemate of compound 18B (30.0 mg, yield: 80.5%) as a white solid. LC-MS: (M+H)⁺ = 651.0. ¹H NMR: (400 MHz, CDCl3) δ 8.92 (d, J = 4.8 Hz, 2H), 7.92 (s, 1H), 7.86 (s, 1H), 7.40 (dd, J = 8.4, 22.0 Hz, 5H), 7.14 (d, J = 7.6 Hz, 1H), 7.01 - 6.96 (m, 1H), 6.91 (s, 1H), 6.57 (d, J=7.6 Hz, 1H), 6.31 (s, 1H), 5.05 (d, J = 6.0 Hz, 1H), 4.45 (d, J = 14.0 Hz, 1H), 4.07 (s, 3H), 3.97 (dd, J = 6.0, 14.0 Hz, 1H), 3.69 (s, 1H), 1.52 (s, 9H).

Step 3: The racemate of compound 18B (30 mg, 0.05 mmol) and LiOH (20.2 mg, 0.23 mmo) were dissolved in THF/H₂O (1 mL/1 mL), and the solution was stirred at room temperature for 6 h. The reaction solution was purified by flash column chromatography (ACN/H₂O) to give a racemate of compound 18C (21.0 mg, yield: 69.0%) as a white solid. LC-MS: [M+H-C₄H₈]⁺ = 581.0.

Step 4: The racemate of compound 18C (20.0 mg, 0.03 mmol), N,O-dimethylhydroxylamine hydrochloride (3.22 mg, 0.03 mmol), HATU (14.3 mg, 0.04 mmol), HOAT (2.25 mg, 0.03 mmol), and TMP (11.4 mg, 0.09 mmol) were added to DMF (5.0 mL). The mixture was stirred for 16 h and purified by flash column chromatography (ACN/H₂O) to give a racemate of compound 18D (20.0 mg, yield: 93.6%) as a white solid. LCMS [M+1]⁺ = 680.1.

Step 5: HCl (6 N, 1 mL) was added to a solution of the racemate of compound 18D (43.0 mg, 0.08 mmol) in THF (1 mL), and the mixture was stirred at room temperature overnight. The reaction solution was purified by flash column chromatography (ACN/H₂O) to give a racemate of compound 18 (70 mg, yield: 41%), which was further resolved on a chiral column to give compound 18 as a white solid. LCMS [M+H]⁺ = 580.0.

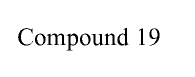

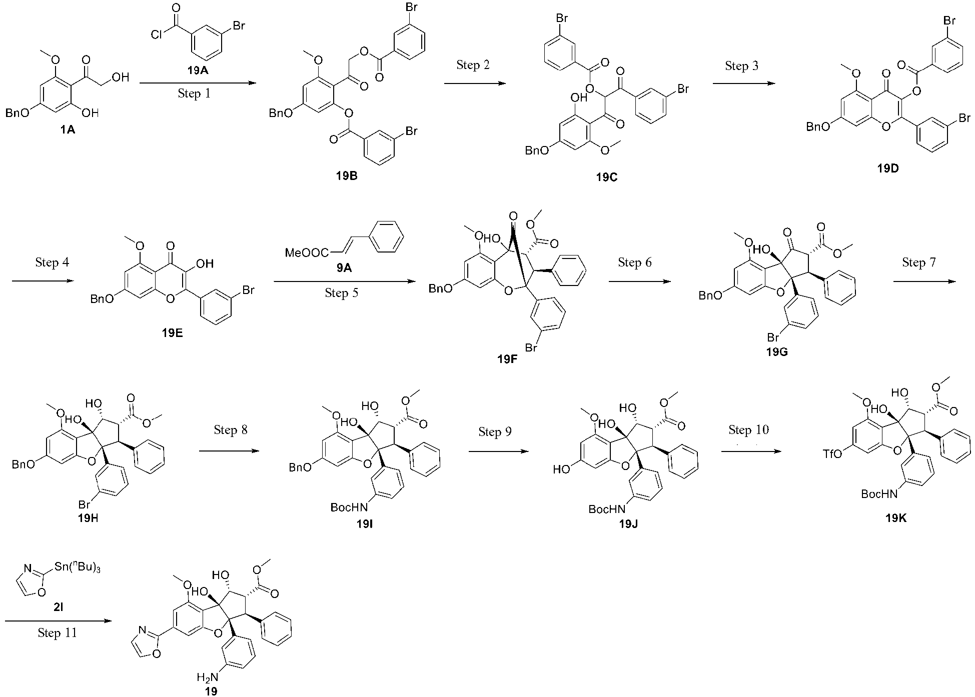

Step 1: Compound 1A (3.5 g, 12.1 mmol), DMAP (148 mg, 1.21 mmol), and TEA (3667 mg, 36.3 mmol) were dissolved in DCM (40 mL). The solution was cooled to 0 °C, and compound 19A (5860 mg, 26.7 mmol) was added in portions. The mixture was stirred at 25 °C for 1 h. The solution was concentrated and extracted with EA, then washed with water and brine, dried over anhydrous Na₂SO₄, and purified on a silica gel column (PE:EA = 3/1) to give 19B (5 g, yield: 63%) as a yellow solid. LCMS: [M+H]⁺ = 655.0.

Step 2: Compound 19B (5 g, 7.64 mmol) was dissolved in THF (50 mL), and the solution was cooled to -78 °C. Under a N₂ atmosphere, LiHMDS (23 mL, 23 mmol) was added dropwise to the above stirred solution, and the mixture was stirred at -78 °C for 2 h. The reaction solution was quenched with a saturated aqueous NH₄Cl solution, extracted with EA, and washed with water and saturated brine. The organic layer was dried over anhydrous Na₂SO₄, concentrated to dryness by rotary evaporation, and purified on a silica gel column (PE:EA = 3/1) to give 19C (4.1 g, yield: 82%) as a yellow solid. LCMS: [M+H]⁺ = 654.9.

Step 3: Compound 19C (6.1 g, 9.32 mmol) and AcONa (1912 mg, 23.31 mmol) were dissolved in AcOH (183 mL), and the solution was stirred at 100 °C overnight. TLC showed that no starting material remained and a new spot was formed. The solution was concentrated to dryness by rotary evaporation, then extracted with EA, washed with water and brine, dried over anhydrous Na₂SO₄, concentrated, and purified on a silica gel column (PE:EA = 1/1) to give 19D (4 g, yield: 67%) as a yellow solid. LCMS: [M+H]⁺ = 636.9.

Step 4: Compound 19D (4 g, 6.29 mmol) was dissolved in CHCl₃ (120 mL). The solution was cooled to 0 °C, and MeONa (3396 mg, 18.86 mmol) was added. The mixture was stirred at 25 °C for 1 h. TLC showed that no starting material remained and a new spot was formed. The reaction solution was adjusted to pH = 5 with acetic acid, concentrated to dryness by rotary evaporation, extracted with EA, and washed with MeOH to give a filter cake, which was 19E (2.5 g, yield: 88%) as a gray solid.

LCMS: [M+H]⁺ = 453.0.

¹H NMR (400 MHz, CDCl₃) δ 8.33 (s, 1H), 8.17 (d, J= 7.9 Hz, 1H), 7.55 *(d, J=* 7.9 Hz, 1H), 7.48 - 7.34 (m, 6H), 6.66 (d, *J =* 1.4 Hz, 1H), 6.44 (s, 1H), 5.17 (s, 2H), 3.97 (s, 3H).

Step 5: Compound 19E (1 g, 2.2 mmol) and compound 9A (2.1 g, 13.25 mmol) were dissolved in CHCl₃/TFE (7/3, 15 mL). The reaction mixture was injected at a rate of 12 mL/h via a syringe using a syringe pump into a coil wound around a jacketed glass cylinder equipped with a 250 W ultraviolet lamp. The temperature of the external circulation cold trap was adjusted to maintain the internal reaction temperature at 0-5 °C, and the UV lamp was turned on simultaneously. The reaction mixture was irradiated for 1 h and then concentrated under reduced pressure to remove the solvent. The residue was purified on a silica gel column (EtOAc/hexane) to remove excess cinnamate to give a racemate of compound 19F (2.9 g, yield: 64%) as a pale yellow solid. LCMS [M+H+H2O]⁺ = 633.1.

Step 6: A mixture of the racemate of compound 19F (2.96 g, 4.81 mmol) in MeOH (50 mL) was added to MeONa (649.76 g, 12.03 mmol). The mixture was stirred at 65 °C for 2 h, concentrated and extracted with EA, then washed with a saturated NH₄Cl solution and brine, dried over anhydrous Na₂SO₄, and separated and purified on a silica gel column (0.5% FA, EA/PE = 31/69) to give a racemate of compound 19G (1.2 g, yield: 75%) as a pale yellow solid. LCMS [M+H]⁺ = 597.1.

Step 7: The racemate of compound 19G (1.7 g, 2.76 mmol) was mixed with MeCN/CHCl₃ (1/1, 50 mL/50 mL), and Na(AcO)₃BH (2.9 g, 13.82 mmol) and AcOH (1.7 g, 27.64 mmol) were added at 0 °C. The mixture was stirred at room temperature for 2 h. The solution was concentrated and extracted with EA, then washed with water and brine, dried over anhydrous Na₂SO₄, and purified on a silica gel column (A: PE, B: DCM/EA = 1/1) to give a racemate of compound 19H (919 mg, yield: 54%).

LCMS [M+H-H₂O]⁺ = 599.0.

¹H NMR (400 MHz, CDCl₃) δ 7.49 - 7.32 (m, 6H), 7.24 (ddd, *J =* 7.9, 2.0, 1.0 Hz, 1H), 7.15 - 7.11 (m, 1H), 7.10 - 7.05 (m, 3H), 7.00 (dd, *J =* 9.1, 6.7 Hz, 1H), 6.89 (dd, *J =* 7.0, 2.2 Hz, 2H), 6.38 (d, *J =* 1.9 Hz, 1H), 6.23 (d, *J =* 1.9 Hz, 1H), 5.09 (s, 2H), 5.02 (d, *J =* 6.5 Hz, 1H), 4.36 (d, *J= 14.2* Hz, 1H), 3.95 - 3.88 (m, 1H), 3.86 (s, 3H), 3.66 (d, *J =* 3.6 Hz, 3H).

Step 8: The racemate of compound 19H (300.0 mg, 0.49 mmol), NH₂Boc (170.7 mg, 1.46 mmol), Pd₂(dba)₃ (44.5 mg, 0.05 mmol), Xantphos (56.2 mg, 0.10 mmol), and Cs₂CO₃ (316.0 mg, 0.97 mmol) were mixed in 1,4-dioxane (20 mL), and the mixture was stirred at 100 °C for 16 h under N₂. The reaction solution was concentrated and purified on a silica gel column (PE/EA) to give a racemate of compound 19I (219 mg, yield: 69%).

LCMS [M-H]⁻ = 652.2.

¹H NMR (400 MHz, CDCl₃) δ 7.48 - 7.35 (m, 6H), 7.10 - 7.03 (m, 4H), 6.96 (t, *J =* 1.8 Hz, 1H), 6.92 - 6.83 (m, 3H), 6.36 (d, *J =* 1.9 Hz, 1H), 6.22 (d, *J=* 1.9 Hz, 1H), 5.09 (s, 2H), 5.03 (d, *J =* 6.7 Hz, 1H), 4.33 (d, *J =* 14.2 Hz, 1H), 3.95 (dd, *J =* 14.2, 6.7 Hz, 1H), 3.86 (s, 3H), 3.65 (d, *J =* 3.7 Hz, 3H), 1.49 (s, 9H).

Step 9: Pd(OH)₂/C (10%, 200 mg) was added to a solution of the racemate of compound 19I (219.0 mg, 0.34 mmol) in EA/MeOH/THF/DCM (20:2:2:0.2, 10 mL). The mixture was purged with H₂ multiple times and stirred at room temperature for 16 h. The suspension was filtered, and the filter cake was washed with MeOH/DCM (1/10). The filtrates were mixed, concentrated, and dried to give a racemate of compound 19J (180 mg, yield: 95%).

¹H NMR (400 MHz, CDCl₃) δ 7.23 - 7.02 (m, 7H), 6.95 - 6.86 (m, 3H), 6.47 (s, 1H), 6.11 (d, *J=* 1.5 Hz, 1H), 5.91 (s, 1H), 4.99 (d, *J =* 7.1 Hz, 1H), 4.31 (d, *J =* 14.1 Hz, 1H), 3.97 (dd, *J =* 14.1, 7.1 Hz, 1H), 3.74 (s, 3H), 3.66 (s, 3H), 1.50 (s, 9H).

LCMS [M+Na]⁺ = 586.1.

Step 10: The racemate of compound 19J (180 mg, 0.32 mmol) and K₂CO₃ (88 mg, 0.64 mmol) were stirred in DMF (10 mL). N-Phenylbis(trifluoromethanesulfonimide) (114 mg, 0.32 mmol) was added, and the mixture was stirred at 60 °C for 20 min. The solution was concentrated and extracted with EA, then washed with water and brine, dried over anhydrous Na₂SO₄, and purified on a silica gel column (DCM/MeOH = 10/1) to give a racemate of compound 19K (190 mg, yield: 86%). LCMS: [M+Na]⁺ = 718.0.

Step 11: Compound 2I (288.55 mg, 0.8 mmol), Pd(PPh₃)₄ (11.6 mg, 0.01 mmol), and CuI (3.84 mg, 0.02 mmol) were added to a solution of the racemate of compound 19K (70 mg, 0.1 mmol) in DMF (3 mL). The reaction solution was purged with nitrogen three times, then allowed to react in a microwave reactor at 100 °C for 1.5 h, and purified by reversed-phase column chromatography (H₂O:ACN = 0-100%) to give a racemate of compound 19 (2.3 mg, yield: 5%), which was further resolved on a chiral column to give compound 19 as a white solid.

LCMS [M+H]⁺ = 515.0.

¹H NMR (400 MHz, DMSO) δ 8.25 (s, 1H), 7.41 (s, 1H), 7.21 - 7.10 (m, 2H), 7.10 - 6.89 (m, 5H), 6.64 (t, *J =* 7.9 Hz, 1H), 6.48 (s, 1H), 6.26 (d, *J =* 8.0 Hz, 1H), 6.17 (d, *J =* 6.4 Hz, 1H), 5.31 (d, *J* = 5.2 Hz, 1H), 5.25 (s, 1H), 4.70 (t, *J =* 5.2 Hz, 2H), 4.20 (d, *J =* 14.1 Hz, 1H), 4.01 (dd, *J =* 14.0, 5.2 Hz, 1H), 3.85 (s, 3H), 3.55 (s, 3H).

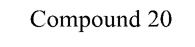

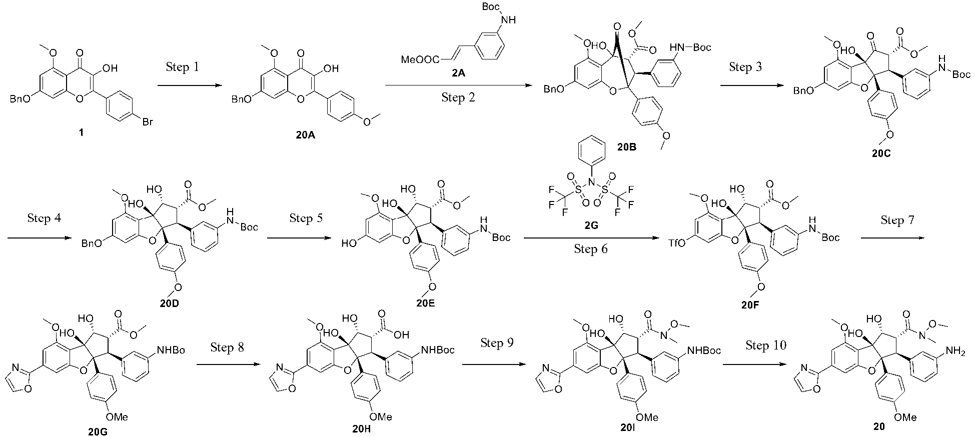

Step 1: Compound 1 (1000 mg, 2.21 mmol) and Cs₂CO₃ (1.08 g, 3.32 mmol) were dissolved in anhydrous methanol (10 mL) and anhydrous toluene (30 mL), and a solution of t-BuXPhosPdG3 (35.13 mg, 0.04 mmol) was added. The mixture was stirred at 85 °C for 13 h. The solution was concentrated and diluted with MeOH/DCM (1/1), and then filtered. The filtrate was concentrated and purified on a silica gel column (DCM/MeOH = 10/1) to give 20A (0.65 g, yield: 73%) as a yellow solid.

Step 2: Compound 20A (1 g, 2.2 mmol) and compound 2A (4.1 g, 13.3 mmol) were dissolved in CHCl₃/TFE (9/1, 15 mL). The reaction mixture was then injected at a rate of 12 mL/h via a 20 mL syringe using a syringe pump into a coil wound around a jacketed glass cylinder equipped with a 250 W ultraviolet lamp. The temperature of the external circulation cold trap was adjusted to maintain the internal reaction temperature at 0-5 °C, and the UV lamp was turned on simultaneously. The reaction mixture was irradiated for 1 h, concentrated under reduced pressure to remove the solvent, and then separated by column chromatography (EA/PE = 1/3) to remove excess cinnamate, thus giving a racemate of the desired compound 20B (3.9 g, yield: 80%) as a yellow oily crude product. LCMS [M+H]⁺ = 682.3.

Step 3: The racemate of compound 20B (3.7 g, 5.43 mmol) was mixed with MeOH (50 mL), and NaOMe (733.5 mg, 13.58 mmol) was added at 0 °C. The mixture was stirred at 65 °C for 1.5 h. The solution was concentrated, washed with H₂O, NH₄Cl (aq.), and brine, then dried over Na₂SO₄, concentrated under reduced pressure, and purified on a silica gel column (0.2% FA, EA/PE = 1/3) to give a racemate of compound 20C (2.5 g, yield: 67%). LCMS [M+H-H₂O]⁺ = 664.2.

Step 4: The racemate of compound 20C (2.5 g, 3.67 mmol) was dissolved in MeCN/CHCl₃ (1/1, 50 mL/50 mL), and Na(AcO)₃BH (3.9 g, 18.36 mmol) and AcOH (2.2 g, 36.71 mmol) were added at 0 °C. The mixture was stirred at room temperature for 2 h. The reaction solution was extracted with EA, washed with water and brine, dried over anhydrous Na₂SO₄, and purified on a silica gel column (A: PE, B: EA = 1/1) to give a racemate of compound 20D (1.5 g, yield: 60%). LCMS [M+H-H₂O]⁺ = 666.3.

Step 5: Pd(OH)₂/C (10%, 100 mg) was added to a solution of the racemate of compound 20D (200 mg, 0.29 mmol) in EA/MeOH/THF/DCM (20:2:2:2:0.2, 10 mL). The mixture was purged with H₂ 3 times and stirred at room temperature for 16 h. The suspension was filtered, and the filter cake was washed with MeOH/DCM (1/10). The filtrate was concentrated and purified on a silica gel column (DCM/MeOH = 10/1) to give a racemate of compound 20E (155 mg, yield: 89%). LCMS [M+Na]⁺ = 616.1.

Step 6: The racemate of compound 20E (155.0 mg, 0.26 mmol) and K₂CO₃ (72.1 mg, 0.52 mmol) were stirred in DMF (2 mL). Compound 2G (93.3 mg, 0.26 mmol) was added. The reaction solution was stirred at 60 °C for 20 min, then diluted with water, extracted with EA, washed with water and brine, dried over anhydrous Na₂SO₄, and purified on a silica gel column (EA/PE = 1/1) to give a racemate of compound 20F (160 mg, yield: 84%). LCMS: [M+Na]⁺ = 748.1.

Step 7: The racemate of compound 20F (50 mg, 0.07 mmol) was dissolved in DMF (1.5 mL), and 2-(tributylstannyl)oxazole (190 mg, 0.54 mmol), tetrakis(triphenylphosphine)palladium (8 mg, 0.007 mmol), and CuI (2.6 mg, 0.014 mmol) were sequentially added to the solution. The solution was stirred under microwave at 100 °C for 1.5 h. The reaction mixture was filtered, washed with MeOH (5 mL), and concentrated. The residue was purified by reversed-phase chromatography (acetonitrile/water = 5%-95%, 214 nm, 20 min) to give a racemate of 20G (17 mg, yield: 38%) as a yellow solid. LCMS: [M+Na]⁺ = 667.2.

Step 8: The racemate of compound 20G (35 mg, 0.05 mmol) was dissolved in tetrahydrofuran/water (3 mL/1 mL), and then lithium hydroxide (4.56 mg, 0.1 mmol) was added to the solution. The reaction mixture was stirred at room temperature for 2 h, then concentrated, and purified by reversed-phase chromatography (acetonitrile/water = 5%-95%, 214 nm, 20 min) to give a racemate of 20H (21 mg, yield: 61%) as a yellow solid. LCMS: [M+Na]⁺ = 653.2.

Step 9: The racemate of compound 20H (21 mg, 0.03 mmol), N,O-dimethylhydroxylamine (3.25 mg, 0.06 mmol), HATU (17.1 mg, 0.05 mmol), and DIEA (11.61 mg, 0.09 mmol) were added to DMF (1.5 mL). The reaction mixture was stirred at room temperature for 2 h. The reaction solution was purified by reversed-phase chromatography (acetonitrile/water = 5%-95%, 214 nm, 20 min) to give a racemate of 20I (12 mg, yield: 54%) as a yellow solid. LCMS: [M+Na]⁺ = 696.3.

Step 10: 6 N/HCl (0.3 mL) was added to a solution of the racemate of compound 20I (12 mg, 0.02 mmol) in tetrahydrofuran (0.5 mL). The mixture was stirred at room temperature for 12 h and then purified by preparative chromatography to give a racemate of 20 (3.2 mg, yield: 22%) as a white solid, which was further resolved on a chiral column to give compound 20. LCMS: [M+H]⁺ = 574.2.

1H NMR (400 MHz, DMSO-*d*₆) δ 8.24 (d, *J =* 0.8 Hz, 1H), 7.40 (d, *J =* 0.8 Hz, 1H), 7.17 (d, *J =* 1.2 Hz, 1H), 7.14 (d, *J =* 1.2 Hz, 1H), 7.08-7.05 (m, 2H), 6.67-6.63 (m, 3H), 6.23 (brs, 1H), 6.16 (d, *J =* 9.2 Hz, 1H), 6.01 (d, *J =* 7.6 Hz, 1H), 5.30 (s, 1H), 4.86 (d, *J =* 4.4 Hz, 1H), 4.80-4.79 (m, 3H), 4.15-4.08 (m, 2H), 3.87 (s, 3H), 3.85 (s, 3H), 3.63 (s, 3H), 3.06 (s, 3H).

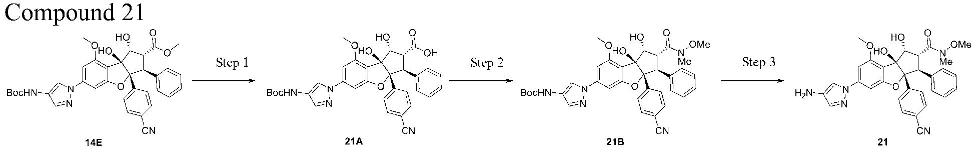

Step 1: An aqueous LiOH solution (12 mg, 0.3 mmol, in 0.5 mL of H₂O) was added to a solution of the racemate of compound 14E (60 mg, 0.1 mol) in THF (2 mL). The reaction solution was stirred at room temperature for 18 h and then purified by reversed-phase column chromatography (H₂O:ACN = 0-100%) to give a racemate of 21A (45 mg, yield: 77.5%) as a white solid. LCMS: [M+H]⁺ = 625.2.

Step 2: TMP (35 mg, 0.28 mmol) was added to a solution of the racemate of compound 21A (45 mg, 0.07 mmol), N,O-dimethylhydroxylamine (14 mg, 0.14 mmol), HATU (54.72 mg, 0.14 mmol), and HOAT (19.58 mg, 0.14 mmol) in DMF (4 mL). The reaction solution was stirred at 50 °C for 18 h and then purified by normal-phase column chromatography (DCM/MeOH = 10/1) to give a racemate of 21B (24 mg, yield: 50%) as a white solid. LCMS: [M+H]⁺ = 668.2.

Step 3: 6 M HCl (2 mL) was added to a solution of the racemate of compound 21B (24 mg, 0.03 mmol) in THF (1 mL). The reaction solution was stirred at room temperature for 18 h and then purified by reversed-phase column chromatography (H₂O:ACN = 0-100%) to give a racemate of 21 (14 mg, yield: 70%) as a white solid, which was further resolved on a chiral column to give compound 21.

LCMS [M+H]⁺ = 568.2.

¹H NMR (400 MHz, DMSO) δ 7.78 (s, 1H), 7.52 (d, *J =* 8.6 Hz, 2H), 7.34 - 7.24 (m, 3H), 7.06 (t, *J =* 7.4 Hz, 2H), 6.99 (t, *J =* 4.4 Hz, 2H), 6.91 (dd, *J =* 18.8, 4.5 Hz, 3H), 5.50 (s, 1H), 5.01 (d, *J =* 4.8 Hz, 1H), 4.80 (t, *J =* 5.1 Hz, 1H), 4.43 (d, *J =* 13.8 Hz, 1H), 4.19 (s, 3H), 3.91 (s, 3H), 3.81 (s, 2H), 3.09 (s, 3H).

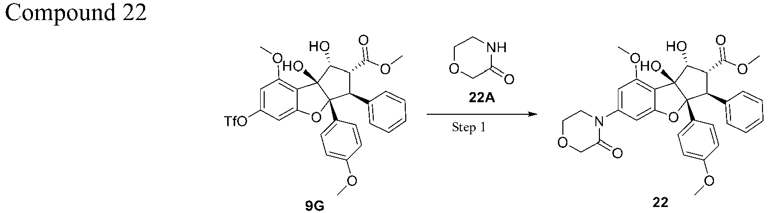

Step 1: The racemate of compound 9G (50.0 mg, 0.08 mmol), compound 22A (24.8 mg, 0.25 mmol), t-BuXPhos PdG3 (13.0 mg, 0.02 mmol), t-Bune phos (7.7 mg, 0.02 mmol), and Na₂CO₃ (26.1 mg, 0.25 mmol) were dissolved in 1,4-dioxane (5 mL). The reaction solution was stirred at 100 °C for 16 h under a N₂ atmosphere, then concentrated, and purified by preparative high-performance liquid chromatography to give a racemate of 22 (1.2 mg, yield: 6.7%) as a white solid, which was further resolved on a chiral column to give compound 22.

LCMS [M+H]⁺ = 562.2.

¹H NMR (400 MHz, DMSO) δ 7.11 - 6.94 (m, 5H), 6.89 (d, *J =* 7.4 Hz, 2H), 6.68 (d, *J =* 1.5 Hz, 1H), 6.62 - 6.54 (m, 3H), 5.29 (s, 1H), 5.26 (d, *J =* 5.0 Hz, 1H), 4.71 (t, *J =* 5.2 Hz, 1H), 4.23 - 4.15 (m, 3H), 4.00 - 3.92 (m, 3H), 3.78 (dd, *J =* 11.2, 6.2 Hz, 1H), 3.74 (s, 3H), 3.60 (s, 3H), 3.55 (s, 3H).

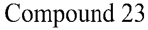

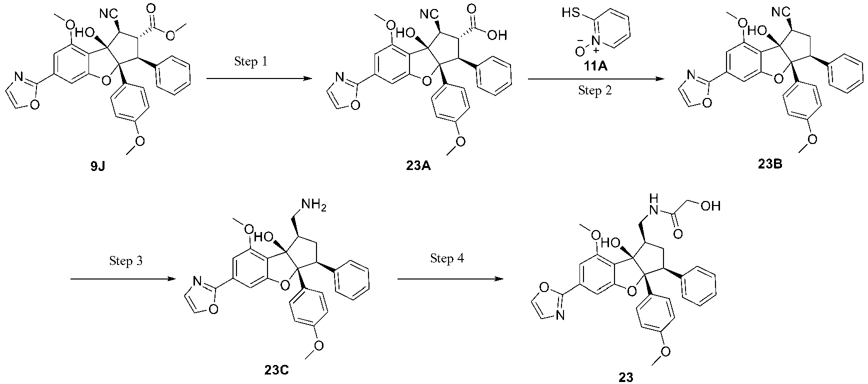

Step 1: An aqueous solution (0.5 mL) of LiOH (47 mg, 1.12 mmol) was added to a solution of the racemate of compound 9J (200 mg, 0.37 mol) in THF (5 mL). The reaction solution was stirred at room temperature for 18 h and then purified by reversed-phase column chromatography (H₂O:ACN = 0-100%) to give a racemate of 23A (180 mg, yield: 92%) as a white solid. LCMS [M+H]⁺ = 525.3.

Step 2: A solution of the racemate of compound 23A (10 mg, 0.02 mmol), compound 11A (24.3 mg, 0.2 mmol), tert-dodecyl mercaptan (38.4 mg, 0.2 mmol), and Zinc meso-tetraphenylporphyrin (1.3 mg, 0.001 mmol) in DMF (1 mL) was added to a microwave tube. The microwave tube was placed in a water bath at 80 °C and irradiated with a 25 W red light lamp. EDCI (36.6 mg, 0.2 mmol, dissolved in DMF) was added dropwise to the reaction system. The mixture was then stirred for 30 min. Three parallel reactions were set up. The reaction solutions were combined and purified by reversed-phase column chromatography (H₂O:ACN = 0-100%) to give a racemate of 23B (10 mg, yield: 30%) as a white solid. LCMS [M+H]⁺ = 481.2.

Step 3: A solution of the racemate of compound 23B (30 mg, 0.06 mmol) in THF (2 mL) was cooled to 0 °C. LiAlH₄ (30 µL, 0.12 mmol) was added dropwise to the reaction system, and then the mixture was allowed to react at 0 °C for 1 h. Na₂SO₄·10H₂O was added to the reaction system, and then the mixture was stirred at room temperature for half an hour. The suspension was filtered, and the filter cake was washed with MeOH/DCM (1/10) three times. The resulting filtrate was concentrated to dryness by rotary evaporation and purified by thin-layer chromatography (DCM/MeOH = 7/1) to give a racemate of 23C (5 mg, yield: 16.7%) as a white solid. LCMS [M+H]⁺ = 485.2.

Step 4: TMP (4 mg, 0.032 mmol) was added to a solution of the racemate of compound 23C (4 mg, 0.008 mmol), glycolic acid (1.2 mg, 0.008 mmol), HATU (6.4 mg, 0.016 mmol), and HOAT (2.4 mg, 0.016 mmol) in DMF (1 mL), and the mixture was allowed to react at room temperature for 1 h. The reaction solution was purified by reversed-phase column chromatography (H₂O:ACN = 0-100%) to give a racemate of 23 (2.2 mg, yield: 50%) as a white solid, which was further resolved on a chiral column to give compound 23.

LCMS [M+H-H₂O]⁺ = 525.0.

¹H NMR (400 MHz, DMSO) δ 8.26 (d, *J =* 0.6 Hz, 1H), 7.79 (t, *J =* 5.7 Hz, 1H), 7.41 (d, *J =* 0.7 Hz, 1H), 7.22 (dd, *J=* 8.9, 1.1 Hz, 2H), 7.14 (d, *J =* 8.9 Hz, 2H), 7.11 - 7.00 (m, 3H), 6.91 *(d, J=* 6.8 Hz, 2H), 6.69 (d, *J =* 9.0 Hz, 2H), 5.64 (t, *J =* 5.5 Hz, 1H), 5.42 (s, 1H), 3.96 (s, 3H), 3.91 (d, *J= 5.3* Hz, 2H), 3.70 (t, *J =* 5.3 Hz, 2H), 3.65 (s, 3H), 3.39 (d, *J =* 6.2 Hz, 1H), 2.90 (td, *J =* 14.0, 6.8 Hz, 1H), 2.24 - 2.01 (m, 2H).

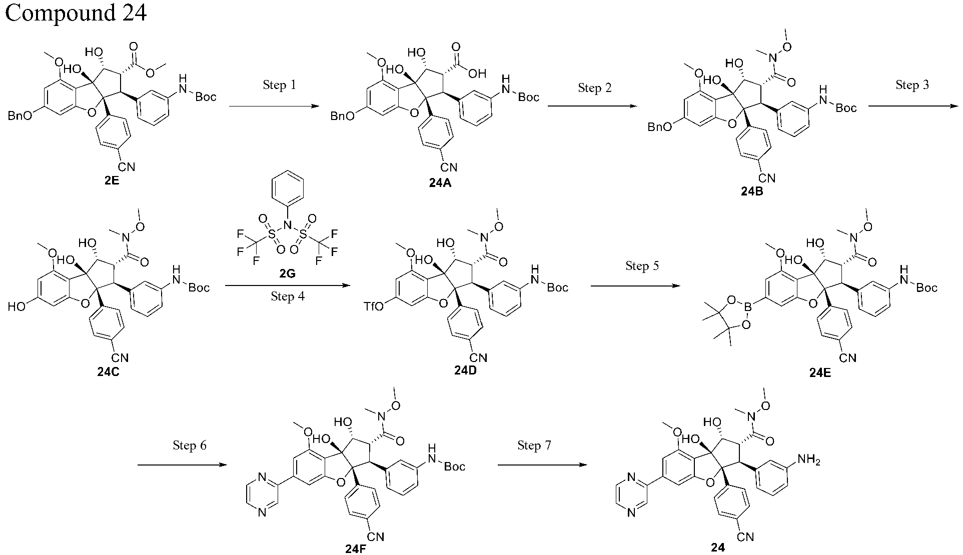

Step 1: The racemate of compound 2E (3200 mg, 4.715 mmol) was dissolved in THF (30 mL), MeOH (10 mL), and H₂O (10 mL), and LiOH (594 mg, 14.14 mmol) was added. The mixture was stirred at room temperature for 8 h under a nitrogen atmosphere. The reaction mixture was adjusted to pH = 5 with 3 M HCl and concentrated to give a crude product, which was then subjected to silica gel column chromatography (DCM/MeOH = 10/1) to give a racemate of 24A (2.8 g, yield: 89%) as a white solid. LCMS [M+H]⁺ = 665.2.

Step 2: The racemate of compound 24A (2800 mg, 4.2124 mmol) was dissolved in DMF (120 mL), and dimethylhydroxylamine hydrochloride (1233 mg, 12.6371 mmol), HATU (4802 mg, 12.6371 mmol), HOAT (1719 mg, 12.6371 mmol), and TMP (1539 mg, 12.6371 mmol) were added. The mixture was heated to 50 °C and stirred for 16 h under a nitrogen atmosphere. Water (100 mL) was added to the reaction solution, and the mixture was extracted with ethyl acetate (150 mL) twice. The organic phases were combined, washed with saturated brine (150 mL), dried over anhydrous sodium sulfate, filtered, and concentrated to give a crude product, which was then purified by silica gel column chromatography (ethyl acetate/petroleum ether = 1/10) to give a racemate of 24B (2.9 g, yield: 97%) as a yellow solid. LCMS [M+H-C₄H₈]⁺ = 652.2.

Step 3: The racemate of compound 24B (2900 mg, 4.10 mmol) was dissolved in a mixed solution (40 mL) of EA/MeOH/THF/DCM (20:2:2:0.2), and Pd(OH)₂ (870 mg) was added. The mixture was stirred at room temperature for 16 h under a hydrogen atmosphere. The reaction solution was filtered and concentrated to give a crude product, which was then purified by silica gel column chromatography (DCM/MeOH = 10/1) to give a racemate of 24C (1.8 g, yield: 71%) as a yellow solid. LCMS [M+H-C₄H₈]⁺ = 562.2.

Step 4: The racemate of compound 24C (1800 mg, 2.9142 mmol) was dissolved in DMF (50 mL), and compound 2G (1041 mg, 2.9142 mmol) and K₂CO₃ (804 mg, 5.8284 mmol) were added. The mixture was stirred at 60 °C for 40 min under a nitrogen atmosphere. The reaction solution was filtered, and water (100 mL) was added to the filtrate. The mixture was extracted with ethyl acetate (150 mL) twice. The organic phases were combined, washed with saturated brine (150 mL), dried over anhydrous sodium sulfate, filtered, and concentrated to give a crude product, which was then purified by silica gel column chromatography (EA/PE = 1/1) to give a racemate of 24D (1.6 g, yield: 74%) as a yellow oil. LCMS [M+H-C₄H₈]⁺ = 694.1.

Step 5: The racemate of compound 24D (860 mg, 1.15 mmol) was dissolved in dioxane (12 mL), and bis(pinacolato)diboron (876 mg, 3.45 mmol), Pd(dppf)Cl₂ (188 mg, 0.23 mmol), and KOAc (432 mg, 4.60 mmol) were added. The mixture was stirred at 85 °C for 4 h under a nitrogen atmosphere. The reaction solution was filtered and concentrated to give a crude product, which was then purified by silica gel column chromatography (EA/PE = 1/1) to give a racemate of 24E (700 mg, yield: 84%) as a yellow oil. LCMS [M+H-C₄H₈]⁺ = 672.2.

Step 6: The racemate of compound 24E (20 mg, 0.0300 mmol) was dissolved in dioxane (1.5 mL) and water (0.3 mL), and 2-bromopyrazine (8.74 mg, 0.0500 mmol), Pd(dppf)Cl₂ (2.24 mg, 0.0030 mmol), and Na₂CO₃ (11.65 mg, 0.1099 mmol) were added. The mixture was allowed to react under microwave at 100 °C for 1 h under a nitrogen atmosphere. The reaction solution was filtered and concentrated to give a racemate of 24F (17 g, yield: 84%) as a yellow solid. LCMS [M+H]⁺ = 680.4.

Step 7: The racemate of compound 24F (17 mg, 0250 mmol) was dissolved in THF (1 mL), and 6 M HCl (1 mL) was added. The mixture was stirred at room temperature for 16 h under a nitrogen atmosphere. The reaction solution was filtered and concentrated to give a crude product. The crude product was then purified on a reversed-phase column (H₂O/ACN) to give a racemate of 24 (1.4 mg, yield: 9.6%) as a white solid, which was further resolved on a chiral column to give compound 24.

LCMS [M+H]⁺ = 580.2.

¹H NMR (400 MHz, DMSO) δ 9.38 (d, J = 0.7 Hz, 2H), 7.70 (d, J = 1.1 Hz, 1H), 7.66 (d, J = 1.0 Hz, 1H), 7.54 (d, J = 8.6 Hz, 2H), 7.33 (d, J = 8.6 Hz, 2H), 6.66 (t, J = 7.8 Hz, 1H), 6.23 (s, 1H), 6.16 (d, J = 7.8 Hz, 1H), 6.05 (d, J = 7.4 Hz, 1H), 5.65 (s, 1H), 5.09 (d, J = 4.9 Hz, 1H), 4.84 - 4.78 (m, 2H), 4.33 (d, J = 13.9 Hz, 1H), 4.13 (dd, J = 15.2, 9.8 Hz, 1H), 3.91 (s, 3H), 3.87 (s, 3H), 3.09 (s, 3H).

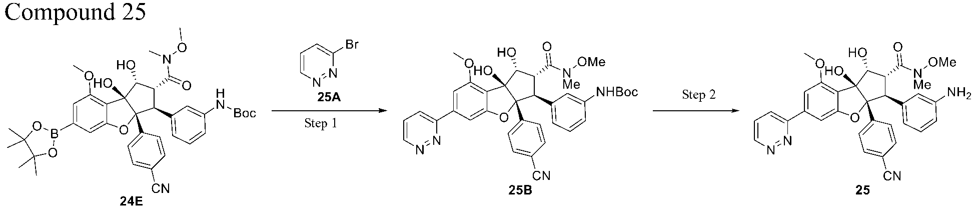

Step 1: The racemate of compound 24E (10 mg, 0.01 mmol) was dissolved in dioxane/water (5 mL/1 mL), and compound 25A (2.19 mg, 0.02 mmol), Na₂CO₃ (4.37 mg, 0.04 mmol), and Pd(dppf)Cl₂ (1.11 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 25B (12 mg) as a yellow solid, which was directly used in the next step. LCMS [M+H-C₄H₈]⁺ = 624.3.

Step 2: 6 N HCl (0.3 mL) was added to a solution of the racemate of compound 25B (12 mg, 0.02 mmol) in tetrahydrofuran (0.5 mL), and the mixture was stirred at room temperature for 12 h. The reaction solution was directly purified by preparative HPLC to give a racemate of 25 (6.3 mg, yield: 73%) as a white solid, which was further resolved on a chiral column to give compound 25. LCMS [M+H]⁺ = 580.0.

¹H NMR (400 MHz, DMSO-*d*₆) δ 9.25-9.23 (m, 1H), 8.33-8.31 (m, 1H), 7.83-7.80 (m, 1H), 7.55 (d, *J =* 8.4 Hz, 2H), 7.40 (d, *J =* 4.8 Hz, 2H), 7.34 (d, *J =* 8.8 Hz, 2H), 6.68 (t, *J =* 7.6 Hz, 1H), 6.22-6.17 (m, 2H), 6.08 (d, *J =* 6.0 Hz, 1H), 5.59 (s, 1H), 4.80 (d, *J* = 4.8 Hz, 1H), 4.33 (d, *J=* 14.0 Hz, 1H), 4.17-4.13 (m, 1H), 3.91 (s, 3H), 3.88 (s, 3H), 3.09 (s, 3H).

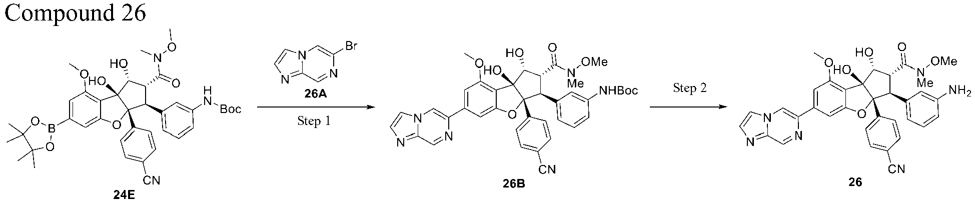

Step 1: The racemate of compound 24E (20 mg, 0.03 mmol) was dissolved in dioxane/water (5 mL/1 mL), and compound 26A (8.17 mg, 0.04 mmol), Na₂CO₃ (8.74 mg, 0.08 mmol), and Pd(dppf)Cl₂ (2.23 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 26B (19 mg) as a yellow solid, which was directly used in the next step. LCMS [M+H]⁺ = 719.3.

Step 2: 6 N HCl (0.3 mL) was added to a solution of the racemate of compound 26B (19 mg, 0.02 mmol) in tetrahydrofuran (0.5 mL), and the mixture was stirred at room temperature for 12 h. The reaction solution was directly purified by preparative HPLC to give a racemate of 26 (2.3 mg, yield: 17%) as a white solid, which was further resolved on a chiral column to give compound 26. LCMS [M+H]⁺ = 619.2.

¹H NMR (400 MHz, DMSO-*d*₆) δ 9.32 (d, *J=* 1.6 Hz, 1H), 9.19 (s, 1H), 8.14 (s, 1H), 7.88 (d, *J=* 0.8 Hz, 1H), 7.55 *(d, J=* 8.4Hz, 2H), 7.33 *(d, J=* 8.4Hz, 3H), 7.28 (s, 1H), 6.67 (t, *J* = 7.8Hz, 1H), 6.20-6.16 (m, 2H), 6.05 (d, *J=* 7.6 Hz, 1H), 5.53 (s, 1H), 4.98 (d, *J=* 4.4 Hz, 1H), 4.82-4.80 (m, 3H), 4.30 (d, *J =* 14.0Hz, 1H), 4.15-4.11 (m, 1H), 3.90 (s, 3H), 3.87 (s, 3H), 3.09 (s, 3H).

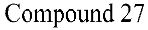

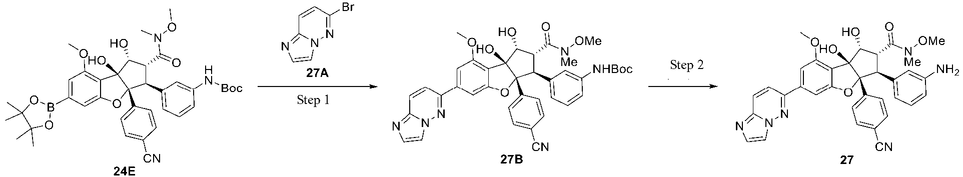

Step 1: The racemate of compound 24E (20 mg, 0.03 mmol) was dissolved in dioxane/water (5 mL/1 mL), and compound 27A (8.17 mg, 0.04 mmol), Na₂CO₃ (8.74 mg, 0.08 mmol), and Pd(dppf)Cl₂ (2.23 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 27B (18 mg) as a yellow solid, which was directly used in the next step. LCMS [M+H]⁺ = 719.3.

Step 2: 6 N HCl (0.3 mL) was added to a solution of the racemate of compound 27B (18 mg, 0.03 mmol) in tetrahydrofuran (0.5 mL), and the mixture was stirred at room temperature for 12 h. The reaction solution was directly purified by preparative HPLC to give a racemate of 27 (1.5 mg, yield: 9.7%) as a white solid, which was further resolved on a chiral column to give compound 27. LCMS [M+H]⁺ = 619.2.

¹H NMR (400 MHz, DMSO-*d*₆) δ 8.38 (s, 1H), 8.24 (d, *J =* 9.6 Hz, 1H), 7.91 (d, *J =* 9.6 Hz, 1H), 7.84 (s, 1H), 7.55 (d, *J =* 8.4 Hz, 2H), 7.35 (s, 2H), 7.32 (s, 1H), 7.23 (s, 1H), 6.67 (t, *J =* 7.8 Hz, 1H), 6.21 (s, 1H), 6.17 (d, *J* = 7.6 Hz, 1H), 6.07 (d, *J =* 7.6 Hz, 1H), 5.60 (s, 1H), 5.05 (d, *J* = 4.8 Hz, 1H), 4.82-4.79 (m, 3H), 4.33 (d, *J =* 14.0 Hz, 1H), 4.17-4.09 (m, 1H), 3.91 (s, 3H), 3.88 (s, 3H), 3.09 (s, 3H).

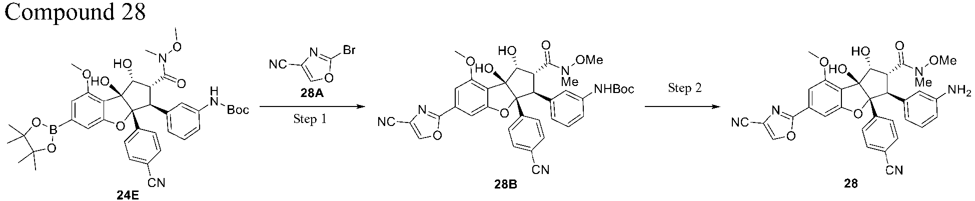

Step 1: The racemate of compound 24E (20 mg, 0.03 mmol) was dissolved in dioxane/water (3 mL/1 mL), and compound 28A (7.13 mg, 0.04 mmol), Na₂CO₃ (8.74 mg, 0.08 mmol), and Pd(dppf)Cl₂ (2.23 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of compound 28B (20 mg, crude product) as a yellow solid, which was directly used in the next step. LCMS [M+H-C₄H₈]⁺ = 638.2.

Step 2: 6 N HCl (0.5 mL) was added to a solution of the racemate of compound 28B (20 mg, 0.03 mmol) in tetrahydrofuran (0.5 mL), and the mixture was stirred at room temperature for 12 h. The reaction solution was directly purified by preparative HPLC to give a racemate of 28 (3.8 mg, yield: 22%) as a white solid, which was further resolved on a chiral column to give compound 28.

LCMS [M+H]⁺ = 594.0.

¹H NMR (400 MHz, DMSO-*d*₆) δ 9.25 (s, 1H), 7.53 (d, *J =* 8.4 Hz, 2H), 7.30 (d, *J =* 8.4 Hz, 2H), 7.24 (d, *J* = 1.2 Hz, 1H), 7.16 (d, *J =* 1.2 Hz, 1H), 6.66 (t, *J =* 15.2 Hz, 1H), 6.20 (s, 1H), 6.16 (d, *J=* 8.0 Hz, 1H), 6.06 (d, *J* = 7.6 Hz, 1H), 5.67 (s, 1H), 5.13 (d, *J =* 4.8 Hz, 1H), 4.83-4.76 (m, 3H), 4.34 (d, *J =* 14.0 Hz, 1H), 4.17-4.10 (m, 1H), 3.90 (s, 3H), 3.85 (s, 3H), 3.09 (s, 3H).

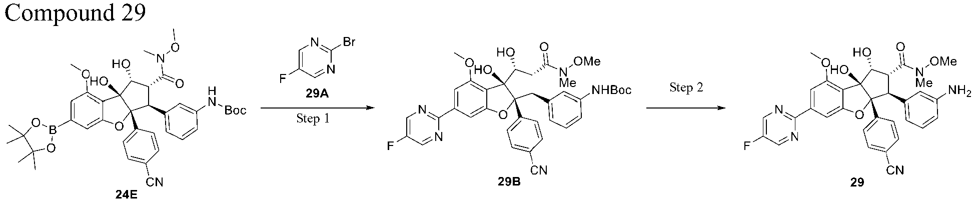

Step 1: The racemate of compound 24E (20 mg, 0.03 mmol) was dissolved in dioxane/water (3 mL/1 mL), and compound 29A (7.30 mg, 0.04 mmol), Na₂CO₃ (8.74 mg, 0.08 mmol), and Pd(dppf)Cl₂ (2.23 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 29B (20 mg) as a yellow solid, which was directly used in the next step. LCMS [M+H-C₄H₈]⁺ = 642.2.

Step 2: 6 N HCl (0.3 mL) was added to a solution of the racemate of compound 29B (20 mg, 0.03 mmol) in tetrahydrofuran (0.5 mL), and the mixture was stirred at room temperature for 12 h. The reaction solution was directly purified by preparative HPLC to give a racemate of 29 (1.5 mg, yield: 9%) as a white solid, which was further resolved on a chiral column to give compound 29. LCMS [M+H]⁺ = 598.2.

¹H NMR (400 MHz, DMSO-*d*₆) δ 9.01 (s, 2H), 7.57-7.53 (m, 4H), 7.32 (d, *J =* 8.8 Hz, 2H), 6.66 (t, *J =* 7.6 Hz, 1H), 6.23 (s, 1H), 6.17 (d, *J =* 12.0 Hz, 1H), 6.04 (d, *J =* 7.6 Hz, 1H), 5.59 (s, 1H), 5.03 (d, *J* = 4.8 Hz, 1H), 4.87-4.79 (m, 3H), 4.30 (d, *J =* 14.0 Hz, 1H), 4.16-4.09 (m, 1H), 3.90 (s, 3H), 3.85 (s, 3H), 3.09 (s, 3H).

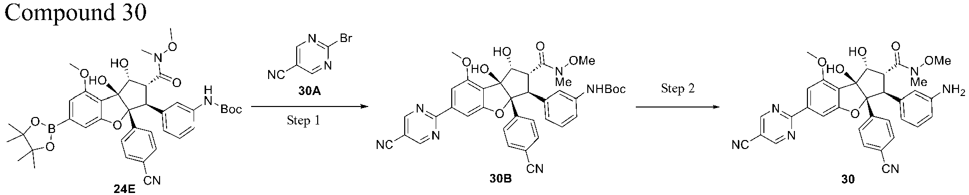

Step 1: The racemate of compound 24E (20 mg, 0.03 mmol) was dissolved in dioxane/water (3 mL/1 mL), and compound 30A (7.59 mg, 0.04 mmol), Na₂CO₃ (8.74 mg, 0.08 mmol), and Pd(dppf)Cl₂ (2.23 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 30B (20 mg) as a yellow solid, which was directly used in the next step. LCMS [M+H-C₄H₈]⁺ = 649.2.

Step 2: 6 N HCl (0.3 mL) was added to a solution of the racemate of compound 30B (20 mg, 0.03 mmol) in tetrahydrofuran (0.5 mL), and the mixture was stirred at room temperature for 12 h. The reaction solution was directly purified by preparative HPLC to give a racemate of 30 (1.1 mg, yield: 6.4%) as a white solid, which was further resolved on a chiral column to give compound 30. LCMS [M+H]⁺ = 605.2.

¹H NMR (400 MHz, DMSO-*d*₆) δ 8.68 (s, 2H), 7.55-7.53 (m, 4H), 7.33 (d, *J =* 8.8 Hz, 2H), 6.66 (t, *J =* 7.6 Hz, 1H), 6.22 (s, 1H), 6.17-6.15 (m, 1H), 6.03 (d, *J =* 8.0 Hz, 1H), 5.54 (s, 1H), 4.98 (d, *J=* 4.8 Hz, 1H), 4.83-4.79 (m, 3H), 4.29 (d, *J =* 14.0 Hz, 1H), 4.16-4.10 (m, 1H), 3.98 (s, 3H), 3.90 (s, 3H), 3.85 (s, 3H), 3.08 (s, 3H).

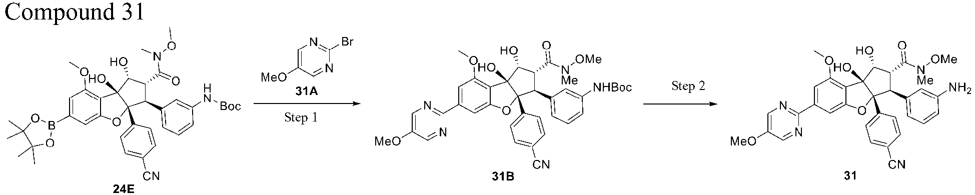

Step 1: The racemate of compound 24E (20 mg, 0.03 mmol) was dissolved in dioxane/water (3 mL/1 mL), and compound 31A (7.80 mg, 0.04 mmol), Na₂CO₃ (8.74 mg, 0.08 mmol), and Pd(dppf)Cl₂ (2.23 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 31B (20 mg) as a yellow solid, which was directly used in the next step. LCMS [M+H-C₄H₈]⁺ = 654.2.

Step 2: 6 N HCl (0.3 mL) was added to a solution of the racemate of compound 31B (20 mg, 0.03 mmol) in tetrahydrofuran (0.5 mL), and the mixture was stirred at room temperature for 12 h. The reaction solution was directly purified by preparative HPLC to give a racemate of 31 (3.3 mg, yield: 19%) as a white solid, which was further resolved on a chiral column to give compound 31. LCMS [M+H]⁺ = 610.2.

¹H NMR (400 MHz, DMSO-*d*₆) δ 8.68 (s, 2H), 7.55-7.53 (m, 4H), 7.33 (d, *J =* 8.8 Hz, 2H), 6.66 (t, *J =* 7.6 Hz, 1H), 6.22 (s, 1H), 6.17-6.15 (m, 1H), 6.03 *(d, J=* 8.0 Hz, 1H), 5.54 (s, 1H), 4.98 (d, *J=* 4.8 Hz, 1H), 4.83-4.79 (m, 3H), 4.29 (d, *J =* 14.0 Hz, 1H), 4.16-4.10 (m, 1H), 3.98 (s, 3H), 3.90 (s, 3H), 3.85 (s, 3H), 3.08 (s, 3H).

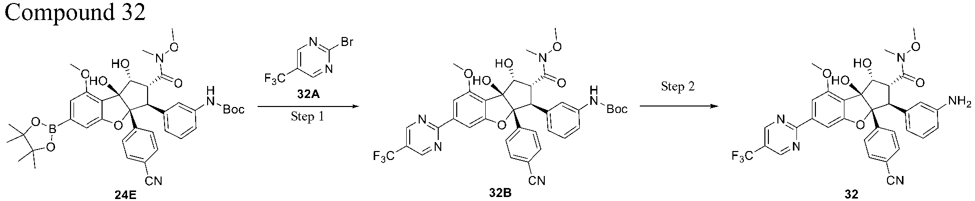

Step 1: The racemate of compound 24E (20 mg, 0.0300 mmol) was dissolved in dioxane (2.0 mL) and water (0.5 mL), and compound 32A (9.00 mg, 0.0550 mmol), Pd(dppf)Cl₂ (2.24 mg, 0.0027 mmol), and Na₂CO₃ (12.00 mg, 0.1099 mmol) were added. The mixture was heated by microwave to 100 °C and allowed to react for 1 h under a nitrogen atmosphere. The reaction solution was filtered and concentrated to give a racemate of 32B (17 mg, yield: 84%) as a yellow solid. LCMS [M+H-C₄H₈]⁺ = 692.2.

Step 2: The racemate of compound 32B (17 mg, 0269 mmol) was dissolved in THF (1 mL), and 6 M HCl (1 mL) was added. The mixture was stirred at room temperature for 16 h. The reaction solution was filtered and concentrated to give a crude product. The crude product was then purified on a preparative reversed-phase column [H₂O (containing 0.1% FA)/ACN] to give a racemate of 32 (1.1 mg, yield: 7.5%) as a white solid, which was further resolved on a chiral column to give compound 32.

LCMS [M+H]⁺ = 648.2.

¹H NMR (400 MHz, DMSO) δ 9.38 (d, J = 0.7 Hz, 2H), 7.70 (d, J = 1.1 Hz, 1H), 7.66 (d, J = 1.0 Hz, 1H), 7.54 (d, J = 8.6 Hz, 2H), 7.33 (d, J = 8.6 Hz, 2H), 6.66 (t, J = 7.8 Hz, 1H), 6.23 (s, 1H), 6.16 (d, J = 7.8 Hz, 1H), 6.05 (d, J = 7.4 Hz, 1H), 5.65 (s, 1H), 5.09 (d, J = 4.9 Hz, 1H), 4.84 - 4.78 (m, 2H), 4.33 (d, J = 13.9 Hz, 1H), 4.13 (dd, J = 15.2, 9.8 Hz, 1H), 3.91 (s, 3H), 3.87 (s, 3H), 3.09 (s, 3H).

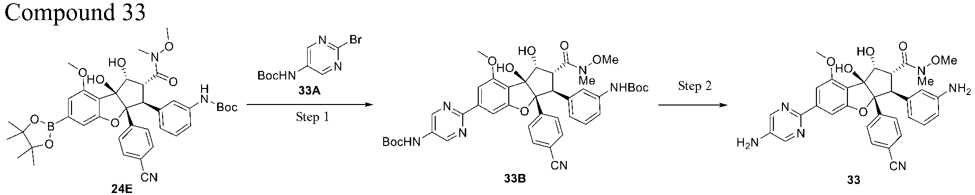

Step 1: The racemate of compound 24E (20 mg, 0.03 mmol) was dissolved in dioxane/water (3 mL/1 mL), and compound 33A (11.26 mg, 0.04 mmol), Na₂CO₃ (8.74 mg, 0.08 mmol), and Pd(dppf)Cl₂ (2.23 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 33B (23 mg) as a yellow solid, which was directly used in the next step. LCMS [M+H-C₄H₈]⁺ = 739.3.

Step 2: 6 N HCl (0.5 mL) was added to a solution of the racemate of compound 33B (23 mg, 0.03 mmol) in tetrahydrofuran (0.5 mL), and the mixture was stirred at room temperature for 12 h. The reaction solution was directly purified by preparative HPLC to give a racemate of 33 (1.1 mg, yield: 6.4%) as a white solid, which was further resolved on a chiral column to give compound 33. LCMS [M+H]⁺ = 595.2.

¹H NMR (400 MHz, DMSO-*d*₆) δ 8.22 (s, 2H), 7.54 (d, *J =* 8.8 Hz, 2H), 7.44 (d, *J =* 4.8 Hz, 2H), 7.32 (d, *J =* 8.8 Hz, 2H), 6.65 (t, *J* = 7.8 Hz, 1H), 6.22 (s, 1H), 6.16 (d, *J=* 8.0 Hz, 1H), 6.01 (d, *J* = 7.6 Hz, 1H), 5.75 (s, 2H), 5.47 (s, 1H), 4.89 (d, *J* = 4.4 Hz, 1H), 4.84-4.77 (m, 3H), 4.26 (d, *J =* 13.6 Hz, 1H), 3.89 (s, 3H), 3.83 (s, 3H), 3.08 (s, 3H).

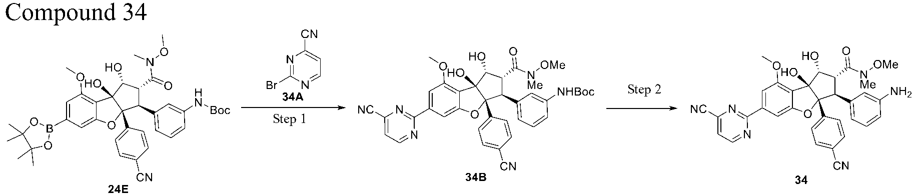

Step 1: The racemate of compound 24E (20 mg, 0.03 mmol) was dissolved in dioxane/water (3 mL/1 mL), and compound 34A (6.51 mg, 0.04 mmol), Na₂CO₃ (5.83 mg, 0.08 mmol), and Pd(dppf)Cl₂ (4.89 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 34B (20 mg) as a yellow solid, which was directly used in the next step. LCMS [M+H-C₄H₈]⁺ = 649.2.

Step 2: 6 N HCl (0.5 mL) was added to a solution of the racemate of compound 34B (20 mg, 0.03 mmol) in tetrahydrofuran (0.5 mL), and the mixture was stirred at room temperature for 12 h. The reaction solution was directly purified by preparative HPLC to give a racemate of 34 (4.6 mg, yield: 27%) as a white solid, which was further resolved on a chiral column to give compound 34. LCMS [M+H]⁺ = 605.2.

¹H NMR (400 MHz, DMSO-*d*₆) δ 9.26 (d, *J =* 4.8 Hz, 1H), 8.10 (d, *J =* 4.8 Hz, 1H), 7.62 (d, *J*= 1.0 Hz, 1H), 7.58 - 7.52 (m, 3H), 7.33 (d, *J =* 8.4 Hz, 2H), 6.66 (t, *J =* 7.6 Hz, 1H), 6.23 (s, 1H), 6.16 (d, *J =* 8.0 Hz, 1H), 6.04 (d, *J =* 7.6 Hz, 1H), 5.64 (s, 1H), 5.08 (d, *J* = 4.8 Hz, 1H), 4.93 - 4.76 (m, 3H), 4.31 (d, *J =* 14.0 Hz, 1H), 4.17 (s, 1H), 3.91(S,3H), 3.89 (s, 3H), 3.09 (s, 3H).

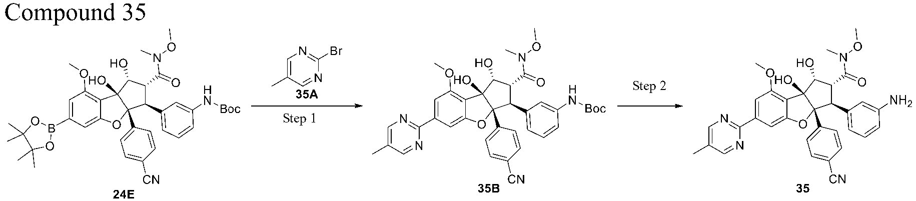

Step 1: The racemate of compound 24E (20 mg, 0.0275 mmol) was dissolved in dioxane (2.0 mL) and water (0.4 mL), and compound 35A (9.00 mg, 0.0550 mmol), Pd(dppf)Cl₂ (2.24 mg, 0.0027 mmol), and Na₂CO₃ (12.00 mg, 0.1099 mmol) were added. The mixture was heated by microwave to 100 °C and allowed to react for 1 h under a nitrogen atmosphere. The reaction solution was filtered and concentrated to give a crude product, which was then purified on a reversed-phase column (H₂O/ACN) to give a racemate of 35B (12 mg, yield: 63%) as a yellow solid. LCMS [M+H-C₄H₈]⁺ = 638.2.

Step 2: Compound 35B (12 mg, 0.0173 mmol) was dissolved in THF (1 mL), and 6 M HCl (1 mL) was added. The mixture was stirred at room temperature for 16 h. The reaction solution was filtered and concentrated to give a crude product. The crude product was then purified on a reversed-phase column [H₂O (containing 0.1% FA)/ACN] to give 35 (2.6 mg, yield: 25%) as a white solid, which was further resolved on a chiral column to give compound 35.

LCMS [M+H]⁺ = 594.2.

¹H NMR (400 MHz, DMSO) δ 9.38 (d, J = 0.7 Hz, 2H), 7.70 (d, J = 1.1 Hz, 1H), 7.66 (d, J = 1.0 Hz, 1H), 7.54 (d, J = 8.6 Hz, 2H), 7.33 (d, J = 8.6 Hz, 2H), 6.66 (t, J = 7.8 Hz, 1H), 6.23 (s, 1H), 6.16 (d, J = 7.8 Hz, 1H), 6.05 (d, J = 7.4 Hz, 1H), 5.65 (s, 1H), 5.09 (d, J = 4.9 Hz, 1H), 4.84 - 4.78 (m, 2H), 4.33 (d, J = 13.9 Hz, 1H), 4.13 (dd, J = 15.2, 9.8 Hz, 1H), 3.91 (s, 3H), 3.87 (s, 3H), 3.09 (s, 3H).

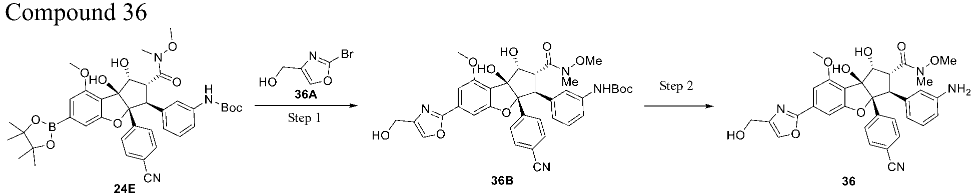

Step 1: Compound 36A (9.8 mg, 0.05 mmol), Na₂CO₃ (8.8 mg, 0.08 mmol), and Pd(dppf)Cl₂ (2.5 mg, 0.00 mmol) were added to a solution of the racemate of compound 24E (20 mg, 0.03 mmol) in dioxane/H₂O (2.5 mL/0.5 mL), and the mixture was heated by microwave to 100 °C and allowed to react for 1 h under a nitrogen atmosphere. The reaction solution was concentrated, and the crude product was purified on a reversed-phase column (ACN/H₂O) to give a racemate of 36B (10.0 mg, yield: 52.1%) as a white solid. LCMS [M+H-C₄H₈]⁺ = 643.2.

Step 2: HCl (6 N, 2 mL) was added to a solution of the racemate of compound 36B (10.0 mg, 0.01 mmol) in THF (2 mL), and the mixture was stirred at room temperature overnight. The reaction solution was directly purified on a reversed-phase column (ACN/H₂O) to give a racemate of 36 (6.4 mg, yield: 74.7%) as a white solid, which was further resolved on a chiral column to give compound 36. LCMS [M+H]⁺ = 599.3.

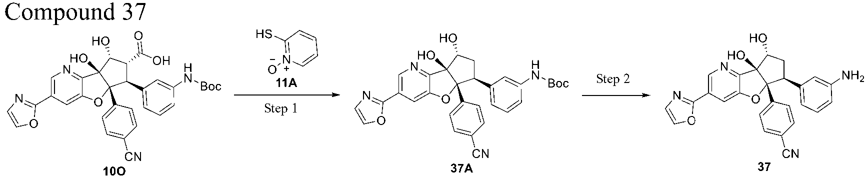

Step 1: The racemate of compound 10O (20 mg, 0.03 mmol), compound 11A (42.6 mg, 0.3 mmol), HOAT (6.12 mg, 0.045 mmol), tert-dodecyl mercaptan (60.6 mg, 0.3 mmol), TMP (5.48 mg, 0.045 mmol), Zinc meso-tetraphenylporphyrin (2.03 mg, 0.003 mmol), and DMF (2 mL) were added to a microwave tube. The reaction system was purged with nitrogen. Then, the microwave tube was placed in a hot water bath at 80 °C, and the system was stirred while being irradiated with 25 W red light. A solution of EDCI (57.3 mg, 0.3 mmol) in DMF (2 mL) was slowly added dropwise to the reaction solution, and then the mixture was stirred for 30 min. After the reaction was completed, the reaction solution was purified on a silica gel column (H₂O/ACN = 40%:60%) to give a racemate of compound 37A (8.6 mg, yield: 46%). LCMS [M+H]⁺ = 553.2.

Step 2: The racemate of compound 37A (8.6 mg, 0.015 mmol) was dissolved in THF (1 mL), and 6 M HCl (2 mL) was added. The reaction solution was stirred at room temperature (20 °C) for 2 h, then concentrated to dryness by rotary evaporation, and purified on a silica gel column (H₂O/ACN = 55:45) to give a racemate of compound 37 (3.7 mg, yield: 52%), which was further resolved on a chiral column to give compound 37.

LCMS [M+H]⁺ = 453.2.

¹H NMR (400 MHz, DMSO) δ 8.80 (d, *J =* 1.7 Hz, 1H), 8.33 (s, 1H), 7.87 (d, *J =* 1.7 Hz, 1H), 7.54 (d, *J =* 8.6 Hz, 2H), 7.47 (d, *J =* 0.6 Hz, 1H), 7.30 (d, *J =* 8.6 Hz, 2H), 6.88 (s, 1H), 6.55 (s, 1H), 6.47 (d, *J =* 23.6 Hz, 2H), 6.03 (s, 1H), 5.21 (s, 1H), 4.51 (d, *J =* 3.9 Hz, 1H), 4.24 (dd, *J =* 14.0, 6.0 Hz, 1H), 2.80 (td, *J =* 13.4, 4.3 Hz, 1H), 2.16 (dd, *J =* 12.6, 6.3 Hz, 1H).

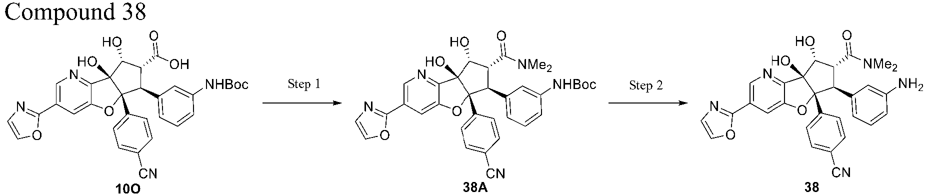

Step 1: A dimethylamine solution (0.1 mL, 0.2 mmol), HATU (15 mg, 0.04 mmol), and TMP (5 mg, 0.04 mmol) were added to a solution of the racemate of compound 10O (24 mg, 0.04 mmol) in DMF (2 mL), and the mixture was stirred at 20 °C for 2 h. The reaction solution was directly purified on a reversed-phase chromatography column (MeCN/H₂O/FA) to give a racemate of 38A (12 mg, yield: 48%) as a white solid. LCMS [M+H]⁺ = 624.3.

Step 2: 6 M HCl (2 mL) was added to a solution of the racemate of compound 38A (12 mg, 0.02 mmol) in THF (2 mL), and the mixture was stirred at 25 °C for 18 h. The reaction solution was directly purified on a reversed-phase chromatography column (MeCN/H₂O/FA) to give a racemate of 38 (4.7 mg, yield: 47%) as a white solid, which was further resolved on a chiral column to give compound 38.

LCMS [M+H]⁺ = 524.2.

¹H NMR (400 MHz, DMSO) δ 8.78 (d, *J =* 1.7 Hz, 1H), 8.33 (s, 1H), 7.89 (d, *J =* 1.7 Hz, 1H), 7.53 (s, 1H), 7.51 (s, 1H), 7.47 (s, 1H), 7.37 (d, *J =* 8.5 Hz, 2H), 6.66 (t, *J =* 7.8 Hz, 1H), 6.22 (s, 1H), 6.20 (s, 1H), 6.14 (dd, *J =* 17.3, 7.6 Hz, 2H), 5.28 (d, *J =* 5.5 Hz, 1H), 4.79 (s, 2H), 4.73 (t, *J* = 5.3 Hz, 1H), 4.54 (d, *J =* 13.3 Hz, 1H), 4.21 (dd, *J =* 13.3, 5.3 Hz, 1H), 3.28 (s, 3H), 2.79 (s, 3H).

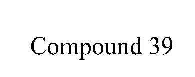

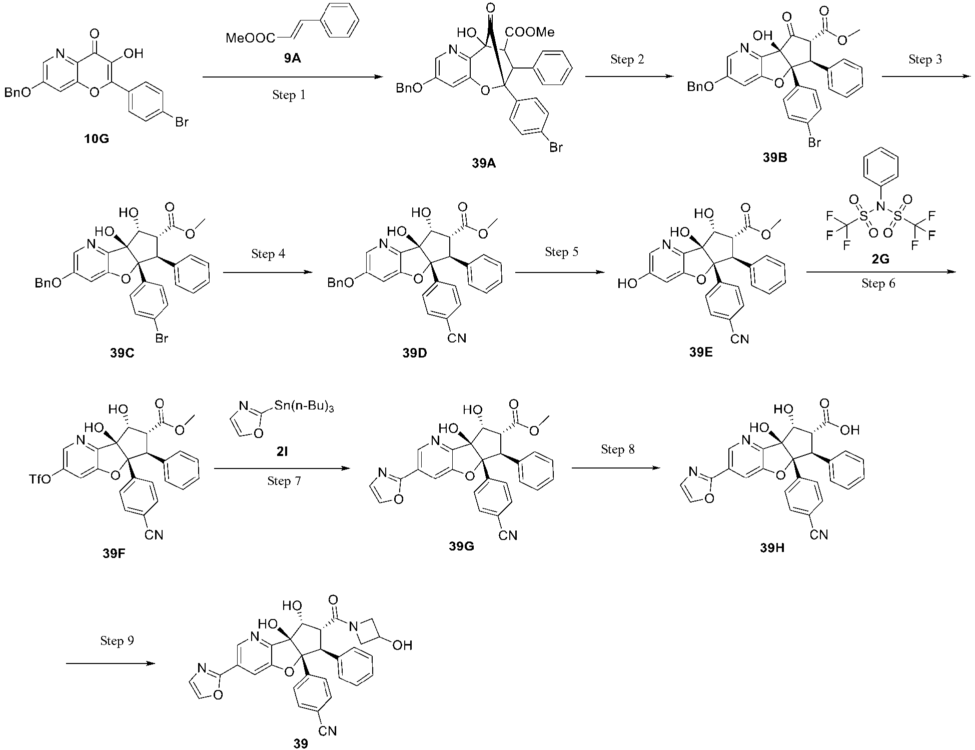

Step 1: Compound 10G (1 g, 2.36 mmol) and compound 9A (2.3 g, 14.14 mmol) were dissolved in CHCl₃/TFE (7/3, 15 mL). The reaction mixture was then injected at a rate of 12 mL/h via a 20 mL syringe using a syringe pump into a coil wound around a jacketed glass cylinder equipped with a 250 W ultraviolet lamp. The temperature of the external circulation cold trap was adjusted to maintain the internal reaction temperature at 0-5 °C, and the UV lamp was turned on simultaneously. The reaction mixture was irradiated for 1 h, concentrated under reduced pressure to remove the solvent, and then separated by column chromatography (EA/PE = 1/3) to remove excess cinnamate, thus giving a racemate of 39A (7 g, yield: 51%) as a light yellow solid. LCMS [M+H-H₂O]⁺ = 568.1.

Step 2: The racemate of compound 39A (4.9 g, 8.37 mmol) was dissolved in methanol (60 mL), and NaOMe (1.135 g, 20.94 mmol) was added at 0 °C. The mixture was stirred at 65 °C for 1.5 h. The reaction solution was concentrated, extracted with EA, washed with water, an NH₄Cl solution, and saturated brine, dried over anhydrous sodium sulfate, then filtered, concentrated again, and purified on a silica gel column (0.2% FA in EA/PE = 1/3) to give a racemate of compound 39B (3.6 g, 73%). LCMS [M+H]⁺ = 586.1.

Step 3: The racemate of compound 39B (3.6 g, 6.15 mmol) was dissolved in a mixed solvent of ACN/CHCl₃ (1/1, 40 mL/40 mL), and Na(AcO)₃BH (6.525 g, 30.77 mmol) and AcOH (3.695 g, 61.53 mmol) were added at 0 °C. The mixture was stirred at room temperature for 2 h. The reaction solution was concentrated, extracted with EA, washed with water and saturated brine, dried over anhydrous sodium sulfate, then filtered, concentrated again, and purified on a silica gel column (0.2% FA in EA/PE = 3/2) to give a racemate of compound 39C (1.9 g, yield: 53%). LCMS [M+H]⁺ = 588.1.

Step 4: Under a nitrogen atmosphere, a solution of the racemate of compound 39C (1500 mg, 2.55 mmol), Zn(CN)₂ (7745.6 mg, 6.37 mmol), Pd₂(dba)₃ (468 mg, 0.5 mmol), and DPPF (564 mg, 1.02 mmol) in DMP (30 mL) was stirred at 150 °C for 1 h. The reaction solution was diluted with water and extracted with EA three times (50 mL × 3). The organic phase was concentrated, and the crude product was purified on a FLASH column (low- to medium-pressure flash preparative liquid chromatography) (DCM/MEOH = 0-10%) to give a racemate of 39D (1100 mg, yield: 80.7%) as a yellow solid. LCMS: [M+H]⁺ = 535.6.

Step 5: The racemate of 39D (1100 mg, 2.06 mmol) and Pd/C (700 mg) were added to a solution of EA/MeOH (20/20 mL), and the mixture was purged with hydrogen three times and stirred at room temperature for 16 h. The reaction solution was purified on a FLASH column (low- to medium-pressure flash preparative liquid chromatography) (MeOH/DCM = 0-10%) to give a racemate of compound 39E (350 mg, yield: 38.2%). LCMS: [M+H]⁺ = 445.6.

Step 6: The racemate of compound 39E (350 mg, 0.79 mmol) and K₂CO₃ (108 mg, 0.79 mmol) were added to DMF (5 mL), and then compound 2G (281 mg, 0.79 mmol) was added. The mixture was stirred at 60 °C for 1.5 h. The reaction solution was diluted with EA (30 mL), washed with H₂O (20 mL × 2), then purified on a FLASH column (low- to medium-pressure flash preparative liquid chromatography) (MeOH/DCM = 0-10%) to give a racemate of compound 39F (310 mg, yield: 68.3%). LCMS [M+H]⁺ = 577.6.

Step 7: The racemate of compound 39F (300 mg, 0.7 mmol), compound 2I (756 mg, 2.1 mmol), and Pd(PPh₃)₄ (81 mg, 0.05 mmol) were added to DMF (4 mL), and CuI (26.5 mg, 0.14 mmol) was added. The mixture was stirred under microwave at 135 °C for 5 h. The reaction solution was diluted with EA (30 mL), and the resulting mixture was washed with H₂O (20 mL × 2). The solution was then purified on a FLASH column (low- to medium-pressure flash preparative liquid chromatography) (MeOH/DCM = 0-15%) to give a racemate of 39G (250 mg, yield: 71%) as a yellow solid. LCMS [M+H]⁺ = 496.2.

Step 8: The racemate of compound 39G (250 mg, 0.5 mmol) and LiOH·H₂O (60 mg, 1.5 mmol) were added to a mixed solution of THF/MeOH/H₂O (1.5/1.5/1.5 mL), and the mixture was stirred at 20 °C for 5 h. The reaction solution was purified on a FLASH column (low- to medium-pressure flash preparative liquid chromatography) (ACN-H₂O) to give a racemate of 39H (170 mg, yield: 70%) as a yellow solid. LCMS [M+H]⁺ = 482.7.

Step 9: The racemate of compound 39H (20 mg, 0.04 mmol), HATU (16 mg, 0.04 mmol), and TMP (15 mg, 0.12 mmol) were dissolved in DMF (2 mL), and 3-hydroxyazetidine (5.8 mg, 0.08 mmol) was added. The mixture was stirred at 20 °C for 2 h. The reaction solution was purified on a FLASH column (low- to medium-pressure flash preparative liquid chromatography) (MeOH/DCM = 0-10%) to give a racemate of 39 (19 mg, yield: 86%) as a white solid, which was further resolved on a chiral column to give compound 39.

LCMS [M+H]⁺ = 537.7.

¹H NMR (400 MHz, DMSO) δ 8.79 (s, 1H), 8.44 (s, 1H), 8.33 (s, 1H), 7.93 (d, *J =* 1.6 Hz, 1H), 7.48 (d, *J =* 6.1 Hz, 3H), 7.37 - 7.30 (m, 2H), 7.06 (t, *J =* 6.8 Hz, 2H), 7.02 - 6.93 (m, 3H), 6.28 (d, *J =* 10.8 Hz, 1H), 5.82 (s, 1H), 5.41 (m, 1H), 4.67 (m, 2H), 4.53 (m, 2H), 4.07 - 3.96 (m, 3H), 3.62 - 3.55 (m, 1H).

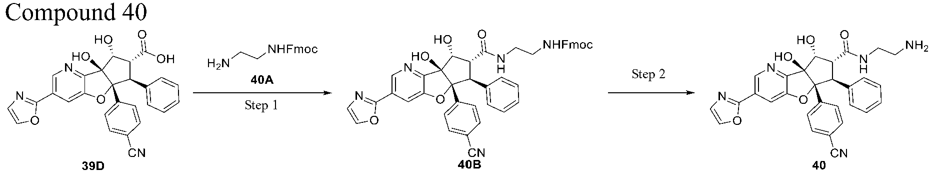

Step 1: The racemate of compound 39D (20 mg, 0.04 mmol), HATU (12 mg, 0.04 mmol), and TMP (14.5 mg, 0.12 mmol) were stirred in DMF (2 mL). Compound 40A (12 mg, 0.04 mmol) was added. The mixture was stirred at 20 °C for 2 h and then purified on a normal-phase chromatography column (MeOH/DCM = 0-10%) to give a racemate of 40B (30 mg, yield: 94%) as a white solid. LCMS [M+H]⁺ = 747.0.

Step 2: The racemate of compound 40B (30 mg, 0.04 mmol) and piperidine (10 mg, 0.12 mol) were dissolved in DMF (1 mL), and the mixture was allowed to react at 20 °C for 1 h. The reaction solution was lyophilized to give a racemate of 40 (20 mg, yield: 87%) as a white solid, which was further resolved on a chiral column to give compound 40.

LCMS [M+H]⁺ = 524.2.

¹H NMR (400 MHz, DMSO) δ 8.80 (d, *J =* 1.7 Hz, 1H), 8.41 (t, *J =* 5.6 Hz, 1H), 8.34 (s, 1H), 7.93 (d, *J =* 1.7 Hz, 1H), 7.52 (d, *J =* 8.6 Hz, 2H), 7.48 (s, 1H), 7.30 (d, *J =* 8.5 Hz, 2H), 7.04 (ddd, *J =* 21.9, 14.9, 7.3 Hz, 6H), 6.26 (s, 1H), 4.67 (dd, *J =* 18.3, 9.1 Hz, 2H), 3.25 (dd, *J =* 12.6, 6.5 Hz, 3H), 2.75 (t, *J =* 6.6 Hz, 2H).

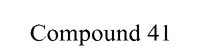

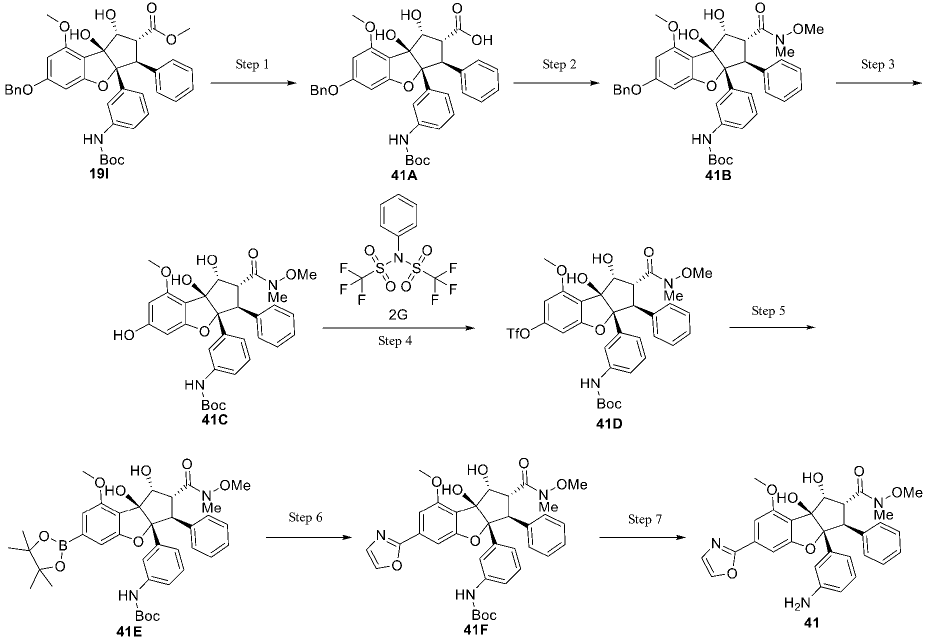

Step 1: LiOH·H₂O (19.3 mg, 0.46 mmol) was added to a solution of the racemate of compound 19I (100.0 mg, 0.15 mmol) in THF/H₂O/MeOH (3 mL/1 mL/1 mL), and the mixture was stirred at 40 °C for 3 h. The reaction solution was adjusted to about pH = 5 with 3 M HCl, concentrated to dryness by rotary evaporation, and purified by normal-phase column chromatography (DCM/MeOH) to give a racemate of compound 41A (75 mg, yield: 77%). LCMS [M-H]⁻ = 638.2.

Step 2: TMP (56.8 mg, 0.47 mmol) was added to a solution of the racemate of compound 41A (75.0 mg, 0.12 mmol), dimethylhydroxylamine hydrochloride (22.8 mg, 0.23 mmol), HOAT (16.0 mg, 0.12 mmol), and HATU (89.2 mg, 0.23 mmol) in DMF (mL). The mixture was stirred at 40 °C for 16 h. The reaction solution was purified by normal-phase column chromatography (DCM/MeOH) to give a racemate of compound 41B (85 mg, yield: 110%). LCMS [M+H]⁺ = 683.1.

Step 3: Pd(OH)₂/C (10%, 50 mg) was added to a mixed solution of the racemate of compound 41B (85.0 mg, 0.13 mmol) in EA/MeOH/THF/DCM (20:2:2:0.2, 10 mL), and the mixture was purged with hydrogen three times and stirred at room temperature for 16 h. The reaction solution was filtered. The filter cake was rinsed with MeOH/DCM (1/10) three times, and the filtrate was concentrated to dryness by rotary evaporation to give a racemate of compound 41C (50 mg, crude product, yield not calculated), which was directly used in the next step. LCMS [M+H]⁺ = 593.1.

Step 4: Compound 2G (30.2 mg, 0.0.84 mmol) was added to a solution of the racemate of compound 41C (50.0 mg, 0.08 mmol) and K₂CO₃ (23.3 mg, 0.169 mmol) in DMF (2 mL), and the mixture was stirred at 60 °C for 1 h. The reaction solution was extracted with water and ethyl acetate. The organic phase was dried over anhydrous sodium sulfate, filtered, and then concentrated. The resulting crude product was purified by normal-phase column chromatography (PE/EA) to give a racemate of compound 41D (45 mg, yield: 73%). LCMS: [M+H]+ = 725.1.

Step 5: The racemate of compound 41D (33.0 mg, 0.05 mmol) was dissolved in a solution of dioxane (5 mL), and Pin₂B₂ (34.5 mg, 0.14 mmol), Pd(dppf)Cl₂ (7.4 mg, 0.01 mmol), and KOAc (13.4 mg, 0.18 mmol) were added. The mixture was stirred at 85 °C for 16 h under a N₂ atmosphere. The reaction solution was filtered. The filter cake was washed with EA, and the filtrate was concentrated and then purified on a silica gel column (DCM/MeOH) to give a racemate of compound 41E (43 mg, crude product, yield not calculated), which was directly used in the next step. LCMS [M+H]⁺ = 703.3.

Step 6: The racemate of compound 41E (43.0 mg, 0.06 mmol), 2-bromooxazole (13.6 mg, 0.09 mmol), Na₂CO₃ (19.5 mg, 0.184 mmol), and Pd(dppf)Cl₂ (5.0 mg, 0.01 mmol) were added to a solution of dioxane/water (2 mL/1 mL). The reaction mixture was degassed under reduced pressure, purged with N₂ three times, and stirred under microwave at 100 °C for 2 h. The reaction solution was concentrated and then purified on a silica gel column (DCM/MeOH) to give a racemate of compound 41F (7 mg, yield: 17.7%). LCMS [M+H]⁺ = 644.1.

Step 7: The racemate of compound 41F (7.0 mg, 0.01 mmol) was mixed with THF (1 mL), and 6 M HCl (2 mL) was added at 0 °C. The mixture was stirred at 10 °C for 16 h. The reaction solution was concentrated and purified by preparative HPLC to give a racemate of 41 (0.8 mg, yield: 13.5%) as a white solid, which was further resolved on a chiral column to give compound 41.

LCMS [M+H]⁺ = 544.2.

¹H NMR (400 MHz, DMSO) δ 8.25 (s, 1H), 7.41 (s, 1H), 7.17 (d, *J =* 14.2 Hz, 2H), 7.04 (t, *J*= 7.4 Hz, 2H), 6.98 (d, *J =* 7.2 Hz, 1H), 6.89 (d, *J =* 7.6 Hz, 2H), 6.69 (t, *J =* 7.8 Hz, 1H), 6.47 (s, 1H), 6.28 (d, *J =* 7.9 Hz, 1H), 6.19 (d, *J =* 8.2 Hz, 1H), 5.23 (s, 1H), 4.90 (s, 1H), 4.84 (s, 1H), 4.76 (s, 1H), 4.21 (s, 1H), 3.89 (s, 3H), 3.86 (s, 3H), 3.06 (s, 3H).

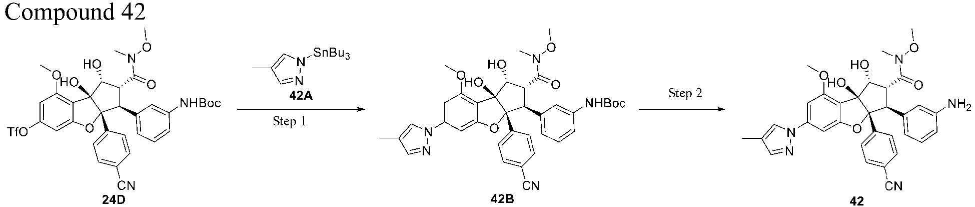

Step 1: The racemate of compound 24D (50 mg, 0.07 mmol), t-BuXPhos-PdG3 (7.94 mg, 0.01 mmol), and K₂CO₃ (450 mg, 3.26 mmol) were added to DMF (3 mL), and compound 42A (582 mg, 1.63 mmol) was added. The mixture was allowed to react at 120 °C for 5 h. The reaction solution was diluted with water and extracted with EA three times (10 mL × 3). The organic phase was concentrated and purified by preparative HPLC (ACN/H₂O) to give a racemate of 42B (8 mg, yield: 18%) as a white solid. LCMS [M+H]⁺ = 682.1.

Step 2: The racemate of compound 42B (10 mg, 0.01 mmol) and 6 M HCl (1.5 mol) were added to THF (1 mL), and the mixture was allowed to react at 40 °C for 5 h. The reaction solution was lyophilized to give a racemate of 42 (8 mg, yield: 94%) as a white solid. LCMS [M+H]⁺ = 582.1. The racemate was further resolved on a chiral column to give compound 42.

¹H NMR (400 MHz, DMSO) δ 8.36 (s, 1H), 7.63 - 7.50 (m, 3H), 7.31 (d, J = 8.5 Hz, 2H), 7.13 - 6.98 (m, 3H), 6.80 (s, 3H), 5.60 (s, 1H), 4.81 (s, 1H), 4.42 (d, J = 13.4 Hz, 1H), 4.17 (d, J = 12.4 Hz, 1H), 3.92 (s, 3H), 3.83 (s, 3H), 3.08 (s, 3H), 2.12 (s, 3H).

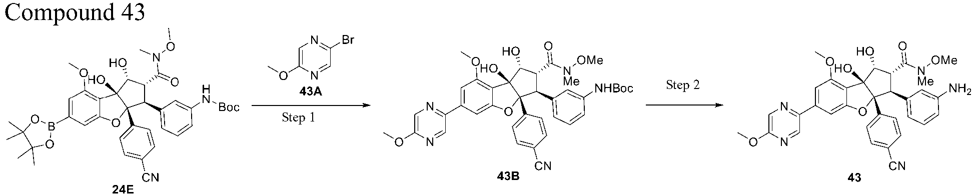

Step 1: The racemate of compound 24E (20 mg, 0.03 mmol) was dissolved in dioxane/water (3 mL/1 mL), and compound 43A (7.79 mg, 0.04 mmol), Na₂CO₃ (5.83 mg, 0.06 mmol), and Pd(dppf)Cl₂ (4.89 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 43B (20 mg) as a yellow solid, which was directly used in the next step. LCMS [M+H]⁺ = 710.2.

Step 2: 6 N HCl (0.5 mL) was added to a solution of the racemate of compound 43B (20 mg, 0.03 mmol) in tetrahydrofuran (0.5 mL), and the mixture was stirred at room temperature for 12 h. The reaction solution was directly purified by preparative HPLC to give a racemate of 43 (3.1 mg, yield: 18%) as a white solid, which was further resolved on a chiral column to give compound 43. LCMS [M+H]+ = 610.2.

¹H NMR (400 MHz, DMSO-*d*₆) δ 8.89 (d, *J =* 1.2 Hz, 1H), 8.41 (d, *J =* 1.2 Hz, 1H), 7.54 (d, *J*= 8.4 Hz, 2H), 7.33 (s, 1H), 7.31 (s, 2H), 7.24 (d, *J =* 0.8 Hz, 1H), 6.67 (t, *J =* 7.8 Hz, 1H), 6.22-6.15 (m, 2H), 6.05 (d, *J =* 8.0 Hz, 1H), 5.51 (s, 1H), 4.98 (d, *J =* 4.4 Hz, 1H), 4.82-4.78 (m, 3H), 4.30 (d, *J* = 14.0 Hz, 1H), 4.13 (d, *J =* 14.8 Hz, 1H), 4.03 (s, 3H), 3.90 (s, 3H), 3.85 (s, 3H).

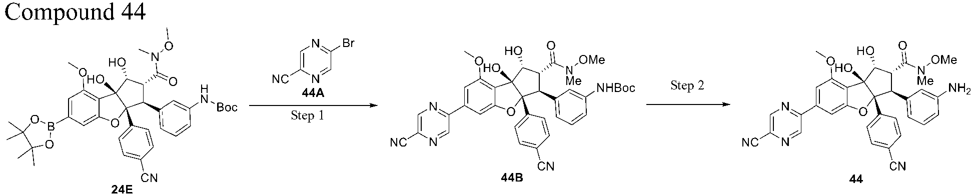

Step 1: The racemate of compound 24E (20 mg, 0.03 mmol) was dissolved in dioxane/water (3 mL/1 mL), and compound 44A (7.59 mg, 0.04 mmol), Na₂CO₃ (5.83 mg, 0.08 mmol), and Pd(dppf)Cl₂ (4.89 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 44B (20 mg) as a yellow solid, which was directly used in the next step. LCMS [M+H]⁺ = 705.0.

Step 2: 6 N HCl (0.5 mL) was added to a solution of the racemate of compound 44B (20 mg, 0.03 mmol) in tetrahydrofuran (0.5 mL), and the mixture was stirred at room temperature for 12 h. The reaction solution was directly purified by preparative HPLC to give a racemate of 44 (1.4 mg, yield: 8.2%) as a white solid, which was further resolved on a chiral column to give compound 44. LCMS [M+H]+ = 605.2.

¹H NMR (400 MHz, DMSO-*d*₆) δ9.57 (d, *J =* 1.6 Hz, 1H), 9.28 (d, *J=* 1.2 Hz, 1H), 7.55-7.53 (m, 3H), 7.42 (s, 1H), 7.33-7.31 (m, 2H), 6.67 (t, *J =* 7.8 Hz, 1H), 6.20-6.15 (m, 2H), 6.07 (d, *J =* 8.0 Hz, 1H), 5.64 (s, 1H), 5.10 (d, *J =* 5.2 Hz, 1H), 4.82-4.78 (m, 3H), 4.34 (d, *J =* 13.6 Hz, 1H), 4.15 (d, *J =* 18.8 Hz, 1H), 3.90 (s, 3H), 3.87 (s, 3H), 3.09 (s, 3H).

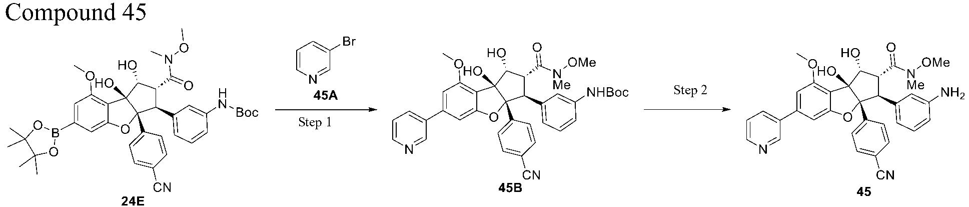

Step 1: The racemate of compound 24E (20 mg, 0.03 mmol) was dissolved in dioxane/water (3 mL/1 mL), and compound 45A (6.51 mg, 0.04 mmol), Na₂CO₃ (5.83 mg, 0.08 mmol), and Pd(dppf)Cl₂ (4.89 mg, 0.001 mmol) were added. The mixture was heated by microwave to 100 °C and allowed to react for 1 h under a nitrogen atmosphere. The reaction solution was filtered. The filter cake was washed with ethyl acetate (2 mL × 3), and the filtrate was concentrated to give a racemate of 45B (20 mg) as a yellow solid, which was directly used in the next step.

Step 2: 6 N HCl (0.5 mL) was added to a solution of the racemate of compound 45B (20 mg, 0.03 mmol) in tetrahydrofuran (0.5 mL), and the mixture was stirred at room temperature for 12 h. The reaction solution was directly purified by preparative HPLC to give a racemate of 45 (1.5 mg, yield: 8.8%) as a white solid, which was further resolved on a chiral column to give compound 45. LCMS [M+H]⁺ = 579.2

¹H NMR (400 MHz, DMSO-*d*₆) δ 8.96 (d, *J* = 2.0 Hz, 1H), 8.59 (d, *J =* 5.6 Hz, 1H), 8.14 (d, *J* = 8.0 Hz, 1H), 7.70 - 7.46 (m, 3H), 7.33 (d, *J =* 8.4 Hz, 2H), 7.00 (s, 1H), 6.88 (s, 1H), 6.79 - 6.57 (m, 1H), 6.23 - 6.12 (m, 2H), 6.06 (d, *J=* 7.6 Hz, 1H), 5.51 (s, 1H), 4.99 (d, *J* = 4.4 Hz, 1H), 4.88 - 4.64 (m, 3H), 4.30 (d, *J =* 13.9 Hz, 1H), 4.14 (d, *J =* 10.5 Hz, 1H), 3.90 (s, 3H), 3.88 (s, 3H), 3.09 (s, 3H).

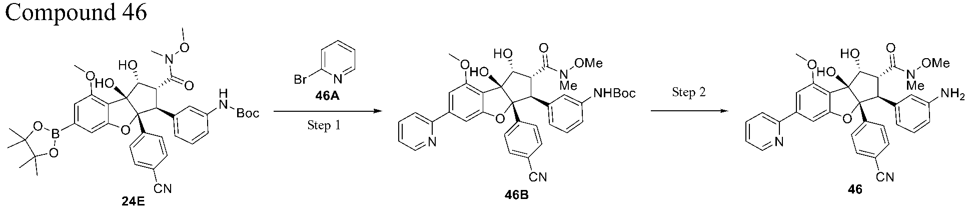

Step 1: The racemate of compound 24E (20 mg, 0.03 mmol) was dissolved in dioxane/water (3 mL/1 mL), and compound 46A (6.51 mg, 0.04 mmol), Na₂CO₃ (5.83 mg, 0.08 mmol), and Pd(dppf)Cl₂ (4.89 mg, 0.001 mmol) were added. The reaction mixture was allowed to react under microwave at 100 °C for 1 h. The reaction solution was filtered. The filter cake was washed with ethyl acetate (2 mL × 3), and the filtrates were combined and concentrated to give a racemate of 46B (20 mg) as a yellow solid, which was directly used in the next step. LCMS [M+H]⁺ = 679.0. Step 2: 6 N HCl (0.5 mL) was added to a solution of the racemate of compound 46B (20 mg, 0.03 mmol) in tetrahydrofuran (0.5 mL), and the mixture was stirred at room temperature for 12 h. The reaction solution was directly purified by preparative HPLC to give a racemate of 46 (1.4 mg, yield: 8.2%) as a white solid, which was further resolved on a chiral column to give compound 46. LCMS [M+H]⁺ = 579.1.

¹H NMR (400 MHz, DMSO-*d*₆) δ 8.69-8.68 (m, 1H), 8.04 (d, *J =* 8.0 Hz, 1H), 7.92-7.88 (m, 1H), 7.54 (d, *J*= 8.8 Hz, 2H), 7.40-7.32 (m, 5H), 6.66 (t, *J* = 7.8 Hz, 1H), 6.20-6.15 (m, 2H), 6.05 (d, *J* = 7.2 Hz, 1H), 5.52 (s, 1H), 4.97 (d, *J =* 4.4 Hz, 1H), 4.82-4.79 (m, 3H), 4.30 (d, *J=* 14.0 Hz, 1H), 4.16-4.11 (m, 1H), 3.90 (s, 3H), 3.86 (s, 3H), 3.09 (s, 3H).

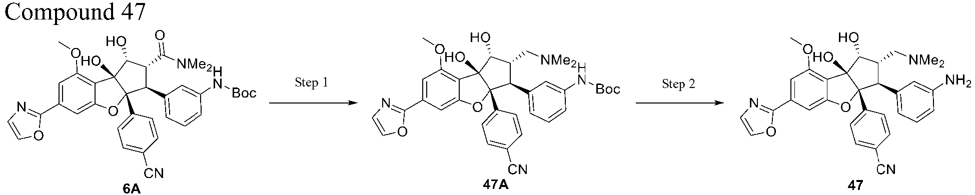

Step 1: BH₃-DMS (70 µL, 0.14 mmol) was added to a solution of the racemate of compound 6A (30 mg, 0.05 mmol) in tetrahydrofuran (5 mL). The reaction system was stirred at 60 °C for 2 h. Methanol (15 mL) was added to the reaction system, and the mixture was stirred at 60 °C for another 18 h. The reaction solution was concentrated to dryness by rotary evaporation and then purified by normal-phase column chromatography (DCM:MeOH) to give a racemate of 47A (20 mg, yield: 69%) as a white solid. LCMS [M+H]⁺ = 639.2.

Step 2: 6 M HCl (1 mL) was added to a solution of the racemate of compound 47A (20 mg, 0.03 mmol) in THF (1 mL). The reaction system was stirred at room temperature for 18 h. The reaction solution was purified by reversed-phase column chromatography (H₂O/ACN) to give a racemate of 47 (14 mg, yield: 69%) as a white solid, which was further resolved on a chiral column to give compound 47.

LCMS [M+H]⁺ = 539.2.

¹H NMR (400 MHz, DMSO) δ 9.68 (s, 1H), 8.25 (s, 1H), 7.57 - 7.36 (m, 5H), 7.18 (s, 2H), 6.85 (s, 1H), 6.45 (d, *J* = 39.4 Hz, 3H), 5.74 (s, 1H), 5.30 (d, *J =* 5.8 Hz, 1H), 4.68 (s, 1H), 3.87 (s, 3H), 3.68 (d, *J =* 13.2 Hz, 1H), 3.24 (d, *J =* 11.1 Hz, 1H), 2.95 (d, *J* = 3.3 Hz, 3H), 2.84 (t, *J =* 12.9 Hz, 4H), 1.24 (s, 1H).

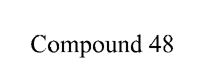

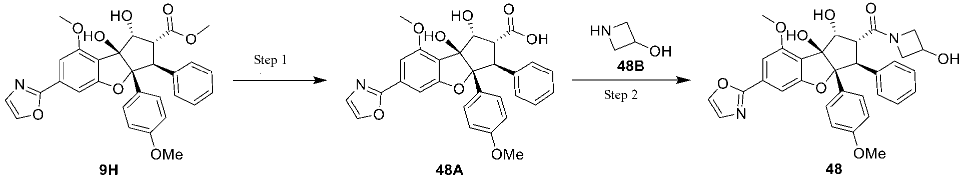

Step 1: The racemate of compound 9H (210 mg, 0.4 mmol) and LiOH·H₂O (48 mg, 1.19 mmol) were added to THF/MeOH/H₂O (1.5/1.5/1.5 mL). The mixture was stirred at 20 °C for 16 h. The solution was purified on a reversed-phase column (MeCN/H₂O/FA) to give a racemate of 48A (150 mg, yield: 75%) as a white solid. LCMS [M+H]⁺ = 516.7.

Step 2: The racemate of compound 48A (20 mg, 0.04 mmol), HATU (15 mg, 0.04 mmol), and TMP (14 mg, 0.12 mmol) were stirred in DMF (2 mL). The racemate of compound 48B (9 mg, 0.08 mmol) was added to the mixture. The resulting mixture was stirred at 20 °C for 2 h and then purified on a silica gel column (MeOH/DCM = 0-10%) to give 48 (10 mg, yield: 50%) as a white solid, which was further resolved on a chiral column to give compound 48.

LCMS [M+H]⁺ = 571.2.

¹H NMR (400 MHz, DMSO) δ 8.25 (s, 1H), 7.41 (s, 1H), 7.17 (d, J = 20.4 Hz, 2H), 7.05 (m, 4H), 7.00 - 6.94 (m, 1H), 6.91 (d, J = 7.2 Hz, 2H), 6.58 (m, 2H), 5.77 (m, 1H), 5.34 (d, J = 7.2 Hz, 1H), 4.91 (t, J = 4.4 Hz, 1H), 4.72 - 4.45 (m, 3H), 4.31 (m, 1H), 4.23 - 4.05 (m, 1H), 4.03 - 3.93 (m, 1H), 3.88 - 3.76 (m, 4H), 3.61 - 3.50 (m, 4H).

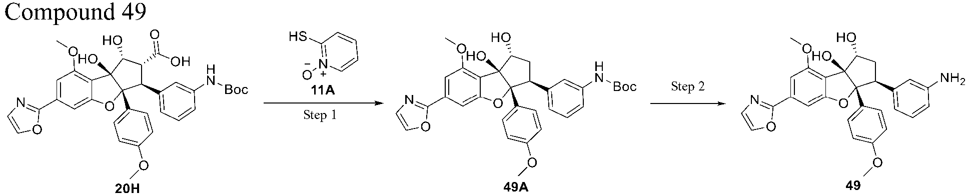

Step 1: The racemate of compound 20H (25 mg, 0.04 mmol), compound 11A (50.38 mg, 0.4 mmol), HOAT (8.09 mg, 0.06 mmol), tert-dodecyl mercaptan (80.13 mg, 0.40 mmol), TMP (7.25 mg, 0.06 mmol), Zinc meso-tetraphenylporphyrin (2.69 mg, 0.001 mmol), and DMF (2 mL) were mixed in a microwave tube. The reaction system was purged with nitrogen. Then, the microwave tube was placed in hot water at 80 °C, and the system was stirred while being irradiated with 25 W red light. EDCI (75.77 mg, 0.40 mmol, dissolved in 2 mL of DMF) was slowly added dropwise to the reaction solution, and then the mixture was stirred for 30 min. After the reaction was completed, the reaction solution was purified on a silica gel column (H₂O/ACN = 40%:60%) to give a racemate of the target product 49A (16 mg, yield: 69%). LCMS [M+H-C₄H₈-H₂O]⁺ = 513.2. Step 2: The racemate of compound 49A (13 mg, 0.02 mmol) was dissolved in THF (2 mL), and 6 M HCl (4 mL) was added. The reaction solution was stirred at room temperature (20 °C) for 4 h, then concentrated to dryness by rotary evaporation, and purified on a silica gel column (H₂O/ACN = 55:45) to give a racemate of compound 49 (10 mg, yield: 93%), which was further resolved on a chiral column to give compound 49.

LCMS [M+H]⁺ = 487.2.

¹H NMR (400 MHz, DMSO) δ 8.24 (d, *J* = 0.7 Hz, 1H), 7.40 (d, *J =* 0.7 Hz, 1H), 7.14 (dd, *J* = 5.1, 1.1 Hz, 2H), 7.04 (d, *J =* 9.0 Hz, 2H), 6.75 (t, *J =* 7.7 Hz, 1H), 6.62 (d, *J =* 9.0 Hz, 2H), 6.40 (s, 1H), 6.26 (t, *J =* 6.3 Hz, 2H), 5.06 (s, 1H), 5.01 (s, 1H), 4.65 (d, *J =* 3.7 Hz, 1H), 4.47 (s, 1H), 3.93 - 3.87 (m, 1H), 3.86 (s, 3H), 3.62 (s, 3H), 2.64 (td, *J =* 13.4, 4.9 Hz, 1H), 2.00 (dd, *J =* 12.7, 6.1 Hz, 1H).

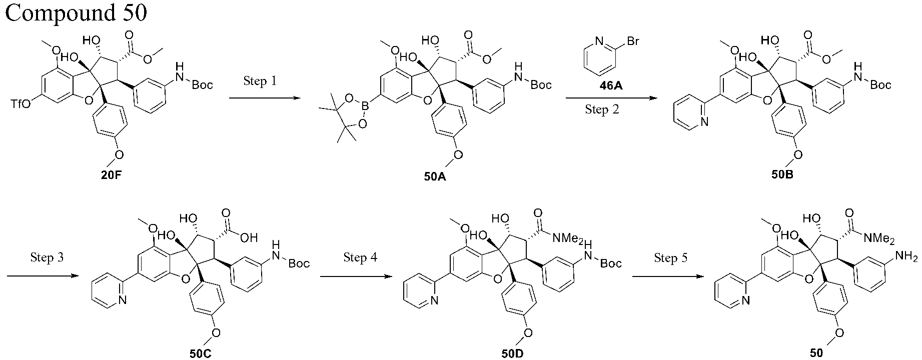

Step 1: The racemate of compound 20F (2.8 g, 3.86 mmol) was dissolved in dioxane (30 mL), and Pin₂B₂ (2.9 g, 11.59 mmol), Pd(dppf)Cl₂ (630.3 mg, 0.77 mmol), and KOAc (1.1 g, 15.45 mmol) were added. The mixture was purged with nitrogen three times and stirred at 85 °C for 16 h. The reaction solution was filtered. The filter cake was washed with EA, and the filtrate was concentrated and then purified on a silica gel column (DCM/MeOH) to give a racemate of compound 50A (1.8 g, yield: 66%). LCMS [M+Na]⁺ = 726.8.

Step 2 and step 3: The racemate of compound 50A (200.0 mg, 0.28 mmol) was dissolved in dioxane/water (2 mL/1 mL), and compound 46A (67.4 mg, 0.43 mmol), Na₂CO₃ (90.5 mg, 0.85 mmol), and Pd(dppf)Cl₂ (23.0 mg, 0.03 mmol) were added. The reaction solution was purged with nitrogen three times, stirred at 130 °C for 2 h, concentrated, and then purified on a silica gel column (DCM/MeOH) to give a racemate of compound 50C (110 mg, two-step yield: 60%). LCMS [M+H]⁺ = 641.8.

Step 4: HATU (26.7 mg, 0.07 mmol), DIEA (30.2 mg, 0.24 mmol), and the racemate of compound 50C (30.0 mg, 0.05 mmol) were dissolved in DMF (2 mL), and the mixture was stirred at 10 °C for 10 min. Dimethylamine (0.1 mL, 0.23 mmol, 2 M in THF) was added to the mixture, and the resulting mixture was allowed to react at 10 °C for 1 h. The solution was diluted with water, extracted with EA, washed with water and brine, dried over anhydrous Na₂SO₄, filtered, concentrated, and then purified on a silica gel column (DCM/MeOH) to give a racemate of compound 50D (20 mg, yield: 64%). LCMS [M+H]⁺ = 669.0.

Step 5: The racemate of compound 50D (20.0 mg, 0.02 mmol) was dissolved in THF (1 mL), and 6 M HCl (1 mL) was added at 0 °C. The mixture was stirred at 25 °C for 16 h, concentrated, and then purified by preparative HPLC to give a racemate of 50 (4.1 mg, yield: 24%) as a white solid, which was further resolved on a chiral column to give compound 50.

LCMS [M+H]⁺ = 568.2.

¹H NMR (400 MHz, DMSO) δ 8.69 (dd, *J* = 4.7, 0.9 Hz, 1H), 8.05 (d, *J =* 8.1 Hz, 1H), 7.90 (td, *J* = 7.8, 1.8 Hz, 1H), 7.41 - 7.34 (m, 1H), 7.32 (dd, *J =* 6.4, 1.1 Hz, 2H), 7.10 (d, *J =* 8.9 Hz, 2H), 6.69 (t, *J =* 7.8 Hz, 1H), 6.64 (d, *J =* 9.0 Hz, 2H), 6.32 (s, 1H), 6.25 (d, *J =* 6.7 Hz, 1H), 6.10 (d, *J* = 7.0 Hz, 1H), 5.23 (s, 1H), 4.79 (d, *J =* 5.5 Hz, 1H), 4.73 (d, *J =* 3.6 Hz, 1H), 4.20 (d, *J =* 13.4 Hz, 1H), 4.01 (dd, *J =* 13.4, 6.1 Hz, 1H), 3.88 (s, 3H), 3.63 (s, 3H), 3.25 (s, 3H), 2.76 (s, 3H).

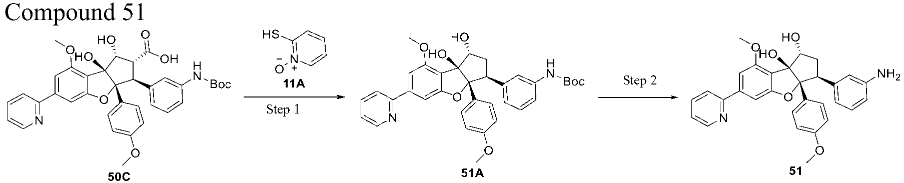

Step 1: The racemate of compound 50C (25 mg, 0.04 mmol), compound 11A (49.59 mg, 0.39 mmol), HOAT (7.97 mg, 0.06 mmol), tert-dodecyl mercaptan (78.88 mg, 0.39 mmol), TMP (7.13 mg, 0.06 mmol), Zinc meso-tetraphenylporphyrin (2.65 mg, 0.004 mmol), and DMF (2 mL) were mixed in a microwave tube. The reaction system was purged with nitrogen. Then, the microwave tube was placed in hot water at 80 °C, and the system was stirred while being irradiated with 25 W red light. A solution of EDCI (74.58 mg, 0.39 mmol) in DMF (2 mL) was slowly added dropwise to the reaction solution, and then the mixture was stirred for 30 min. After the reaction was completed, the reaction solution was purified on a silica gel column (H₂O/ACN = 40:60) to give a racemate of the target product 51A (14 mg, yield: 60%). LCMS [M+H]⁺ = 597.2.

Step 2: The racemate of compound 51A (12 mg, 0.02 mmol) was dissolved in THF (2 mL), and 6 M HCl (4 mL) was added. The reaction solution was stirred at room temperature (20 °C) for 4 h, then concentrated to dryness by rotary evaporation, and purified on a silica gel column (H₂O/ACN = 55%:45%) to give a racemate of the target product 51 (3 mg, yield: 30%), which was further resolved on a chiral column to give compound 51.

LCMS [M+H]⁺ = 497.2.

¹H NMR (400 MHz, DMSO) δ 8.68 (d, J = 3.9 Hz, 1H), 8.03 (d, J = 8.1 Hz, 1H), 7.89 (td, J = 7.8, 1.8 Hz, 1H), 7.40 - 7.35 (m, 1H), 7.33 - 7.27 (m, 2H), 7.07 (d, J = 9.0 Hz, 2H), 6.73 (t, J = 7.7 Hz, 1H), 6.63 (d, J = 9.0 Hz, 2H), 6.37 (s, 1H), 6.23 (d, J = 7.8 Hz, 2H), 5.33 (t, J = 4.6 Hz, 1H), 4.92 (s, 1H), 4.80 (s, 1H), 4.57 (d, J = 3.6 Hz, 1H), 4.50 (d, J = 4.1 Hz, 1H), 3.87 (s, 3H), 3.63 (s, 3H), 2.00 (dd, J = 15.0, 7.3 Hz, 3H).

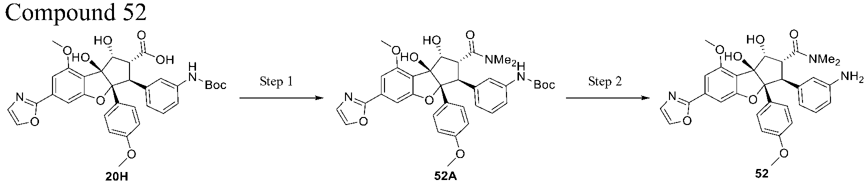

Step 1: The racemate of compound 20H (30 mg, 0.048 mmol) was dissolved in DMF (3 mL), and a dimethylamine solution (0.12 mL, 0.24 mmol, 2 M in THF), HATU (36.18 mg, 0.095 mmol), HOAT (12.95 mg, 0.095 mmol), and TMP (17.27 mg, 0.14 mmol) were added. The mixture was stirred at 25 °C for 2 h. Water was added to quench the reaction, and the mixture was extracted with ethyl acetate twice. The organic phase was concentrated and then purified on a silica gel column (EA/PE) to give a racemate of 52A (20 mg, yield: 65%) as a yellow solid. LCMS [M+H]⁺ = 658.2.

Step 2: The racemate of compound 52A (20 mg, 0.030 mmol) was dissolved in THF (1 mL), and 6 M HCl (1 mL) was added. The mixture was stirred at 25 °C for 16 h. The reaction solution was filtered and purified on a reversed-phase column (ACN:0.1% FA-H₂O) to give a racemate of 52 (9.4 mg, yield: 55%) as a yellow solid, which was further resolved on a chiral column to give compound 52.

LCMS [M+H]⁺ = 558.2.

¹H NMR (400 MHz, DMSO) δ 8.24 (s, 1H), 7.40 (s, 1H), 7.16 (d, *J =* 15.0 Hz, 2H), 7.08 (d, *J*= 8.9 Hz, 2H), 6.63 (t, *J =* 8.4 Hz, 3H), 6.23 (s, 1H), 6.15 (d, *J =* 7.9 Hz, 1H), 6.01 (d, *J =* 7.6 Hz, 1H), 5.28 (s, 1H), 4.80 - 4.69 (m, 4H), 4.20 (d, *J =* 13.4 Hz, 1H), 4.02 (dd, *J =* 13.5, 5.5 Hz, 1H), 3.85 (s, 3H), 3.62 (s, 3H), 3.25 (s, 3H), 2.77 (s, 3H).

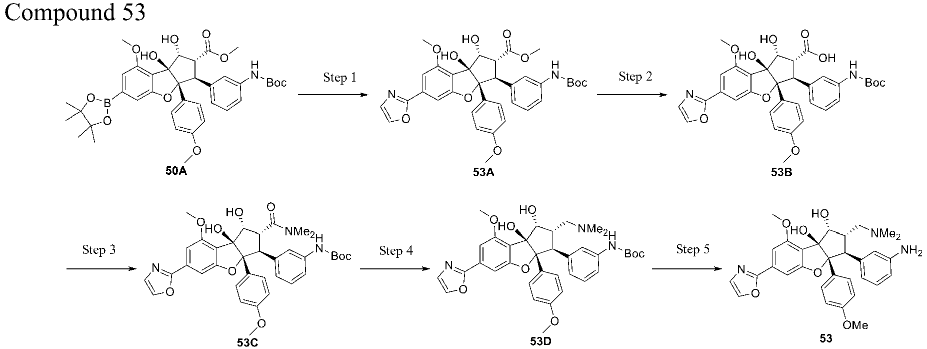

Step 1: The racemate of compound 50A (250 mg, 0.3553 mmol) was dissolved in dioxane (3 mL) and water (0.6 mL), and 2-bromooxazole (525.73 mg, 3.5532 mmol), Pd(dppf)Cl₂ (28.99 mg, 0.0355 mmol), and Na₂CO₃ (150.78 mg, 1.4225 mmol) were added. The mixture was stirred under microwave at 130 °C for 2.5 h. The reaction solution was filtered, concentrated, and then purified on a silica gel column (EA/PE) to give a racemate of 53A (200 mg, yield: 87%) as a yellow solid. LCMS [M+H-C₄H₈]⁺ = 589.2.

Step 2: The racemate of compound 53A (200 mg, 0.3102 mmol) was dissolved in THF (6 mL) and H₂O (2 mL), and LiOH (40 mg, 0.9307 mmol) was added. The mixture was stirred at 20 °C for 16 h. The reaction solution was adjusted to pH = 5 with 3 M HCl, concentrated, and then purified on a silica gel column (DCM:MeOH = 10:1) to give a racemate of 53B (150 mg, yield: 77%) as a yellow solid. LCMS [M-C₄H₈+H]⁺ = 575.1.

Step 3: The racemate of compound 53B (150 mg, 0.238 mmol) was dissolved in DMF (5 mL), and a dimethylamine solution (0.6 mL, 1.2 mmol, 2 M in THF), HATU (180.99 mg, 0.476 mmol), HOAT (64.79 mg, 0.476 mmol), and TMP (86.43 mg, 0.714 mmol) were added. The mixture was stirred at 25 °C for 2 h. The reaction solution was filtered, concentrated, and then purified on a reversed-phase column (ACN:0.1% FA-H₂O) to give a racemate of 53C (120 mg, yield: 77%) as a yellow solid. LCMS [M+H]⁺ = 658.2.

Step 4: The racemate of compound 53C (60 mg, 0.091 mmol) was dissolved in THF (2 mL). Under a nitrogen atmosphere, BH₃·THF (0.45 mL) was added, and the mixture was stirred at 60 °C for 3 h. Then, MeOH was added, and the mixture was stirred at 60 °C for 16 h. The reaction solution was concentrated and purified on a reversed-phase column (ACN:0.1% FA-H₂O) to give a racemate of 53D (30 mg, yield: 52%) as a yellow solid. LCMS [M+H]⁺ = 644.2.

Step 5: The racemate of compound 53D (30 mg, 0.05 mmol) was dissolved in THF (1 mL), and 6 M HCl (1 mL) was added. The mixture was stirred at 25 °C for 16 h. The reaction solution was directly purified on a reversed-phase column (ACN:0.1%FA-H₂O) to give a racemate of 53 (17 mg, yield: 68%) as a white solid, which was further resolved on a chiral column to give compound 53.

LCMS [M+H]⁺ = 544.2.

¹H NMR (400 MHz, DMSO) δ 8.25 (d, J = 0.7 Hz, 1H), 7.41 (d, J = 0.8 Hz, 1H), 7.19 - 7.15 (m, 4H), 6.88 (s, 3H), 6.64 (d, J = 9.0 Hz, 3H), 5.41 (d, J = 111.7 Hz, 2H), 4.71 (s, 1H), 3.87 (s, 3H), 3.62 (s, 3H), 3.24 (s, 1H), 2.95 (d, J = 4.3 Hz, 4H), 2.81 (d, J = 4.7 Hz, 4H), 1.24 (s, 1H).

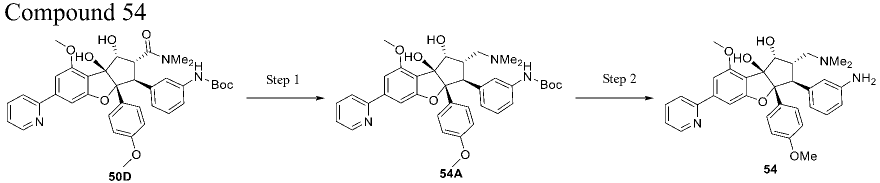

Step 1: The racemate of compound 50D (25.0 mg, 0.04 mmol) was dissolved in THF (1 mL), and BH₃·DMS (0.04 mL, 0.11 mmol) was added. The mixture was stirred at 60 °C for 2 h. Methanol (1 mL) was added, and the mixture was stirred at 60 °C for 18 h. The reaction solution was concentrated and purified on a silica gel column (DCM/MeOH) to give a racemate of compound 54A (10 mg, yield: 41%). LCMS [M+H]⁺ = 654.2.

Step 2: The racemate of compound 54A (10.0 mg, 0.015 mmol) was mixed with THF (1 mL), and 6 M HCl (1 mL) was added at 0 °C. The mixture was stirred at 25 °C for 16 h. The reaction solution was concentrated and then purified by preparative HPLC to give a racemate of 54 (1.2 mg, yield: 14%) as a white solid, which was further resolved on a chiral column to give compound 54.

LCMS [M+H]+ = 554.2.

¹H NMR (400 MHz, DMSO) δ 8.67 (d, *J=* 3.8 Hz, 1H), 8.01 (d, *J=* 8.0 Hz, 1H), 7.89 (t, *J* = 7.8 Hz, 1H), 7.39 - 7.33 (m, 1H), 7.27 (s, 2H), 7.18 (d, *J =* 8.9 Hz, 2H), 6.70 (d, *J =* 7.8 Hz, 1H), 6.62 (d, *J* = 9.0 Hz, 2H), 6.30 (s, 1H), 6.20 (d, *J =* 8.3 Hz, 1H), 6.10 (d, *J =* 7.6 Hz, 1H), 5.33 (d, *J =* 4.7 Hz, 2H), 5.14 (s, 1H), 4.81 (s, 1H), 4.54 (s, 1H), 3.86 (s, 3H), 3.62 (s, 3H), 3.48 (d, *J =* 13.7 Hz, 1H), 2.04 - 1.98 (m, 8H), 1.29 - 1.29 (m, 1H).

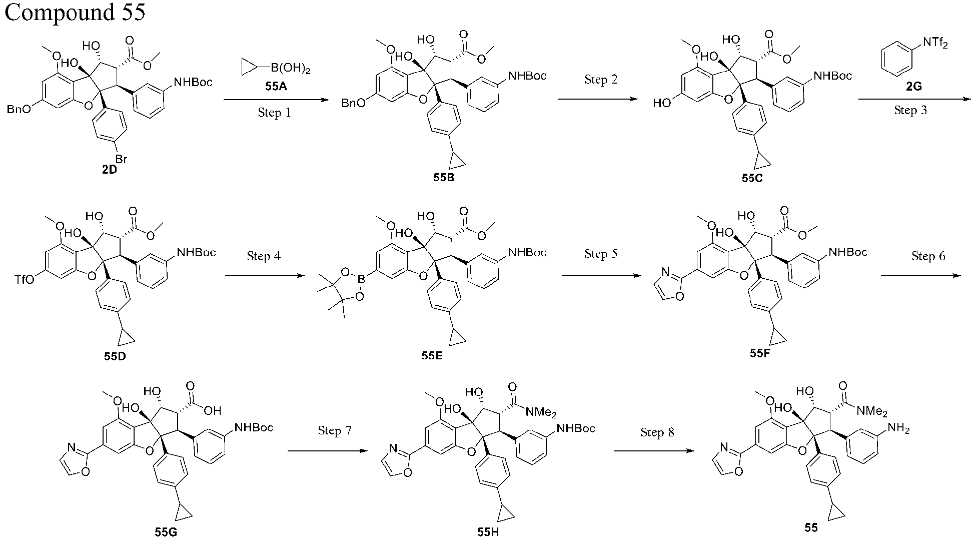

Step 1: Compound 55A (212 mg, 2.46 mmol), K₃PO₄ (695 mg, 3.28 mmol), Cy₃P (92 mg, 0.33 mmol), and Pd(OAc)₂ (37 mg, 0.164 mmol) were added to a mixture of the racemate of compound 2D (600 mg, 0.82 mmol) in toluene (25 mL) and H₂O (10 mL), and the reaction solution was stirred at 120 °C for 3 h under a nitrogen atmosphere. The reactants were poured into water (20 mL) and extracted with EA (50 mL). The organic phase was washed with 20 mL of brine, dried, concentrated, and purified by silica gel column chromatography (PE/EA = 2/1) to give a racemate of 55B (450 mg, yield: 79%) as a yellow oil. LCMS [M+Na]⁺ = 716.4.

Step 2: Pd(OH)₂/C (10%, 100 mg) was added to a mixed solution of the racemate of compound 55B (450 mg, 0.65 mol) in EA/MeOH (1:1, 25 mL). The mixture was purged with hydrogen three times and stirred at 25 °C for 18 h. The operation was repeated three times until the starting materials were completely consumed. The reaction solution was filtered, and the filter cake was washed with MeOH/DCM (1/10). The filtrates were combined and concentrated to give a racemate of 55C (380 mg, yield: 97%) as a white solid. LCMS [M+Na]⁺ = 626.2.

Step 3: K₂CO₃ (174 mg, 1.26 mmol) was added to a solution of the racemate of compound 55C (380 mg, 0.63 mmol) and compound 2G (225 mg, 0.63 mmol) in DMF (15 mL), and the mixture was stirred at 60 °C for 2 h, then poured into H₂O (15 mL), and extracted with EtOAc (30 mL). The organic phase was washed with water (15 mL) and brine (15 mL), dried over anhydrous Na₂SO₄, filtered, and then concentrated. The crude product was purified by silica gel column chromatography (DCM/EA = 0-30%) to give a racemate of 55D (350 mg, yield: 76%) as a white solid. LCMS [M+Na]⁺ = 758.0.

Step 4: Under a nitrogen atmosphere, bis(pinacolato)diboron (69 mg, 0.272 mmol), potassium acetate (40 mg, 0.408 mmol), and Pd(dppf)Cl₂·DCM (17 mg, 0.02 mmol) were added to a solution of the racemate of compound 55D (50 mg, 0.068 mmol) in dioxane (20 mL), and the mixture was stirred at 105 °C for 18 h. The reaction solution was concentrated and purified by silica gel column chromatography (DCM/EA = 3/1) to give a racemate of 55E (40 mg, yield: 83%) as an oil. LCMS [M+Na]⁺ = 736.4.

Step 5: Under a nitrogen atmosphere, 2-bromooxazole (50 mg, 0.34 mmmol), Pd(dppf)Cl₂·DCM (14 mg, 0.017 mmol), and Na₂CO₃ (27 mg, 0.25 mmol) were added to a mixture of the racemate of compound 55E (58 mg, 0.084 mmol) in dioxane (4 mL) and H₂O (2 mL), and the resulting mixture was stirred under microwave at 90 °C for 2 h. Water (10 mL) was added, and the mixture was extracted with EA (50 mL). The organic phase was washed with water (20 mL) and brine (20 mL), dried, and concentrated. The crude product was purified by flash silica gel column chromatography (DCM/EA = 2:1) to give a racemate of 55F (38 mg, yield: 70%) as a white solid. LCMS [M+Na]⁺ = 677.3.

Step 6: H₂O (2 mL), MeOH (2 mL), and LiOH (8 mg, 0.174 mmol) were added to a solution of the racemate of compound 55F (38 mg, 0.058 mmol) in THF (2 mL), and the mixture was stirred at 25 °C for 18 h. The reaction solution was directly purified on a reversed-phase column (MeCN/FA/H₂O) to give a racemate of 55G (25 mg, yield: 68%) as a white solid. LCMS [M+Na]⁺ = 663.3.

Step 7: A dimethylamine solution (0.1 mL, 0.2 mmol, 2 M in THF), HATU (15 mg, 0.04 mol), and TMP (10 mg, 0.08 mmol) were added to a solution of the racemate of compound 55G (25 mg, 0.04 mmol) in DMF (2 mL), and the mixture was stirred at 20 °C for 2 h. The reaction solution was directly purified on a reversed-phase column (MeCN/H₂O/FA) to give a racemate of 55H (18 mg, yield: 69%) as a white solid. LCMS [M+H]⁺ = 668.3.

Step 8: The racemate of compound 55H (18 mg, 0.027 mmol) was dissolved in THF (2 mL), and 6 M HCl (2 mL) was added. The mixture was stirred at 25 °C for 18 h. The reaction solution was purified by reversed-phase column chromatography (MeCH/FA/H₂O) to give a racemate of 55 (6.0 mg, yield: 40%) as a white solid, which was further resolved on a chiral column to give compound 55.

LCMS [M+H]⁺ = 568.3.

¹H NMR (400 MHz, DMSO) δ 8.25 (s, 1H), 7.41 (s, 1H), 7.18 (s, 1H), 7.14 (s, 1H), 7.04 (d, *J* = 8.3 Hz, 2H), 6.76 (d, *J* = 8.3 Hz, 2H), 6.63 (t, *J* = 7.7 Hz, 1H), 6.24 (s, 1H), 6.15 (d, *J* = 7.5 Hz, 1H), 6.02 (d, *J =* 7.4 Hz, 1H), 5.29 (s, 1H), 4.78 (d, *J* = 4.3 Hz, 2H), 4.74 (d, *J* = 5.2 Hz, 1H), 4.22 (d, *J* = 13.4 Hz, 1H), 4.08 - 4.00 (m, 1H), 3.85 (s, 3H), 3.26 (s, 3H), 2.77 (s, 3H), 1.74 (d, *J* = 4.9 Hz, 1H), 0.87 - 0.81 (m, 2H), 0.53 (d, *J=* 5.1 Hz, 2H).

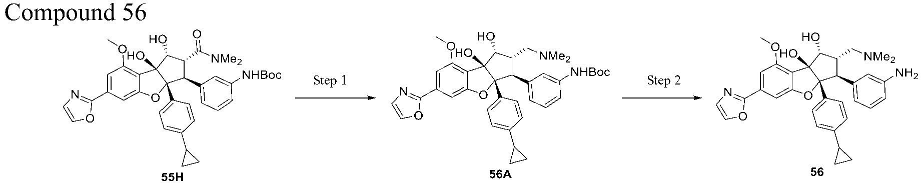

Step 1: A 1 M DIBAL-H solution (0.1 mL, 0.1 mmol) was added to a mixture of the racemate of compound 55H (3 mg, 0.004 mmol) in THF (1 mL). The resulting mixture was stirred at 20 °C for 5 h. The DIBAL-H solution (0.1 mL, 0.1 mmol) was further added, and the mixture was stirred at 60 °C for another 4 h. The reaction was quenched with methanol, and the reaction solution was concentrated. The crude product was purified by preparative TLC (DCM/MeOH) to give a racemate of 56A (2.5 mg, yield: 80%) as a white solid. LCMS [M+H]⁺ = 654.3.

Step 2: 6 M HCl (2 mL) was added to a solution of the racemate of compound 56A (3 mg, 0.005 mmol) in THF (2 mL), and the mixture was stirred at 25 °C for 18 h. The reaction solution was purified on a reversed-phase chromatography column (MeCN/FA/H₂O) to give a racemate of 56 (1.5 mg, yield: 60%) as a white solid, which was further resolved on a chiral column to give compound 56.

LCMS: [M+H]⁺ = 554.2.

¹H NMR (400 MHz, DMSO) δ 8.37 (s, 4H), 8.22 (s, 1H), 7.39 (s, 1H), 7.21 (s, 1H), 7.10 (d, *J* = 9.0 Hz, 4H), 6.75 (d, *J =* 8.5 Hz, 2H), 6.68 (t, *J =* 7.7 Hz, 2H), 6.28 (s, 1H), 6.18 (d, *J* = 7.6 Hz, 1H), 6.11 (d, *J* = 7.9 Hz, 1H), 5.33 (t, *J* = 4.6 Hz, 1H), 5.16 (s, 1H), 4.77 (s, 1H), 4.46 (d, *J* = 4.4 Hz, 1H), 3.83 (s, 3H), 3.53 (s, 2H), 2.20 (s, 6H), 1.97 (s, 1H), 1.73 (d, *J* = 5.4 Hz, 1H), 0.85-0.83 (m, 2H), 0.53-0.51 (m, 2H).

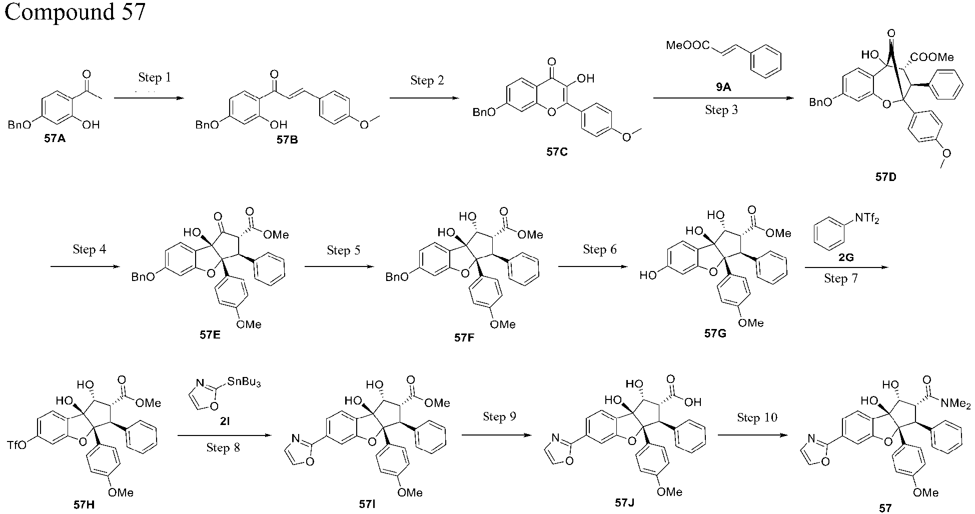

Step 1: Compound 57A (24 g, 99.06 mmol) and 4-methoxybenzaldehyde (16.19 g, 118.87 mmol) were dissolved in EtOH (1 L), and 50% NaOH (80 mL) was added at 50 °C. The mixture was stirred at 50 °C for 12 h. The reaction solution was concentrated, and the resulting crude product was diluted with H₂O (500 mL), extracted with DCM (500 mL × 2), washed with brine (500 mL × 2), dried over Na₂SO₄, filtered, and concentrated to give 57B (19.87 g, yield: 56%) as a yellow solid. LCMS [M+H]⁺ = 361.2.

Step 2: Compound 57B (18.8 g, 52.16 mmol) was dissolved in MeOH/H₂O (100 mL/50 mL), and NaOH (14.61 g, 365.10 mmol) and H₂O₂ (80 mL) were added. The mixture was stirred at 50 °C for 12 h. The reaction solution was filtered, and the filter cake was washed with diethyl ether (100 mL) and then dissolved in 200 mL of DCM. The resulting solution was adjusted to pH = 5 with 1N HCl (100 mL), washed with brine (100 mL × 2), dried over Na₂SO₄, filtered, and concentrated to give 57C (8.9 g, yield: 46%) as a yellow solid. LCMS [M+H]⁺ = 375.0.

Step 3: Compound 57C (2 g, 5.35 mmol) and compound 9A (5.197 g, 32.09 mmol) were dissolved in CHCl₃/TFE (7/3, 15 mL × 2). The reaction mixture was injected (12 mL/h) via a syringe using a syringe pump into a coil wound around a jacketed glass cylinder equipped with a 250 W ultraviolet lamp. The temperature was adjusted to maintain the internal reaction temperature at 0-5 °C, while the UV lamp was turned on simultaneously. The reaction mixture was irradiated for 1 h and then concentrated under reduced pressure to remove the solvent. The residue was purified by a silica gel plug (EtOAc/hexane) to remove excess cinnamate, thus giving a racemate of 57D (8.1 g, total yield: 75%) as a pale yellow solid. LCMS [M+H]⁺ = 537.7.

Step 4: The racemate of compound 57D (8.6 g, 16.03 mmol) was dissolved in methanol (120 mL), and sodium methoxide solid (2.163 g, 40.07 mmol) was slowly added under an ice-water bath (0 °C). The reaction solution was placed in an oil bath at 65 °C and stirred for 1.5 h. After the reaction was completed, the reaction solution was concentrated to dryness by rotary evaporation to remove the methanol, and then extracted with ethyl acetate (200 mL × 3). The organic phase was washed once with an ammonium chloride solution (100 mL) and once with saturated brine (100 mL), dried over anhydrous sodium sulfate, filtered, concentrated, and then purified on a silica gel column (0.2% FA in EA/PE (65%/35%)) to give a racemate of compound 57E (7.0 g, yield: 81%). LCMS [M+H-H₂O]⁺ = 519.7.

Step 5: The racemate of compound 57E (7.0 g, 9.39 mmol) was dissolved in a mixed solution of acetonitrile/chloroform (80 mL/80 mL), and acetic acid (7.827 g, 130.46 mmol) and STAB (13.828 g, 65.23 mmol) were slowly added under an ice-water bath (0 °C). The reaction solution was stirred at room temperature for 2 h. After the reaction was completed, the reaction solution was concentrated to dryness by rotary evaporation to remove the solvent, and then extracted with ethyl acetate (200 mL × 3). The organic phase was washed with saturated brine (100 mL), dried over anhydrous sodium sulfate, filtered, concentrated, and then purified on a silica gel column (0.2% FA in EA/PE = 50%/50%) to give a racemate of compound 57F (3.8 g, yield: 54%). LCMS [M+H-H₂O]⁺ = 521.8.

Step 6: The racemate of compound 57F (3.8 g, 7.06 mmol) was dissolved in methanol (50 mL), and 10% Pd/C (1.14 g) was added. The mixture was purged with hydrogen three times and stirred at 20 °C for 16 h. The reaction solution was filtered, concentrated, and purified on a silica gel column (PE:EA = 40:60) to give a racemate of compound 57G (3.0 g, yield: 85%). LCMS [M+H-H₂O]⁺ = 431.2.

Step 7: Under a nitrogen atmosphere, the racemate of compound 57G (2.8 g, 6.24 mmol) was dissolved in DMF (30 mL), then compound 2G (2.23 g, 6.24 mmol) and K₂CO₃ (1.723 g, 12.49 mmol) were added, and the mixture was heated to 60 °C and allowed to react for 1 h. After the reaction was completed, the reaction solution was quenched with water (100 mL) and extracted with EA (200 mL) three times. The organic phase was washed with saturated brine, dried over anhydrous sodium sulfate, filtered, concentrated, and then purified on a silica gel column (PE:EA = 60:40) to give a racemate of compound 57H (2.7 g, yield: 75%). LCMS [M+H-H₂O]⁺ = 563.0. Step 8: Under a nitrogen atmosphere, the racemate of compound 57H (200.0 mg, 0.34 mmol) was dissolved in DMF (3 mL), then compound 2I (333.3 mg, 0.93 mmol), Pd(PPh₃)₄ (139.9 mg, 0.03 mmol), and CuI (13.1 mg, 0.069 mmol) were added, and the mixture was stirred at 100 °C for 1 h. The reaction solution was quenched with water and extracted with EA. The organic layer was washed with water and brine, dried over anhydrous Na₂SO₄, filtered, concentrated, and then purified on a silica gel column (DCM/MeOH = 20:1) to give a racemate of compound 57I (100 mg, yield: 58%). LCMS [M+H]+ = 500.7.

Step 9: The racemate of compound 57I (100.0 mg, 0.20 mmol) was dissolved in THF/H₂O/MeOH (3 mL/1 mL/1 mL), and LiOH (25.3 mg, 0.60 mmol) was added. The mixture was stirred at 40 °C for 3 h, adjusted to pH = 5 with 3 M hydrochloric acid, concentrated, and then purified on a silica gel column (DCM/MeOH) to give a racemate of compound 57J (90 mg, yield: 92%). LCMS [M+H]⁺ = 486.7.

Step 10: The racemate of compound 57J (90.0 mg, 0.19 mmol), HATU (105.8 mg, 0.28 mmol), and DIEA (119.7 mg, 0.93 mmol) were dissolved in DMF (2 mL), and the solution was stirred at 25 °C for 10 min. DMA (0.6 µL, 0.93 mmol, 2 M in THF) was added, and the mixture was allowed to react at 25 °C for 1 h. The solution was diluted with water, extracted with EA, washed with water and brine, dried over anhydrous Na₂SO₄, filtered, concentrated, and then purified on a silica gel column (DCM/MeOH) to give a racemate of compound 57 (6.5 mg, yield: 6.8%), which was further resolved on a chiral column to give compound 57.

LCMS [M+H]+ = 513.8.

¹H NMR (400 MHz, DMSO) δ 8.25 (s, 1H), 7.73 - 7.50 (m, 3H), 7.41 (s, 1H), 7.12 (d, *J =* 8.8 Hz, 2H), 7.06 - 6.95 (m, 3H), 6.83 (d, *J =* 7.1 Hz, 2H), 6.68 (d, *J* = 8.9 Hz, 2H), 4.94 (d, *J* = 8.1 Hz, 1H), 4.19 (d, *J* = 13.2 Hz, 1H), 4.02 (dd, *J* = 13.1, 8.1 Hz, 1H), 3.65 (s, 3H), 3.20 (s, 3H), 2.72 (s, 3H).

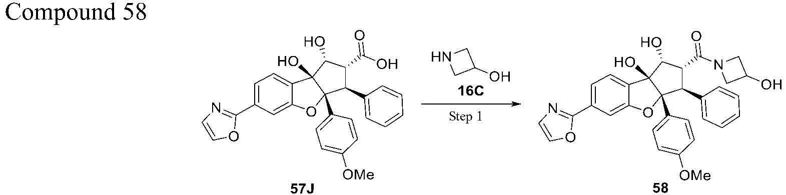

Step 1: The racemate of compound 57J (30.0 mg, 0.06 mmol), HATU (70.5 mg, 0.19 mmol), and DIEA (39.9 mg, 0.31 mmol) were dissolved in DMF (2 mL), and the mixture was stirred at 25 °C for 10 min. Compound 16C (34.0 mg, 0.31 mmol) was added, and the mixture was allowed to react at 25 °C for 1 h. The reaction solution was diluted with water, extracted with EA, washed with water and brine, dried over anhydrous Na₂SO₄, and purified on a silica gel column (DCM/MeOH) to give a racemate of compound 58 (14.1 mg, yield: 42%), which was further resolved on a chiral column to give compound 58.

LCMS [M+H]⁺ = 541.2.

¹H NMR (400 MHz, DMSO) δ 8.24 (s, 1H), 7.58 (d, *J =* 14.3 Hz, 3H), 7.40 (s, 1H), 7.05 (ddd, *J* = 21.5, 11.8, 6.0 Hz, 5H), 6.86 (d, *J* = 7.2 Hz, 2H), 6.67 (dd, *J* = 8.6, 7.0 Hz, 2H), 5.73 (dd, *J* = 10.7, 5.7 Hz, 1H), 5.59 (dd, *J* = 5.8, 2.5 Hz, 1H), 5.53 (d, *J =* 3.3 Hz, 1H), 4.83 (dt, *J =* 10.3, 7.7 Hz, 1H), 4.53 (dt, *J* = 40.9, 11.6 Hz, 2H), 4.15 (dd, *J* = 13.2, 7.7 Hz, 1H), 4.10 - 4.03 (m, 1H), 3.93 (ddd, *J* = 25.7, 13.2, 8.3 Hz, 1H), 3.78 - 3.66 (m, 1H), 3.65 (t, *J* = 5.7 Hz, 3H), 3.50 (ddd, *J* = 33.5, 10.0, 4.2 Hz, 1H).

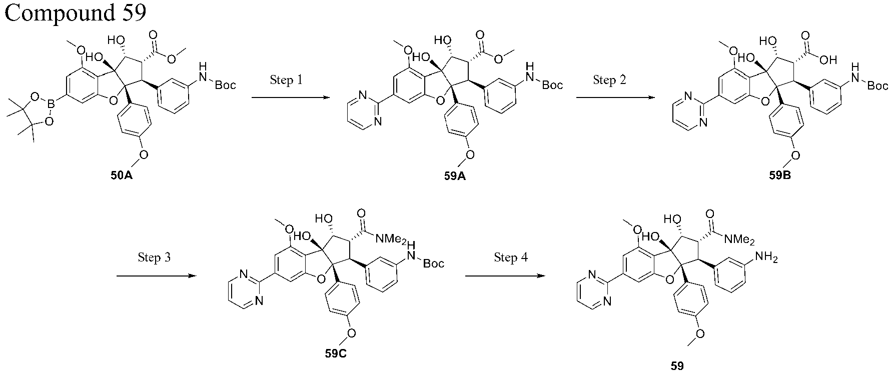

Step 1: The racemate of compound 50A (100 mg, 0.1421 mmol) was dissolved in dioxane (2 mL) and water (0.4 mL), and 2-bromopyrimidine (112.98 mg, 0.7106 mmol), Pd(dppf)Cl₂ (11.60 mg, 0.0142 mmol), and Na₂CO₃ (60.26 mg, 0.5685 mmol) were added. The mixture was stirred under microwave at 130 °C for 2 h. The reaction solution was filtered, concentrated, and then purified on a silica gel column (EA/PE) to give a racemate of 59A (70 mg, yield: 75%) as a yellow solid. LCMS [M+H-H₂O]⁺ = 638.2.

Step 2: The racemate of compound 59A (60 mg, 0.09 mmol) was dissolved in THF (2 mL) and H₂O (0.7 mL), and LiOH (11.5 mg, 0.27 mmol) was added. The mixture was stirred at 20 °C for 16 h. The reaction solution was adjusted to pH = 5 with 3 M HCl, concentrated, and then purified on a silica gel column (DCM:MeOH = 10:1) to give a racemate of 59B (40 mg, yield: 69%) as a yellow solid. LCMS [M+H-C₄H₈]⁺ = 586.2.

Step 3: The racemate of compound 59B (40 mg, 0.062 mmol) was dissolved in DMF (2 mL), and a dimethylamine solution (0.16 mL, 0.32 mmol, 2 M in THF), HATU (47.40 mg, 0.312 mmol), HOAT (16.97 mg, 0.125 mmol), and TMP (22.62 mg, 0.187 mmol) were added. The mixture was stirred at 25 °C for 2 h. The reaction solution was filtered, concentrated, and then purified on a reversed-phase column (ACN:0.1% FA-H₂O) to give a racemate of 59C (31 mg, yield: 76%) as a yellow solid. LCMS [M+H]⁺ = 669.2.

Step 4: The racemate of compound 59C (7 mg, 0.01 mmol) was dissolved in THF (1 mL), and 6 M HCl (0.5 mL) was added. The mixture was stirred at 20 °C for 16 h. The reaction solution was directly purified on a reversed-phase column (ACN:0.1% FA-H₂O) to give a racemate of 59 (4.0 mg, yield: 67%) as a white solid, which was further resolved on a chiral column to give compound 59.

LCMS [M+H]⁺ = 569.2.

¹H NMR (400 MHz, DMSO) δ 8.94 (d, J = 4.9 Hz, 2H), 7.65 - 7.61 (m, 2H), 7.48 (t, J = 4.9 Hz, 1H), 7.10 (d, J = 8.9 Hz, 2H), 6.65 - 6.60 (m, 3H), 6.27 (s, 1H), 6.17 (d, J = 7.1 Hz, 1H), 6.00 (d, J = 7.8 Hz, 1H), 5.26 (s, 1H), 4.78 (d, J = 5.8 Hz, 1H), 4.74 (d, J = 4.0 Hz, 1H), 4.19 (d, J = 13.5 Hz, 1H), 4.01 (dd, J = 13.6, 6.0 Hz, 1H), 3.87 (s, 3H), 3.63 (s, 3H), 3.25 (s, 3H), 2.76 (s, 3H).

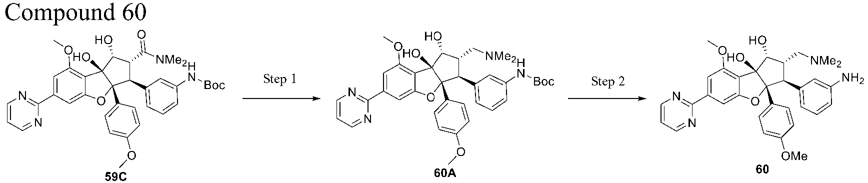

Step 1: The racemate of compound 59C (10 mg, 0.015 mmol) was dissolved in THF (1.5 mL), and DIBAL-H (0.045 mL, 0.045 mmol) was added at 0 °C under a nitrogen atmosphere. The mixture was allowed to react at 22 °C for 1 h. DIBAL-H (0.045 mL, 0.045 mmol) was then added at 0 °C under a nitrogen atmosphere, and the mixture was allowed to react at 22 °C for 1 h. DIBAL-H (0.045 mL, 0.045 mmol) was then added at 0 °C under a nitrogen atmosphere, and the mixture was allowed to react at 22 °C for 1 h. The reaction solution was quenched with ice water, concentrated, and then purified on a reversed-phase column (ACN:0.1% FA-H₂O) to give a racemate of 60A (5 mg, yield: 51%) as a yellow solid. LCMS [M+H]⁺ = 655.5.

Step 2: The racemate of compound 60A (5 mg, 0.008 mmol) was dissolved in THF (1 mL), and 6 M HCl (1 mL) was added. The mixture was stirred at 25 °C for 16 h. The reaction solution was directly purified on a reversed-phase column (ACN:0.1% FA-H₂O) to give a racemate of 60 (2.5 mg, yield: 59%) as a white solid, which was further resolved on a chiral column to give compound 60.

LCMS [M+H]⁺ = 555.5.

¹H NMR (400 MHz, ) δ 8.92 (d, J = 4.8 Hz, 2H), 8.13 (s, 1H), 7.57 (d, J = 16.0 Hz, 2H), 7.46 (t, J = 4.8 Hz, 1H), 7.17 (d, J = 8.9 Hz, 2H), 6.69 (t, J = 7.7 Hz, 1H), 6.62 (d, J = 8.9 Hz, 2H), 6.28 (s, 1H), 6.20 (d, J = 7.7 Hz, 1H), 6.07 (d, J = 7.2 Hz, 1H), 5.20 (s, 1H), 4.81 (s, 2H), 4.54 (s, 1H), 3.86 (s, 3H), 3.62 (s, 3H), 3.47 (d, J = 13.8 Hz, 1H), 3.06 (s, 2H), 2.37 (s, 6H).

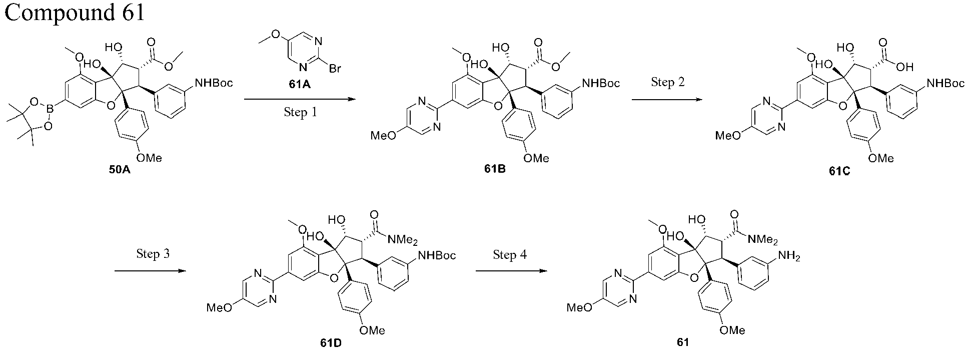

Step 1: Compound 61A (189 mg, 0.71 mmol), Pd(dppf)Cl₂ (11.6 mg, 0.014 mmol), and an aqueous solution (0.4 mL) of Na₂CO₃ (30.13 mg, 0.2842 mmol) were added to a solution of the racemate of compound 50A (100 mg, 0.142 mmol) in dioxane (2 mL). Under a nitrogen atmosphere, the mixture was stirred at 100 °C for 16 h. The reaction solution was directly purified on a silica gel column (PE/EA) to give a racemate of compound 61B (50 mg, yield: 50%). LCMS [M+H]⁺ = 687.0.

Step 2: The racemate of compound 61B (50 mg, 0.07 mmol) and LiOH·H₂O (9 mg, 0.22 mmol) were added to a mixed solution of THF/MeOH/H₂O (1/1/1, 3 mL), and the mixture was stirred at 20 °C for 16 h. The reaction solution was purified on a reversed-phase column (ACN-H₂O) (FA) to give a racemate of 61C (34 mg, yield: 69%) as a white solid. LCMS [M+H]⁺ = 673.0.

Step 3: The racemate of compound 61C (34 mg, 0.05 mmol), HATU (19 mg, 0.05 mmol), and TMP (14 mg, 0.12 mmol) were stirred in DMF (2 mL). Dimethylamine (11 mg, 0.1 mmol) was added to the mixture, and the resulting mixture was stirred at 20 °C for 2 h. The reaction solution was purified on a reversed-phase column (ACN/H₂O = 0-100%) to give a racemate of 61D (10 mg, yield: 50%) as a white solid. LCMS [M+H]⁺ = 700.1.

Step 4: The racemate of compound 61D (6 mg, 0.0086 mmol) was dissolved in THF (1 mL), and 6 M HCl (1 mL) was added. The mixture was stirred at 20 °C for 5 h. The reaction solution was directly purified on a reversed-phase column (ACN-H₂O) to give a racemate of 61 (3.8 mg, yield: 70%) as a white solid, which was further resolved on a chiral column to give compound 61. LCMS [M+H]⁺ = 600.0.

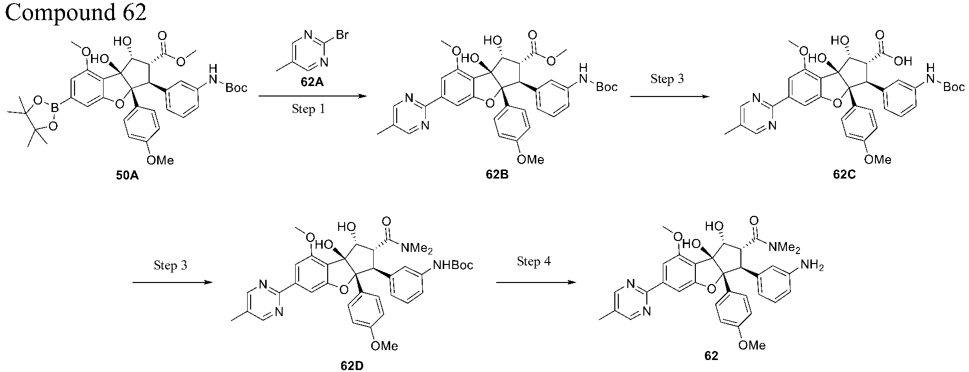

Step 1: Compound 62A (25.82 mg, 0.15 mmol), Na₂CO₃ (31.64 mg, 0.3 mmol), and Pd(dppf)Cl₂ (8.06 mg, 0.01 mmol) were added to a solution of the racemate of compound 50A (70 mg, 0.1 mmol) in dioxane/water (3 mL/0.6 mL). The reaction system was purged with nitrogen three times and allowed to react under microwave at 130 °C for 1 h. The reaction solution was purified by reversed-phase column chromatography (H₂O + 0.1% FA)/ACN) to give a racemate of 62B (50 mg, yield: 75%) as a yellow solid. LCMS [M+H]⁺ = 670.9.

Step 2: LiOH·H₂O (6.41 mg, 0.15 mmol) was added to a solution of the racemate of compound 62B (50 mg, 0.08 mmol) in THF/H₂O (1/1, 2 mL), and the mixture was stirred at room temperature for 18 h. The reaction solution was purified by reversed-phase column chromatography (H₂O/ACN) to give a racemate of 62C (25 mg, yield: 51%) as a white solid. LCMS [M+H-C₄H₈]⁺ = 600.9. Step 3: DIEA (7.88 mg, 0.06 mmol) was added to a solution of the racemate of compound 62C (20 mg, 0.03 mmol), dimethylamine (12.44 mg, 0.15 mmol), and HATU (17.4 mg, 0.05 mmol) in DMF (1 mL), and the mixture was stirred at room temperature for 1 h. The reaction solution was purified by reversed-phase column chromatography (H₂O/ACN) to give a racemate of 62D (25 mg, yield: 90%) as a white solid. LCMS [M+H]⁺ = 684.1.

Step 4: 6 M HCl (1 mL) was added to a solution of the racemate of compound 62D (5 mg, 0.01 mmol) in THF (1 mL), and the mixture was stirred at room temperature for 5 h. The reaction solution was purified by reversed-phase column chromatography (H₂O/ACN) to give a racemate of 62 (2.5 mg, yield: 60%) as a white solid, which was further resolved on a chiral column to give compound 62.

LCMS [M+H]⁺ = 583.2.

¹H NMR (400 MHz, DMSO) δ 8.78 (s, 2H), 7.59 (d, *J* = 4.3 Hz, 2H), 7.10 (d, *J* = 8.8 Hz, 2H), 6.63 (t, *J* = 7.8 Hz, 3H), 6.25 (s, 1H), 6.16 (d, *J* = 7.6 Hz, 1H), 5.98 (d, *J* = 7.4 Hz, 1H), 5.25 (s, 1H), 4.86 - 4.63 (m, 4H), 4.17 (d, *J* = 13.5 Hz, 1H), 3.99 (dd, *J* = 13.4, 6.0 Hz, 1H), 3.87 (s, 3H), 3.63 (s, 3H), 3.25 (s, 3H), 2.76 (s, 3H), 2.34 (s, 3H).

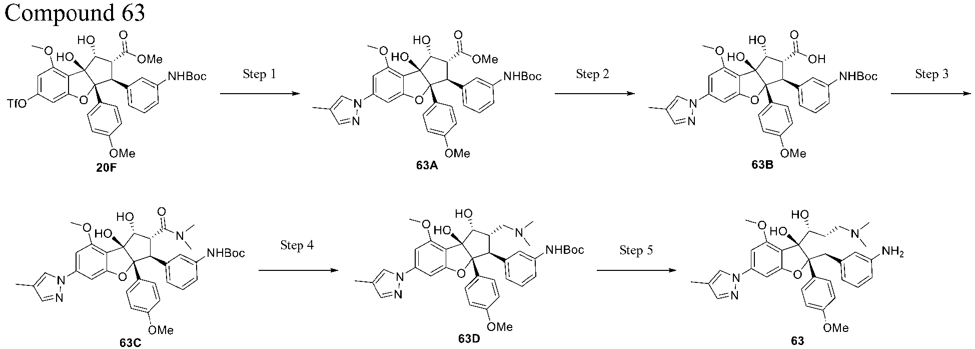

Step 1: The racemate of compound 20F (200 mg, 0.28 mmol) was dissolved in 1,4-dioxane (20 mL), and then K₂CO₃ (77.28 mg, 1.38 mmol), 4-methyl-1H-pyrazole (113.70 mg, 1.38 mol), Pd₂(dba)₃ (51.28 mg, 0.01 mmol), and t-Buxphos (67.31 mg, 0.14 mmol) were added. The mixture was stirred at 120 °C for 12 h. The reaction solution was concentrated, and the resulting crude product was purified on a FLASH column (low- to medium-pressure flash preparative liquid chromatography) (PE/EA = 2/1) to give a racemate of 63A (133.7 mg, yield: 73%) as a yellow solid.LCMS [M+H-C₄H₈]⁺ = 602.2.

Step 2: The racemate of compound 63A (133.7 mg, 0.20 mmol) was dissolved in THF/H₂O (3 mL/1 mL), and LiOH·H₂O (25.60 mg, 0.60 mmol) was added. The mixture was allowed to react at 15 °C for 12 h. The reaction solution was purified by preparative HPLC (H₂O/ACN) to give a racemate of 63B (92.9 mg, yield: 71%) as a yellow solid. LCMS [M+H-C₄H₈]⁺ = 588.2.

Step 3: The racemate of compound 63B (92.9 mg, 0.14 mmol) was dissolved in DMF (2 mL), and dimethylamine/THF (0.1 mL, 0.29 mmol), HATU (65.86 mg, 0.17 mmol), HOAT (23.57 mg, 0.17 mol), and TMP (52.47 mg, 0.43 mmol) were added. The mixture was allowed to react at room temperature for 2 h. The reaction solution was purified by preparative HPLC (H₂O/ACN) to give a racemate of 63C (71.9 mg, yield: 74%) as a white solid. LCMS [M+H]⁺ = 671.3.

Step 4: BH₃/DMS (0.13 mL, 0.33 mmol) and TFA (12.50 mg, 0.11 mmol) were added to a solution of the racemate of compound 63C (72 mg, 0.11 mmol) in THF (2.5 mL), and the mixture was allowed to react at 20 °C for 12 h. The reaction solution was purified by preparative HPLC (H₂O/ACN) to give a racemate of 63D (28.4 mg, yield: 40%) as a yellow oil. LC-MS: [M+H]⁺=657.2.

Step 5: 6 N HCl (1 mL) was added to a solution of the racemate of compound 63D (28.3 mg, 0.04 mmol) in THF (1 mL), and the mixture was allowed to react at 15 °C for 12 h. The reaction solution was purified by preparative HPLC (H₂O/ACN) to give a racemate of 63 (5.8 mg, yield: 24%) as a white solid, which was further resolved on a chiral column to give compound 63.

LCMS [M+H]⁺ = 557.2.

¹H NMR (400 MHz, DMSO-*d*₆) δ 8.31 (s, 1H), 8.24 (s, 1H), 7.54 (s, 1H), 7.14 (d, *J =* 8.8 Hz, 2H), 6.95 (s, 2H), 6.68 (t, *J* = 7.8 Hz, 1H), 6.62 (d, *J* = 9.2 Hz, 2H), 6.25 (s, 1H), 6.19-6.17 (m, 1H), 6.07 (d, *J* = 7.6 Hz, 1H), 5.05 (s, 1H), 4.80 (brs, 1H), 4.45 (d, *J* = 4.4 Hz, 1H), 3.82 (s, 1H), 3.62 (s, 3H), 3.48-3.44 (m, 2H), 3.02-2.95 (m, 1H), 2.33 (s, 6H), 2.11 (s, 3H).

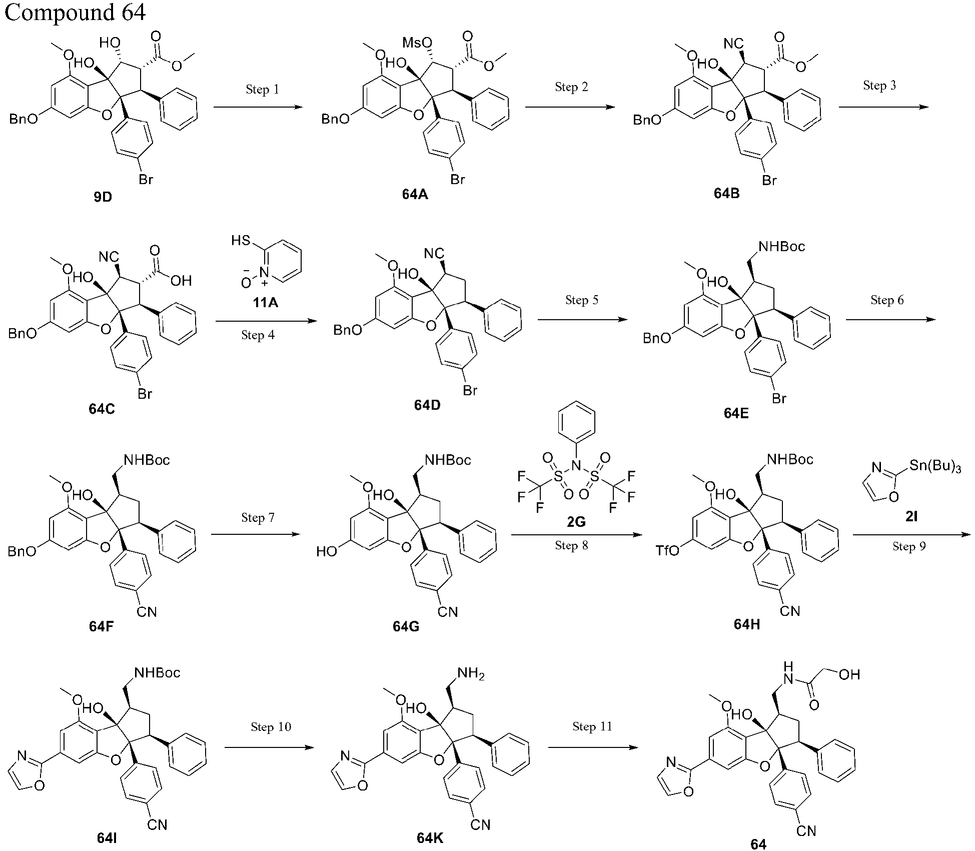

Step 1: MsCl (4250 mg, 37.3 mmol) was added to a mixture of the racemate of compound 9D (2300 mg, 3.73 mmol) in pyridine (30 mL) at 0 °C, and the resulting mixture was allowed to react at 0 °C for 3 h. EA (50 mL) was added to the reaction solution, and the mixture was washed with water (20 mL). The organic phase was dried, filtered, and concentrated, and then the crude product was purified on a silica gel column (PE/EA = 2/1) to give a racemate of 64A (2400 mg, yield: 93%) as a white solid. LCMS [M+Na]⁺ = 717.1.

Step 2: NaCN (423 mg, 8.63 mmol) was added to a solution of the racemate of compound 64A (2.4 g, 3.45 mmol) in DMF (20 mL), and the mixture was allowed to react at 25 °C for 18 h. EA (30 mL) was added to the reaction solution, and the mixture was washed separately with water (20 mL) and saturated brine. The organic phase was dried, concentrated, and purified on a FLASH column (low- to medium-pressure flash preparative liquid chromatography) (PE/EA = 2/1) to give a racemate of 64B (2200 mg, yield: 100%) as a white solid. LCMS [M+H]⁺ = 626.1.

Step 3: H₂O (5 mL), MeOH (5 mL), and LiOH·H₂O (443 mg, 10.54 mmol) were added to a solution of the racemate of compound 64B (2200 mg, 3.51 mmol) in THF (5 mL), and the mixture was allowed to react at 25 °C for 18 h. The reaction solution was purified on a reversed-phase chromatography column (MeCN/FA/H₂O) to give a racemate of 64C (1.9 g, yield: 88%) as a white solid. LCMS [M+H]⁺ = 612.2.

Step 4: The racemate of compound 64C (100 mg, 0.16 mmol), compound 11A (207.3 mg, 1.63 mmol), HOAT (33.3 mg, 0.24 mmol), tert-dodecyl mercaptan (329.8 mg, 1.63 mmol), TMP (29.8 mg, 0.24 mmol), Zinc meso-tetraphenylporphyrin (11.0 mg, 0.02 mmol), and DMF (2 mL) were mixed in a microwave tube. The reaction system was purged with nitrogen. Then, the microwave tube was placed in hot water at 80 °C, and the system was stirred while being irradiated with 25 W red light. A solution of EDCI (311.8 mg, 1.63 mmol) in DMF (4 mL) was slowly added dropwise to the reaction solution, and the mixture was stirred for 30 min and then purified on a reversed-phase chromatography column (H₂O/ACN = 40%-60%) to give a racemate of 64D (35 mg, yield: 38%) as a white solid. LCMS [M+H-H₂O]⁺ = 550.4.

Step 5: A 10 M BH₃·DMS solution (0.4 mL, 3.7 mmol) was added to a mixture of the racemate of compound 64D (210 mg, 0.37 mmol) in THF (10 mL), and the resulting mixture was stirred at 70 °C for 3 h. The reaction solution was cooled, and MeOH was slowly added. The mixture was concentrated to give a crude product. The crude product was dissolved in DCM (15 mL), and DIPEA (90 mg, 0.70 mmol) and Boc₂O (150 mg, 0.70 mmol) were added. The mixture was stirred at 25 °C for 5 h. The reaction solution was concentrated and purified on a FLASH column (low-to medium-pressure flash preparative liquid chromatography) (PE:EA = 5:1) to give a racemate of 64E (200 mg, yield: 85%) as a white solid. LCMS [M+Na]⁺ = 694.2.

Step 6: The racemate of compound 64E (160 mg, 0.24 mmol), Pd₂(dba)₃ (46 mg, 0.05 mmol), DPPF (55 mg, 0.1 mmol), and Zn(CN)₂ (74 mg, 0.63 mmol) were added to NMP (20 mL), and the mixture was heated to 140 °C and stirred for 2 h under a N₂ atmosphere. The reaction solution was diluted with water and extracted with EA. The organic phase was washed separately with water and brine, dried, and concentrated. The crude product was purified on a FLASH column (low- to medium-pressure flash preparative liquid chromatography) (PE:EA = 2:1) to give a racemate of 64F (130 mg, yield: 88%) as a white solid. LCMS: [M+H-H₂O-C₄H₈]⁺ = 545.3.

Step 7: Pd(OH)₂/C (10%, 50 mg) was added to a solution of the racemate of compound 64F (130 mg, 0.21 mol) in EA/MeOH (1:1, 25 mL), and the mixture was purged with H₂ several times and stirred at 25 °C for 5 h. The reaction solution was filtered, and the filter cake was washed with MeOH/DCM (1/10). The filtrates were combined, concentrated, and purified on a FLASH column (low- to medium-pressure flash preparative liquid chromatography) to give a racemate of 64G (60 mg, yield: 54%) as a white solid. LCMS: [M+Na]⁺ = 551.0.

Step 8: K₂CO₃ (32 mg, 0.228 mmol) was added to a solution of the racemate of compound 64G (60 mg, 0.114 mmol) and compound 2G (41 mg, 0.114 mol) in DMF (5 mL), and the mixture was stirred at 60 °C for 1 h, then poured into water, and extracted with EA. The organic phase was washed with water and brine, dried, and concentrated. The concentrate was purified on a silica gel chromatography column (EA-PE.DCM/0-50%), and the resulting crude product was further purified on a reversed-phase chromatography column to give a racemate of 64H (40 mg, yield: 53%) as a white solid. LCMS [M+H-CO₂-C₄H₈]⁺ = 561.0.

Step 9: Compound 2I (11 mg, 0.03 mmol), Pd(PPh₃)₄ (3 mg, 0.002 mmol), and CuI (1 mg, 0.004 mmol) were added to a solution of the racemate of compound 64H (5 mg, 0.007 mmol) in DMF (1 mL), and the mixture was stirred at 100 °C for 2 h under a nitrogen atmosphere. EA (10 mL) was added, and the mixture was washed separately with water (10 mL) and brine (10 mL). The organic phase was dried and concentrated. The crude product was purified on a preparative silica gel plate to give a racemate of 64I (3 mg, yield: 75%) as a white solid. LCMS [M+Na]⁺ = 602.2. Step 10: The racemate of compound 64I (3 mg, 0.044 mmol) was dissolved in 6 M HCl (1 mL) and THF (1 mL), and the solution was stirred at 25 °C for 5 h. The reaction solution was purified on a reversed-phase chromatography column (MeCN/FA/H₂O) to give a racemate of 64K (3 mg, yield: 100%) as a white solid. LCMS [M+Na]⁺ = 480.2.

Step 11: Glycolic acid (4 mg, 0.05 mmol), HATU (4 mg, 0.01 mmol), and TMP (3 mg, 0.02 mmol) were added to a mixture of the racemate of compound 64K (3 mg, 0.01 mmol) in DMF (1 mL), and the resulting mixture was stirred at 20 °C for 2 h. The reaction solution was purified on a reversed-phase chromatography column (MeCN/H₂O/FA) to give a racemate of 64 (2 mg, yield: 59%) as a white solid, which was further resolved on a chiral column to give compound 64.

LCMS [M+Na]⁺ = 560.0.

¹H NMR (400 MHz, DMSO) δ 8.41 (s, 1H), 8.27 (s, 1H), 7.79 (s, 1H), 7.59 (d, *J* = 8.4 Hz, 2H), 7.45 - 7.37 (m, 3H), 7.26 (d, *J =* 16.1 Hz, 2H), 7.07 (td, *J* = 14.1, 7.0 Hz, 3H), 6.95 (d, *J* = 7.2 Hz, 2H), 5.66 (s, 2H), 5.33 (d, *J* = 4.5 Hz, 1H), 3.98-3.90 (m, 3H), 3.92 (d, *J =* 5.0 Hz, 2H), 3.79 (d, *J* = 6.8 Hz, 1H), 3.73 - 3.66 (m, 1H), 2.29 - 2.20 (m, 1H), 2.19-2.13 (m, 1H).

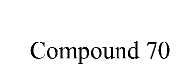

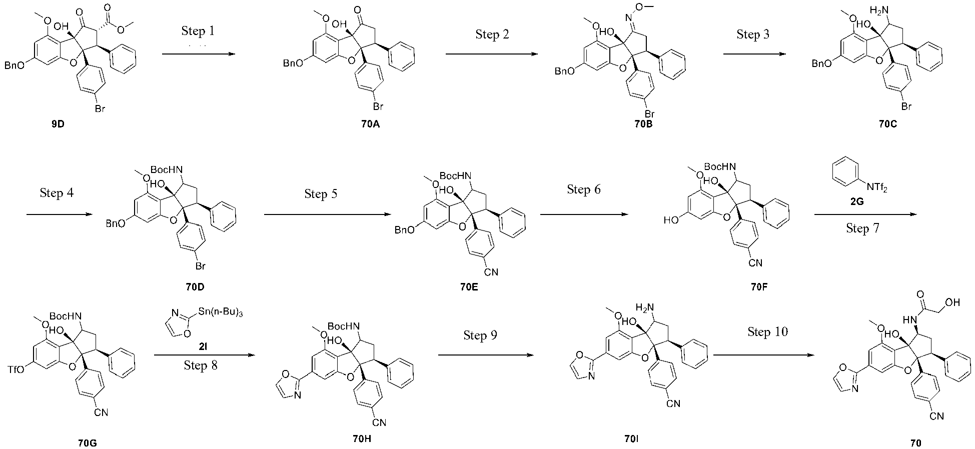

Step 1: DMAP (113 mg, 0.93 mmol) was added to a mixture of the racemate of compound 9D (1900 mg, 3.18 mmol) in H₂O/toluene (6 mL/30 mL), and then the solution was stirred at 110 °C for 2 h. The mixture was concentrated and purified on a silica gel column (PE/EA = 2/1) to give a racemate of the target product 70A (1200 mg, yield: 70%). LCMS [M+H]⁺ = 557.2.

Step 2: The racemate of compound 70A (1000 mg, 1.8 mmol) and dimethylhydroxylamine hydrochloride (500 mg, 5.4 mmol) were dissolved in pyridine (20 mL) and EtOH (20 mL), and the solution was heated to 70 °C and stirred for 4 h under a nitrogen atmosphere, then diluted with water, and extracted with EA. The organic phase was washed with brine, dried, concentrated, and purified by silica gel column chromatography (EA/PE = 0-1/2) to give a racemate of 70B (650 mg, yield: 71%) as a yellow solid. LCMS [M+H-H₂O]⁺ = 568.1.

Step 3: A BH₃·DMS solution (2.78 mL, 5.55 mmol) was added to a solution of the racemate of compound 70B (650 mg, 0.17 mmol) in THF (10 mL), and the mixture was stirred at 65 °C for 4 h. The reaction solution was cooled to room temperature, slowly quenched with MeOH, and concentrated to give a racemate of 70C (420 mg, yield: 68%) as a white solid. LCMS [M+H]⁺ = 558.2.

Step 4: DIPEA (291 mg, 2.26 mmol) and Boc₂O (194 mg, 0.9 mmol) were added to a solution of the racemate of compound 70C (420 mg, 0.75 mmol) in DCM (10 mL), and the mixture was stirred at 25 °C for 4 h, then concentrated, and purified by flash chromatography to give a racemate of 70D (300 mg, yield: 61%) as a white solid. LCMS [M+Na]⁺ = 680.2.

Step 5: The racemate of compound 70D (345 mg, 0.52 mmol), Pd₂(dba)₃ (92 mg, 0.1 mmol), DPPF (111 mg, 0.2 mmol), and Zn(CN)₂ (152 mg, 1.3 mmol) were suspended in NMP (20 mL), and the suspension was heated to 130 °C and stirred for 2 h under a nitrogen atmosphere, then diluted with water, and extracted with EA. The organic phase was washed with water and brine, dried, concentrated, and purified by silica gel column chromatography (EA/DCM = 0-1/1) to give a racemate of 70E (280 mg, yield: 89%) as a yellow solid. LCMS [M+H-H₂O]⁺ = 587.2.

Step 6: A mixture of the racemate of compound 70E (230 mg, 0.38 mmol) and Pd(OH)₂ (80 mg) in EA (10 mL) and MeOH (10 mL) was stirred at 25 °C for 3 h under a hydrogen atmosphere. The mixture was filtered, and the filtrate was concentrated to give a racemate of 70F (170 mg, yield: 87%) as a white solid. LCMS [M+Na]⁺ = 537.2.

Step 7: The racemate of compound 70F (200 mg, 0.39 mmol), K₂CO₃ (135 mg, 0.98 mmol), and compound 2G (167 mg, 0.47 mmol) were added to DMF (10 mL), and the mixture was stirred at 60 °C for 2 h. 30 mL of EA was added, and the mixture was washed with 10 mL of water. The organic phase was washed with 10 mL of water and 10 mL of brine, dried, concentrated, and purified by flash column chromatography (PE/EA = 3/1) to give a racemate of 70G (150 mg, yield: 60%) as a white solid. LCMS [M+Na]⁺ = 669.0.

Step 8: Compound 2I (78 mg, 0.216 mmmol), Pd(PPh₃)₄ (12 mg, 0.01 mmol), and CuI (4 mg, 0.02 mmol) were added to a mixture of the racemate of compound 70G (35 mg, 0.054 mmol) in DMF (3 mL), and the mixture was stirred at 100 °C for 1.5 h under a nitrogen atmosphere. The reaction solution was cooled to room temperature and poured into 30 mL of EA, and the mixture was washed with 10 mL of water. The organic phase was then washed with 10 mL of water and 10 mL of brine, dried, concentrated, and purified by preparative TLC to give a racemate of 70H (25 mg, yield: 83%) as a white solid. LCMS [M+Na]⁺ = 588.2.

Step 9: The racemate of compound 70H (25 mg, 0.044 mmol) and 6 M HCl (1 mL) were added to THF (1 mL), and the mixture was stirred at 25 °C for 5 h. The reaction solution was directly purified on a reversed-phase chromatography column (MeCN/FA/H₂O) to give a racemate of 70I (25 mg, yield: 100%) as a white solid. LCMS [M+Na]⁺ = 466.0.

Step 10: Glycolic acid (4 mg, 0.05 mmol), HATU (4 mg, 0.01 mmol), and TMP (3 mg, 0.02 mmol) were added to a mixture of the racemate of compound 70I (5 mg, 0.01 mmol) in DMF (1 mL). The resulting mixture was stirred at 20 °C for 2 h. The reaction solution was purified on a reversed-phase chromatography column (MeCN/H₂O/FA) to give a racemate of 70 (4 mg, yield: 67%) as a white solid, which was further resolved on a chiral column to give compound 70.

LCMS [M+Na]⁺ = 546.0.

¹H NMR (400 MHz, DMSO) δ 8.26 (s, 1H), 8.02 (d, *J* = 6.9 Hz, 1H), 7.58 (d, *J* = 8.5 Hz, 2H), 7.42 (s, 1H), 7.37 (d, *J =* 8.5 Hz, 2H), 7.29 (d, *J =* 0.8 Hz, 1H), 7.19 (s, 1H), 7.12 - 7.07 (m, 2H), 7.03 (dd, *J =* 15.2, 7.3 Hz, 3H), 6.15 (s, 1H), 5.81 (t, *J* = 5.5 Hz, 1H), 4.65 - 4.52 (m, 1H), 3.95 (d, *J* = 5.5 Hz, 2H), 3.82 (s, 3H), 3.68 (dd, *J* = 14.3, 5.7 Hz, 1H), 2.40-2.38 (m, 1H), 2.03-1.97 (m, 1H).

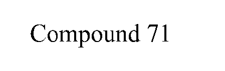

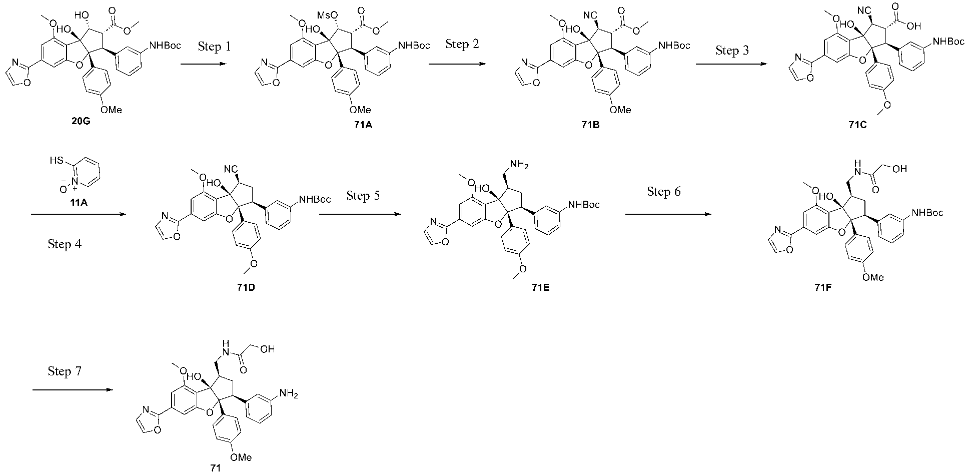

Step 1: The racemate of compound 20G (197 mg, 0.31 mmol) was dissolved in pyridine (2 mL), and MsCl (1.07 g, 9.30 mmol) was added under an ice-water bath. The reaction solution was stirred at 10 °C for 3 h, then diluted with 6 N HCl (20 mL), extracted with EA (20 mL × 2), washed with brine (20 mL), and purified by preparative HPLC (H₂O (0.1% FA)/ACN) to give a racemate of 71A (150 mg, yield: 68%) as a white solid. LCMS [M+H]⁺ = 723.2.

Step 2: The racemate of compound 71A (150 mg, 0.21 mmol) was dissolved in DMF (2 mL), and NaCN (51.45 mg, 1.05 mmol) was added. The reaction solution was stirred at 10 °C for 12 h, then diluted with a NaHCO₃ solution (20 mL), extracted with EA (20 mL × 2), washed with brine (20 mL), and purified by preparative HPLC (H₂O (0.1% FA)/ACN) to give a racemate of 71B (115 mg, yield: 85%) as a yellow solid. LCMS [M+H]⁺ = 654.2.

Step 3: The racemate of compound 71B (115 mg, 0.18 mmol) was dissolved in a solution of THF (2 mL), and LiOH·H₂O (22.68 mg, 0.54 mmol) was added. The reaction solution was stirred at 10 °C for 3 h, thenconcentrated, and purified by preparative HPLC (H₂O (0.1% FA)/ACN) to give a racemate of 71C (95 mg, yield: 84%) as a yellow solid. LCMS [M+H-C₄H₈]⁺ = 584.7.

Step 4: A solution of the racemate of compound 71C (10 mg, 0.02 mmol), compound 11A (19.85 mg, 0.16 mmol), tert-dodecyl mercaptan (31.58 mg, 0.16 mmol), TMP (2.86 mg, 0.02 mmol), and Zinc meso-tetraphenylporphyrin (1.06 mg, 0.001 mmol) in DMF (1 mL) was placed in a Schlenk tube. The reaction system was irradiated with 25 W red light, with the reaction temperature maintained at 80 °C. EDCI (29.86 mg, 0.16 mmol) was dissolved in 1 mL of DMF, and the solution was added dropwise to the reaction system. The reaction solution was stirred for 0.5 h and then purified by reversed-phase column chromatography (H₂O/ACN) to give a racemate of 71D (20 mg, yield: 24.7%) as a white solid. LCMS [M+H-C₄H₈]⁺ = 541.2.

Step 5: A solution of the racemate of compound 71D (15 mg, 0.03 mmol) in THF (1 mL) was cooled to 0 °C. LiAlH₄ (12.6 µL, 0.05 mmol) was added dropwise to the reaction system, and the mixture was stirred at 0 °C for 1 h. Na₂SO₄·10H₂O was added to the reaction system, and the mixture was stirred at room temperature for another 0.5 h. The reaction solution was filtered, and the filter cake was washed with DMF. The resulting filtrate was concentrated to dryness by rotary evaporation to give a racemate of 71E (20 mg, crude product, yield not calculated) as a yellow solid, which was directly used in the next step. LCMS [M+H]⁺ = 600.9.

Step 6: TMP (14.64 mg, 0.121 mmol) was added to a solution of the racemate of compound 71E (18 mg, 0.03 mmol), glycolic acid (4.57 mg, 0.06 mmol), HATU (22.84 mg, 0.06 mmol), and HOAT (8.17 mg, 0.06 mmol) in DMF (1 mL), and the mixture was stirred at room temperature for 1 h. The reaction solution was purified by reversed-phase column chromatography (H₂O/ACN) to give a racemate of 71F (4 mg, yield: 13.8%) as a white solid. LCMS [M+H-H₂O]⁺ = 640.9.

Step 7: 6 M HCl (1 mL) was added to a solution of the racemate of compound 71F (4 mg, 0.01 mmol) in THF (1 mL), and the mixture was stirred at room temperature overnight. The reaction solution was purified by reversed-phase column chromatography (H₂O/ACN) to give a racemate of 71 (1.2 mg, yield: 36.4%) as a white solid, which was further resolved on a chiral column to give compound 71.

LCMS [M+H- H₂O]⁺ = 540.9.

¹H NMR (400 MHz, DMSO) δ 8.25 (s, 1H), 7.76 (s, 1H), 7.41 (s, 1H), 7.19 (dd, *J* = 12.7, 6.6 Hz, 4H), 6.73 (dd, *J* = 18.4, 7.8 Hz, 3H), 6.42 - 6.24 (m, 2H), 6.16 (s, 1H), 5.63 (s, 1H), 5.35 (s, 1H), 3.95 (s, 3H), 3.90 (s, 3H), 3.66 (s, 5H), 2.86 (s, 1H), 2.15 - 1.98 (m, 2H).

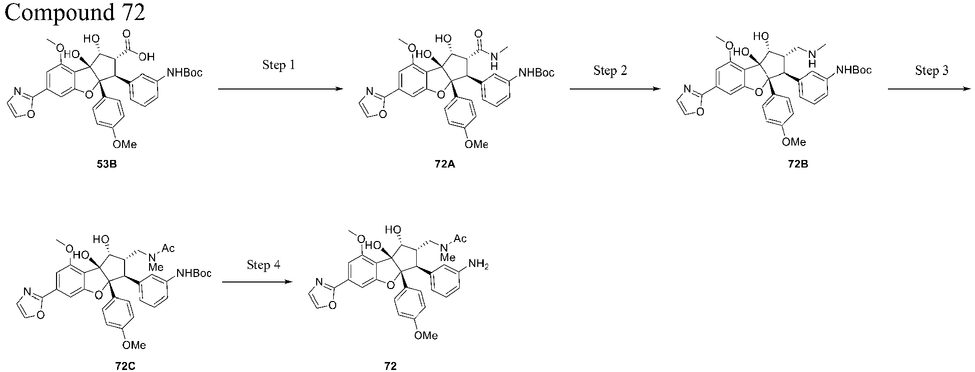

Step 1: The racemate of 53B (100 mg, 0.16 mmol), HATU (90 mg, 0.24 mmol), HOAT (32 mg, 0.24 mmol), and TMP (58 mg, 0.48 mmol) were added to a solution of DMF (5 mL), and the mixture was stirred at 17 °C for 0.5 h. Methylamine hydrochloride (21 mg, 0.32 mmol) was added, and the mixture was stirred at 28 °C for 1 h. The reaction solution was purified on a silica gel column (DCM/MeOH) to give a racemate of 72A (90 mg, yield: 82%) as a yellow solid. LCMS [M+H]⁺ = 644.4.

Step 2: The racemate of compound 72A (80 mg, 0.124 mmol) was dissolved in a solution of THF (1 mL), and a solution of BH₃·DMS (0.19 mL, 2 M in THF) was added. The mixture was stirred at 60 °C for 4 h under a nitrogen atmosphere. MeOH (3 mL) was added, and the mixture was stirred at 60 °C for 16 h. The reaction solution was purified on a silica gel column (DCM/MeOH) to give a racemate of 72B (15 mg, yield: 19%) as a yellow solid. LCMS [M+H]⁺ = 630.4.

Step 3: Acetic anhydride (3.7 mg, 0.035 mmol) was added to a solution of the racemate of compound 72B (15 mg, 0.023 mmol) and TEA (7.06 mg, 0.070 mmol) in DMF (1 mL), and the mixture was stirred at 27 °C for 2 h. The reaction solution was purified on a silica gel column (DCM/MeOH) to give a racemate of 72C (8 mg, yield: 54%) as a yellow solid. LCMS [M+H]⁺ = 672.3.

Step 4: The racemate of compound 72C (8 mg, 0.008 mmol) was added to THF/6 M HCl = 1/1 (3 mL), and the mixture was stirred at 28 °C for 5 h. The reaction solution was concentrated and purified on a reversed-phase column (H₂O/ACN) to give a racemate of 72 (2.9 mg, yield: 39%) as a white solid, which was further resolved on a chiral column to give compound 72. LCMS [M+H]⁺ = 572.4.

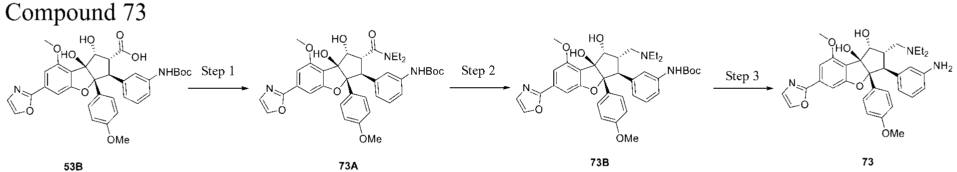

Step 1: The racemate of compound 53B (50 mg, 0.08 mmol), HATU (60.29 mg, 0.16 mmol), and DIEA (30.74 mg, 0.24 mmol) were dissolved in DMF (2 mL), and then diethylamine (5.8 mg, 0.08 mmol) was added. The mixture was stirred at room temperature for 0.5 h. The reaction solution was concentrated to dryness by rotary evaporation and purified on a reversed-phase silica gel column (FA-H₂O/ACN = 45:55) to give a racemate of the target product 73A (35 mg, yield: 74%). LCMS [M+H]⁺ = 686.3.

Step 2: The racemate of compound 73A (25 mg, 0.04 mmol) was dissolved in THF (2 mL), and BH₃·DMS (0.12 mL, 2 M in THF) was added. The mixture was stirred at 60 °C for 2 h. MeOH (2 mL) was added, and the mixture was stirred for 14 h. The reaction solution was concentrated to dryness by rotary evaporation and then purified on a silica gel column (DCM:MeOH = 85:15) to give a racemate of compound 73B (20 mg, yield: 74.5%). LCMS [M+H]⁺ = 672.3.

Step 3: The racemate of compound 73B (15 mg, 0.02 mmol) was dissolved in THF (3 mL), and 6 M HCl (3 mL) was added. The mixture was stirred at room temperature for 2 h. The reaction solution was concentrated to dryness by rotary evaporation and purified on a reversed-phase silica gel column (FA-H₂O/ACN = 70:30) to give a racemate of the target product 73 (1 mg, yield: 8.7%), which was further resolved on a chiral column to give compound 73.

LCMS [M+H]⁺ = 572.2.

1H NMR (400 MHz, DMSO) δ 8.23 (s, 1H), 8.16 (s, 1H), 7.39 (d, J = 0.7 Hz, 1H), 7.16 (d, J = 8.8 Hz, 2H), 7.11 (s, 2H), 6.69 (t, J = 7.7 Hz, 1H), 6.62 (d, J = 8.9 Hz, 2H), 6.29 (s, 1H), 6.19 (d, J = 8.1 Hz, 1H), 6.10 (d, J = 7.2 Hz, 1H), 5.22 (s, 1H), 4.81 (s, 1H), 4.51 (s, 1H), 3.85 (s, 3H), 3.62 (s, 3H), 3.05 (s, 2H), 2.74 (s, 2H), 1.24 (s, 4H), 1.00 (d, J = 5.2 Hz, 6H).

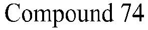

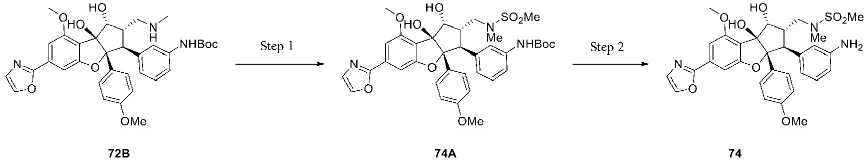

Step 1: MsCl (1.5 mg, 0.014 mmol) was added to a solution of the racemate of 72B (6 mg, 0.009 mmol) and TEA (2.82 mg, 0.028 mmol) in DMF (1 mL), and the mixture was stirred at 27 °C for 2 h. The reaction solution was concentrated and purified by preparative TLC (DCM/MeOH = 10/1) to give a racemate of 74A (3.3 mg, yield: 50%) as a yellow solid. LCMS [M+H]⁺ = 708.5.

Step 2: The racemate of compound 74A (3 mg, 0.004 mmol) was dissolved in THF/6 M HCl (1/1, 3 mL), and the solution was stirred at 28 °C for 5 h. The reaction solution was concentrated and purified on a reversed-phase column (H₂O/ACN) to give a racemate of 74 (1 mg, yield: 39%) as a white solid, which was further resolved on a chiral column to give compound 74.

LCMS [M+H]⁺ = 608.5.

¹H NMR (400 MHz, DMSO) δ 8.23 (s, 1H), 7.40 (s, 1H), 7.21 (s, 1H), 7.14 (s, 1H), 7.11 (d, *J* = 2.1 Hz, 3H), 6.70 (t, *J =* 7.8 Hz, 2H), 6.61 (d, *J* = 9.0 Hz, 2H), 6.32 (s, 1H), 6.22 - 6.18 (m, 2H), 5.33 (d, *J =* 4.7 Hz, 1H), 5.27 (s, 1H), 4.78 (d, *J* = 5.0 Hz, 3H), 4.47 (s, 1H), 3.85 (s, 3H), 3.61 (s, 3H), 2.90 (s, 3H), 2.83 (s, 3H), 2.00 (dd, *J* = 14.4, 6.8 Hz, 6H), 1.46 (d, *J* = 7.4 Hz, 3H).

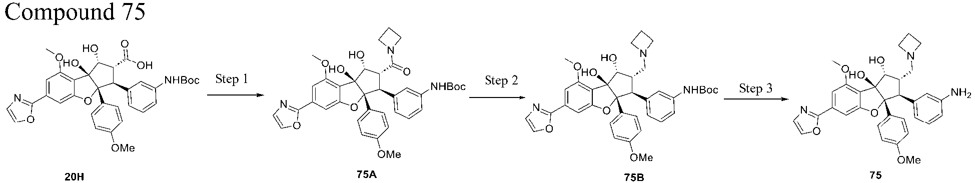

Step 1: The racemate of compound 20H (50 mg, 0.08 mmol), HATU (30.15 mg, 0.08 mmol), HOAT (10.79 mg, 0.08 mmol), and TMP (38.43 mg, 0.32 mmol) were dissolved in DMF (2 mL), and cyclobutylamine (4.53 mg, 0.08 mmol) was added. The mixture was stirred at room temperature for 2 h. The reaction solution was concentrated to dryness by rotary evaporation and purified on a reversed-phase silica gel column (H₂O/ACN = 50:50) to give a racemate of the target product 75A (25 mg, yield: 70%). LCMS [M+H]⁺ = 670.2.

Step 2: The racemate of compound 75A (25 mg, 0.04 mmol) was dissolved in THF (2 mL), and BH₃·DMS (0.12 mL, 2 N in THF) was added. The mixture was stirred at 60 °C for 2 h. MeOH (2 mL) was added, and the mixture was stirred for 14 h. The reaction solution was concentrated to dryness by rotary evaporation and then purified on a silica gel column (DCM:MeOH = 85:15) to give a racemate of compound 75B (9 mg, yield: 39%). LCMS [M+H]⁺ = 656.2.

Step 3: The racemate of compound 75B (9 mg, 0.01 mmol) was dissolved in THF (1 mL), and 6 M HCl (1 mL) was added. The mixture was stirred at room temperature for 2 h. The reaction solution was concentrated to dryness by rotary evaporation and purified on a reversed-phase silica gel column (H₂O/ACN = 70:30) to give a racemate of compound 75 (1 mg, yield: 18%), which was further resolved on a chiral column to give compound 75.

LCMS [M+H]⁺ = 556.2.

¹H NMR (400 MHz, DMSO) δ 8.23 (d, J = 0.7 Hz, 1H), 8.21 (s, 1H), 7.39 (d, J = 0.7 Hz, 1H), 7.15 - 7.08 (m, 4H), 6.70 (t, J = 7.7 Hz, 1H), 6.61 (d, J = 9.0 Hz, 2H), 6.27 (s, 1H), 6.19 (d, J = 7.8 Hz, 1H), 6.10 (d, J = 7.6 Hz, 1H), 5.17 (s, 1H), 4.79 (s, 1H), 4.43 (d, J = 4.2 Hz, 1H), 3.84 (s, 3H), 3.61 (d, J = 2.2 Hz, 3H), 3.51 (d, J = 14.1 Hz, 1H), 2.82 (d, J = 12.6 Hz, 1H), 2.69 (dd, J = 17.5, 6.7 Hz, 1H), 2.42 (d, J = 10.4 Hz, 1H), 2.03 - 1.92 (m, 3H), 1.24 (s, 5H).

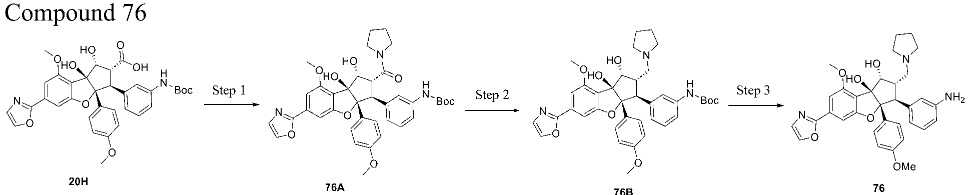

Step 1: TMP (19.2 mg, 0.16 mmol) was added to a solution of the racemate of compound 20H (50.0 mg, 0.08 mmol), cyclopentylamine (5.6 mg, 0.08 mmol), HATU (120.6 mg, 0.32 mmol), and HOAT (21.6 mg, 0.16 mmol) in DMF (2 mL), and the mixture was stirred at 25 °C for 2 h. The reaction solution was purified on a silica gel column (DCM/MeOH) to give a racemate of compound 76A (40 mg, yield: 74%). LCMS [M+H]⁺ = 684.6.

Step 2: BH₃·DMS (0.09 mL, 0.18 mmol, 2 N in THF) was added to a mixture of the racemate of compound 76A (40.0 mg, 0.06 mmol) in THF (2 mL), and the mixture was stirred at 60 °C for 2 h. Methanol (8 mL) was added, and the mixture was stirred at 60 °C for 18 h. The reaction solution was concentrated and purified on a silica gel column (DCM/MeOH) to give a racemate of compound 76B (30 mg, yield: 76%). LCMS [M+H]⁺ = 670.2.

Step 3: The racemate of compound 76B (30.0 mg, 0.04 mmol) was dissolved in THF (1 mL), and 6 M HCl (2 mL) was added at 0 °C. The mixture was stirred at 25 °C for 1 h. The reaction solution was purified on a silica gel column (DCM/MeOH) to give a racemate of compound 76 (23.3 mg, yield: 91%), which was further resolved on a chiral column to give compound 76.

LCMS [M+H]⁺ = 570.2.

¹H NMR (400 MHz, DMSO) δ 10.57 (s, 1H), 10.01 (s, 2H), 8.25 (d, *J* = 0.7 Hz, 1H), 7.41 (d, *J =* 0.7 Hz, 1H), 7.21 - 7.09 (m, 6H), 7.09 - 6.94 (m, 2H), 6.62 (d, *J* = 9.0 Hz, 2H), 5.53 (s, 1H), 5.37 (s, 1H), 4.74 (d, *J* = 3.4 Hz, 1H), 3.86 (s, 3H), 3.79 (s, 1H), 3.70 (d, *J* = 13.6 Hz, 1H), 3.62 (s, 3H), 3.32 (dd, *J =* 16.9, 6.7 Hz, 4H), 3.06 (s, 1H), 2.96 - 2.86 (m, 1H), 2.10 - 1.91 (m, 4H).

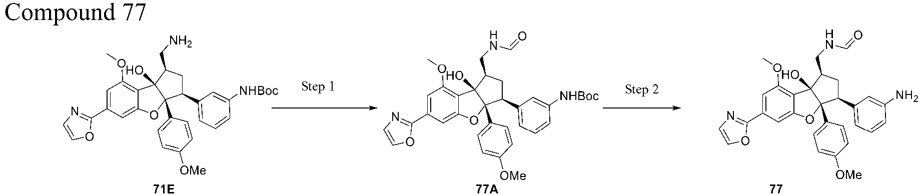

Step 1: The racemate of compound 71E (10 mg, 0.017 mmol) and ethyl formate (63 mg, 0.85 mmol) were dissolved in DMF (5 mL), and the solution was stirred at 60 °C for 18 h. The reaction solution was purified on a reversed-phase chromatography column (MeCN/FA/H₂O) to give a racemate of 77A (2.5 mg, yield: 23%) as a white solid. LCMS [M+H-H₂O]⁺ = 610.2.

Step 2: A mixture of the racemate of compound 77A (2.5 mg, 0.034 mmol) and formic acid (4 mL) in THF (0.5 mL) was stirred at 25 °C for 5 h. The reaction solution was purified on a reversed-phase chromatography column (MeCN/FA/H₂O) to give a racemate of 77 (1.5 mg, yield: 71%) as a white solid, which was further resolved on a chiral column to give compound 77.

LCMS [M+Na]⁺ = 550.2.

¹H NMR (400 MHz, DMSO) δ 8.25 (s, 1H), 8.14 (s, 1H), 7.86 (s, 1H), 7.41 (s, 1H), 7.19 (d, *J* = 6.0 Hz, 2H), 7.15 (d, *J =* 8.9 Hz, 2H), 6.69 (t, *J =* 7.9 Hz, 4H), 6.28 (s, 1H), 6.23 (d, *J* = 7.8 Hz, 1H), 6.09 (d, *J* = 8.0 Hz, 1H), 5.32 (t, *J =* 4.6 Hz, 1H), 5.21 (s, 1H), 4.80 (s, 1H), 3.93 (s, 3H), 3.66 (s, 3H), 2.09 (d, *J* = 8.2 Hz, 1H), 2.00 (d, *J =* 7.5 Hz, 3H).

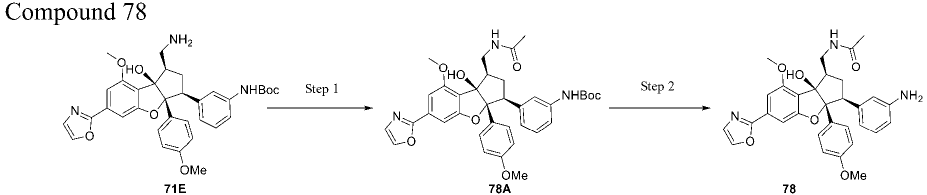

Step 1: A mixture of the racemate of compound 71E (10 mg, 0.017 mmol) and Ac₂O (48 mg, 0.034 mmol) in DMF (5 mL) was stirred at 25 °C for 18 h. The reaction solution was purified on a reversed-phase chromatography column (MeCN/FA/H₂O) to give a racemate of 78A (5 mg, yield: 45%) as a white solid. LCMS [M+Na]⁺ = 664.3.

Step 2: A mixture of the racemate of compound 78A (5 mg, 0.008 mmol) and 6 M HCl (1 mL) in THF (1 mL) was stirred at 25 °C for 5 h. The reaction solution was purified on a reversed-phase chromatography column (MeCN/FA/H₂O) to give a racemate of 78 (2 mg, yield: 50%) as a white solid, which was further resolved on a chiral column to give compound 78.

LCMS [M+Na]⁺ = 564.2.

¹H NMR (400 MHz, DMSO) δ 8.25 (s, 1H), 7.65 (s, 1H), 7.41 (s, 1H), 7.20 (d, *J =* 4.5 Hz, 3H), 7.15 (d, *J =* 8.9 Hz, 2H), 6.69 (dd, *J =* 8.3, 5.0 Hz, 4H), 6.29 (s, 1H), 6.23 (d, *J =* 7.8 Hz, 1H), 6.10 (d, *J* = 7.2 Hz, 1H), 5.33 (t, *J* = 4.6 Hz, 1H), 5.16 (s, 1H), 4.80 (s, 1H), 3.93 (s, 3H), 3.66 (s, 3H), 3.51 (s, 2H), 2.00 (d, *J* = 7.6 Hz, 2H), 1.87 (s, 3H).

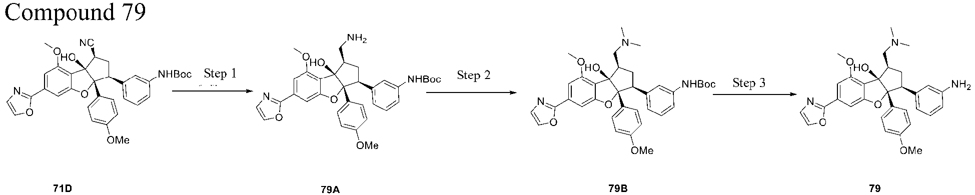

Step 1: Aqueous ammonia (1 drop) was added to a mixture of the racemate of compound 71D (20 mg, 0.034 mmol) and Raney nickel (100 mg) in DMF (5 mL), and the mixture was stirred at 25 °C for 18 h. The reaction solution was filtered, and the filtrate was concentrated to give a racemate of 79A (20 mg, crude product, yield not calculated) as a white crude product, which was directly used in the next step. LCMS [M+H]⁺ = 600.2.

Step 2: Sodium triacetoxyborohydride (20 mg, 0.09 mmol) was added to a solution of the racemate of compound 79A (18 mg, 0.03 mmol), AcOH (18 mg, 0.3 mmol), and 30% formaldehyde (150 mg, 1.5 mmol) in DMF (2 mL), and the mixture was stirred at 25 °C for 18 h. The reaction solution was purified on a reversed-phase chromatography column (MeCN/FA/H₂O) to give a racemate of 79B (8 mg, yield: 42%) as a white solid. LCMS [M+H]⁺ = 628.2.

Step 3: 6 M HCl (1 mL) was added to a solution of the racemate of compound 79B (8 mg, 0.013 mmol) in THF (0.5 mL), and the mixture was stirred at 25 °C for 5 h. The reaction solution was purified on a reversed-phase chromatography column (MeCN/FA/H₂O) to give a racemate of 79 (3 mg, yield: 45%) as a white solid, which was further resolved on a chiral column to give compound 79.

LCMS [M+H]⁺ = 528.2.

¹H NMR (400 MHz, DMSO) δ 8.25 (s, 1H), 8.22 (s, 1H), 7.41 (s, 1H), 7.19 (d, *J* = 4.5 Hz, 2H), 7.15 (d, *J* = 8.9 Hz, 2H), 6.73 - 6.66 (m, 4H), 6.32 (s, 1H), 6.23 (d, *J* = 7.7 Hz, 1H), 6.13 (d, *J* = 7.4 Hz, 1H), 5.32 (s, 1H), 4.80 (s, 1H), 3.93 (s, 3H), 3.66 (s, 3H), 2.89 (s, 1H), 2.37 (s, 6H), 2.20-2.18 (m, 1H), 2.08 - 1.95 (m, 3H).

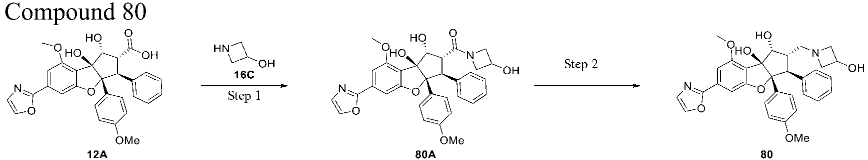

Step 1: DIEA (46.94 mg, 0.388 mmol) was added to a solution of the racemate of compound 12A (50 mg, 0.097 mol), compound 16C (12.75 mg, 0.116 mmol), HATU (55.28 mg, 0.145 mmol), and HOAt (19.79 mg, 0.145 mmol) in DMF (5 mL), and the mixture was stirred at 20 °C for 1 h. The reaction solution was diluted with water (100 mL) and extracted with ethyl acetate (50 mL × 3). The organic phase was dried over anhydrous sodium sulfate, filtered, concentrated, and then purified by silica gel column chromatography (DCM:MeOH = 10:1) to give a racemate of 80A (45 mg, yield: 73.7%) as a white solid. LCMS [M+H]⁺ = 571.

Step 2: A solution of BH₃·DMS (0.8 mL, 1.665 mmol, 2 N in THF) and trifluoroacetic acid (63 mg, 0.549 mmol) in THF (1 mL) was added to a solution of the racemate of compound 80A (95 mg, 0.166 mol) in THF (10 mL), and the mixture was stirred at 0 °C for 1 h. MeOH (20 mL) was added to the reaction solution, and the mixture was stirred for 1 h. The reaction solution was concentrated and purified on a preparative reversed-phase column (H₂O (0.1% FA)/CH₃CN) to give a racemate of compound 80 (80 mg, yield: 86.3%), which was further resolved on a chiral column to give compound 80.

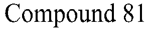

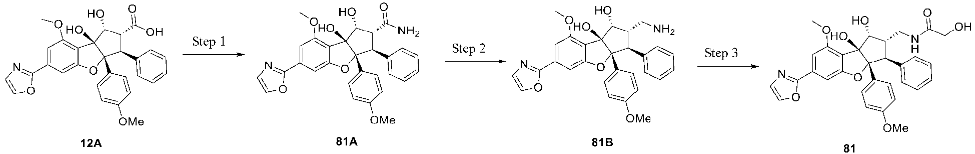

Step 1: The racemate of compound 12A (100.0 mg, 0.19 mmol), HATU (88.5 mg, 0.23 mmol), and DIEA (30.1 mg, 0.23 mmol) were dissolved in DMF (2 mL), and NH₄Cl (20.58 mg, 0.39 mmol) was added. The mixture was stirred at 25 °C for 2 h, then diluted with water, and extracted with DCM. The organic phase was washed with water and brine, dried over anhydrous Na₂SO₄, filtered, concentrated, and then purified on a silica gel column (DCM/MeOH) to give a racemate of compound 81A (90 mg, yield: 90%). LCMS [M+H]⁺ = 515.5.

Step 2: The racemate of compound 81A (70.0 mg, 0.14 mmol) was dissolved in THF (2 mL), and TFA (15.5 mg, 0.14 mmol) was added. BH₃·DMS (0.21 mL, 0.41 mmol) was added, and the mixture was stirred at 25 °C for 2 h. The solution was diluted with an aqueous NaOH solution, stirred at 25 °C for 30 min, concentrated, and then purified on a silica gel column (DCM/MeOH) to give a racemate of compound 81B (23 mg, yield: 33%). LCMS [M+H]⁺ = 501.2.

Step 3: TMP (9.7 mg, 0.08 mmol) was added to a solution of the racemate of compound 81B (10.0 mg, 0.02 mmol), glycolic acid (1.5 mg, 0.02 mmol), HATU (15.2 mg, 0.04 mmol), and HOAT (5.4 mg, 0.04 mmol) in DMF (0.5 mL), and the mixture was stirred at 25 °C for 2 h. The reaction solution was purified on a reversed-phase chromatography column (0.02% FA/H₂O:acetonitrile = 0-100%) to give a racemate of compound 81 (1.1 mg, yield: 9.8%), which was further resolved on a chiral column to give compound 81.

LCMS [M+H]⁺ = 559.2.

¹H NMR (400 MHz, DMSO) δ 8.23 (d, *J* = 0.6 Hz, 1H), 8.05 (s, 1H), 7.40 (d, *J =* 0.7 Hz, 1H), 7.15 - 7.07 (m, 7H), 7.05 - 6.93 (m, 2H), 6.57 (t, *J* = 8.1 Hz, 2H), 5.69 (s, 1H), 5.32 (t, *J* = 4.7 Hz, 1H), 5.27 (s, 1H), 5.09 (d, *J* = 4.4 Hz, 1H), 4.39 (t, *J* = 4.1 Hz, 1H), 3.91 - 3.82 (m, 5H), 3.65 (d, *J* = 13.6 Hz, 1H), 3.59 (s, 3H), 3.27 - 3.25 (m, 1H), 1.29 (s, 1H).

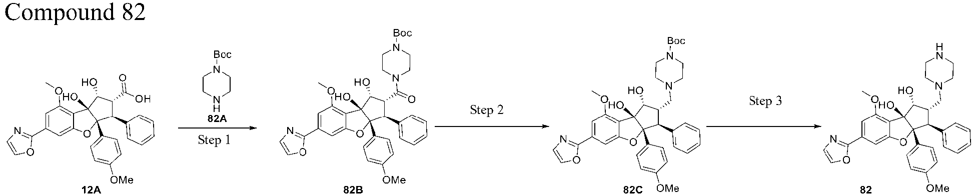

Step 1: The racemate of compound 12A (25 mg, 0.05 mmol), HATU (36.88 mg, 0.05 mmol), and DIEA (18.8 mg, 0.15 mmol) were dissolved in DMF (2 mL), and then compound 82A (9.03 mg, 0.05 mmol) was added. The mixture was stirred at room temperature for 0.5 h. The reaction solution was concentrated to dryness by rotary evaporation and purified on a reversed-phase silica gel column (H₂O/ACN = 50:50) to give a racemate of the target product 82B (20 mg, yield: 60%). LCMS [M+H]⁺ = 684.5.

Step 2: The racemate of compound 82B (10 mg, 0.015 mmol) was dissolved in THF (0.5 mL), and BH₃·DMS (3.4 mg, 2 N in THF) was added. The mixture was stirred at room temperature for 2 h. The reaction solution was concentrated to dryness by rotary evaporation and then purified by preparative TLC (DCM:MeOH = 15:1) to give a racemate of compound 82C (11.5 mg, yield: 65%). LCMS [M+H]⁺ = 670.3.

Step 3: The racemate of compound 82C (11.5 mg, 0.02 mmol) was dissolved in THF (2 mL), and 6 M HCl (2 mL) was added. The mixture was stirred at room temperature for 2 h. The reaction solution was concentrated to dryness by rotary evaporation and purified on a reversed-phase silica gel column (FA-H₂O/ACN = 80:20) to give a racemate of compound 82 (3 mg, yield: 26.3%), which was further resolved on a chiral column to give compound 82.

LCMS [M+H]⁺ = 570.2.

1H NMR (400 MHz, DMSO) δ 8.23 (d, J = 0.7 Hz, 1H), 8.20 (s, 1H), 7.39 (d, J = 0.8 Hz, 1H), 7.16 - 7.04 (m, 7H), 7.00 (d, J = 7.3 Hz, 1H), 6.96 (d, J = 7.3 Hz, 2H), 6.59 (d, J = 9.0 Hz, 2H), 5.26 (s, 1H), 4.79 (s, 1H), 4.50 (d, J = 3.8 Hz, 1H), 3.85 (s, 3H), 3.67 (d, J = 13.9 Hz, 1H), 3.59 (s, 3H), 3.13 (s, 2H), 2.94 (s, 4H), 2.44 - 2.35 (m, 4H), 2.05 (d, J = 9.7 Hz, 1H).

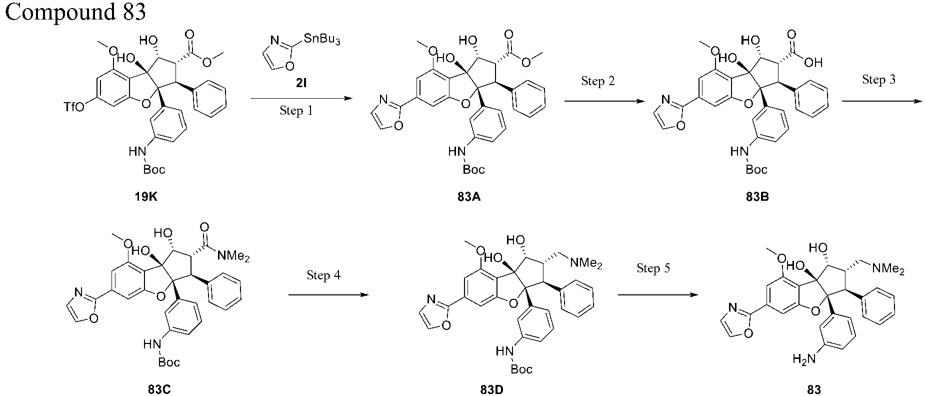

Step 1: Compound 2I (695 mg, 1.9 mmol), Pd(PPh₃)₄ (83 mg, 0.07 mmol), and CuI (27 mg, 0.14 mmol) were added to a solution of the racemate of compound 19K (500 mg, 0.72 mmol) in DMF (5 mL), and the mixture was stirred at 100 °C for 1 h. The reaction solution was filtered, concentrated, and then purified on a silica gel column (DCM/MeOH = 10/1) to give a racemate of 83A (370 mg, yield: 84%) as a grayish-yellow solid. LCMS [M+H]⁺ = 615.6.

Step 2: The racemate of compound 83A (400 mg, 0.65 mmol) and LiOH (68 mg, 1.63 mmol) were dissolved in THF/H₂O/MeOH (3/1/1, 10 mL), and the solution was stirred at 30 °C for 3 h. The reaction solution was adjusted to pH = 5 with 3 M HCl, concentrated, and then purified on a silica gel column (DCM/MeOH) to give a racemate of 83B (250 mg, yield: 64%) as a white solid. LCMS [M+Na]⁺ = 623.6.

Step 3: The racemate of compound 83B (100 mg, 0.17 mmol), HATU (95 mg, 0.25 mmol), HOAT (34 mg, 0.25 mmol), and TMP (60 mg, 0.50 mmol) were added to DMF (5 mL), and the mixture was stirred at 25 °C for 0.5 h. Dimethylamine (27 mg, 0.33 mmol) was added, and the mixture was stirred at 28 °C for 1 h. The reaction solution was concentrated and then purified on a silica gel column (DCM/MeOH) to give a racemate of 83C (43 mg, yield: 41%) as a yellow solid. LCMS [M+H]⁺ = 628.7.

Step 4: The racemate of compound 83C (20 mg, 0.032 mmol) was dissolved in THF (1 mL), and TFA (3.6 mg, 0.032 mmol) and BH₃·DMS (0.048 mL, 2 N in THF) were added. The mixture was stirred at 25 °C for 2 h under a nitrogen atmosphere. A saturated aqueous NaOH solution (1 mL) was added, and the mixture was stirred at 25 °C for 2 h. The reaction solution was purified by preparative TLC (DCM/MeOH = 10/1) to give a racemate of 83D (10 mg, yield: 51%) as a white solid. LCMS [M+H]⁺ = 614.2.

Step 5: The racemate of compound 83D (10 mg, 0.016 mmol) was dissolved in a solution of THF/6 M HCl (1/1, 3 mL), and the mixture was stirred at 28 °C for 5 h. The reaction solution was concentrated and then purified on a reversed-phase column (H₂O/ACN) to give a racemate of 83 (4.6 mg, yield: 55%) as a white solid, which was further resolved on a chiral column to give compound 83.

LCMS [M+H]⁺ = 514.5.

¹H NMR (400 MHz, DMSO) δ 9.46 (s, 1H), 8.25 (s, 1H), 7.41 (s, 1H), 7.18 - 7.05 (m, 5H), 7.01 (d, *J* = 7.4 Hz, 2H), 6.72 (t, *J* = 7.9 Hz, 1H), 6.55 (s, 1H), 6.45 (d, *J* = 7.7 Hz, 1H), 6.22 (d, *J* = 7.6 Hz, 1H), 5.37 (s, 1H), 5.10 (s, 1H), 4.67 (s, 2H), 3.88 (s, 3H), 3.61 (d, *J* = 13.9 Hz, 1H), 2.91 (s, 7H).

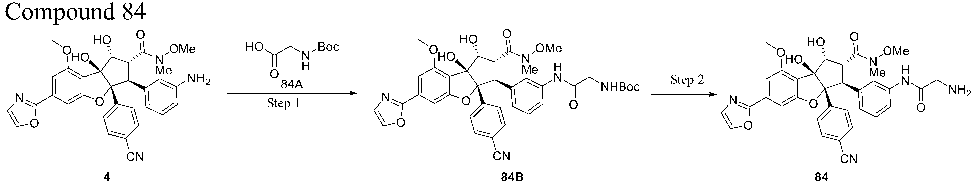

Step 1: The racemate of compound 4 (25 mg, 0.044 mmol) was dissolved in DMF (2 mL), and compound 84A (11.54 mg, 0.066 mmol), HATU (33.44 mg, 0.088 mmol), HOAT (11.97 mg, 0.088 mmol), and TMP (15.96 mg, 0.132 mmol) were added. The mixture was stirred at 25 °C for 1 h under a nitrogen atmosphere. The reaction solution was concentrated and purified on a reversed-phase column (ACN/0.1% FA-H₂O) to give a racemate of 84B (25 mg, yield: 78%) as a white solid. LCMS [M+H]⁺ = 726.6.

Step 2: The racemate of compound 84B (25 mg, 0.034 mmol) was dissolved in THF (2 mL), and 6 M HCl (2 mL) was added. The mixture was stirred at 25 °C for 2 h under a nitrogen atmosphere. The reaction solution was concentrated and purified on a reversed-phase column (ACN/0.1% FAH₂O) to give a racemate of 84 (19.4 mg, yield: 90%) as a white solid, which was further resolved on a chiral column to give compound 84.

LCMS [M+H]⁺ = 626.2.

¹H NMR (400 MHz, DMSO) δ 9.74 (s, 1H), 8.25 (d, J = 0.6 Hz, 1H), 7.52 (d, J = 8.6 Hz, 2H), 7.41 (d, J = 0.7 Hz, 1H), 7.36 - 7.29 (m, 3H), 7.23 (dd, J = 10.3, 4.4 Hz, 2H), 7.16 (d, J = 1.1 Hz, 1H), 6.97 (t, J = 7.9 Hz, 1H), 6.58 (d, J = 7.7 Hz, 1H), 5.68 (s, 1H), 5.17 (d, J = 4.2 Hz, 1H), 4.83 (s, 1H), 4.43 (d, J = 13.8 Hz, 1H), 4.20 (d, J = 9.8 Hz, 1H), 3.93 (s, 3H), 3.85 (s, 3H), 3.35 (s, 2H), 3.09 (s, 3H).

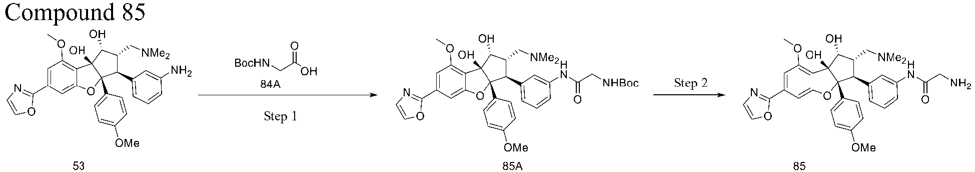

Step 1: TMP (20.19 mg, 0.17 mmol) was added to a solution of the racemate of compound 53 (45 mg, 0.08 mmol), compound 84A (29.01 mg, 0.17 mmol), HATU (62.98 mg, 0.17 mmol) and HOAT (22.54 mg, 0.17 mmol) in DMF (3 mL), and the mixture was stirred at room temperature for 1 h. The reaction solution was extracted with water and ethyl acetate. The organic phase was washed with saturated brine, dried over anhydrous sodium sulfate, filtered, concentrated, and then purified by silica gel column chromatography (DCM/MeOH = 10/1) to give a racemate of 85A (36 mg, yield: 62%) as a yellow solid. LCMS [M+H]⁺ = 701.6.

Step 2: 6 M HCl (3 mL) was added to a solution of the racemate of compound 85A (36 mg, 0.05 mmol) in THF (3 mL), and the mixture was stirred at room temperature overnight. The reaction solution was purified by reversed-phase column chromatography (H₂O/ACN) to give a racemate of 85 (12 mg, yield: 38.7%) as a white solid, which was further resolved on a chiral column to give compound 85.

LCMS [M+H]⁺ = 601.5.

¹H NMR (400 MHz, DMSO) δ 8.26 (d, *J* = 5.0 Hz, 2H), 8.23 (s, 1H), 7.41 (d, *J* = 8.7 Hz, 1H), 7.31 (dd, *J =* 21.2, 10.2 Hz, 2H), 7.20 - 7.08 (m, 5H), 7.01 (td, *J* = 7.9, 4.0 Hz, 1H), 6.71 - 6.63 (m, 2H), 6.60 (d, *J =* 8.9 Hz, 2H), 4.53 (d, *J =* 4.2 Hz, 1H), 3.85 (s, 3H), 3.79 (d, *J =* 3.1 Hz, 1H), 3.64 (s, 1H), 3.62 - 3.58 (m, 3H), 3.52 (s, 2H), 3.03 (s, 1H), 2.31 (d, *J =* 8.3 Hz, 6H), 2.10 (s, 3H).

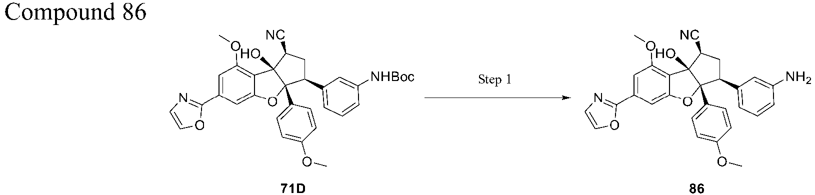

Step 1: The racemate of compound 71D (18 mg, 0.02 mmol) was dissolved in THF (2 mL), and 6 M HCl (2 mL) was added. The mixture was stirred at room temperature for 2 h. The reaction solution was concentrated and purified on a reversed-phase silica gel column (FA-H₂O/ACN = 65%:35%) to give a racemate of the target product 86 (6.1 mg, yield: 37%), which was further resolved on a chiral column to give compound 86.

LCMS [M+H]⁺ = 496.2.

¹H NMR (400 MHz, DMSO) δ 8.27 (s, 1H), 7.43 (d, *J* = 0.6 Hz, 1H), 7.24 (d, *J* = 1.7 Hz, 2H), 7.17 (d, *J =* 8.9 Hz, 2H), 6.76 (d, *J =* 9.0 Hz, 2H), 6.71 (t, *J =* 7.7 Hz, 1H), 6.28 (d, *J =* 8.1 Hz, 1H), 6.25 (s, 1H), 6.01 (d, *J* = 7.1 Hz, 2H), 4.94 (s, 2H), 4.04 (dd, *J* = 10.8, 8.4 Hz, 1H), 3.97 (s, 3H), 3.68 (s, 3H), 3.28 - 3.22 (m, 1H), 2.41 (dd, *J* = 12.4, 8.3 Hz, 1H), 1.24 (s, 1H).

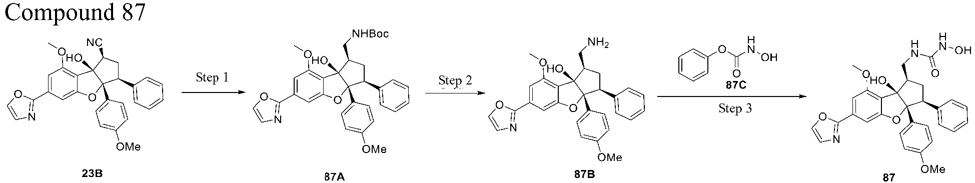

Step 1: The racemate of compound 23B (60 mg, 0.54 mmol), Raney-Ni (21.75 mg, 0.375 mmol), TEA (25 mg, 0.25 mmol), and (Boc)₂O (136 mg, 0.625 mmol) were dissolved in DMF/THF (2.0 mL/2.0 mL), and the solution was stirred at 25 °C for 12 h under a hydrogen atmosphere. The reaction solution was filtered. Water was added, and the mixture was extracted twice with ethyl acetate. The organic phase was concentrated and purified on a normal-phase column (DCM/MeOH) to give a racemate of 87A (40 mg, yield: 55%) as a white solid. LCMS [M+H-H₂O-C₄H₈]⁺ = 511.3. Step 2: The racemate of compound 87A (40 mg, 0.068 mmol) was dissolved in THF (2 mL), and 6 M HCl (2 mL) was added. The mixture was stirred at 25 °C for 2 h under a nitrogen atmosphere. The reaction solution was concentrated and purified on a reversed-phase column (ACN:0.1% FAH₂O) to give a racemate of 87B (27 mg, yield: 82%) as a white solid. LCMS [M+H-H₂O]⁺ = 467.3. Step 3: The racemate of compound 87B (10.0 mg, 0.021 mmol) was dissolved in THF (2 mL), and compound 87C (9.47 mg, 0.062 mmol) and imidazole (14 mg, 0.21 mmol) were added. The mixture was stirred at 70 °C for 5 h under a nitrogen atmosphere. The reaction solution was concentrated and purified on a reversed-phase column (ACN/0.1% FA-H₂O) to give a racemate of 87 (27 mg, yield: 24%) as a white solid, which was further resolved on a chiral column to give compound 87.

LCMS [M+H]⁺ = 526.2.

¹H NMR (400 MHz, DMSO) δ 8.63 (s, 1H), 8.41 (s, 1H), 8.25 (d, J = 0.7 Hz, 1H), 7.41 (d, J = 0.7 Hz, 1H), 7.22 (dd, J = 2.8, 1.1 Hz, 1H), 7.21 - 7.19 (m, 1H), 7.14 (t, J = 6.2 Hz, 2H), 7.10 - 7.03 (m, 3H), 6.91 (d, J = 6.7 Hz, 2H), 6.78 (t, J = 5.7 Hz, 1H), 6.70 - 6.66 (m, 2H), 5.42 (s, 1H), 3.95 (s, 3H), 3.68 - 3.61 (m, 5H), 3.42 - 3.34 (m, 1H), 2.89 (dd, J = 11.2, 5.7 Hz, 1H), 2.23 - 2.12 (m, 1H), 2.10 - 2.01 (m, 1H).

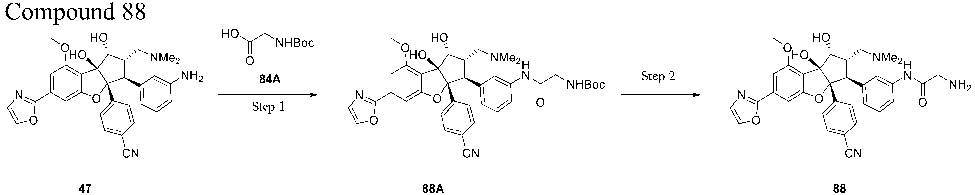

Step 1: The racemate of compound 47 (18 mg, 0.03 mmol), HATU (12.72 mg, 0.03 mmol), HOAT (4.55 mg, 0.03 mmol), and TMP (16.21 mg, 0.13 mmol) were dissolved in DMF (3 mL), and compound 84A (5.86 mg, 0.03 mmol) was added. The mixture was stirred at room temperature for 2 h. The reaction solution was concentrated to dryness by rotary evaporation and purified on a reversed-phase silica gel column (ACN:FA-H₂O = 30%:70%) to give a racemate of the target product 88A (18 mg, yield: 75%). LCMS [M+H]⁺ = 696.3.

Step 2: The racemate of compound 88A (16 mg, 0.02 mmol) was dissolved in THF (3 mL), and 6 M HCl (3 mL) was added. The mixture was stirred at room temperature for 2 h. The reaction solution was concentrated to dryness by rotary evaporation and purified on a reversed-phase silica gel column (ACN:FA-H₂O = 30:70) to give a racemate of the target product 88 (14 mg, yield: 93%), which was further resolved on a chiral column to give compound 88.

LCMS [M+H]⁺ = 596.2.

1H NMR (400 MHz, DMSO) δ 9.76 (s, 1H), 8.24 (d, J = 0.7 Hz, 1H), 8.20 (s, 2H), 7.49 (d, J = 8.7 Hz, 2H), 7.40 (dd, J = 4.7, 3.9 Hz, 3H), 7.36 (s, 1H), 7.29 (d, J = 8.2 Hz, 1H), 7.15 - 7.12 (m, 2H), 7.02 (t, J = 7.9 Hz, 1H), 6.72 (d, J = 8.0 Hz, 1H), 5.57 (s, 1H), 4.51 (d, J = 4.1 Hz, 1H), 3.84 (s, 3H), 3.81 - 3.75 (m, 1H), 3.44 (s, 2H), 3.16 (s, 2H), 2.62 (t, J = 11.3 Hz, 1H), 2.25 (s, 7H), 2.04 (d, J = 11.4 Hz, 1H).

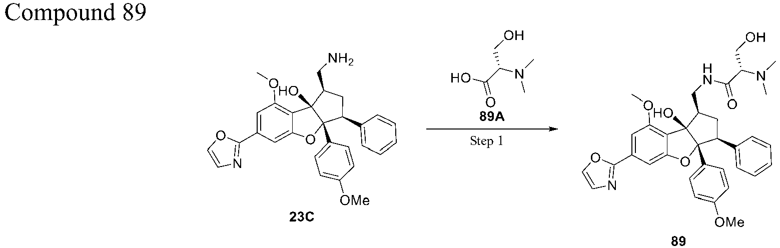

Step 1: TMP (15.0 mg, 0.12 mmol) was added to a solution of the racemate of compound 23C (15.0 mg, 0.03 mmol), compound 89A (4.1 mg, 0.03 mmol), HATU (23.6 mg, 0.06 mmol), and HOAT (8.4 mg, 0.06 mmol) in DMF (0.5 mL), and the mixture was stirred at 25 °C for 2 h. The reaction solution was directly purified on a reversed-phase column (H₂O:acetonitrile = 0-100%) to give a racemate of compound 89 (2.8 mg, yield: 15%), which was further resolved on a chiral column to give compound 89.

LCMS [M+H]⁺ = 600.2.

¹H NMR (400 MHz, DMSO) δ 8.25 (d, *J* = 0.6 Hz, 1H), 7.41 (d, *J* = 0.6 Hz, 1H), 7.22 (d, *J* = 7.2 Hz, 2H), 7.17 - 7.00 (m, 5H), 6.92 (d, *J* = 6.6 Hz, 2H), 6.68 (dd, *J* = 9.0, 2.6 Hz, 2H), 5.25 (dd, *J* = 5.5, 2.0 Hz, 1H), 3.97 (d, *J* = 3.6 Hz, 3H), 3.79 (dd, *J* = 18.1, 9.4 Hz, 2H), 3.64 (s, 4H), 3.35 (s, 2H), 2.84 (s, 1H), 2.66 (dd, *J* = 6.7, 4.8 Hz, 1H), 2.32 (dd, *J* = 5.6, 3.8 Hz, 6H), 2.22 (d, *J* = 4.0 Hz, 2H).

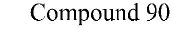

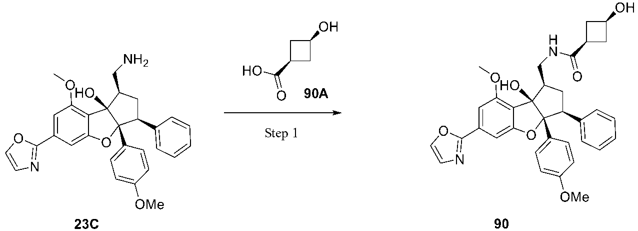

Step 1: TMP (15.0 mg, 0.12 mmol) was added to a solution of the racemate of compound 23C (15.0 mg, 0.03 mmol), compound 90A (3.6 mg, 0.03 mmol), HATU (23.6 mg, 0.06 mmol), and HOAT (8.4 mg, 0.06 mmol) in DMF (0.5 mL), and the mixture was stirred at 25 °C for 2 h. The reaction solution was directly purified on a reversed-phase column (H₂O:acetonitrile = 0-100%) to give a racemate of compound 90 (4.7 mg, yield: 26%), which was further resolved on a chiral column to give compound 90.

LCMS [M+H]⁺ = 565.2.

¹H NMR (400 MHz, DMSO) δ 8.25 (d, *J =* 0.6 Hz, 1H), 7.55 (t, *J =* 5.5 Hz, 1H), 7.41 (d, *J =* 0.7 Hz, 1H), 7.21 (dd, *J* = 8.9, 1.0 Hz, 2H), 7.14 - 7.01 (m, 5H), 6.90 (d, *J* = 6.9 Hz, 2H), 6.68 (d, *J =* 9.0 Hz, 2H), 5.26 (s, 1H), 5.09 (d, *J* = 7.1 Hz, 1H), 3.98 - 3.87 (m, 4H), 3.70 (dt, *J* = 13.3, 5.2 Hz, 1H), 3.65 (s, 3H), 3.62 - 3.53 (m, 1H), 3.37 (d, *J =* 6.4 Hz, 1H), 2.81 (d, *J =* 8.0 Hz, 1H), 2.47 - 2.37 (m, 1H), 2.37 - 2.25 (m, 2H), 2.10 (dt, *J =* 13.1, 9.1 Hz, 2H), 1.99 (dd, *J =* 18.2, 9.5 Hz, 2H).

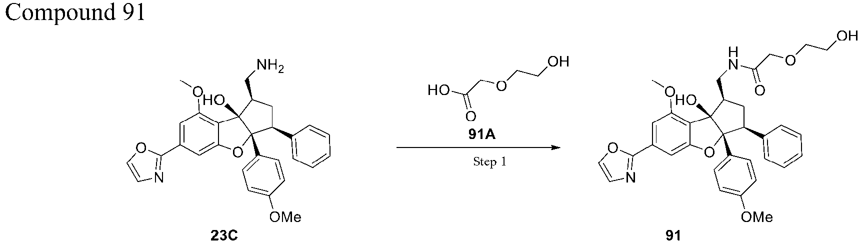

Step 1: TMP (15.0 mg, 0.12 mmol) was added to a solution of the racemate of compound 23C (15.0 mg, 0.03 mmol), compound 91A (3.7 mg, 0.03 mmol), HATU (23.6 mg, 0.06 mmol), and HOAT (8.4 mg, 0.06 mmol) in DMF (0.5 mL), and the mixture was stirred at 25 °C for 2 h. The reaction solution was directly purified on a reversed-phase column (H₂O:acetonitrile = 0-100%) to give a racemate of compound 91 (4.8 mg, yield: 26%), which was further resolved on a chiral column to give compound 91.

LCMS [M+H]⁺ = 569.2.

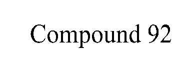

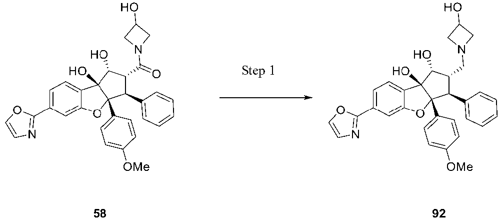

Step 1: The racemate of compound 58 (10 mg, 0.018 mmol) was dissolved in THF (1 mL), and BH₃·DMS (0.028 mL, 2 N in THF) and TFA (2.0 mg, 0.018 mmol) were added. The mixture was stirred at 25 °C for 2 h under a nitrogen atmosphere. The reaction solution was quenched with an aqueous NaOH solution (0.5 mL), filtered, concentrated, and then purified on a reversed-phase column (ACN:0.1% FA-H₂O) to give a racemate of 92 (3.2 mg, yield: 51%) as a white solid, which was further resolved on a chiral column to give compound 92.

LCMS [M+1]⁺ = 527.2.

¹H NMR (400 MHz, DMSO) δ 8.26 (s, 1H), 8.23 (d, J = 0.7 Hz, 1H), 7.57 (dd, J = 7.8, 1.4 Hz, 1H), 7.49 (dd, J = 10.1, 4.5 Hz, 2H), 7.39 (d, J = 0.7 Hz, 1H), 7.09 (dd, J = 8.1, 5.9 Hz, 4H), 7.03 (t, J = 7.2 Hz, 1H), 6.87 (d, J = 7.1 Hz, 2H), 6.64 (d, J = 9.0 Hz, 2H), 5.55 (s, 1H), 4.52 (d, J = 5.7 Hz, 1H), 4.10 (s, 1H), 3.61 (d, J = 7.7 Hz, 5H), 3.53 (d, J = 13.9 Hz, 2H), 2.85 (s, 2H), 2.62 (s, 2H), 2.22 (d, J = 11.9 Hz, 1H).

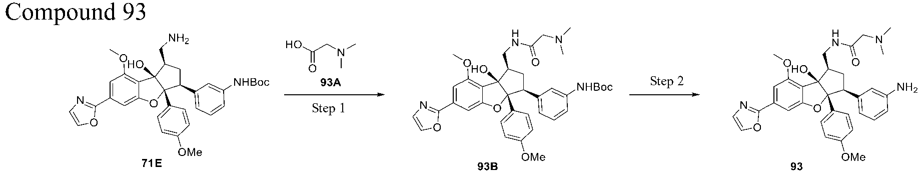

Step 1: Compound 93A (2.89 mg, 0.03 mmol), HATU (12.19 mg, 0.03 mmol), HOAT (4.36 mg, 0.03 mmol), and TMP (9.71 mg, 0.08 mmol) were added to a solution of the racemate of compound 71E (16.0 mg, 0.03 mmol) in DMF (1 mL), and the mixture was stirred at 30 °C for 2 h. The reaction solution was directly purified on a reversed-phase column (H₂O/ACN) to give a racemate of 93B (8.0 mg, yield: 38.4%) as a white solid. LC-MS: (M+H)⁺ = 685.7.

Step 2: 6 N HCl (0.5 mL) was added to a solution of the racemate of compound 93B (8.0 mg, 2.21 mmol) in THF (0.5 mL), and the mixture was stirred at 30 °C for 2 h. The reaction solution was purified on a reversed-phase column (H₂O/ACN) to give a racemate of 93 (3.0 mg, yield: 43.9%) as a white solid, which was further resolved on a chiral column to give compound 93.

LCMS [M+H]⁺ = 585.5.

¹H NMR (400 MHz, DMSO) δ 9.79 (s, 1H), 8.56 (s, 1H), 8.26 (s, 1H), 7.42 (d, *J* = 0.6 Hz, 1H), 7.22 (s, 2H), 7.15 (d, *J* = 8.8 Hz, 2H), 7.04 (s, 1H), 6.71 (d, *J* = 8.9 Hz, 4H), 5.31 (s, 1H), 3.96 (s, 5H), 3.90-3.86 (m, 1H), 3.66 (s, 3H), 3.63-3.57 (m, 1H), 2.83 (s, 7H), 2.19-2.10 (m, 2H), 1.24 (s, 1H).

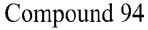

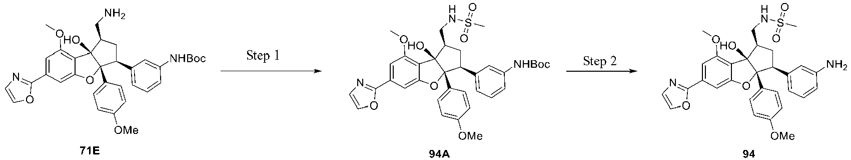

Step 1: TMP (0.1 mL) was added to a solution of the racemate of compound 71E (16.0 mg, 0.03 mmol) in DCM (1 mL), and then MsCl (0.1 mL) was slowly added dropwise under an ice bath. The mixture was stirred at 0 °C for 1 h. The reaction solution was purified on a reversed-phase column (H₂O/ACN) to give a racemate of 94A (2.0 mg, yield: 29.4%) as a white solid. LC-MS: [M+H-H₂O]⁺ = 660.6.

Step 2: HCl (6 N, 0.1 mL) was added to a solution of the racemate of compound 94A (4.0 mg, 0.01 mmol) in THF (0.1 mL), and the mixture was stirred at 25 °C for 2 h. The reaction solution was concentrated and purified by preparative TLC to give a racemate of 94 (2.0 mg, yield: 58.6%) as a white solid, which was further resolved on a chiral column to give compound 94.

LCMS [M+H]⁺ = 579.2.

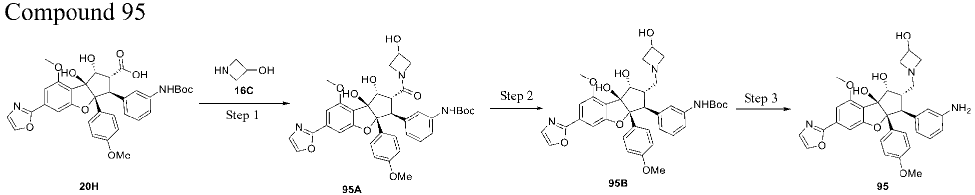

Step 1: The racemate of compound 20H (200 mg, 0.32 mmol), HATU (181 mg, 0.48 mmol), HOAT (65 mg, 0.48 mmol), and TMP (115 mg, 0.95 mmol) were added to DMF (10 mL), and the mixture was stirred at 25 °C for 0.5 h. Compound 16C (69 mg, 0.63 mmol) was added, and the mixture was stirred at 28 °C for 1 h. The reaction solution was concentrated and purified on a silica gel column (DCM/MeOH) to give a racemate of 95A (150 mg, yield: 69%) as a yellow solid. LCMS [M+H]⁺ = 686.6.

Step 2: The racemate of compound 95A (10 mg, 0.015 mmol) was dissolved in a solution of THF (1 mL), and TFA (1.7 mg, 0.015 mmol) and BH₃·DMS (0.022 mL, 2 N in THF) were added. The mixture was stirred at 25 °C for 2 h under a nitrogen atmosphere. A saturated aqueous NaOH solution (1 mL) was added, and the mixture was stirred at 25 °C for 2 h. The reaction solution was concentrated and then purified by silica gel column chromatography (DCM/MeOH = 10/1) to give a racemate of 95B (5 mg, yield: 51%) as a white solid. LCMS [M+H]⁺ = 672.2.

Step 3: The racemate of compound 95B (5 mg, 0.007 mmol) was dissolved in THF/6 M HCl (1/1, 3 mL), and the solution was stirred at 28 °C for 5 h. The reaction solution was concentrated and purified on a reversed-phase column (H₂O/ACN) to give a racemate of 95 (3.2 mg, yield: 74%) as a white solid, which was further resolved on a chiral column to give compound 95.

LCMS [M+H]⁺ = 572.5.

¹H NMR (400 MHz, DMSO) δ 8.24 (d, *J* = 0.6 Hz, 1H), 7.40 (s, 1H), 7.13 (t, *J* = 6.9 Hz, 4H), 6.71 (t, *J =* 7.7 Hz, 1H), 6.62 (d, *J* = 8.8 Hz, 2H), 6.32 (s, 1H), 6.22 (d, *J* = 7.8 Hz, 1H), 6.14 (d, *J =* 6.9 Hz, 1H), 5.31 (d, *J* = 8.2 Hz, 2H), 4.85 (s, 2H), 4.49 (s, 1H), 4.36 (s, 1H), 4.16 (s, 2H), 3.85 (s, 3H), 3.62 (s, 3H), 3.47 (d, *J* = 13.5 Hz, 1H), 3.07 (s, 2H), 2.80 (s, 1H).

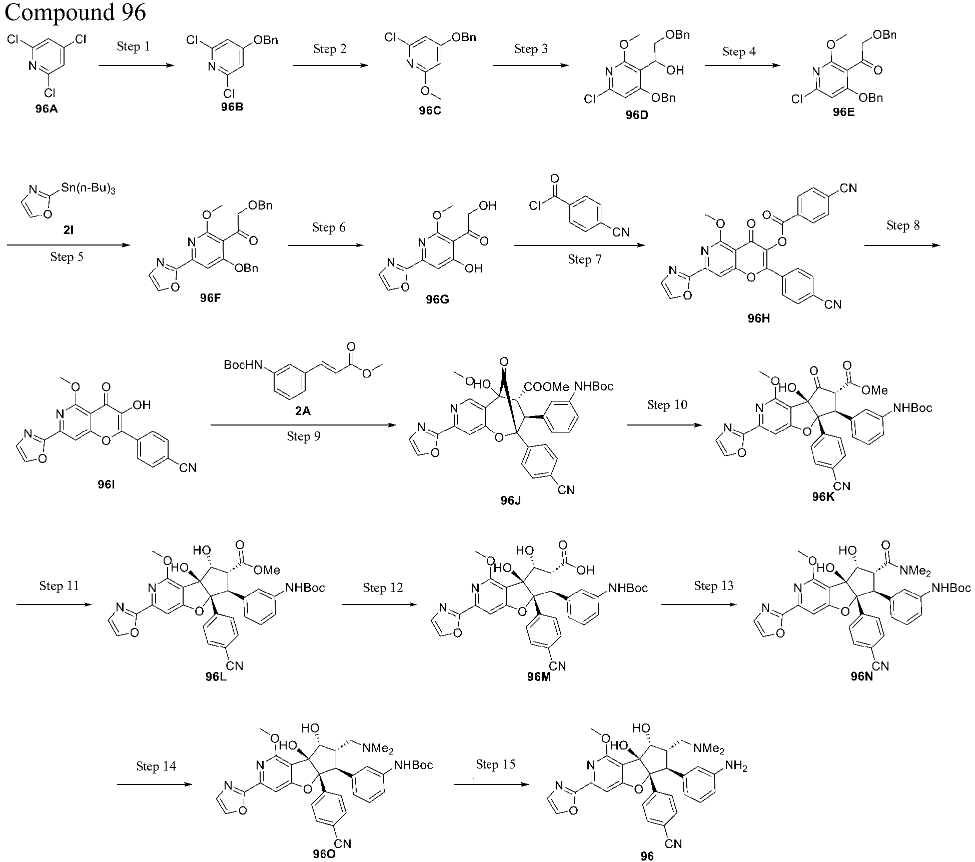

Step 1: At 0 °C, 60% NaH (50.00 g, 301.49 mmol) was added to a solution of compound 96A (50.00 g, 274.08 mmol) in DMF (250 mL), and the mixture was stirred for 20 min. Then, benzyl alcohol (31.12 g, 287.78 mmol) was added dropwise, and the mixture was stirred for another 3 h. The reaction solution was poured into water, and a large amount of white solid was precipitated. The mixture was filtered to give a filter cake, which was then slurried with petroleum ether. The slurry was filtered to give 96B (50 g, yield: 72%) as a white solid. LCMS [M+H]⁺ = 254.0.

Step 2: Compound 96B (118.00 g, 464.37 mmol) was dissolved in toluene (300 mL), and sodium methoxide (67.99 g, 1.26 mol) was added. The mixture was stirred at 60 °C for 5 h. The reaction solution was quenched with water and extracted with ethyl acetate. The organic phases were combined, concentrated, and then slurried with petroleum ether to give compound 96C (90.0 g, yield: 77%). LCMS [M+H]⁺ = 250.2.

Step 3: Compound 96C (10.00 g, 40.05 mmol) was dissolved in diethyl ether (300 mL), and n-butyllithium (16.82 mL, 42.05 mmol, 2.50 M) was added dropwise at -78 °C. The mixture was then stirred at -78 °C for 2 h. 2-(Benzyloxy)acetaldehyde (6.62 g, 44.05 mmol) was dissolved in diethyl ether, and the resulting solution was added dropwise to the above reaction solution. The mixture was stirred overnight, quenched with water, extracted, concentrated to dryness by rotary evaporation, and purified on a silica gel column to give compound 96D (5.30 g, yield: 33.1%). LCMS [M+H]⁺ = 400.0.

Step 4: Compound 96D (10.00 g, 25.01 mmol) was dissolved in DMSO (60 mL). At 10 °C, acetic anhydride (39.57 g, 387.63 mmol) was added, and the mixture was stirred overnight. The reaction solution was quenched with water, extracted, concentrated to dryness by rotary evaporation, and purified by column chromatography to give compound 96E (6.0 g, yield: 60%). LCMS [M+H]⁺ = 398.2.

Step 5: Compound 96E (4000 mg, 10.054 mmol) was dissolved in a solution of NMP (40 mL), and compound was added. The mixture was stirred at 150 °C for 4 h. The reaction solution was concentrated to dryness by rotary evaporation. Water (150 mL) was added, and the mixture was extracted with EA (200 mL) three times. The organic phase was washed with saturated brine (150 mL), dried over anhydrous sodium sulfate, concentrated to dryness by rotary evaporation, and then purified on a silica gel column (EA/PE = 50%/50%) to give compound 96F (1000 mg, yield: 23%). LCMS [M+H]⁺ = 431.3

Step 6: Compound 96F (800.0 mg, 1.858 mmol) was dissolved in a solution of DCM (10 mL), and BCl₃ (11.15 mL) was slowly added at -10 °C. The mixture was stirred at -10 °C for 1 h. The reaction solution was concentrated to dryness by rotary evaporation. MeOH (10 mL) was added, and the mixture was concentrated to dryness by rotary evaporation and purified on a reversed-phase column (ACN/0.1% FA-H₂O = 40%/60%) to give compound 96G (260 mg, yield: 45%). LCMS [M+H]⁺ = 251.2.

Step 7: Compound 96G (150 mg, 0.5995 mmol) was dissolved in a solution of DCM (10 mL), and DBU (274 mg, 1.8 mmol) was added. Then, 4-cyanobenzoyl chloride (248 mg, 1.5 mmol) was added in portions, and the mixture was stirred at room temperature (25 °C) for 16 h. The reaction solution was concentrated to dryness by rotary evaporation and purified on a silica gel column (DCM:EA = 0:1) to give compound 96H (150 mg, yield: 51%). LCMS [M+H]⁺ = 491.2.

Step 8: Compound 96H (250 mg, 0.51 mmol) was dissolved in a solution of MeOH (6 mL), and sodium methoxide (83 mg, 1.53 mmol) was added. The mixture was stirred at 60 °C for 1 h. The reaction solution was adjusted to pH < 7 with acetic acid and filtered. The filter cake was collected to give compound 96I (147 mg, yield: 80%). LCMS [M+H]⁺ = 362.3

Step 9: Compound 96I (170 mg, 0.47 mmol) and compound 2A (781.99 mg, 2.82 mmol) were dissolved in CHCl₃/TFE (7/3, 15 mL). The reaction mixture was injected (12 mL/h) via a syringe into a coil wound around a jacketed glass cylinder equipped with a 250 W ultraviolet lamp. The jacket temperature was adjusted to maintain the internal reaction temperature at 0-5 °C, while the UV lamp was turned on simultaneously. The reaction mixture was irradiated for 1 h and then concentrated under reduced pressure to remove the solvent. The residue was purified by a silica gel plug (EtOAc/hexane) to remove excess cinnamate, thus giving a racemate of 96J (120 mg, yield: 75%) as a pale yellow solid. LCMS [M+H]⁺ = 639.5.

Step 10: The racemate of compound 96J (130 mg, 0.20 mmol) was dissolved in methanol (5 mL), and sodium methoxide (27.48 mg, 0.51 mmol) was added at 0 °C. The reaction solution was stirred at 65 °C for 1.5 h, then concentrated to dryness by rotary evaporation, and directly purified on a silica gel column (0.2% FA in EA/PE = 60%/40%) to give a racemate of compound 96K (68 mg, yield: 52%). LCMS: [M+H]⁺ = 639.6.

Step 11: The racemate of compound 96K (68 mg, 0.11 mmol) was dissolved in a mixed solution of ACN/CHCl₃ (4 mL/4 mL), and AcOH (63.89 mg, 1.06 mmol) and STAB (112.87 mg, 0.53 mmol) were slowly added at 0 °C. The mixture was stirred at room temperature (20 °C) for 2 h. The reaction solution was concentrated to dryness by rotary evaporation. Water (30 mL) was added, and the mixture was extracted with EA (100 mL) three times. The organic phase was washed with saturated brine (50 mL), dried over anhydrous sodium sulfate, concentrated to dryness by rotary evaporation, and then purified on a silica gel column (0.2% FA in EA/PE = 55%/45%) to give a racemate of the product 96L (35 mg, yield: 51%). LCMS:[M+H]⁺ = 641.7.

Step 12: LiOH·H₂O (6 mg, 0.02 mmol) was added to a solution of the racemate of compound 96L (30 mg, 0.05 mmol) in THF/MeOH/H₂O (3/1/1, 2 mL), and the mixture was stirred at room temperature for 1 h. The reaction solution was adjusted to about pH 5 with 3 M HCl, concentrated to dryness by rotary evaporation, and purified on a preparative silica gel plate (DCM/MeOH = 8:1) to give a racemate of 96M (30 mg, yield: 100%) as a white solid. LCMS [M+H]⁺ = 627.5.

Step 13: DIEA (14.42 mg, 0.11 mmol) was added to a solution of the racemate of compound 96M (28 mg, 0.04 mmol), DMA (4.03 mg, 0.09 mmol), and HATU (33.99 mg, 0.09 mmol) in DMF (1 mL), and the mixture was stirred at room temperature for 1 h. The reaction solution was extracted with water and ethyl acetate. The organic phase was washed with saturated brine, dried over anhydrous sodium sulfate, filtered, and concentrated, and then the crude product was purified on a preparative silica gel plate (DCM/MeOH = 14/1) to give a racemate of 96N (30 mg, yield: 96.15%) as a white solid. LCMS: [M+H] = 654.6.

Step 14: BH₃·DMS (60 µL, 0.12 mmol) was added to a solution of the racemate of compound 96N (26 mg, 0.04 mmol) in THF (1 mL), and the mixture was stirred at room temperature for 2 h. Methanol was added to the reaction solution, and the mixture was stirred for another 2 h. The reaction solution was purified on a normal-phase chromatography column (PE/EA = 0-100%) to give a racemate of 96Q (7 mg, yield: 27.48%) as a white solid. LCMS: [M+H]⁺ = 640.6.

Step 15: 6 M HCl (1 mL) was added to a solution of the racemate of compound 96Q (7 mg, 0.01 mmol) in THF (1 mL), and the mixture was stirred at room temperature for 1 h. The reaction solution was concentrated to dryness by rotary evaporation, and the resulting mixture was slurried with diethyl ether three times to give a racemate of compound 96 (5 mg, yield: 84.7%), which was further resolved on a chiral column to give compound 96. LCMS: [M+H]⁺ = 540.2.

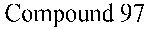

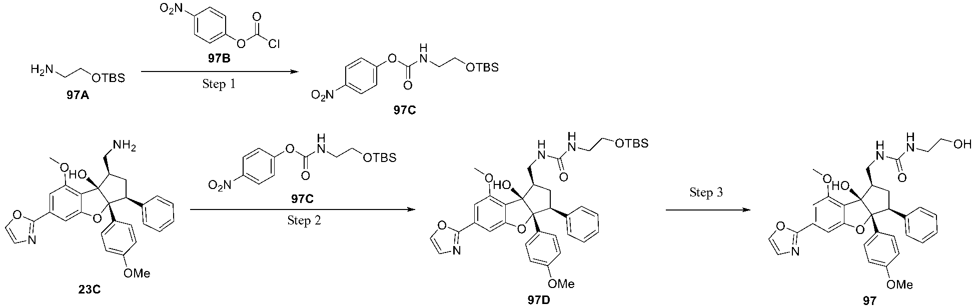

Step 1: The racemate of compound 97A (50 mg, 0.285 mmol) was dissolved in DCM (8 mL), and compound 97B (86 mg, 0.428 mmol) and TEA (87 mg, 0.86 mmol) were added. Under a nitrogen atmosphere, the mixture was stirred at 25 °C for 3 h. The reaction solution was concentrated under reduced pressure and purified on a normal-phase column (PE:EA = 2:1) to give a racemate of 97C (60 mg, yield: 66%) as a white solid. LCMS [M+H-56]⁺ = 341.3.

Step 2: The racemate of compound 23C (10.0 mg, 0.021 mmol) was dissolved in THF (1.5 mL), and 97C (8.4 mg, 0.025 mmol) and TEA (4.1 mg, 0.041 mmol) were added. Under a nitrogen atmosphere, the mixture was stirred at 25 °C for 2 h. The reaction solution was concentrated under reduced pressure and purified on a reversed-phase column (ACN:0.1% FA-H₂O) to give a racemate of 97D (10 mg, yield: 72%) as a white solid. LCMS [M+H-C₄H₈]⁺ = 668.2.

Step 3: The racemate of compound 97D (9.0 mg, 0.013 mmol) was dissolved in THF (0.5 mL), and formic acid (0.5 mL) was added. Under a nitrogen atmosphere, the mixture was stirred at 25 °C for 1 h. The reaction solution was concentrated under reduced pressure and purified on a reversed-phase column (ACN:0.1% FA-H₂O) to give a racemate of 97 (2.6 mg, yield: 35%) as a white solid, which was further resolved on a chiral column to give compound 97. LCMS [M+H]⁺ = 572.5.

¹H NMR (400 MHz, DMSO) δ 8.25 (d, *J* = 0.7 Hz, 1H), 7.41 (d, *J* = 0.7 Hz, 1H), 7.20 (dd, *J* = 11.3, 1.1 Hz, 2H), 7.16 - 7.00 (m, 5H), 6.90 (d, *J* = 6.8 Hz, 2H), 6.68 (d, *J =* 9.0 Hz, 2H), 6.14 (t, *J* = 5.6 Hz, 1H), 5.97 (t, *J =* 5.8 Hz, 1H), 5.33 (s, 1H), 4.69 (t, *J =* 5.1 Hz, 1H), 3.93 (s, 3H), 3.73 - 3.61 (m, 4H), 3.50 (dt, *J =* 13.1, 6.5 Hz, 1H), 3.42 (dd, *J* = 10.8, 5.6 Hz, 2H), 3.37 (d, *J* = 6.3 Hz, 1H), 3.12 (qd, *J =* 5.9, 2.0 Hz, 2H), 2.82 - 2.71 (m, 1H), 2.23 - 2.00 (m, 2H).

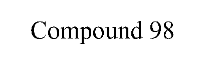

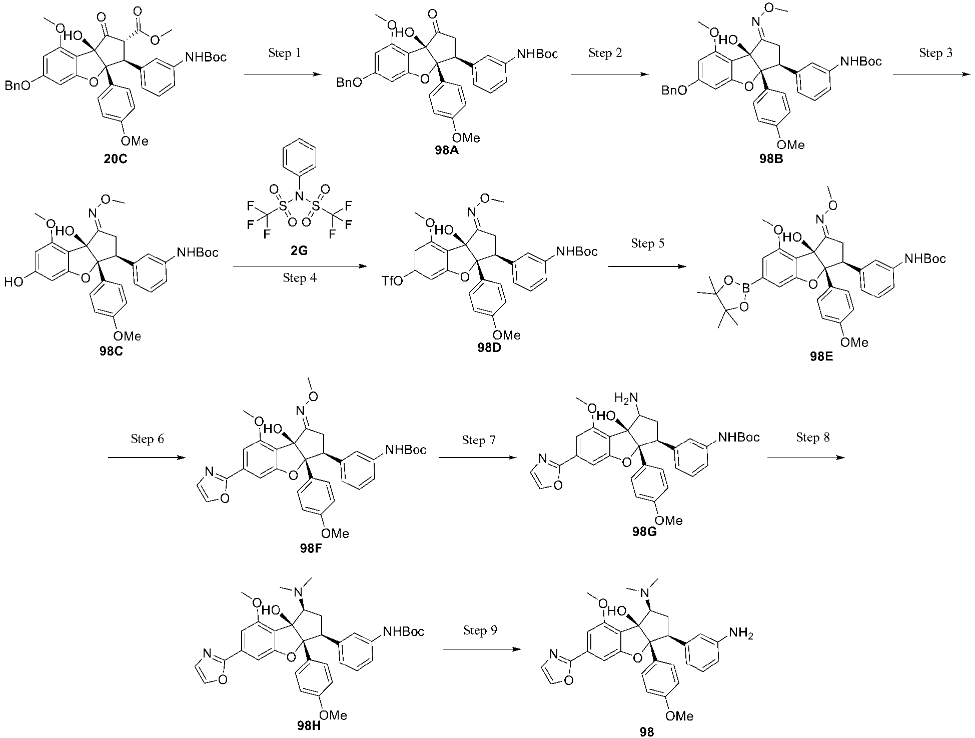

Step 1: DMAP (268 mg, 2.2 mmol) was added to a solution of the racemate of compound 20C (5 g, 7.33 mmol) in H₂O/toluene (10/50 mL), and the mixture was stirred at 60 °C for 1 h. The reaction solution was concentrated to dryness by rotary evaporation and extracted with water and ethyl acetate. The organic phases were combined, washed with saturated brine, dried over anhydrous sodium sulfate, filtered, concentrated, and then purified by normal-phase column chromatography (A: PE, B: EA) to give a racemate of 98A (4 g, yield: 87.45%) as a yellow solid. LCMS: [M+Na]⁺ = 646.6.

Step 2: The racemate of compound 98A (200 mg, 0.32 mmol) and O-methylhydroxylamine hydrochloride (134 mg, 1.61 mmol) in EtOH/pyridine (1/1 mmL) were heated to 60 °C and stirred for 2 h. The reaction solution was concentrated to dryness by rotary evaporation and purified by normal-phase column chromatography (PE, EA) to give a racemate of 98B (120 mg, yield: 57.4%) as a white solid. LCMS: [M+Na]⁺ = 675.7.

Step 3: Pd(OH)₂/C (300 mg) was added to a solution of the racemate of compound 98B (600 mg, 0.92 mmol) in AcOH (10 mL), and the mixture was purged with hydrogen three times and stirred at room temperature for 1 h. The reaction solution was filtered, and the filtrate was directly concentrated to dryness by rotary evaporation to give a racemate of compound 98C (600 mg, crude product, yield not calculated), which was directly used in the next step. LCMS: [M+H]⁺ = 563.6. Step 4: K₂CO₃ (674.96 mg, 1.78 mmol) was added to a solution of the racemate of compound 98C (500 mg, 0.89 mmol) and compound 2G (317.05 mg, 0.89 mmol) in DMF, and the mixture was allowed to react at 60 °C for 0.5 h. The reaction solution was concentrated to dryness by rotary evaporation and extracted with water and ethyl acetate. The organic phases were combined, washed with saturated brine, dried over anhydrous sodium sulfate, filtered, concentrated, and then purified by normal-phase column chromatography (PE:EA) to give a racemate of 98D (330 mg, yield: 47.55%) as a yellow solid. LCMS: [M+H] = 695.6.

Step 5: Bis(pinacolato)diboron (351.27 mg, 1.38 mmol), Pd(dppf)Cl₂·DCM (75.25 mg, 0.09 mmol), and KOAc (173.63 mg, 1.84 mmol) were added to a solution of the racemate of compound 98D (320 mg, 0.46 mmol) in 1,4-dioxane (5 mL), and the mixture was stirred at 85 °C for 5 h. The reaction solution was filtered, and the filtrate was purified by normal-phase column chromatography (PE/EA = 1/1) to give a racemate of 98E (300 mg, yield: 96.77%) as a yellow solid. LCMS [M+H-H₂O]⁺ = 655.2.

Step 6: 2-Bromooxazole (231.08 mg, 1.56 mmol), Pd(dppf)Cl₂ (25.49 mg, 0.03 mmol), and Na₂CO₃ (66.21 mg, 0.62 mmol) were added to a mixed solution of the racemate of compound 98E (210 mg, 0.31 mmol) in 1,4-dioxane (10 mL) and H₂O (2 mL), and the mixture was allowed to react in a microwave reactor at 135 °C for 2.5 h. The reaction solution was filtered, and the filtrate was purified by normal-phase column chromatography (PE/EA = 1/1) to give a racemate of 98F (90 mg, yield: 50.33%) as a yellow solid. LCMS [M+H]⁺ = 614.6.

Step 7: Raney Ni (30 mg) was added to a solution of the racemate of compound 98F (70 mg, 0.11 mmol) in AcOH (5 mL), and the mixture was purged with hydrogen three times and stirred at room temperature for 1 h. The reaction solution was filtered, and the filtrate was concentrated to give a racemate of 98G (60 mg, crude product, yield not calculated) as a yellow solid, which was directly used in the next step. LCMS: [M+H]+ = 600.6.

Step 8: An aqueous formaldehyde solution (1 mL) was added to a solution of the racemate of compound 98G (20 mg, 0.03 mmol) in DMF (1 mL), and the mixture was stirred at room temperature for 1 h. Na(AcO)₃BH (36.23 mg, 0.17 mmol) was added to the reaction solution, and the mixture was stirred for another 1 h. The reaction solution was concentrated to dryness by rotary evaporation and purified by normal-phase column chromatography (PE, EA) to give a racemate of 98H (15 mg, yield: 71.4%) as a white solid. LCMS [M+H]⁺ = 614.6.

Step 9: A solution of HCl in 1,4-dioxane (1 mL) was added to a solution of the racemate of compound 98H (10 mg, 0.02 mmol) in DCM (1 mL), and the mixture was allowed to react at room temperature for 1 h. The reaction solution was purified by reversed-phase column chromatography (H₂O/ACN) to give a racemate of 98 (1.1 mg, yield: 13.75%) as a white solid, which was further resolved on a chiral column to give compound 98.

LCMS [M+H]⁺ = 514.2

¹H NMR (400 MHz, DMSO) δ 8.23 (s, 1H), 7.39 (s, 1H), 7.06 (dd, *J* = 29.7, 11.4 Hz, 4H), 6.74 (t, *J* = 7.9 Hz, 1H), 6.58 (d, *J =* 8.9 Hz, 2H), 6.39 (s, 1H), 6.25 (d, *J* = 7.7 Hz, 2H), 5.08 (s, 1H), 4.82 (s, 2H), 3.83 (s, 3H), 3.71 (s, 1H), 3.61 (s, 3H), 2.19 (s, 6H), 1.20 (d, *J* = 31.1 Hz, 2H).

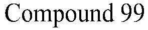

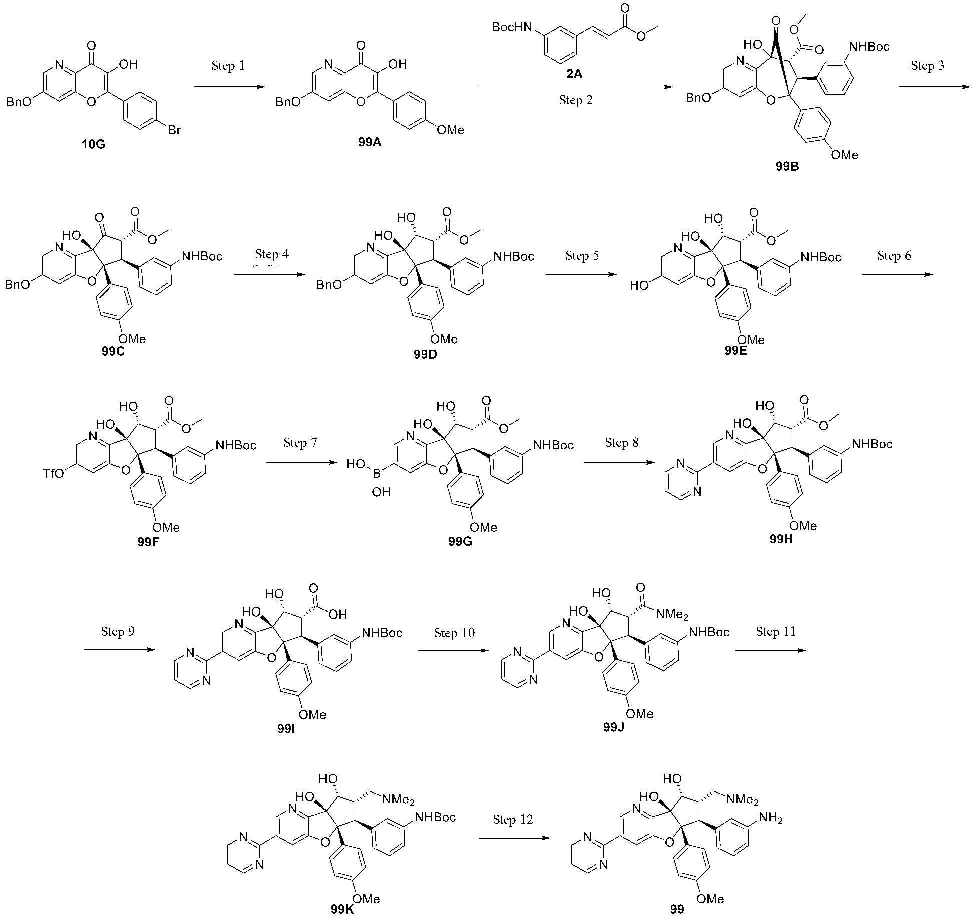

Step 1: At room temperature, tBuXPhos Pd G3 (225.0 mg, 0.28 mmol) and Cs₂CO₃ (6.936 g, 21.28 mmol) were added to a solution of compound 10G (6.0 g, 14.18 mmol) in MeOH (45 mL) and toluene (90 mL). The mixture was purged with nitrogen 3 times and allowed to react at 85 °C for 5 h. The reaction solution was filtered and purified on a normal-phase column to give 99A (2.9 g, yield: 54.5%) as a yellow solid. LC-MS: [M+H]⁺ = 376.3.

Steps 2-4: A racemate of compound 99D was synthesized according to a synthetic method similar to that described for compound 96 in steps 9-11. LCMS: [M+H]⁺ = 655.6.

Step 5: Pd/C (10%, 0.2 g) and AcOH (0.22 g, 3.67 mmol) were added to a solution of the racemate of compound 99D (0.8 g, 1.22 mmol) in MeOH (10 mL), and the mixture was purged with hydrogen three times and stirred at room temperature for 12 h. The reaction solution was filtered. The filter cake was rinsed with DCM/MeOH (10:1), and the filtrate was concentrated to give a racemate of 99E (0.5 g, yield: 72.5%) as a white solid. LCMS: [M+H]⁺ = 565.2.

Step 6: Compound 2G (316.49 mg, 0.89 mmol) and K₂CO₃ (244.68 mg, 1.77 mmol) were added to a solution of the racemate of compound 99E (0.5 g, 0.89 mmol) in DMF (7 mL), and the mixture was stirred at 60 °C for 2 h. The reaction solution was extracted with water and ethyl acetate, dried, and concentrated. The resulting crude product was purified on a normal-phase column to give a racemate of 99F (0.5 g, yield: 81.0%) as a white solid. LCMS: [M+H]⁺ = 697.5

Step 7: Bis(pinacolato)diboron (545.26 mg, 2.16 mmol), Pd(dppf)Cl₂ (117.24 mg, 0.14 mmol), and KOAc (270.11 mg, 2.87 mmol) were added to a solution of the racemate of compound 99F (0.5 g, 0.72 mmol) in 1,4-dioxane (10 mL). Under a nitrogen atmosphere, the mixture was stirred at 85 °C for 24 h. The reaction solution was filtered, and the filter cake was washed with ethyl acetate. The filtrate was concentrated and purified on a normal-phase column to give a racemate of 99G (0.2 g, yield: 47.0%) as a white solid. LCMS: [M+H]⁺ = 593.5.

Step 8: 2-Bromopyrimidine (63.6 mg, 0.4 mmol), Pd(PPh₃)₄ (34.65 mg, 0.03 mmol), and Na₂CO₃ (71.44 mg, 0.67 mmol) were added to a solution of the racemate of compound 99G (0.2 g, 0.337 mmol) in ACN (1 mL) and H₂O (0.25 mL), and the mixture was bubbled with nitrogen for 1 min and stirred at 110 °C for 0.5 h. The reaction solution was filtered, and the filter cake was washed with ethyl acetate. The filtrate was concentrated and purified on a normal-phase column to give a racemate of 99H (0.1 g, yield: 47.2%) as a white solid. LCMS: [M+H]⁺ = 627.6.

Step 9: LiOH·H₂O (20.03 mg, 0.477 mmol) was added to a solution of the racemate of compound 99H (0.1 g, 0.159 mmol) in THF/H₂O/MeOH (1 mL/0.25 mL/0.25 mL), and the mixture was stirred at 40 °C for 3 h. The reaction solution was adjusted to pH = 5 with 1 M HCl and concentrated. The crude product was purified on a normal-phase column to give a racemate of 99I (90.0 mg, yield: 92.0%) as a white solid. LCMS: [M+H]⁺ = 613.5

Step 10: Dimethylamine (9.9 mg, 0.22 mmol), HATU (64.6 mg, 0.17 mmol), HOAT (23.12 mg, 0.17 mmol), and TMP (53.2 mg, 0.44 mmol) were added to a solution of the racemate of compound 99I (90.0 mg, 0.147 mmol) in DMF (1 mL), and the mixture was stirred at room temperature for 2 h. The reaction solution was purified on a reversed-phase column to give a racemate of 99J (70.0 mg, yield: 74.4%) as a white solid. LCMS: [M+H]⁺ = 640.6.

Step 11: Under a nitrogen atmosphere, DIBAL-H (0.36 mg, 0.36 mmol) was slowly added dropwise to a solution of the racemate of compound 99J (60.0 mg, 0.09 mmol) in THF (0.5 mL) at 0 °C, and the mixture was stirred at room temperature for 2 h. The reaction solution was quenched with water and purified on a reversed-phase column to give a racemate of 99K (4.0 mg, yield: 6.8%) as a white solid. LCMS: [M+H]⁺ = 626.6.

Step 12: 6 N HCl (0.5 mL) was added dropwise to a solution of the racemate of compound 99K (4.0 mg, 0.01 mmol) in THF (0.5 mL), and the mixture was stirred at room temperature for 2 h. The reaction solution was purified on a preparative silica gel plate to give a racemate of 99 (0.8 mg, yield: 23.8%) as a white solid, which was further resolved on a chiral column to give compound 99. LCMS: [M+H]⁺ = 526.2.

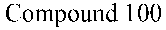

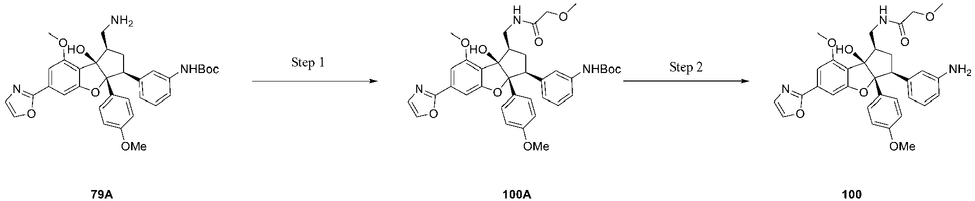

Step 1: Methoxyacetic acid (5 mg, 0.051 mmol), HATU (7 mg, 0.018 mmol), and TMP (4 mg, 0.034 mmol) were added to a mixture of the racemate of compound 79A (10 mg, 0.017 mmol) in DMF (1 mL), and the mixture was stirred at 20 °C for 2 h. The reaction solution was purified on a reversed-phase chromatography column (MeCN/H₂O/FA) to give a racemate of 100A (8 mg, yield: 73%) as a white solid. LCMS [M+H-H₂O]⁺ = 654.2.

Step 2: The racemate of compound 100A (8 mg, 0.012 mmol) was dissolved in a mixed solution of 6 M HCl (1 mL) and THF (1 mL), and the mixture was stirred at 25 °C for 5 h. The reaction solution was purified on a reversed-phase chromatography column (MeCN/FA/H₂O) to give a racemate of 100 (3 mg, yield: 43%) as a white solid, which was further resolved on a chiral column to give compound 100.

LCMS [M+H-H₂O]⁺ = 554.0.

¹H NMR (400 MHz, DMSO) δ 8.25 (s, 1H), 7.65 (s, 1H), 7.41 (s, 1H), 7.21 (d, *J* = 3.0 Hz, 2H), 7.17 (s, 1H), 7.15 (s, 1H), 6.70 (d, *J =* 8.9 Hz, 3H), 6.31 (s, 1H), 6.26 (s, 1H), 6.13 (s, 1H), 5.33 (d, *J* = 4.8 Hz, 2H), 3.94 (s, 3H), 3.89 (d, *J* = 4.8 Hz, 2H), 3.64-3.66 (m, 4H), 3.38 (s, 3H), 2.02 - 1.97 (m, 3H).

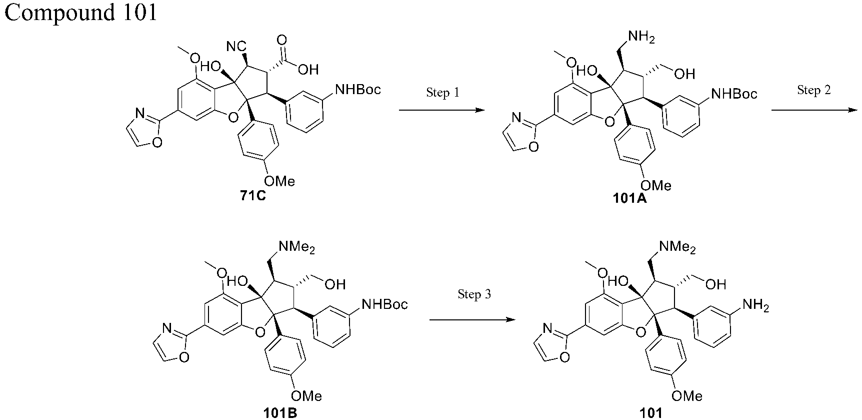

Step 1: BH₃·THF (1 M, 2 mL) was added to a solution of the racemate of the starting material 71C (100 mg, 0.156 mmol) in THF (2 mL) at 0 °C, and the mixture was allowed to react at 0 °C to 20 °C for 4 h. MeOH (2 mL) was slowly added dropwise to the reaction solution, and the mixture was stirred at room temperature for another 1 h. The reaction solution was concentrated to give a crude product of 101A (100 mg, crude product, yield not calculated), which was directly used in the next step. LCMS [M+H]⁺ = 630.6.

Step 2: Pd/C (100 mg) and an aqueous formaldehyde solution (3 mL, 37%) were added to a solution of the racemate of compound 101A (100 mg, 0.156 mmol) in MeOH (3 mL), and the mixture was purged with hydrogen three times and allowed to react at 20 °C for 8 h. The reaction solution was filtered to remove palladium on carbon and purified on a reversed-phase column (MeCN/0.1% FA/H₂O) to give a racemate of 101B (65 mg, yield: 63%) as a yellow solid. LCMS [M+H]⁺ = 658.2.

Step 3: A solution of HCl in 1,4-dioxane (4 N, 1 mL) was added to the racemate of compound 101B (65 mg, 0.099 mmol) in DCM (1 mL), and the mixture was allowed to react at 20 °C for 2 h. The reaction solution was concentrated and slurried with EA twice to give a racemate of 101 (45 mg, yield: 69%) as a yellow solid, which was further resolved on a chiral column to give compound 101.

LCMS [M+H]⁺ = 658.2

1H NMR (400 MHz, DMSO) δ 10.34 (s, 2H), 8.89 (s, 1H), 8.29 (s, 1H), 7.44 (s, 1H), 7.30 - 7.24 (m, 2H), 7.20 - 7.10 (m, 4H), 7.03 (s, 1H), 6.74 (d, J = 8.9 Hz, 2H), 6.66 (d, J = 7.2 Hz, 1H), 6.02 (s, 1H), 4.01 (s, 3H), 3.69 (s, 3H), 3.54 - 3.43 (m, 3H), 3.28 - 3.14 (m, 4H), 3.10 (d, J = 4.7 Hz, 3H), 3.00 (d, J = 4.6 Hz, 3H), 2.72 - 2.59 (m, 1H), 1.23 (s, 1H).

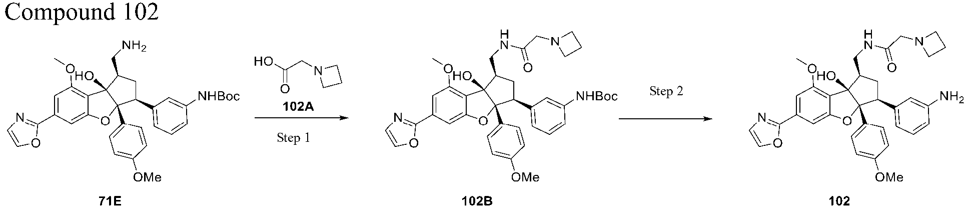

Step 1: Compound 102A (6 mg, 0.051 mmol), HATU (7 mg, 0.018 mmol), and TMP (4 mg, 0.034 mmol) were added to a solution of the racemate of compound 71E (10 mg, 0.017 mmol) in DMF (1 mL), and the mixture was stirred at 20 °C for 2 h. The reaction solution was purified by reversed-phase column chromatography (MeCN/H₂O/FA) to give a racemate of 102B (7 mg, yield: 58%) as a white solid. LCMS [M+H]⁺ = 697.2.

Step 2: The racemate of compound 102B (7 mg, 0.01 mmol) was dissolved in a mixed solution of 6 M HCl (1 mL) and THF (1 mL), and the mixture was stirred at 25 °C for 5 h. The reaction solution was purified by reversed-phase column chromatography (MeCN/FA/H₂O) to give a racemate of 102 (3 mg, yield: 50%) as a white solid, which was further resolved on a chiral column to give compound 102.

LCMS [M+H]⁺ = 597.0.

¹H NMR (400 MHz, DMSO) δ 8.26 (s, 2H), 7.68 (s, 1H), 7.42 (s, 1H), 7.21 (s, 2H), 7.16 (d, *J* = 8.8 Hz, 2H), 6.70 (dd, *J =* 8.0, 6.0 Hz, 3H), 6.30 (s, 1H), 6.23 (d, *J* = 7.8 Hz, 1H), 6.10 (d, *J* = 7.3 Hz, 1H), 5.29 (s, 1H), 3.99 (s, 3H), 3.66-3.60 (m, 4H), 3.24 - 3.20 (m, 4H), 3.08 (d, *J* = 5.0 Hz, 2H), 2.84 (s, 1H), 2.06-2.00 (m, 5H).

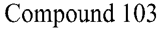

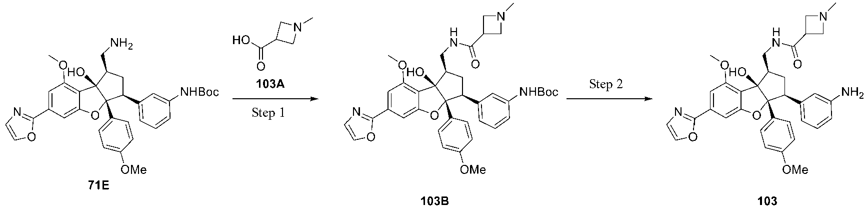

Step 1: Compound 103A (6 mg, 0.051 mmol), HATU (7 mg, 0.018 mmol), and TMP (4 mg, 0.034 mmol) were added to a solution of the racemate of compound 71E (10 mg, 0.017 mmol) in DMF (1 mL), and the mixture was stirred at 20 °C for 2 h. The reaction solution was purified by reversed-phase column chromatography (MeCN/H₂O/FA) to give a racemate of 103B (8 mg, yield: 67%) as a white solid. LCMS [M+H]⁺ = 697.2.

Step 2: The racemate of compound 103B (8 mg, 0.012 mmol) was dissolved in a mixed solution of 6 M HCl (1 mL) and THF (1 mL), and the mixture was stirred at 25 °C for 5 h. The reaction solution was purified by reversed-phase chromatography (MeCN/FA/H₂O) to give a racemate of 103 (3 mg, yield: 43%) as a white solid, which was further resolved on a chiral column to give compound 103.

LCMS [M+H]+ = 597.2.

¹H NMR (400 MHz, DMSO) δ 8.25 (s, 1H), 8.17 (s, 1H), 7.77 (s, 1H), 7.41 (s, 1H), 7.19 (d, *J* = 3.5 Hz, 2H), 7.15 (d, *J* = 8.9 Hz, 2H), 6.69-6.60 (m, 3H), 6.28 (s, 1H), 6.23 (d, *J* = 8.0 Hz, 1H), 6.08 (d, *J* = 7.7 Hz, 1H), 5.32 (s, 1H), 5.17 (s, 1H), 4.79 (s, 1H), 3.97-3.90 (m, 4H), 3.71 (s, 1H), 3.68-3.60 (m, 4H), 3.61 (s, 3H), 2.74 (s, 1H), 2.37 (s, 3H), 2.01 - 1.97 (m, 2H).

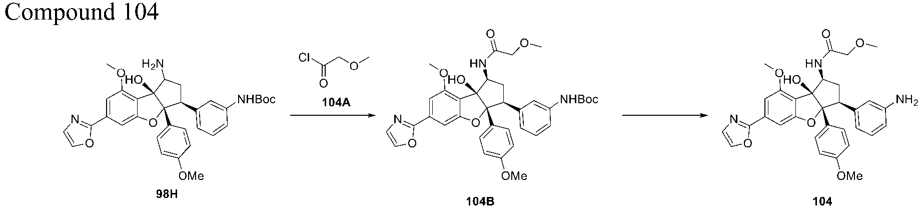

A racemate of compound 104 was synthesized according to a synthetic method similar to that for compound 100, and further resolved on a chiral column to give compound 104.

LCMS: [M+Na]⁺ = 580.2

¹H NMR (400 MHz, DMSO) δ 8.27 (s, 1 H), 8.07 (d, J = 7.4 Hz, 1 H), 7.43 (s, 1 H), 7.21 (d, J = 6.2 Hz, 2 H), 7.11 (d, J = 8.8 Hz, 2 H), 6.75 (t, J = 7.8 Hz, 1 H), 6.67 (d, J = 8.8 Hz, 2 H), 6.35 (s, 1 H), 6.30 - 6.23 (m, 2 H), 5.45 (s, 1 H), 5.32 (s, 1 H), 3.88 (s, 3 H), 3.81 (d, J = 4.3 Hz, 2 H), 3.65 (s, 3 H), 3.29 (s, 3 H), 2.03 - 1.99 (m, 2 H).

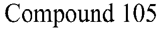

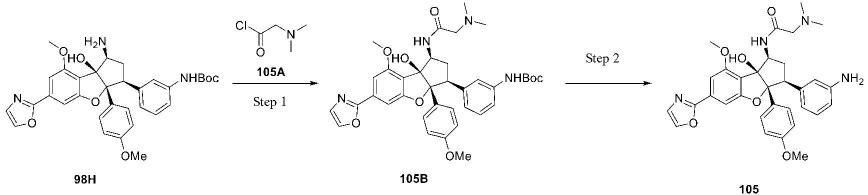

A racemate of compound 105 was synthesized according to a synthetic method similar to that for compound 100, and further resolved on a chiral column to give compound 105.

LCMS: [M+H]⁺ = 571.2.

¹H NMR (400 MHz, DMSO) δ 8.33 (s, 1 H), 8.26 (d, *J* = 6.5 Hz, 1 H), 8.16 (d, *J =* 7.1 Hz, 1 H), 7.42 (d, *J =* 5.0 Hz, 1 H), 7.21 (d, *J =* 8.1 Hz, 2 H), 7.10 (d, *J* = 8.8 Hz, 2 H), 6.76 (t, *J* = 7.8 Hz, 1 H), 6.66 (d, *J =* 8.9 Hz, 2 H), 6.36 (s, 1 H), 6.30 (d, *J* = 7.8 Hz, 1 H), 6.24 (d, *J* = 7.9 Hz, 1 H), 5.45 (s, 1 H), 4.76 (dd, *J* = 13.8, 6.9 Hz, 1 H), 3.88 (d, *J* = 5.8 Hz, 3 H), 3.64 (s, 3 H), 2.83 (s, 2 H), 2.13 (s, 6 H), 2.00 (d, *J* = 7.6 Hz, 1 H), 1.89 (dd, *J =* 12.8, 6.9 Hz, 1 H).

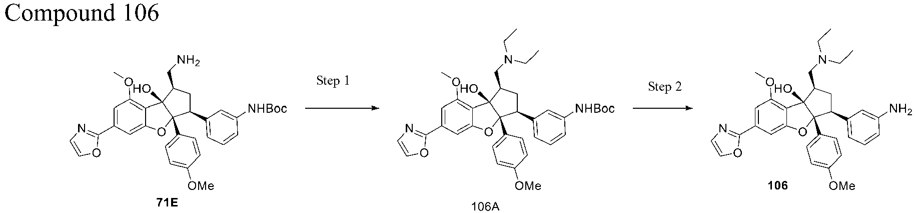

A racemate of compound 106 was synthesized according to a synthetic method similar to that for compound 79, and further resolved on a chiral column to give compound 106.

LCMS [M+H]⁺ = 557.2.

¹H NMR (400 MHz, DMSO) δ 8.34 (s, 1H), 8.25 (s, 1H), 7.41 (s, 1H), 7.17 (m 5H), 6.70 (d, *J* = 8.8 Hz, 3H), 6.34 (s, 1H), 6.23 (m, 1H), 6.17 (d, *J =* 7.6 Hz, 1H), 4.80 (s, 1H), 3.92 (s, 3H), 3.66 (s, 3H), 3.00 (dd, *J =* 12.8, 5.4 Hz, 1H), 2.91 - 2.83 (m, 1H), 2.63 - 2.58 (m, 2H), 2.23 - 1.97 (m, 4H), 1.01 (t, *J* = 7.2 Hz, 6H).

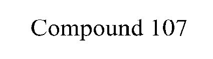

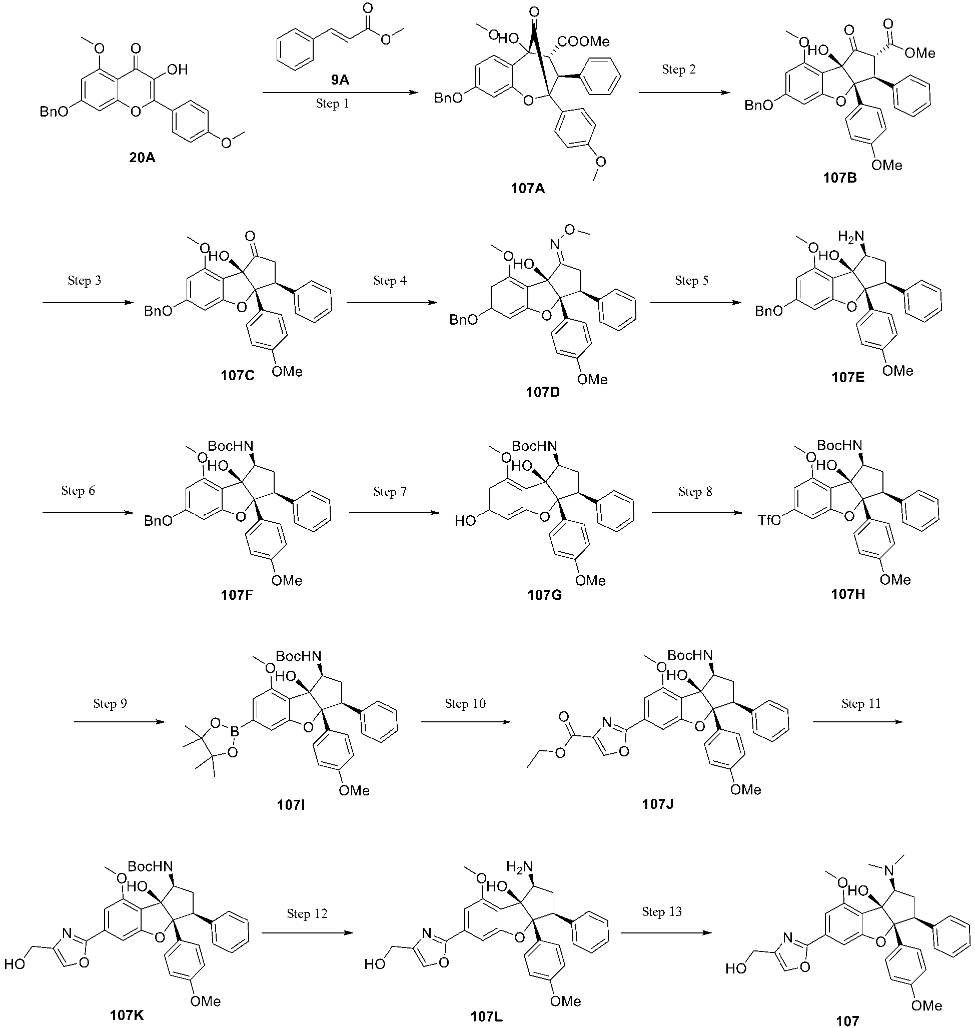

Steps 1 and 2: A racemate of compound 107B was synthesized according to a synthetic method similar to that described for compound 96 in steps 9 and 10. LCMS [M+H]⁺ = 549.6.

Steps 3 and 4: A racemate of compound 107D was synthesized according to a synthetic method similar to that described for compound 98 in steps 1 and 2. LCMS: [M+H-H₂O]⁺ = 520.5.

Step 5: 10 M BH3·DMS (1.9 mL) was added to a solution of the racemate of compound 107D (1 g, 1.86 mmol) in THF (20 mL), and the mixture was stirred at 65 °C for 6 h. The reaction solution was quenched with methanol and concentrated to give a racemate of compound 107E, which was directly used in the next step. LCMS [M+H]⁺ = 510.5.

Step 6: Boc₂O (1.01 g, 4.65 mmol) was added to a solution of the racemate of compound 107E (948 mg, 1.86 mmol) and TEA (752 mg, 7.4 mmol) in DCM (20 mL), and the mixture was stirred at room temperature for 2 h. The reaction solution was concentrated and extracted with water and ethyl acetate. The organic phases were combined, washed with saturated brine, dried over anhydrous sodium sulfate, filtered, concentrated, and then purified on a normal-phase chromatography column (PE/EA) to give a racemate of 107F (350 mg, yield: 31%) as a white solid. LCMS [M-H₂O-t-Bu+H]⁺ = 536.5.

Steps 7-10: A racemate of compound 107J was synthesized according to a synthetic method similar to that described for compound 98 in steps 3-6.LCMS [M+H-C₄H₈]⁺ = 587.4.

Step 11: A solution of the racemate of compound 107J (30 mg, 0.046 mmol) in THF was cooled to 0 °C, and a solution of LiAlH₄ (0.48 mL, 0.46 mmol, 1 N in THF) was slowly added dropwise to the reaction solution. The mixture was stirred at 0 °C for 2 h. Methanol was added to the reaction solution to quench the reaction, and the reaction solution was purified by normal-phase column chromatography (DCM/MeOH = 10/1) to give a racemate of 107K (20 mg, yield: 71.4%) as a white solid. LCMS [M+H]⁺ = 601.2.

Step 12: 4 M HCl (0.3 mL) was added to a solution of the racemate of compound 107K (20 mg, 0.033 mmol) in THF (1 mL), and the mixture was stirred at room temperature for 2 h.The reaction solution was purified by reversed-phase column chromatography to give a racemate of 107L (15 mg, yield: 90.4%) as a white solid.LCMS [M+H]⁺ = 501.5.

Step 13: Two drops of acetic acid were added to a solution of the racemate of compound 107L (15 mg, 0.03 mmol) and an aqueous formaldehyde solution (30%, 0.3 mL) in DMF (2 mL), and the mixture was stirred at room temperature for 2 h.NaBH(AcO)₃ (19 mg, 0.09 mmol) was added to the reaction solution, and the mixture was stirred at room temperature for another 2 h. The reaction solution was purified by reversed-phase column chromatography (H₂O/ACN) to give a racemate of 107 (1 mg, yield: 13.1%) as a white solid, which was further resolved on a chiral column to give compound 107.

LCMS [M+H]⁺ = 529.2.

¹H NMR (400 MHz, DMSO) δ 8.05 (s, 1H), 7.21 (t, *J* = 6.8 Hz, 2H), 7.10 (dd, *J* = 9.5, 7.0 Hz, 5H), 7.00 (d, *J* = 6.8 Hz, 2H), 6.69 (t, *J* = 6.8 Hz, 2H), 5.31 (dd, *J* = 6.6, 4.5 Hz, 2H), 4.46 (d, *J* = 5.6 Hz, 2H), 3.94 (d, *J* = 12.0 Hz, 3H), 3.66 (s, 3H), 2.53 (s, 6H), 2.31 - 2.24 (m, 1H), 1.97 (s, 1H).

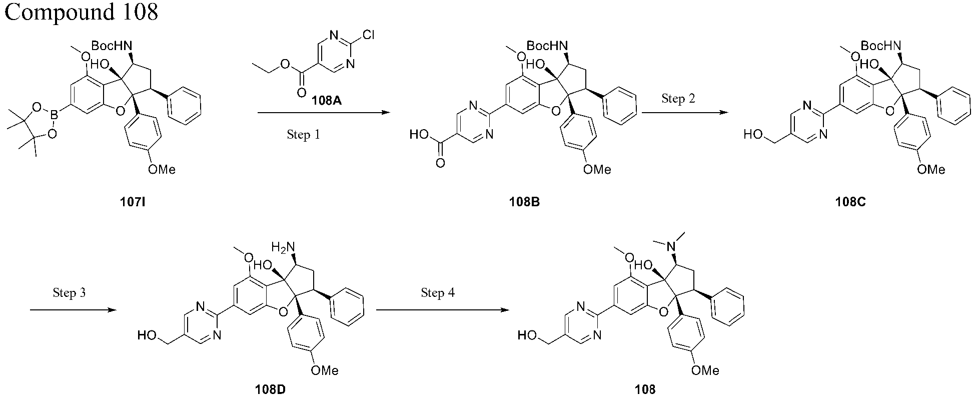

Step 1: The racemate of compound 107I (20.0 mg, 31.8 µmol), compound 108A (17.8 mg, 95.3 µmol), Pd(PPh₃)₄ (3.7 mg, 3.2 µmol), and K₂CO₃ (13.17 mg, 95.3 µmol) were dissolved in a mixed solution of 1,4-dioxane (1.5 mL) and H₂O (0.5 mL). Under a nitrogen atmosphere, the mixture was heated by microwave to 70 °C and allowed to react for 30 min. The reaction solution was purified by preparative HPLC (CH₃CN/H₂O, 0.1% FA) to give a racemate of 108B (15.0 mg, yield: 75.5%) as a white solid. LCMS: [M/2+H]⁺ = 626.5.

Step 2: The racemate of compound 108B (14.0 mg, 22.4 *µ*mol) was dissolved in THF (1 mL), and LiAlH₄ (2 drops) was added at 0 °C. The mixture was stirred at 0 °C for 2 h. Water (1 drop), an aqueous NaOH solution (1 drop), and water (1 drop) were added. The resulting mixed solution was dried over anhydrous sodium sulfate, filtered, and concentrated to give a crude product of a racemate of compound 108C, which was directly used in the next step. LCMS: [M+H]⁺ = 566.5. Step 3: The crude product of the racemate of compound 108C was dissolved in DCM (1 mL), and TFA (1 mL) was added with stirring. The mixture was stirred at 20 °C for 1 h. The reaction solution was concentrated to dryness by rotary evaporation to give a crude product of a racemate of compound 108D, which was directly used in the next step. LCMS: [M+H]⁺ = 512.5.

Step 4: The crude product of the racemate of compound 108E was dissolved in MeOH (1.5 mL) and formaldehyde (1.5 mL), and the solution was stirred. Then, Pd/C (10.0 mg) was added, and the mixture was purged with hydrogen three times and stirred at 20 °C for 2 h. The reaction solution was filtered, and the filtrate was concentrated and purified by preparative HPLC (CH₃CN/H₂O, 0.1% FA) to give a racemate of 108E (1.0 mg) as a white solid, which was further resolved on a chiral column to give compound 108. LCMS: [M+H]⁺ = 540.5.

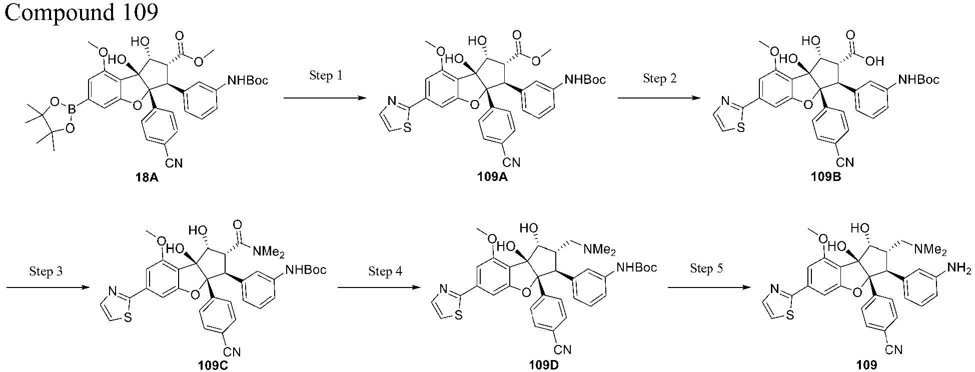

Step 1: The racemate of compound 18A (30.0 mg, 0.04 mmol) was dissolved in dioxane/water (1 mL/2 mL), and 2-bromothiazole (10.6 mg, 0.06 mmol), Na₂CO₃ (13.7 mg, 0.13 mmol), and Pd(dppf)Cl₂ (3.5 mg, 0.004 mmol) were added. The mixture was purged with nitrogen three times and stirred at 85 °C for 16 h. The reaction solution was concentrated and purified by silica gel column chromatography (DCM/MeOH) to give a racemate of compound 109A (20.0 mg, yield: 35%). LCMS [M+H]⁺ = 600.5.

Step 2: The racemate of compound 109A (20.0 mg, 0.03 mmol) and LiOH·H₂O (5.1 mg, 0.12 mmol) were dissolved in THF/H₂O/MeOH (1 mL/0.1 mL/0.1 mL), and the solution was stirred at 40 °C for 3 h. The reaction solution was adjusted to pH = 5 with 3 M hydrochloric acid and purified on a silica gel column (DCM/MeOH) to give a racemate of compound 109B (10 mg, yield: 52%). LCMS [M+H]⁺ = 642.7.

Step 3: The racemate of compound 109B (10.0 mg, 0.02 mmol), HATU (17.8 mg, 0.05 mmol), and DIEA (10.1 mg, 0.08 mmol) were added to DMF (1 mL), and the mixture was stirred at 25 °C for 10 min. DMA (0.04 mL, 0.08 mmol, 2 M in THF) was added, and the mixture was stirred at 25 °C for 1 h. The reaction solution was diluted with water and extracted with EA. The organic phase was washed with water and saturated brine, dried over anhydrous Na₂SO₄, filtered, concentrated, and then purified on a silica gel column (DCM/MeOH) to give a racemate of compound 109C (8 mg, yield: 80%). LCMS [M+H]⁺ = 669.3.

Step 4: The racemate of compound 109C (8.0 mg, 0.01 mmol) and TFA (1.4 mg, 0.012 mmol) were added to THF (1 mL), and the mixture was stirred at 60 °C for 30 min. BH₃·DMS (0.04 mL, 0.036 mmol) was added, and the mixture was stirred at 25 °C for 2 h. The reaction solution was diluted with NaOH (aq), stirred at 25 °C for 30 min, concentrated, and then purified on a silica gel column (DCM/MeOH) to give a racemate of compound 109D (6 mg, yield: 77%). LCMS: [M+H]⁺ = 655.7.

Step 5: The racemate of compound 109D (6.0 mg, 0.01 mmol) was mixed with THF (1 mL), and 6 M HCl (0.5 mL) was added at 0 °C. The mixture was stirred at 25 °C for 1 h. The reaction solution was purified on a silica gel column (DCM/MeOH) to give a racemate of compound 109 (0.8 mg, yield: 15%), which was further resolved on a chiral column to give compound 109.

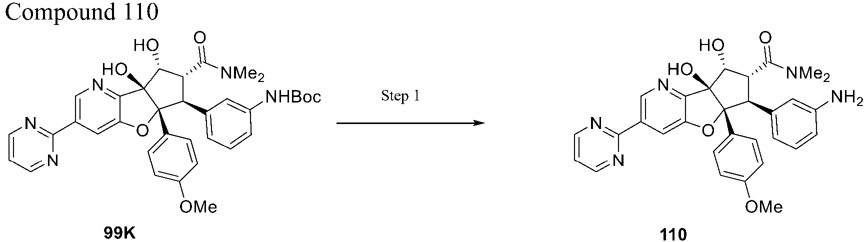

Step 1: 6 N HCl (0.5 mL) was added dropwise to a solution of the racemate of compound 99K (5.0 mg, 0.01 mmol) in THF (0.5 mL), and the mixture was stirred at room temperature for 2 h. The reaction solution was purified on a preparative silica gel plate to give 110 (3.0 mg, yield: 71.1%) as a white solid, which was further resolved on a chiral column to give compound 110.

LCMS: [M+H]⁺ = 540.2.

¹HNMR (400 MHz, DMSO-*d*₆) δ 9.18 (d, *J* = 1.3 Hz, 1H), 8.99 (d, *J* = 4.8 Hz, 2H), 8.26 (d, *J* = 1.3 Hz, 1H), 7.56 (t, *J* = 4.8 Hz, 1H), 7.22 - 6.96 (m, 6H), 6.66 (d, *J =* 8.8 Hz, 2H), 4.88 (d, *J =* 5.6 Hz, 1H), 4.55-4.51 (m, 1H), 4.27-4.22 (m, 1H), 3.62 (s, 3H), 3.39 (s, 1H), 3.28 (s, 3H), 2.78 (s, 3H), 1.77 (s, 1H).

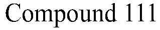

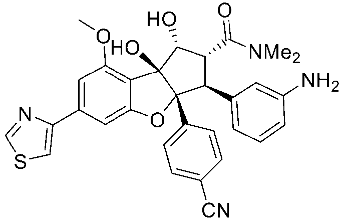

A racemate of compound 111 was synthesized according to a synthetic method similar to that for compound 18, and further resolved on a chiral column to give compound 111. LCMS [M+H]⁺ = 569.2.

Control compound 1 (rocaglamide):

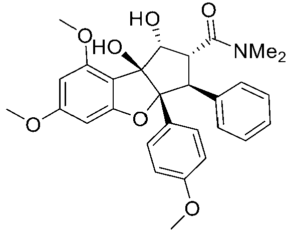

CAS: 84573-16-0, purchased from MedChemExpress LLC.

Santagata S, Mendillo ML, Tang YC, Subramanian A, Perley CC, Roche SP, Wong B, Narayan R, Kwon H, Koeva M, Amon A, Golub TR, Porco JA Jr, Whitesell L, Lindquist S. Tight coordination of protein translation and HSF1 activation supports the anabolic malignant state. Science. 2013 Jul 19; 341(6143):1238303. doi: 10.1126/science.1238303.

Control compound 2:

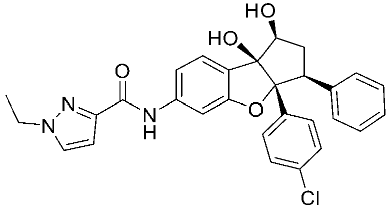

It was synthesized with reference to the patent WO2005113529A2.

### Example 2: Assay for Inhibition of In Vitro Proliferation of Tumor Cells by Small Molecule Compounds (Toxins)

Assay objective: To detect the inhibitory effects of the drug compounds on tumor cell proliferation, for example, the inhibitory activity thereof on the *in vitro* proliferation of NCI-H716, NCI-H1975, NCI-H1373, NCI-H647, NCI-H520, BT474, DLD-1, NCI-N87, NUGC-4, SNU-423, MKN-45, SK-OV-3, OVCAR3, Ls174T, HCC1954, and ES2 tumor cells. The cells were treated with the compounds at different concentrations *in vitro.* After 5 days of culture, the proliferation of the cells was detected using the CTG (CellTiter-Glo^{®} Luminescent Cell Viability Assay, Promega, Cat. No. G7558) reagent, and the *in vitro* activity of the compounds was evaluated based on the IC₅₀ value.
1. Cell preparation: NCI-H716, NCI-H1975, NCI-H1373, NCI-H647, NCI-H520, BT474, DLD-1, NCI-N87, NUGC-4, SNU-423, MKN-45, SK-OV-3, OVCAR3, Ls174T, HCC1954, and ES2 cells in the logarithmic phase were each taken, washed once with PBS, and then digested with 2-3 mL of trypsin for 2-3 min. After the cells were digested completely, 10-15 mL of a cell culture medium was added to elute the digested cells. The eluate was centrifuged at 1000 rpm for 5 min, and the supernatant was discarded. Subsequently, the resulting cells were resuspended in 10-20 mL of the cell culture medium to prepare a single-cell suspension.
2. Cell plating: The NCI-H716, NCI-H1975, NCI-H1373, NCI-H647, NCI-H520, NCI-N87, BT474, DLD-1, NCI-N87, NUGC-4, SNU-423, MKN-45, SK-OV-3, OVCAR3, Ls174T, HCC1954, and ES2 single-cell suspensions were each well mixed and adjusted to a viable cell density of 6 × 10⁴ cells/mL with the cell culture medium. The density-adjusted cell suspensions were each well mixed and added to a 96-well cell culture plate at 50 µL/well. The culture plate was incubated in an incubator overnight (37 °C, 5% CO₂).
3. Compound preparation: Each compound was dissolved in DMSO to prepare a stock solution with an initial concentration of 10 mM. There were 6 concentrations in total for the small molecule compound: 1000 nM, 333 nM, 111 nM, 37.03 nM, 12.34 nM, and 4.11 nM.
4. Sample addition: The prepared test samples at different concentrations were added to the culture plate, with two duplicate wells set for each sample. The culture plate was incubated in the incubator for 5 days (37 °C, 5% CO₂).
5. Color development: The 96-well cell culture plate was taken out, and 50 µL of the CTG reagent was added to each well. The plate was then incubated at room temperature for 10 min.
6. Plate reading: The 96-well cell culture plate was taken out and placed in a microplate reader, and the chemiluminescence was measured using the microplate reader.
7. Data analysis: The cell viability was calculated according to the formula: (Lum_{test drug} - Lum_{blank control})/(Lum_{solvent blank control} - Lum_{blank control}) × 100%. Using a nonlinear regression model in the GraphPad Prism software, a sigmoidal dose-survival rate curve was plotted, and the IC₅₀ values were calculated. The results are shown in Table 2.

**Table 2. IC₅₀ values for the inhibition of the in vitro proliferation of tumor cells by the racemates of the small molecule compounds of the present application**

| Compound No. | Proliferation inhibitory activity IC₅₀ (nM) | | | | | | | | |
|---|---|---|---|---|---|---|---|---|---|
| | BT474 | DLD-1 | NCI-N87 | NUGC-4 | SNU-423 | MKN-45 | SK-OV-3 | Ls174T | HCC1954 |
| Rocaglamide | 5.4861 | 8.1856 | 9.3239 | 8.2530 | 14.4404 | 5.7543 | 8.5247 | 6.0846 | 2.6620 |
| Racemate of control compound 2 | >100 | >100 | / | / | / | / | / | / | / |
| Racemate of compound 2 | 3.3268 | / | 2.0679 | 1.9845 | 3.6709 | / | 2.0485 | / | / |
| Racemate of compound 3 | / | / | 3.6290 | 3.5126 | 8.1778 | / | / | / | / |
| Racemate of compound 4 | 3.0366 | / | / | / | / | 1.4044 | / | / | / |
| Racemate of compound 8 | / | 5.0368 | / | / | / | 2.1198 | / | / | / |
| Racemate of compound 9 | 0.8287 | / | / | / | / | 0.6949 | / | / | / |
| Racemate of compound 11 | / | / | / | / | / | 1.4427 | / | / | / |
| Racemate of compound 14 | / | / | / | / | / | 0.8174 | / | / | / |
| Racemate of compound 15 | / | / | / | / | / | 2.6460 | / | / | / |
| Racemate of compound 16 | / | / | / | / | / | 2.3389 | / | / | / |
| Racemate of compound 18 | / | / | / | / | / | 1.9069 | / | / | / |
| Racemate of compound 21 | / | / | / | / | / | 1.1019 | / | / | / |
| Racemate of compound 23 | 1.8021 | 1.2848 | 0.8970 | / | / | 1.5023 | / | 1.1043 | / |
| Racemate of compound 29 | / | / | / | / | / | 1.2286 | / | / | / |
| Racemate of compound 30 | / | / | / | / | / | 1.5008 | / | / | / |
| Racemate of compound 31 | / | / | / | / | / | 1.3219 | / | / | / |
| Racemate of compound 32 | / | / | / | / | / | / | / | 5.4461 | / |
| Racemate of compound 35 | / | / | / | / | / | / | / | 3.0302 | / |
| Racemate of compound 41 | 4.649 | / | / | / | / | / | / | / | / |
| Racemate of compound 43 | / | / | / | / | / | 2.0029 | / | / | / |
| Racemate of compound 47 | 4.4376 | / | 2.0096 | / | / | 3.1216 | / | / | / |
| Racemate of compound 48 | 2.2505 | / | / | / | / | / | / | / | / |
| Racemate of compound 57 | / | 2.3515 | / | / | / | / | / | / | / |
| Racemate of compound 64 | / | 1.5363 | / | / | / | / | / | / | / |
| Racemate of compound 70 | / | 2.0437 | / | / | / | / | / | / | / |
| Racemate of compound 75 | 4.25 | / | / | / | / | / | / | / | / |
| Racemate of compound 79 | / | 2.486 | / | / | / | / | / | / | 0.7217 |
| Racemate of compound 86 | / | 2.989 | / | / | / | / | / | / | 2.1475 |
| Racemate of compound 87 | / | 2.839 | / | / | / | / | / | / | / |
| Racemate of compound 89 | / | 1.35 | / | / | / | / | / | / | / |
| Racemate of compound 90 | / | 2.747 | / | / | / | / | / | / | / |
| Racemate of compound 91 | / | 2.161 | / | / | / | / | / | / | / |
| Racemate of compound 93 | / | / | / | / | / | / | / | / | 1.4969 |
| Racemate of compound 97 | / | 3.083 | / | / | / | / | / | / | / |
| Racemate of compound 107 | / | 0.9205 | / | / | / | / | / | / | / |
| Racemate of compound 111 | / | / | / | / | / | / | / | / | 2.981 |

| | | | | | | | | | |
|---|---|---|---|---|---|---|---|---|---|
| "/" indicates that no detection was performed. | | | | | | | | | |

**Table 3. IC₅₀ values for the inhibition of the in vitro proliferation of tumor cells by the small molecule compounds of the present application**

| Compound No. | Proliferation inhibitory activity IC₅₀ (nM) | |
|---|---|---|
| | BT474 | DLD-1 |
| Control compound 2 | >50 | >50 |
| Compound 4 | <10 | <10 |
| Compound 11 | <10 | / |
| Compound 47 | <10 | / |
| Compound 49 | <10 | / |
| Compound 79 | <10 | <10 |
| Compound 93 | <10 | <10 |
| Compound 98 | <10 | <10 |
| Compound 106 | <10 | <10 |

| | | |
|---|---|---|
| "/" indicates that no detection was performed. | | |

Conclusion: According to the results in Tables 2 and 3, the small molecule compounds of the present application exhibited significantly enhanced inhibitory activity against the proliferation of BT474, DLD-1, NCI-N87, NUGC-4, SNU-423, MKN-45, SK-OV-3, Ls174T, and HCC1954.

### Example 3: In Vivo Pharmacodynamic Study of Small Molecule Compounds in BALB/c Nude Mouse Subcutaneous Xenograft Tumor Model

Assay objective: To evaluate the efficacy of the test compounds in a BALB/c nude mouse subcutaneous xenograft tumor model. The tumor cell line was selected from NCI-H716, BT474, DLD-1, NCI-N87, NUGC-4, SNU-423, MKN-45, SK-OV-3, Ls174T, or HCC1954.

### 3.1. Experimental procedures:

NCI-H716, BT474, DLD-1, NCI-N87, NUGC-4, SNU-423, MKN-45, SK-OV-3, Ls174T, or HCC1954 cells were taken from the cell bank, thawed, then added to a complete medium (RPMI1640 + 10% FBS + 1% P/S), and cultured in a CO₂ incubator (incubator temperature: 37 °C, CO₂ concentration: 5%). When the number of the cells was expanded to the quantity required for *in vivo* inoculation, the NCI-H716 cells were collected. The cells were counted using a full-automatic cell counter. According to the counting results, the cells were resuspended in a mixture of PBS and matrigel (1:1) to prepare a cell suspension (density: 5 × 10⁷/mL), which was then placed in an ice box for later use.

Female BALB/c nude mice aged 6-8 weeks (18-20 g, Laboratory Animal Management Department, Shanghai Institute of Planned Parenthood Research) were used. The mice were housed within separate cages in an SPF animal facility, with 5 mice in each cage. All cages, bedding, and water were sterilized at a high temperature before use, and all animals had free access to food and water. Before the start of the experiment, the nude mice were marked with disposable universal ear tags for rats and mice. Prior to inoculation, the skin at the inoculation site was disinfected with 75% medical alcohol. Then, 0.1 mL (containing 5 × 10⁶ cells) of NCI-H716, BT474, DLD-1, NCI-N87, NUGC-4, SNU-423, MKN-45, SK-OV-3, Ls174T, or HCC1954 cells were subcutaneously inoculated into the right back of each mouse. When the tumor volume reached 60-200 mm³, the mice were grouped for administration, with 5 mice in each group. Each test compound was administered via the tail vein twice a week, for a total of 5 doses (on D0, D4, D7, D11, and D14, respectively). The tumor volume and body weight of the mice were measured twice a week, and the TGI (%) was calculated.

### 3.2. Data processing:

Tumor volume (mm³) was calculated according to the formula: V = 0.5 × D × d × d, where D and d represent the long diameter and short diameter of the tumor, respectively.

### Calculation of TGI (%):

When there was no tumor regression, TGI (%) = [(1 - (average tumor volume at the end of administration in a treatment group - average tumor volume at the start of administration in the treatment group))/(average tumor volume at the end of treatment in the solvent control group - average tumor volume at the start of treatment in the solvent control group)] × 100%.

When there was tumor regression, TGI (%) = [1 - (average tumor volume at the end of administration in a treatment group - average tumor volume at the start of administration in the treatment group)/average tumor volume at the start of administration in the treatment group] × 100%.

### 3.3. Experimental conclusion:

The data show that the examples of the present disclosure exhibited relatively significant tumor inhibitory effects after administration.

### Example 4: Pharmacokinetic Evaluation Assay in Mice

Assay objective: To study the PKPD behavior of the compounds following a single tail vein administration in mice (tumors).

### 4.1. Test animals:

Species: Mouse
Strain: BALB/c nude mouse
Week age and body weight: Aged 8-10 weeks, weighing 17-25 g
Sex: Female
Supplier: Shanghai Sippe-Bk Lab Animal Co., Ltd.

### 4.2. Drug preparation:

1 mg of each test compound was weighed out, and 0.098 mL of DMA was added to fully dissolve the drug. 0.045 mL of the resulting solution was added to 2.55 mL of a vehicle, and the mixture was vortexed to give a drug solution at a concentration of 1.5 mg/mL.

### 4.3. Administration regimen:

4.3.1. After tumor inoculation, the PDPK experiment was initiated when the tumor volume reached 300-500 mm³.

4.3.2. Tumor-bearing mice were fasted overnight with free access to water. They were then euthanized at designated time points after administration.

### 4.4. Sample collection:

4.4.1. After euthanasia, the mice were subjected to cardiac blood collection, and the collected blood was added to a centrifuge tube containing EDTA-2K. The centrifuge tube was manually inverted several times, then placed on ice, left to stand, and centrifuged at 8000 rpm for 5 min at 4 °C. After centrifugation, 100 µL of plasma was transferred to a new, labeled centrifuge tube, flash-frozen on dry ice, and then stored in a freezer at -80 °C for PK analysis.

4.4.2. After blood collection, the tumor tissue was excised. The excised tumor tissue was divided into 2 portions (~0.1 g each), which were then placed into pre-labeled 2 mL centrifuge tubes, respectively. The tubes were then transferred to the freezer at -80 °C for storage. One portion was used for PK analysis (weighing was required), and the other portion was used for PD analysis.

### 4.5. Liquid chromatography

Liquid chromatography condition: Shimadzu LC-20AD pump
Mass spectrometry condition: AB Sciex API 4000 mass spectrometer
Chromatography column: XBridge^{®} C18 (50 × 4.6 mm, 5 µm particle size)
Mobile phase: solution A: 0.1% formic acid in water; solution B: methanol
Flow rate: 1.0 mL/min
Elution time: 0-4.0 min; the eluent was as follows:

| Time/min | Solution A | Solution B |
|---|---|---|
| 0.2 | 90% | 10% |
| 1.2 | 2% | 98% |
| 3.0 | 2% | 98% |
| 3.1 | 90% | 10% |
| 4.0 | Stop | |

### 4.6. Assay results and analysis

The main pharmacokinetic parameters were calculated using WinNonlin 8.2.

### 4.7. Experimental conclusion:

The data show that in the pharmacokinetic evaluation experiment in mice, the compounds of the examples of the present disclosure, following tail vein administration, exhibited relatively high exposure and a more advantageous distribution in tumors.

Finally, it should be noted that the above examples are provided solely to illustrate the technical solutions of the present disclosure and are not intended to limit the present disclosure. Although the present disclosure has been described in detail with reference to preferred examples, those of ordinary skill in the art should understand that modifications to the specific embodiments of the present disclosure or equivalent replacements of some technical features of the present disclosure can still be made without departing from the spirit of the technical solutions of the present disclosure, and all such modifications and equivalent replacements shall fall within the scope of the technical solutions claimed by the present disclosure.

## Claims

1. A compound represented by formula II or a racemic mixture, an enantiomer, a diastereoisomer, a pharmaceutically acceptable salt, or an isotopically labeled compound thereof, or a mixture of the aforementioned forms: 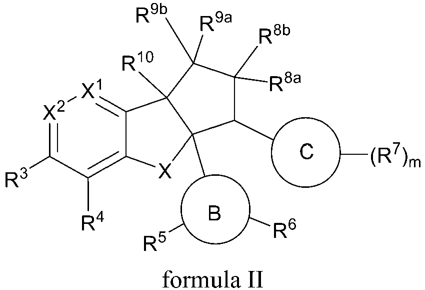 wherein
X¹ is selected from N and C(R¹), wherein R¹ is selected from hydrogen, halogen, cyano, -OH, - (C₁-C₆)alkyl, and -O-(C₁-C₆)alkyl;
X² is selected from N and C(R²), wherein R² is selected from hydrogen, halogen, cyano, -OH, - (C₁-C₆)alkyl, and -O-(C₁-C₆)alkyl;
R³ is selected from -(C₃-C₆)cycloalkyl, -(3- to 8-membered)heterocyclyl, -(C₆-C₁₀)aryl, and -(5-to 12-membered)heteroaryl, wherein the -(C₃-C₆)cycloalkyl, -(3- to 8-membered)heterocyclyl, - (C₆-C₁₀)aryl, and -(5- to 12-membered)heteroaryl are optionally substituted with one or more R³¹;
each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, -(C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(Cₗ-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, - (C₆-C₁₀)aryl, -(5- to 12-membered)heteroaryl, -S(=O)₂-NH₂, -S(=O)₂-NH(C₁-C₆)alkyl, -S(=O)₂-N[(C₁-C₆)alkyl]₂, -NH-C(=O)H, -NH-C(=O)-(C₁-C₆)alkyl, -N[(C₁-C₆)alkyl]-C(=O)-(C₁-C₆)alkyl, N[(C₁-C₆)alkyl]-C(=O)H, -NH-S(=O)₂-(C₁-C₆)alkyl, -N[(C₁-C₆)alkyl]-S(=O)₂-(C₁-C₆)alkyl, - P(=O)[(C₁-C₆)alkyl]₂, -P(=O)[(C₁-C₆)alkyl]-NH₂, -P(=O)[(C₁-C₆)alkyl]-NH(C₁-C₆)alkyl, and - P(=O)[(C₁-C₆)alkyl]-N[(C₁-C₆)alkyl]₂, wherein the -(C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], -(C₁-C₆)alkylene-N[(C₁-C₆)alkyl]₂, - (C₆-C₁₀)aryl, -(5- to 12-membered)heteroaryl, -(C₁-C₆)alkylene-O(C₁-C₆)alkyl, and oxo;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4- to 10-membered)heterocyclyl, -(C₆-C₁₀)aryl, -O-(C₆-C₁₀)aryl, -(5-to 12-membered)heteroaryl, and -O-(5- to 12-membered)heteroaryl;
X is selected from O, S, NH, N[(C₁-C₆)alkyl], N[C(=O)-R^{c}], C(R^{a})(R^{b}), C(=O), C[=C(R^{a})R^{b}], S(=O), and S(=O)₂;
R^{a} and R^{b} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4-to 10-membered)heterocyclyl, -(C₆-C₁₀)aryl, -O-(C₆-C₁₀)aryl, -(5- to 12-membered)heteroaryl, and -O-(5- to 12-membered)heteroaryl;
ring B is selected from -(C₆-C₁₀)aryl and -(5- to 12-membered)heteroaryl;
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, oxo, -OH, -O-(C₁-C₄)alkyl, -NH₂, -NH(C₁-C₄)alkyl, and N[(C₁-C₄)alkyl]₂; or
R⁵ and R⁶, together with the carbon atoms to which they are attached, form 5-membered heterocyclyl;
ring C is selected from -(C₆-C₁₀)aryl and -(5- to 12-membered)heteroaryl;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, and -NHC(=O)-(C₁-C₆)alkyl, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, oxo, -OH, -O-(C₁-C₄)alkyl, -NH₂, -NH(C₁-C₄)alkyl, and -N[(C₁-C₄)alkyl]₂; m is 1, 2, 3, 4, 5, 6, 7, 8, or 9;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -[(C₁-C₈)alkylene ]N(R)C(=O)R, -[(C₁-C₈)alkylene]N(R)C(=O)N(R)R, -C(=O)R, -C(=O)N(R)R, -C(=O)[(C₁-C₈)alkylene]N(R)R, - C(=O)OR, -C(=S)N(R)R, -SR, -S(=O)R, -S(=O)₂R, -S(=O)₂N(R)R, -N(R)C(=O)R, - N(R)C(=O)N(R)R, -P(=O)(OR)(OR), -(C₆-C₁₀)aryl, -(5- to 12-membered)heteroaryl, -(C₃-C₆)cycloalkyl, and -(4- to 10-membered)heterocyclyl;
each R is independently selected from hydrogen, -OH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -(C₁-C₄)alkylene-O-(C₁-C₆)alkyl, -O-(C₁-C₆)haloalkyl, -S-(C₁-C₆)alkyl, -S(=O)₂-(C₁-C₆)alkyl, -(C₃-C₆)cycloalkyl, -(4- to 10-membered)heterocyclyl, -O-(C₃-C₆)cycloalkyl, -O-(4- to 10-membered)heterocyclyl, -C(=O)-H, - C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(C₃-C₆)cycloalkyl, -[(C₁-C₆)alkylene]-(4- to 10-membered)heterocyclyl, -(C₆-C₁₀)aryl, -[(C₁-C₆)alkylene]-(C₆-C₁₀)aryl, and -(5- to 12-membered)heteroaryl, wherein the -(C₁-C₆)alkylene-, -(C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, - NH₂, -NH(C₁-C₄)alkyl, N[(C₁-C₄)alkyl]₂, and (C₁-C₄)alkyl;
or R^{8a} and R^{8b} are combined to form oxo, -(C₂-C₆)alkenyl, -(C₃-C₆)cycloalkyl, or -(4- to 10-membered)heterocyclyl;
or R^{9a} and R^{9b} are combined to form oxo, -(C₂-C₆)alkenyl, -(C₃-C₆)cycloalkyl, or -(4- to 10-membered)heterocyclyl;
or R^{8a} and R^{9a}, together with the carbon atoms to which they are attached, form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, - O-(C₁-C₄)alkyl, -NH₂, -NH(C₁-C₄)alkyl, -N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
or R^{8b} and R^{9b}, together with the carbon atoms to which they are attached, form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, - O-(C₁-C₄)alkyl, -NH₂, -NH(C₁-C₄)alkyl, -N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
R¹⁰ is selected from hydrogen, cyano, -OH, and -NH₂; or R¹⁰ and R^{9a}, together with the atoms to which they are attached, form (5- to 6-membered)heterocyclyl, wherein the (5- to 6-membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

2. The compound or the racemic mixture, the enantiomer, the diastereoisomer, the pharmaceutically acceptable salt, or the isotopically labeled compound thereof, or the mixture of the aforementioned forms according to claim 1, wherein the compound is selected from a compound represented by formula I: 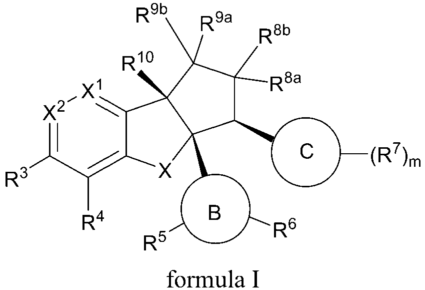 wherein
X¹ is selected from N and C(R¹), wherein R¹ is selected from hydrogen, halogen, cyano, -OH, - (C₁-C₆)alkyl, and -O-(C₁-C₆)alkyl;
X² is selected from N and C(R²), wherein R² is selected from hydrogen, halogen, cyano, -OH, - (C₁-C₆)alkyl, and -O-(C₁-C₆)alkyl;
R³ is selected from -(C₃-C₆)cycloalkyl, -(3- to 8-membered)heterocyclyl, -(C₆-C₁₀)aryl, and -(5-to 12-membered)heteroaryl, wherein the -(C₃-C₆)cycloalkyl, -(3- to 8-membered)heterocyclyl, - (C₆-C₁₀)aryl, and -(5- to 12-membered)heteroaryl are optionally substituted with one or more R³¹; each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, -(C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, - (C₆-C₁₀)aryl, -(5- to 12-membered)heteroaryl, -S(=O)₂-NH₂, -S(=O)₂-NH(C₁-C₆)alkyl, -S(=O)₂-N[(C₁-C₆)alkyl]₂, -NH-C(=O)H, -NH-C(=O)-(C₁-C₆)alkyl, -N[(C₁-C₆)alkyl]-C(=O)-(C₁-C₆)alkyl, N[(C₁-C₆)alkyl]-C(=O)H, -NH-S(=O)₂-(C₁-C₆)alkyl, -N[(C₁-C₆)alkyl]-S(=O)₂-(C₁-C₆)alkyl, - P(=O)[(C₁-C₆)alkyl]₂, -P(=O)[(C₁-C₆)alkyl]-NH₂, -P(=O)[(C₁-C₆)alkyl]-NH(C₁-C₆)alkyl, and - P(=O)[(C₁-C₆)alkyl]-N[(C₁-C₆)alkyl]₂, wherein the -(C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], -(C₁-C₆)alkylene-N[(C₁-C₆)alkyl]₂, - (C₆-C₁₀)aryl, -(5- to 12-membered)heteroaryl, -(C₁-C₆)alkylene-O(C₁-C₆)alkyl, and oxo;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4- to 10-membered)heterocyclyl, -(C₆-C₁₀)aryl, -O-(C₆-C₁₀)aryl, -(5-to 12-membered)heteroaryl, and -O-(5- to 12-membered)heteroaryl;
X is selected from O, S, NH, N[(C₁-C₆)alkyl], N[C(=O)-R^{c}], C(R^{a})(R^{b}), C(=O), C[=C(R^{a})R^{b}], S(=O), and S(=O)₂;
R^{a} and R^{b} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4- to 10-membered)heterocyclyl, -O-(4-to 10-membered)heterocyclyl, -(C₆-C₁₀)aryl, -O-(C₆-C₁₀)aryl, -(5- to 12-membered)heteroaryl, and -O-(5- to 12-membered)heteroaryl;
ring B is selected from -(C₆-C₁₀)aryl and -(5- to 12-membered)heteroaryl;
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, oxo, -OH, -O-(C₁-C₄)alkyl, -NH₂, -NH(C₁-C₄)alkyl, and N[(C₁-C₄)alkyl]₂; or
R⁵ and R⁶, together with the carbon atoms to which they are attached, form 5-membered heterocyclyl;
ring C is selected from -(C₆-C₁₀)aryl and -(5- to 12-membered)heteroaryl;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4- to 10-membered)heterocyclyl, -O-(C₃-C₆)cycloalkyl, -O-(4- to 10-membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, and -NHC(=O)-(C₁-C₆)alkyl, wherein the -(C₁-C₆)alkyl, -(C₂-C₆)alkenyl, -(C₂-C₆)alkynyl, -(C₃-C₆)cycloalkyl, and -(4- to 10-membered)heterocyclyl are optionally substituted with one or more groups selected from hydrogen, halogen, oxo, -OH, -O-(C₁-C₄)alkyl, -NH₂, -NH(C₁-C₄)alkyl, and -N[(C₁-C₄)alkyl]₂;
m is 1, 2, 3, 4, 5, 6, 7, 8, or 9;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -[(C₁-C₈)alkylene]NHC(=O)R, -[(C₁-C₈)alkylene]N(R)C(=O)N(R)R, -C(=O)R, -C(=O)N(R)R, -C(=O)[(C₁-C₈)alkylene]N(R)R, - C(=O)OR, -C(=S)N(R)R, -SR, -S(=O)R, -S(=O)₂R, -S(=O)₂N(R)R, -N(R)C(=O)R, - N(R)C(=O)N(R)R, -P(=O)(OR)(OR), -(C₆-C₁₀)aryl, -(5- to 12-membered)heteroaryl, -(C₃-C₆)cycloalkyl, and -(4- to 10-membered)heterocyclyl;
each R is independently selected from hydrogen, -OH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -(C₁-C₄)alkylene-O-(C₁-C₆)alkyl, -O-(C₁-C₆)haloalkyl, -S-(C₁-C₆)alkyl, -S(=O)₂-(C₁-C₆)alkyl, -(C₃-C₆)cycloalkyl, -(4- to 10-membered)heterocyclyl, -O-(C₃-C₆)cycloalkyl, -O-(4- to 10-membered)heterocyclyl, -C(=O)-H, - C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(C₃-C₆)cycloalkyl, -[(C₁-C₆)alkylene]-(4- to 10-membered)heterocyclyl, -(C₆-C₁₀)aryl, -[(C₁-C₆)alkylene]-(C₆-C₁₀)aryl, and -(5- to 12-membered)heteroaryl, wherein the -(C₁-C₆)alkylene-, -(C₁-C₆)alkyl, -(C₂-C₆)alkenyl, -(C₂-C₆)alkynyl, -(C₃-C₆)cycloalkyl, -(4- to 10-membered)heterocyclyl, -(C₆-C₁₀)aryl, and -(5- to 12-membered)heteroaryl are optionally substituted with one or more groups selected from hydrogen, halogen, -OH, -O-(C₁-C₄)alkyl, - NH₂, -NH(C₁-C₄)alkyl, N[(C₁-C₄)alkyl]₂, and (C₁-C₄)alkyl;
or R^{8a} and R^{8b} are combined to form oxo, -(C₂-C₆)alkenyl, -(C₃-C₆)cycloalkyl, or -(4- to 10-membered)heterocyclyl;
or R^{9a} and R^{9b} are combined to form oxo, -(C₂-C₆)alkenyl, -(C₃-C₆)cycloalkyl, or -(4- to 10-membered)heterocyclyl;
or R^{8a} and R^{9a}, together with the carbon atoms to which they are attached, form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, - O-(C₁-C₄)alkyl, -NH₂, -NH(C₁-C₄)alkyl, -N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
or R^{8b} and R^{9b}, together with the carbon atoms to which they are attached, form (C₃-C₆)cycloalkyl, (4- to 10-membered)heterocyclyl, or (5- to 10-membered)heteroaryl, wherein the aforementioned group is optionally substituted with one or more groups selected from hydrogen, halogen, -OH, - O-(C₁-C₄)alkyl, -NH₂, -NH(C₁-C₄)alkyl, -N[(C₁-C₄)alkyl]₂, -(C₃-C₆)cycloalkyl, -(4- to 8-membered)heterocyclyl, -(5- to 6-membered)heteroaryl, and -(C₆-C₁₀)aryl;
R¹⁰ is selected from hydrogen, cyano, -OH, and -NH₂; or R¹⁰ and R^{9a}, together with the atoms to which they are attached, form (5- to 6-membered)heterocyclyl, wherein the (5- to 6-membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

3. The compound or the racemic mixture, the enantiomer, the diastereoisomer, the pharmaceutically acceptable salt, or the isotopically labeled compound thereof, or the mixture of the aforementioned forms according to claim 1 or 2, wherein R¹ is selected from hydrogen, halogen, cyano, -OH, -(C₁-C₄)alkyl, and -O-(C₁-C₄)alkyl;
preferably, R¹ is selected from hydrogen, fluorine, chlorine, cyano, -OH, methyl, ethyl, propyl, - O-methyl, -O-ethyl, and -O-propyl;
preferably, R¹ is selected from hydrogen and -O-methyl;
preferably, R¹ is -O-methyl, and X¹ is selected from N and C(OCH₃).

4. The compound or the racemic mixture, the enantiomer, the diastereoisomer, the pharmaceutically acceptable salt, or the isotopically labeled compound thereof, or the mixture of the aforementioned forms according to any one of claims 1-3, wherein R² is selected from hydrogen, halogen, cyano, -OH, -(C₁-C₄)alkyl, and -O-(C₁-C₄)alkyl;
preferably, R² is selected from hydrogen, fluorine, chlorine, cyano, -OH, methyl, ethyl, propyl, - O-methyl, -O-ethyl, and -O-propyl;
preferably, R² is hydrogen, and X² is selected from N and CH.

5. The compound or the racemic mixture, the enantiomer, the diastereoisomer, the pharmaceutically acceptable salt, or the isotopically labeled compound thereof, or the mixture of the aforementioned forms according to any one of claims 1-4, wherein
R³¹ is selected from oxo, hydrogen, halogen, cyano, nitro, -OH, -SH, -NH₂, -(C₁-C₄)alkyl, -(C₁-C₄)alkylene-OH, -(C₁-C₄)alkylene-N[(C₁-C₄)alkyl]₂, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4- to 8-membered)heterocyclyl, -O-(C₃-C₆)cycloalkyl, -O-(4- to 8-membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, and -C(=O)-N[(C₁-C₄)alkyl]₂;
preferably, R³¹ is selected from hydrogen, fluorine, chlorine, cyano, -OH, -SH, -NH₂, -(C₁-C₄)alkyl, -O-(C₁-C₄)alkyl, -NH-(C₁-C₄)alkyl, -(C₁-C₄)alkylene-OH, -(C₁-C₄)haloalkyl, -(C₁-C₄)alkylene-NH₂, -(C₁-C₄)alkylene-NH(C₁-C₄)alkyl, and -(C₁-C₄)alkylene-N[(C₁-C₄)alkyl]₂;
preferably, R³¹ is selected from hydrogen, fluorine, chlorine, cyano, -OH, -SH, -NH₂, methyl, halomethyl, -O-methyl, -CH₂OH, -CH₂NH₂, and -CH₂CH₂N(CH₃)₂;
preferably, R³¹ is selected from hydrogen, fluorine, chlorine, cyano, -OH, -SH, and -NH₂;
R³ is selected from -(C₃-C₆)cycloalkyl, -(3- to 6-membered)heterocyclyl, -(C₆-C₁₀)aryl, and -(5-to 12-membered)heteroaryl, wherein the -(C₃-C₆)cycloalkyl, -(3- to 6-membered)heterocyclyl, - (C₆-C₁₀)aryl, and -(5- to 12-membered)heteroaryl are optionally substituted with one or more R³¹ and R³¹ is as defined in the present claim;
preferably, R³ is selected from -(C₆-C₁₀)aryl and -(5- to 12-membered)heteroaryl, wherein the - (C₆-C₁₀)aryl and -(5- to 12-membered)heteroaryl are optionally substituted with one or more R³¹ and R³¹ is as defined in the present claim;
preferably, R³ is selected from phenyl and -(5- to 10-membered)heteroaryl, wherein the phenyl and -(5- to 10-membered)heteroaryl are optionally substituted with one or more R³¹, and R³¹ is as defined in the present claim;
preferably, R³ is selected from -(5- to 6-membered)heteroaryl, wherein the -(5- to 6-membered)heteroaryl is optionally substituted with 1, 2, 3, or 4 R³¹, and R³¹ is as defined in the present claim;
preferably, R³ is selected from oxazolyl, thiazolyl, pyrazolyl, isoxazolyl, isothiazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl, wherein the oxazolyl, thiazolyl, pyrazolyl, isoxazolyl, isothiazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl are optionally substituted with 1, 2, or 3 R³¹, and R³¹ is as defined in the present claim;
preferably, R³ is selected from 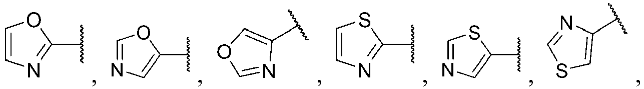 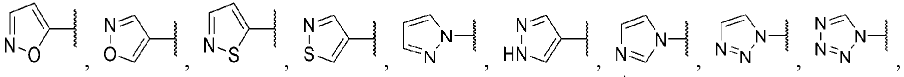 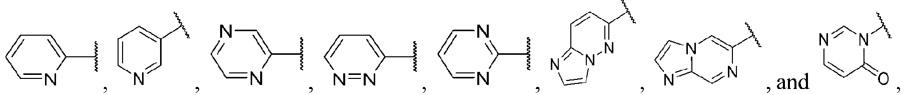
wherein the 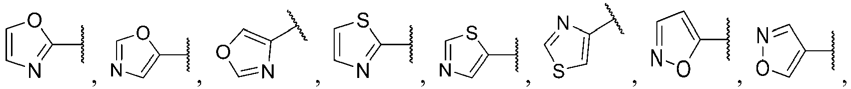 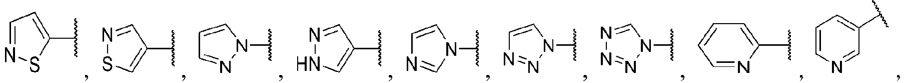 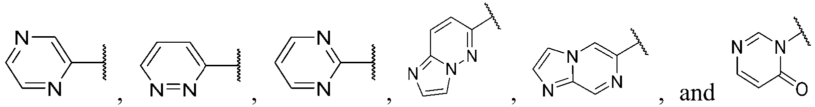 are optionally substituted with 1, 2, or 3 R³¹, and R³¹ is as defined in the present claim;
preferably, R³ is selected from 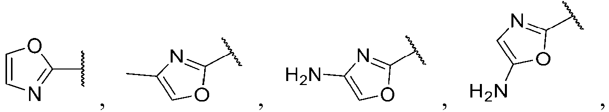 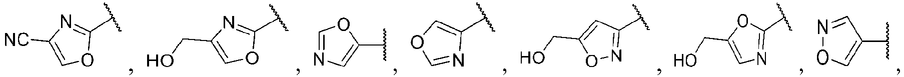 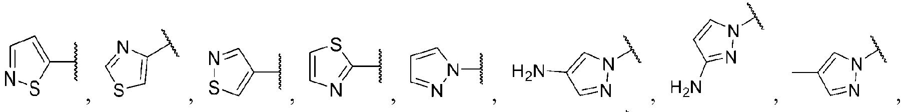 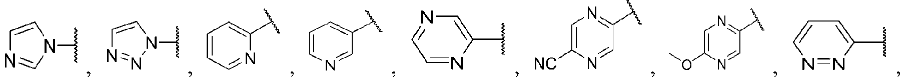 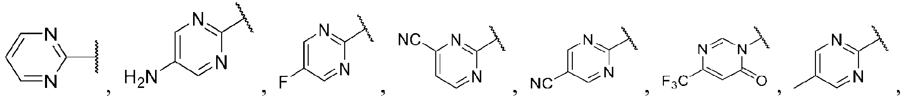 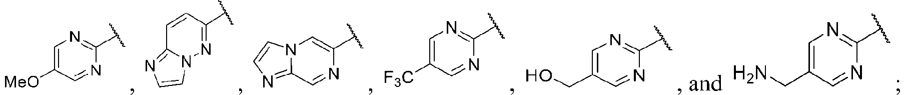
further preferably, R³ is selected from oxazolyl, pyrazolyl, pyridinyl, pyrimidinyl, pyrazinyl, pyridazinyl, pyridazinoimidazolyl, and pyrazinoimidazolyl, wherein the oxazolyl, pyrazolyl, pyridinyl, pyrimidinyl, pyrazinyl, pyridazinyl, pyridazinoimidazolyl, and pyrazinoimidazolyl are optionally substituted with 1, 2, or 3 R³¹, and R³¹ is as defined in the present claim;
further preferably, R³ is selected from 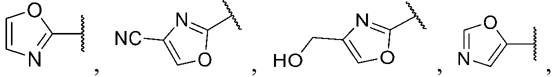 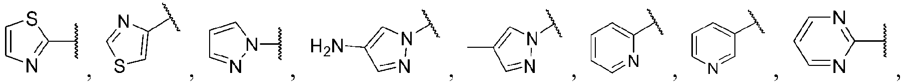 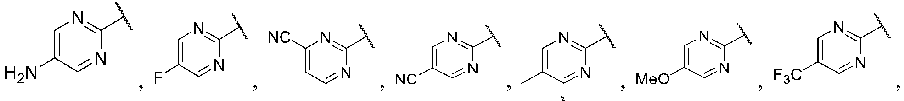 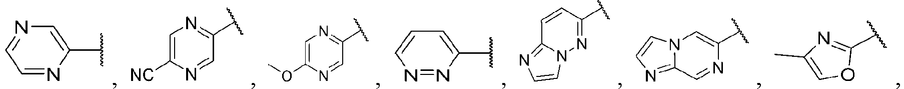 and 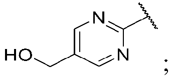
further preferably, R³ is selected from oxazolyl, pyrazolyl, pyrimidinyl, and pyrazinyl, wherein the oxazolyl, pyrazolyl, pyrimidinyl, and pyrazinyl are optionally substituted with 1, 2, or 3 R³¹ (as defined in the present claim), for example, substituted with fluorine, cyano, amino, methyl, trifluoromethyl, -methylene-OH, and -O-methyl;
further preferably, R³ is selected from 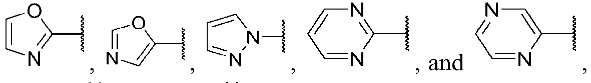 wherein the 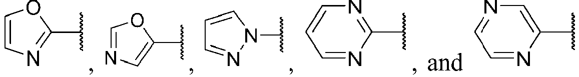 are optionally substituted with 1, 2, or 3 R³¹ (as defined in the present claim), for example, substituted with fluorine, cyano, amino, methyl, trifluoromethyl, -methylene-OH, and -O-methyl;
further preferably, R³ is selected from 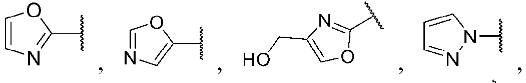 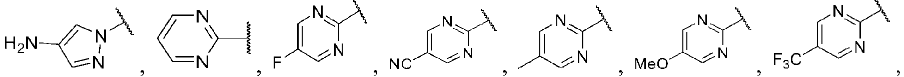 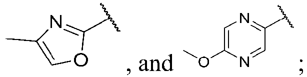
or R³ is selected from -(C₃-C₆)cycloalkyl and -(3- to 6-membered)heterocyclyl, wherein the -(C₃-C₆)cycloalkyl and -(3- to 6-membered)heterocyclyl are optionally substituted with one or more R³¹, and R³¹ is as defined in the present claim;
preferably, R³ is selected from cyclopropyl, oxetanyl, azetidinyl, tetrahydrofuranyl, pyrrolidinyl, tetrahydropyranyl, piperidinyl, piperazinyl, piperazinonyl, morpholinyl, and morpholinonyl, wherein the aforementioned groups are optionally substituted with one or more R³¹, and R³¹ is as defined in the present claim;
preferably, R³ is selected from

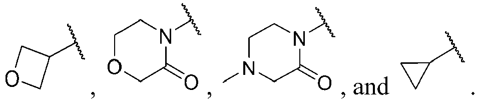

6. The compound or the racemic mixture, the enantiomer, the diastereoisomer, the pharmaceutically acceptable salt, or the isotopically labeled compound thereof, or the mixture of the aforementioned forms according to any one of claims 1-5, wherein R⁴ is selected from hydrogen, halogen, cyano, -OH, -NH₂, -(C₁-C₄)alkyl, -(C₁-C₄)haloalkyl, -O-(C₁-C₄)alkyl, -O-(C₁-C₄)haloalkyl, -(C₃-C₆)cycloalkyl, -O-(C₃-C₆)cycloalkyl, -(4- to 8-membered)heterocyclyl, -O-(4-to 8-membered)heterocyclyl, -(C₆-C₁₀)aryl, -O-(C₆-C₁₀)aryl, -(5- to 10-membered)heteroaryl, and -O-(5- to 10-membered)heteroaryl;
preferably, R⁴ is selected from hydrogen, fluorine, chlorine, cyano, -OH, -NH₂, methyl, halomethyl, -O-methyl, -NH-methyl, and cyclopropyl;
preferably, R⁴ is selected from hydrogen, fluorine, chlorine, cyano, -OH, and -NH₂;
preferably, R⁴ is hydrogen.

7. The compound or the racemic mixture, the enantiomer, the diastereoisomer, the pharmaceutically acceptable salt, or the isotopically labeled compound thereof, or the mixture of the aforementioned forms according to any one of claims 1-6, wherein
preferably, X is selected from O, S, NH, N(CH₃), N(CH₂CH₃), N[C(=O)-CH₃], CH₂, C(CH₃)₂, C(CH₃)(CH₂CH₃), C(CH₂CH₃)₂, C(=O), C(=CH₂), C(=CF₂), C(=CHCF₃), S(=O), and S(=O)₂;
preferably, X is selected from O, S, and NH;
further preferably, X is O.

8. The compound or the racemic mixture, the enantiomer, the diastereoisomer, the pharmaceutically acceptable salt, or the isotopically labeled compound thereof, or the mixture of the aforementioned forms according to any one of claims 1-7, wherein
ring B is selected from -(C₆-C₁₀)aryl and 5- to 10-membered heteroaryl;
preferably, ring B is selected from phenyl, naphthyl, oxazolyl, thiazolyl, pyrazolyl, imidazolyl, triazolyl, tetrazolyl, pyridinyl, pyrimidinyl, benzofuranyl, benzothienyl, indolyl, quinolinyl, and isoquinolinyl;
preferably, ring B is phenyl;
preferably, the structural unit 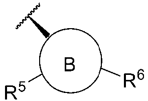 is 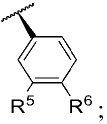
R⁵ and R⁶ are each independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4- to 8-membered)heterocyclyl, -O-(C₃-C₆)cycloalkyl, -O-(4- to 8-membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, - C(=O)-NH₂, -C(=O)-NH(C₁-C₄)alkyl, -C(=O)-N[(C₁-C₄)alkyl]₂, -C(=O)-H, and -C(=N-OH)-H, wherein the -(C₁-C₄)alkyl, -(C₂-C₄)alkenyl, -(C₂-C₄)alkynyl, -(C₃-C₆)cycloalkyl, and -(4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, and -N(ethyl)-propyl;
preferably, R⁵ and R⁶ are each independently selected from hydrogen, fluorine, chlorine, bromine, cyano, -OH, -NH₂, methyl, ethyl, -O-methyl, -NH-methyl, -N(methyl)₂, -N(methyl)-ethyl, cyclopropyl, azetidinyl, -O-cyclopropyl, -C(=O)-methyl, -C(=O)-cyclopropyl, -C(=O)-H, and - C(=N-OH)-H;
preferably, R⁵ and R⁶ are each independently selected from hydrogen, fluorine, chlorine, bromine, cyano, -OH, -NH₂, -(C₁-C₄)alkyl, -(C₁-C₄)alkyl-OH, -(C₁-C₄)alkyl-NH₂, -(C₁-C₄)haloalkyl, -O-(C₁-C₄)alkyl, -O-(C₁-C₄)haloalkyl, and -(C₃-C₆)cycloalkyl;
preferably, R⁵ is selected from hydrogen, fluorine, chlorine, bromine, cyano, -OH, -NH₂, and - CH₂NH₂;
preferably, R⁵ is selected from hydrogen and -NH₂;
preferably, R⁶ is selected from hydrogen, fluorine, chlorine, bromine, cyano, -OH, -NH₂, -O-methyl, and cyclopropyl;
or R⁵ and R⁶, together with the atoms to which they are attached, form 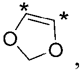 wherein * indicates the position of the attached atom.

9. The compound or the racemic mixture, the enantiomer, the diastereoisomer, the pharmaceutically acceptable salt, or the isotopically labeled compound thereof, or the mixture of the aforementioned forms according to any one of claims 1-8, wherein
ring C is selected from -(C₆-C₁₀)aryl and -(5- to 10-membered)heteroaryl;
preferably, ring C is selected from phenyl, naphthyl, oxazolyl, thiazolyl, pyrazolyl, imidazolyl, triazolyl, tetrazolyl, pyridinyl, pyrimidinyl, benzofuranyl, benzothienyl, indolyl, quinolinyl, and isoquinolinyl;
preferably, ring C is phenyl;
preferably, the structural unit 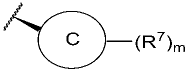 is 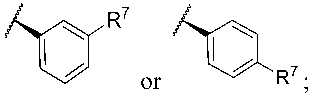
each R⁷ is independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4- to 8-membered)heterocyclyl, -O-(C₃-C₆)cycloalkyl, -O-(4- to 8-membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, - C(=O)-NH₂, -C(=O)-NH(C₁-C₄)alkyl, -C(=O)-N[(C₁-C₄)alkyl]₂, and -NHC(=O)-(C₁-C₄)alkyl, wherein the -(C₁-C₄)alkyl, -(C₂-C₄)alkenyl, -(C₂-C₄)alkynyl, -(C₃-C₆)cycloalkyl, and -(4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, and -N(ethyl)-propyl; preferably, each R⁷ is independently selected from hydrogen, fluorine, chlorine, bromine, cyano, - OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, -(C₁-C₄)alkylene-NH₂, and -NHC(=O)-(C₁-C₄)alkylene-NH₂;
preferably, each R⁷ is independently selected from hydrogen, fluorine, chlorine, bromine, -OH, - NH₂, -methylene-OH, -ethylene-OH, -propylene-OH, -methylene-NH₂, -ethylene-NH₂, - propylene-NH₂, -NHC(=O)-methylene-NH₂, and -NHC(=O)-ethylene-NH₂;
preferably, each R⁷ is independently selected from hydrogen, -OH, and -NH₂;
further preferably, each R⁷ is independently selected from hydrogen and -NH₂;
preferably, m is 1, 2, or 3.

10. The compound or the racemic mixture, the enantiomer, the diastereoisomer, the pharmaceutically acceptable salt, or the isotopically labeled compound thereof, or the mixture of the aforementioned forms according to any one of claims 1-9, wherein
each R is independently selected from hydrogen, -OH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -(C₁-C₄)alkylene-O-(C₁-C₄)alkyl, -O-(C₁-C₄)haloalkyl, -S-(C₁-C₄)alkyl, -S(=O)₂-(C₁-C₄)alkyl, -(C₃-C₆)cycloalkyl, -(4- to 8-membered)heterocyclyl, -O-(C₃-C₆)cycloalkyl, -O-(4- to 8-membered)heterocyclyl, -C(=O)-H, - C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, -C(=O)-N[(C₁-C₄)alkyl]₂, -[(C₁-C₄)alkylene]-(C₃-C₆)cycloalkyl, -[(C₁-C₄)alkylene]-(4- to 8-membered)heterocyclyl, -(C₆-C₁₀)aryl, -[(C₁-C₄)alkylene]-(C₆-C₁₀)aryl, and -(5- to 10-membered)heteroaryl, wherein the -(C₁-C₄)alkylene-, -(C₁-C₄)alkyl, -(C₂-C₄)alkenyl, -(C₂-C₄)alkynyl, -(C₃-C₆)cycloalkyl, -(4- to 8-membered)heterocyclyl, -(C₆-C₁₀)aryl, and -(5- to 10-membered)heteroaryl are optionally substituted with 1, 2, 3, 4, 5, 6, or 7 groups selected from hydrogen, halogen, -OH, -O-(C₁-C₄)alkyl, -NH₂, -NH(C₁-C₄)alkyl, N[(C₁-C₄)alkyl]₂, and (C₁-C₄)alkyl;
preferably, each R is independently selected from hydrogen, -OH, -NH₂, -(C₁-C₄)alkyl, -(C₁-C₄)haloalkyl, -O-(C₁-C₄)alkyl, -(C₁-C₄)alkylene-O-(C₁-C₆)alkyl, -O-(C₁-C₄)haloalkyl, -S(=O)₂-(C₁-C₄)alkyl, -(C₃-C₆)cycloalkyl, -(4- to 8-membered)heterocyclyl, -C(=O)-H, -C(=O)-(C₁-C₄)alkyl, -C(=O)-(C₃-C₆)cycloalkyl, -[(C₁-C₄)alkylene]-(C₃-C₆)cycloalkyl, and -[(C₁-C₄)alkylene]-(4- to 8-membered)heterocyclyl, wherein the -(C₁-C₄)alkylene-, -(C₁-C₄)alkyl, -(C₃-C₆)cycloalkyl, and -(4- to 8-membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, -N(ethyl)₂, -N(ethyl)-propyl, methyl, and ethyl;
preferably, each R is independently selected from hydrogen, -OH, -NH₂, methyl, ethyl, propyl, halomethyl, haloethyl, halopropyl, -methylene-O-methyl, -methylene-O-ethyl, -ethylene-O-methyl, -ethylene-O-ethyl, -O-methyl, -O-ethyl, -O-propyl, -O-halomethyl, -O-haloethyl, -O-halopropyl, -C(=O)-H, -C(=O)-methyl, -C(=O)-ethyl, -C(=O)-propyl, -S(=O)₂-methyl, -S(=O)₂-ethyl, -S(=O)₂-propyl, cyclopropyl, cyclobutyl, cyclopentyl, cyclohexyl, oxetanyl, tetrahydrofuranyl, tetrahydropyranyl, azetidinyl, pyrrolidinyl, piperidinyl, piperazinyl, morpholinyl, -methylene-cyclobutyl, -methylene-oxetanyl, and -methylene-azetidinyl, wherein the methyl, ethyl, propyl, cyclopropyl, cyclobutyl, cyclopentyl, cyclohexyl, oxetanyl, tetrahydrofuranyl, tetrahydropyranyl, azetidinyl, pyrrolidinyl, piperidinyl, piperazinyl, and morpholinyl are optionally substituted with 1, 2, or 3 groups selected from -OH, -OCH₃, -NH₂, NHCH₃, N(CH₃)₂, and -CH₃;
preferably, each R is independently selected from hydrogen, -OH, -NH₂, -CH₃, -CH₂CH₃, -CH₂OH, -CH₂CH₂OH, -CH₂OCH₃, -CH₂NH₂, -CH₂CH₂NH₂, -CH₂CH₂NHCH₃, -CH₂N(CH₃)₂, - CH(N(CH₃)₂)CH₂OH, -CH₂OCH₂CH₂OH, -OCH₃, -C(=O)-H, -C(=O)CH₃, -C(=O)CH₂OH, - S(=O)₂CH₃, cyclobutyl-OH, oxetanyl, azetidinyl, azetidinyl-OH, pyrrolidinyl, piperazinyl, azetidinyl-CH₃, piperazinyl-CH₃, and -CH₂-azetidinyl;
further preferably, each R is independently selected from hydrogen, -OH, -NH₂, -(C₁-C₄)alkyl, - O-(C₁-C₄)alkyl, -C(=O)-(C₁-C₄)alkyl, -S(=O)₂(C₁-C₄)alkyl, -(C₃-C₆)cycloalkyl, -(4- to 8-membered)heterocyclyl, -methylene-(C₃-C₆)cycloalkyl, and -methylene-(4- to 8-membered)heterocyclyl, wherein the -(C₁-C₄)alkyl, -(C₃-C₆)cycloalkyl, and -(4- to 8-membered)heterocyclyl are each optionally substituted with 1, 2, 3, 4, or 5 groups selected from hydrogen, halogen, -OH, -NH₂, -NH(C₁-C₄)alkyl, -N[(C₁-C₄)alkyl]₂, -O-(C₁-C₄)alkyl, and -(C₁-C₄)alkyl;
further preferably, each R is independently selected from hydrogen, -OH, methyl, ethyl, propyl, - O-methyl, -O-ethyl, -O-propyl, -methylene-O-ethyl, -C(=O)-methyl, -S(=O)₂-methyl, cyclobutyl, oxetanyl, azetidinyl, pyrrolidinyl, piperazinyl, -methylene-cyclobutyl, -methylene-oxetanyl, and - methylene-azetidinyl, wherein the aforementioned groups are each independently optionally substituted with 1, 2, or 3 groups selected from -OH, -NH₂, OCH₃, NHCH₃, N(CH₃)₂, and CH₃; R^{8a}, R^{8b}, R^{9a}, and R^{9b} are each independently selected from hydrogen, halogen, cyano, -(C₁-C₄)alkyl, -(C₁-C₄)haloalkyl, -(C₂-C₄)alkenyl, -(C₂-C₄)alkynyl, -OR, -N(R)R, -[(C₁-C₆)alkylene]R, -[(C₁-C₆)alkylene]OR, -[(C₁-C₆)alkylene]N(R)R, -[(C₁-C₆)alkylene]N(R)C(=O)R, -[(C₁-C₆)alkylene]N(R)C(=O)N(R)R, -C(=O)R, -C(=O)N(R)R, -C(=O)[(C₁-C₆)alkylene]N(R)R, - C(=O)OR, -C(=S)N(R)R, -SR, -S(=O)R, -S(=O)₂R, -S(=O)₂N(R)R, -N(R)C(=O)R, - N(R)C(=O)N(R)R, -P(=O)(OR)(OR), -(C₆-C₁₀)aryl, -(5- to 10-membered)heteroaryl, -(C₃-C₆)cycloalkyl, and -(4- to 8-membered)heterocyclyl, wherein R is as defined in the present claim;
preferably, R^{8a}, R^{8b}, R^{9a}, and R^{9b} are each independently selected from hydrogen, cyano, -OR, - N(R)R, -[(C₁-C₄)alkylene]R, -[(C₁-C₄)alkylene]OR, -[(C₁-C₄)alkylene]N(R)R, -[(C₁-C₄)alkylene]N(R)C(=O)R, -[(C₁-C₄)alkylene]N(R)C(=O)N(R)R, -C(=O)R, -C(=O)N(R)R, - C(=O)[(C₁-C₄)alkylene]N(R)R, -C(=O)OR, -C(=S)N(R)R, -S(=O)R, -S(=O)₂R, -S(=O)₂N(R)R, - N(R)C(=O)R, and -N(R)C(=O)N(R)R, wherein R is as defined in the present claim;
preferably, R^{8a} and R^{8b} are each independently selected from hydrogen, -methylene-R, -ethylene-R, -propylene-R, -methylene-OR, -ethylene-OR, -propylene-OR, -methylene-N(R)R, -ethylene-N(R)R, -propylene-N(R)R, -C(=O)R, -C(=O)N(R)R, and -C(=O)OR, wherein R is as defined in the present claim;
preferably, R^{8a} and R^{8b} are each independently selected from hydrogen, -methylene-R, -methylene-OR, -methylene-N(R)R, -C(=O)R, -C(=O)OR, and -C(=O)N(R)R, wherein R is as defined in the present claim; for example, each R is independently selected from hydrogen, -OH, -NH₂, -(C₁-C₄)alkyl, -O-(C₁-C₄)alkyl, -C(=O)-(C₁-C₄)alkyl, -S(=O)₂(C₁-C₄)alkyl, and -(4- to 8-membered)heterocyclyl, wherein the -(C₁-C₄)alkyl and -(4- to 8-membered)heterocyclyl are each optionally substituted with 1, 2, 3, 4, or 5 groups selected from hydrogen, halogen, -OH, -NH₂,-NHCH₃, and CH₃;
preferably, R^{8a} is hydrogen, preferably 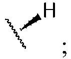
preferably, R^{8b} is selected from hydrogen, 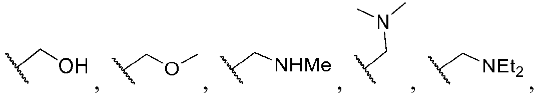 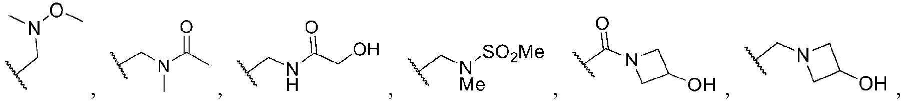 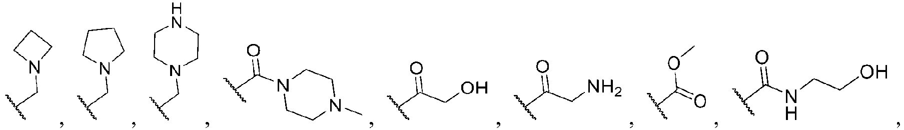 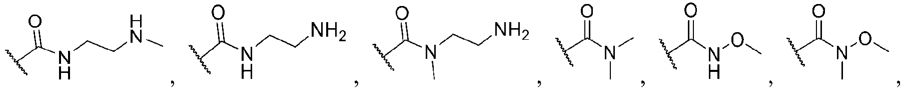 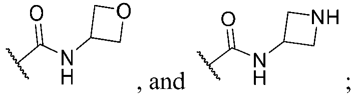
preferably, R^{8b} is selected from hydrogen, 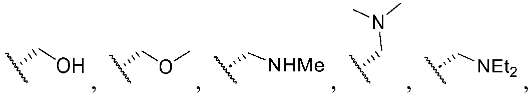 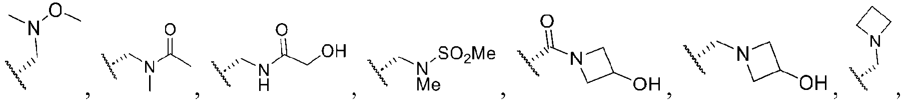 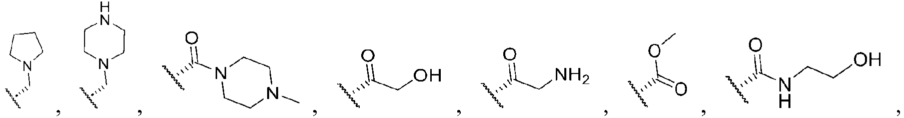 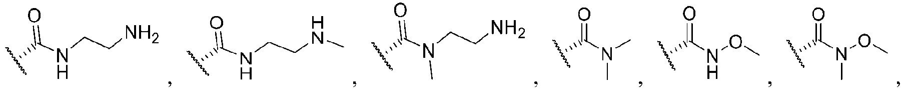 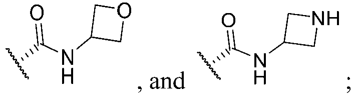
preferably, R^{8b} is selected from hydrogen, -methylene-R, -methylene-N(R)R, -C(=O)OR, - C(=O)N(R)R, and -C(=O)R, wherein R is as defined in the present claim; for example, each R is independently selected from hydrogen, -OH, methyl, ethyl, -O-methyl, -C(=O)-methyl, -S(=O)₂-methyl, azetidinyl, pyrrolidinyl, and piperazinyl, wherein the aforementioned groups are each independently optionally substituted with 1, 2, or 3 groups selected from -OH and -NH₂;
further preferably, R^{8b} is selected from hydrogen, 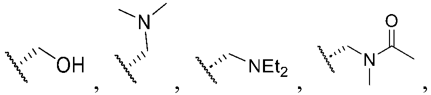 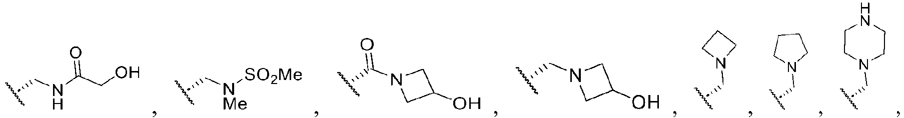 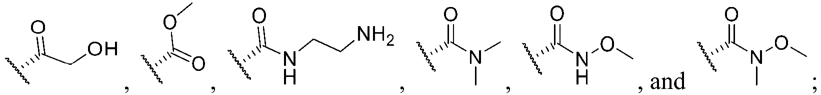
further preferably, R^{8b} is selected from hydrogen, 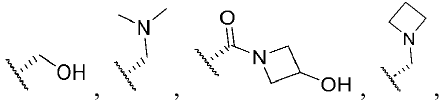 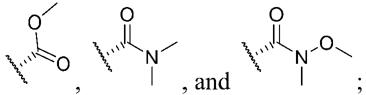
preferably, R^{9a} and R^{9b} are each independently selected from hydrogen, -CN, -OR, -NHR, -N(R)R, -NHC(=O)R, -methylene-R, -ethylene-R, -propylene-R, -methylene-N(R)R, -ethylene-N(R)R, - propylene-N(R)R, -methylene-NHC(=O)R, and -methylene-NHC(=O)NHR, wherein R is as defined in the present claim;
preferably, R^{9a} and R^{9b} are each independently selected from hydrogen, -CN, -OR, -N(R)R, - NHC(=O)R, -methylene-N(R)R, -methylene-NHC(=O)R, and -methylene-NHC(=O)NHR, wherein R is as defined in the present claim; for example, each R is independently selected from hydrogen, -OH, -(C₁-C₄)alkyl, -(C₁-C₄)alkylene-O-(C₁-C₄)alkyl, -S(=O)₂(C₁-C₄)alkyl, -(C₃-C₆)cycloalkyl, -(4- to 8-membered)heterocyclyl, -methylene-(C₃-C₆)cycloalkyl, and -methylene-(4- to 8-membered)heterocyclyl, wherein the -(C₁-C₄)alkyl, -(C₃-C₆)cycloalkyl, and -(4- to 8-membered)heterocyclyl are each optionally substituted with 1, 2, 3, 4, or 5 groups selected from hydrogen, halogen, -OH, -NH₂, -NH(C₁-C₄)alkyl, -N[(C₁-C₄)alkyl]₂, -O-(C₁-C₄)alkyl, and -(C₁-C₄)alkyl;
preferably, R^{9a} is selected from hydrogen, -CN, -N(R)R, -NHC(=O)R, -methylene-N(R)R, - methylene-NHC(=O)R, and -methylene-NHC(=O)NHR, wherein R is as defined in the present claim; for example, each R is independently selected from hydrogen, -OH, methyl, ethyl, - methylene-O-ethyl, -S(=O)₂-methyl, cyclobutyl, azetidinyl, -methylene-cyclobutyl, and - methylene-azetidinyl, wherein the aforementioned groups are each independently optionally substituted with 1, 2, or 3 groups selected from -OH, OCH₃, NHCH₃, N(CH₃)₂, and CH₃;
preferably, R^{9a} is selected from hydrogen, -CN, -NHCH₃, -N(CH₃)₂, -NHC(=O)H, - NHC(=O)CH₂OH, -NHC(=O)CH₂OCH₃, -NHC(=O)CH₂NHCH₃, -NHC(=O)CH₂N(CH₃)₂, - CH₂NH₂, -CH₂NHCH₃, -CH₂N(CH₃)₂, -CH₂N(CH₂CH₃)₂, -CH₂N(CH₃)(CH₂CH₃), - CH₂NHC(=O)CH₂OH, -CH₂NHC(=O)CH₂OCH₃, -CH₂NHC(=O)CH₂NHCH₃, - CH₂NHC(=O)CH₂N(CH₃)₂, -CH₂NHC(=O)H, -CH₂NHC(=O)CH₃, -CH₂NHC(=O)NHOH, - CH₂NHC(=O)NHCH₂CH₂OH, -CH₂NHC(=O)CH(N(CH₃)₂)CH₂OH, -CH₂NHC(=O)CH₂NHCH₃, -CH₂NHC(=O)CH₂N(CH₃)₂, -CH₂NHC(=O)CH₂N(CH₂CH₃)₂, -CH₂NHC(=O)CH₂OCH₂CH₂OH, -CH₂NHS(=O)₂CH₃, -CH₂NHC(=O)cyclobutyl-OH, -CH₂NHC(=O)-azetidinyl-CH₃, and - CH₂NHC(=O)CH₂-azetidinyl;
preferably, R^{9a} is selected from hydrogen, 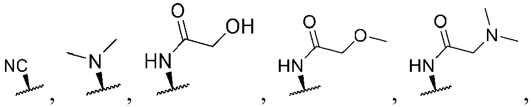 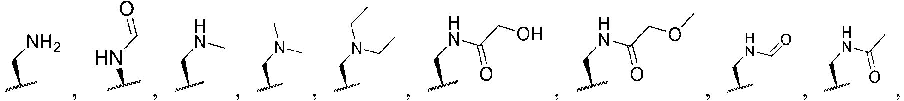 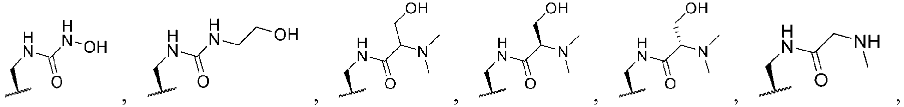 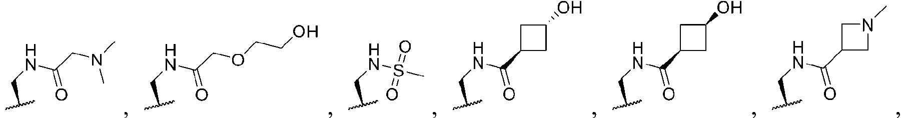 and 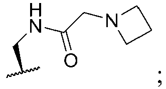
preferably, R^{9a} is selected from hydrogen, 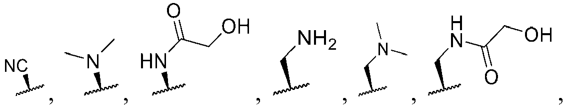 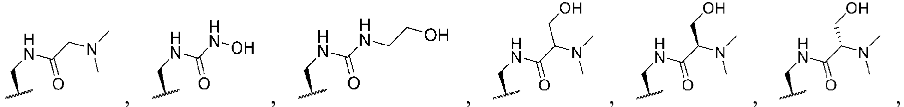 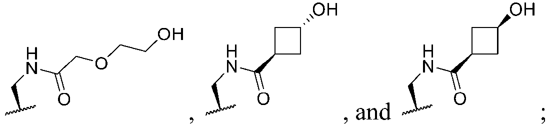
preferably, R^{9a} is 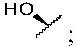
preferably, R^{9b} is selected from hydrogen and 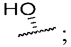
or R^{8a} and R^{8b} are combined to form oxo, -(C₂-C₄)alkenyl, -(C₃-C₆)cycloalkyl, or -(4- to 8-membered)heterocyclyl;
or R^{9a} and R^{9b} are combined to form oxo, -(C₂-C₄)alkenyl, -(C₃-C₆)cycloalkyl, or -(4- to 8-membered)heterocyclyl;
preferably, R^{8a} and R^{8b} are combined to form oxo, vinyl, propenyl, cyclopropyl, cyclobutyl, cyclopentyl, oxetanyl, tetrahydrofuranyl, tetrahydropyranyl, azetidinyl, pyrrolidinyl, piperidinyl, or piperazinyl;
preferably, R^{9a} and R^{9b} are combined to form oxo, vinyl, propenyl, cyclopropyl, cyclobutyl, cyclopentyl, oxetanyl, tetrahydrofuranyl, tetrahydropyranyl, azetidinyl, pyrrolidinyl, piperidinyl, or piperazinyl;
or R^{8a} and R^{9a}, together with the carbon atoms to which they are attached, form (C₃-C₆)cycloalkyl, (4- to 8-membered)heterocyclyl, or (5- to 9-membered)heteroaryl, wherein the aforementioned group is optionally substituted with 1, 2, 3, 4, 5, 6, or 7 groups selected from hydrogen, halogen, - OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl;
or R^{8b} and R^{9b}, together with the carbon atoms to which they are attached, form (C₃-C₆)cycloalkyl, (4- to 8-membered)heterocyclyl, or (5- to 9-membered)heteroaryl, wherein the aforementioned group is optionally substituted with 1, 2, 3, 4, 5, 6, or 7 groups selected from hydrogen, halogen, - OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl;
preferably, R^{8a} and R^{9a}, together with the carbon atoms to which they are attached, form (4- to 8-membered)heterocyclyl or (5- to 9-membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl;
preferably, R^{8b} and R^{9b}, together with the carbon atoms to which they are attached, form (4- to 8-membered)heterocyclyl or (5- to 9-membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl;
preferably, R^{8a} and R^{9a}, together with the carbon atoms to which they are attached, form oxetanyl, tetrahydrofuranyl, tetrahydropyranyl, azetidinyl, pyrrolidinyl, piperidinyl, piperazinyl, pyridinonyl, pyrimidinonyl, oxazolyl, thiazolyl, pyrazolyl, imidazolyl, triazolyl, tetrazolyl, pyridinyl, pyrimidinyl, benzothienyl, or indolyl, wherein the aforementioned group is optionally substituted with 1, 2, or 3 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl;
preferably, R^{8b} and R^{9b}, together with the carbon atoms to which they are attached, form oxetanyl, tetrahydrofuranyl, tetrahydropyranyl, azetidinyl, pyrrolidinyl, piperidinyl, piperazinyl, pyridinonyl, pyrimidinonyl, oxazolyl, thiazolyl, pyrazolyl, imidazolyl, triazolyl, tetrazolyl, pyridinyl, pyrimidinyl, benzothienyl, or indolyl, wherein the aforementioned group is optionally substituted with 1, 2, or 3 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl;
preferably, R^{8a} and R^{9a} are absent, and R^{8b} and R^{9b}, together with the atoms to which they are attached, form 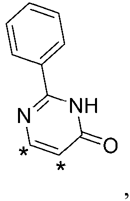 wherein * indicates the position of the attached atom.

11. The compound or the racemic mixture, the enantiomer, the diastereoisomer, the pharmaceutically acceptable salt, or the isotopically labeled compound thereof, or the mixture of the aforementioned forms according to any one of claims 1-10, wherein R¹⁰ is selected from -OH; or R¹⁰ and R^{9a}, together with the atoms to which they are attached, form 5-membered nitrogen-containing heterocyclyl, wherein the 5-membered nitrogen-containing heterocyclyl is optionally substituted with 1, 2, 3, 4, or 5 groups selected from hydrogen, halogen, -OH, methyl, ethyl, -O-methyl, -O-ethyl, -NH₂, -NH-methyl, -NH-ethyl, -N(methyl)₂, -N(methyl)-ethyl, and -N(ethyl)₂; preferably, R¹⁰ is -OH; or R¹⁰ and R^{9a}, together with the atoms to which they are attached, form imidazolinyl, wherein the imidazolinyl is optionally substituted with 1, 2, or 3 groups selected from halogen, -OH, methyl, -NH₂, -NH-methyl, and -N(methyl)₂;
preferably, R¹⁰ is -OH; or R¹⁰ and R^{9a}, together with the atoms to which they are attached, form 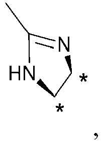 wherein * indicates the position of the attached atom;
preferably, R¹⁰ is -OH, preferably

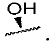

12. The compound or the racemic mixture, the enantiomer, the diastereoisomer, the pharmaceutically acceptable salt, or the isotopically labeled compound thereof, or the mixture of the aforementioned forms according to any one of claims 1-11, wherein the compound is selected from a compound represented by formula I-1: 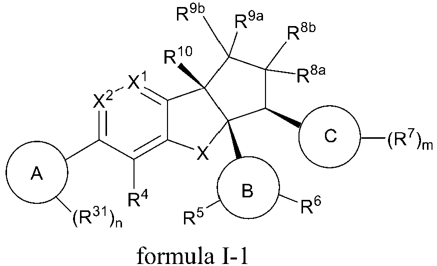 wherein
X, X¹, X², ring B, ring C, R³¹, R⁴, R⁵, R⁶, R⁷, R^{8a}, R^{8b}, R^{9a}, R^{9b}, R¹⁰, and m are as defined in any one of claims 1-11;
n is 0, 1, 2, 3, 4, or 5;
the structural fragment 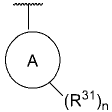 is as defined for R³, and R³ is as defined in any one of claims 1-11;
preferably, ring A is selected from 5- to 10-membered heteroaryl, preferably 5- to 9-membered heteroaryl, preferably 5- to 6-membered heteroaryl, preferably oxazolyl, thiazolyl, pyrazolyl, imidazolyl, triazolyl, tetrazolyl, pyridinyl, and pyrimidinyl, preferably 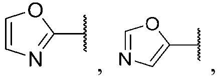 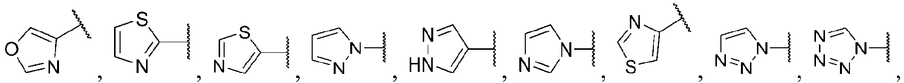 and 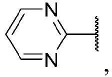 preferably oxazolyl and pyrimidinyl, for example, 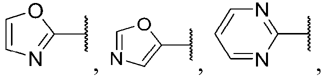 and 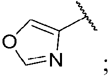
preferably, the compound is selected from a compound represented by formula I-1-A: 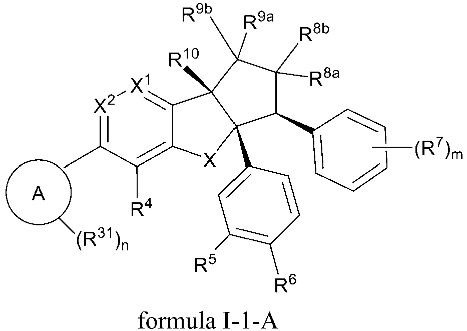 wherein
X, X¹, X², R³, R⁴, R⁵, R⁶, R⁷, R^{8a}, R^{8b}, R^{9a}, R^{9b}, R¹⁰, and m are as defined in any one of claims 1-11, and ring A and n are as defined in the present claim;
the structural unit 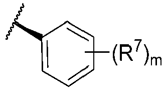 is preferably 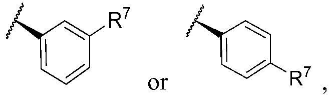 preferably 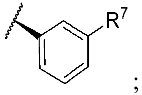
preferably, the compound is selected from a compound represented by formula I-1-B: 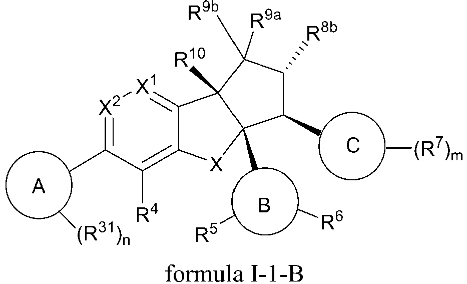 wherein
X, X¹, X², ring B, ring C, R³¹, R⁴, R⁵, R⁶, R⁷, R^{8b}, R^{9a}, R^{9b}, R¹⁰, and m are as defined in any one of claims 1-11, and ring A and n are as defined in the present claim;
preferably, the compound is selected from a compound represented by formula I-1-AB-1D: 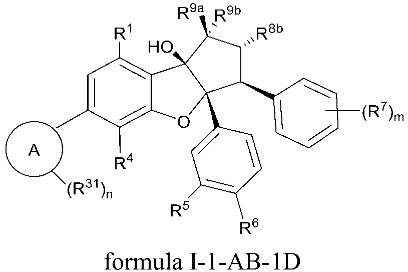 wherein
R³¹, R¹, R⁴, R⁵, R⁶, R⁷, R^{8b}, R^{9a}, R^{9b}, and m are as defined in any one of claims 1-11, and ring A and n are as defined in the present claim;
R^{8b1} is selected from R and N(R)R, wherein R is as defined in claim 1, 2, or 10.

13. The compound or the racemic mixture, the enantiomer, the diastereoisomer, the pharmaceutically acceptable salt, or the isotopically labeled compound thereof, or the mixture of the aforementioned forms according to any one of claims 1-12, wherein the compound is selected from

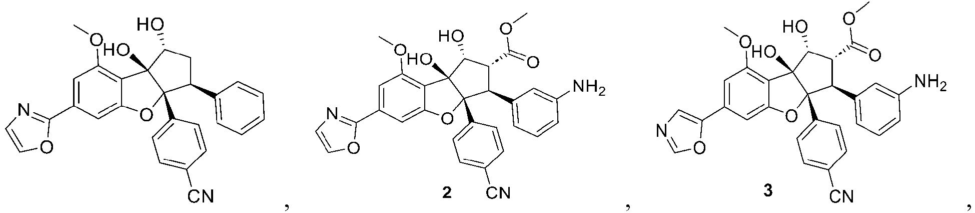

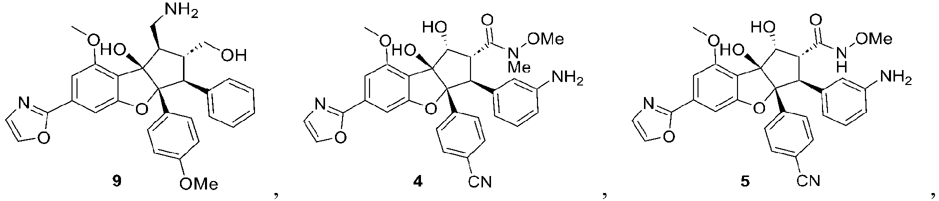

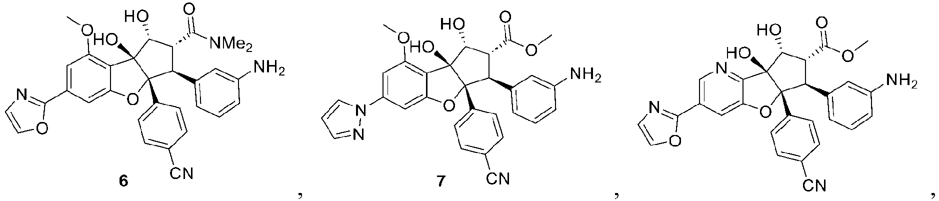

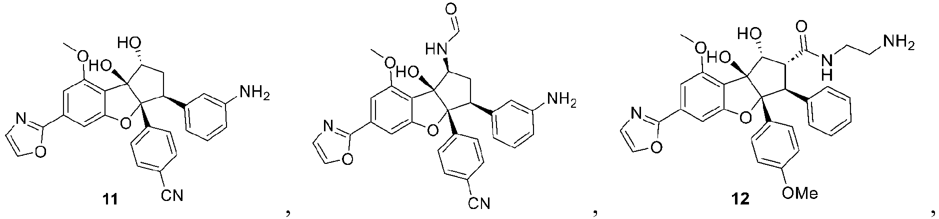

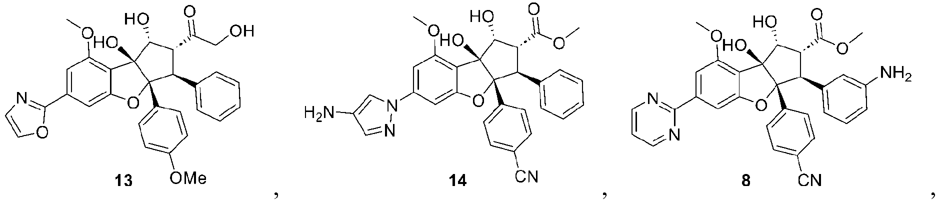

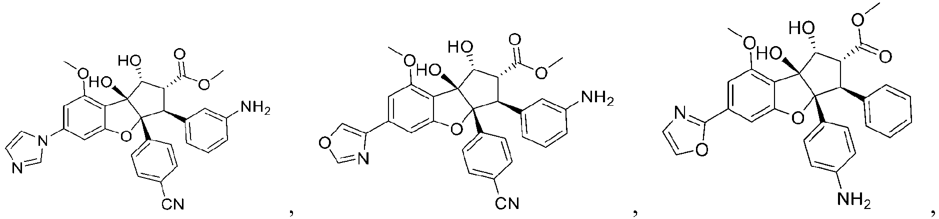

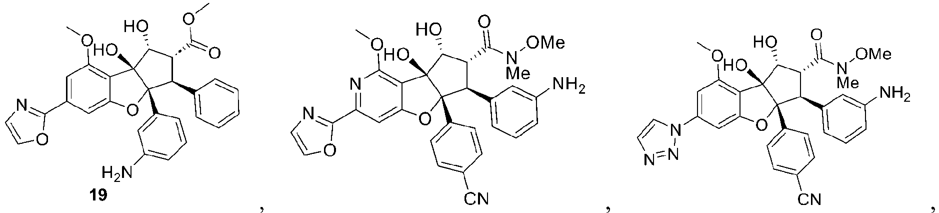

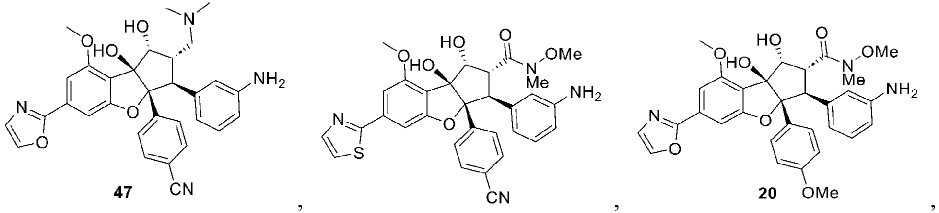

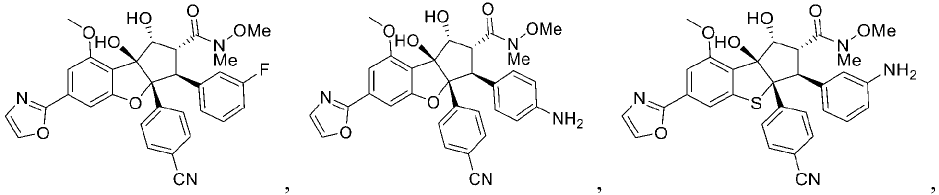

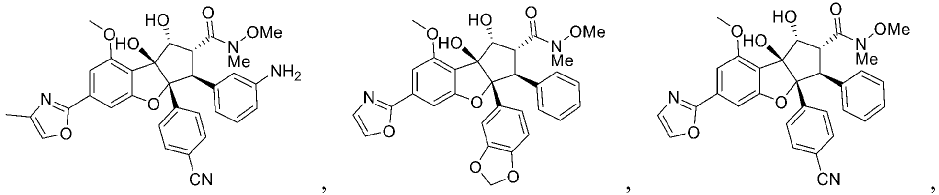

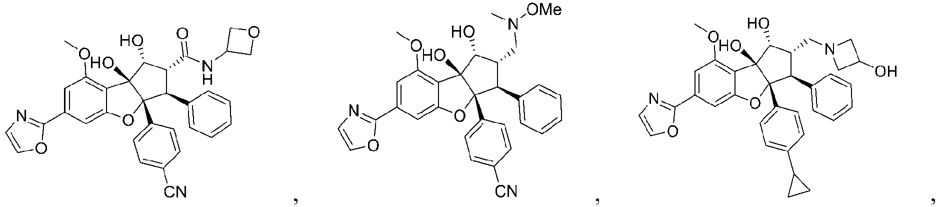

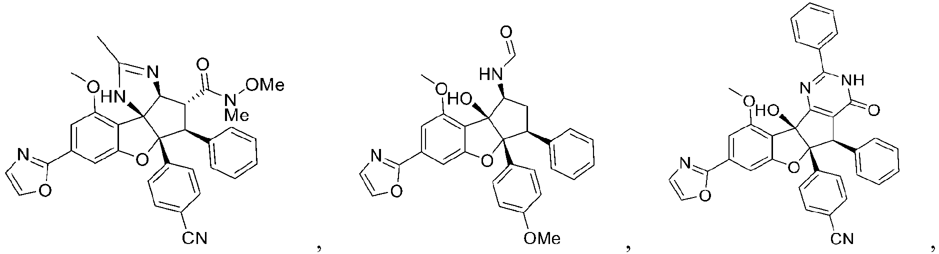

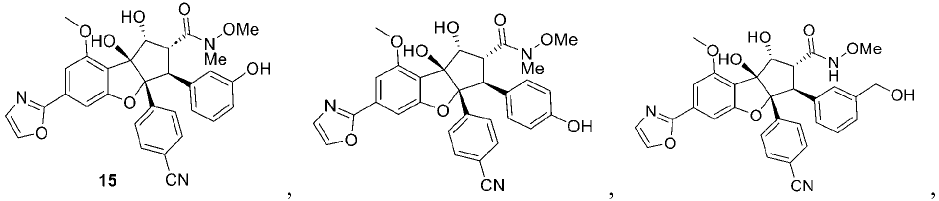

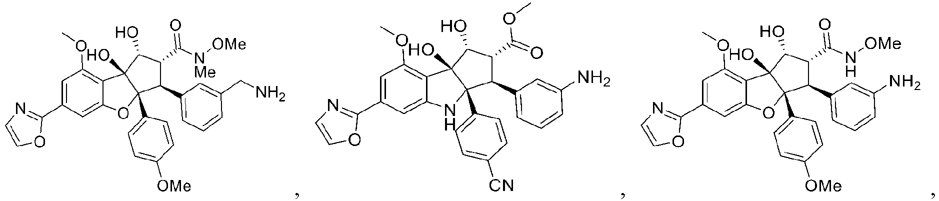

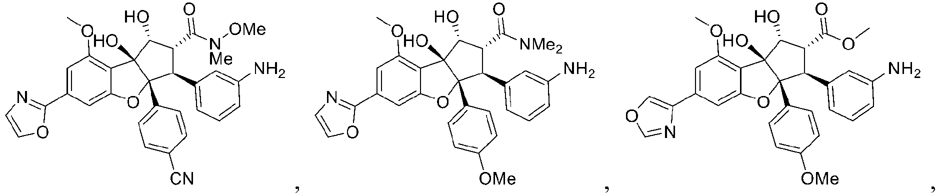

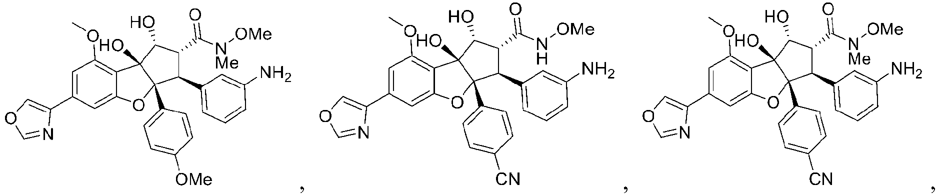

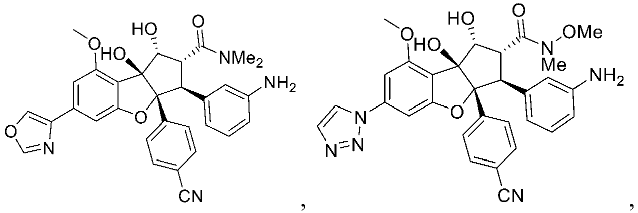

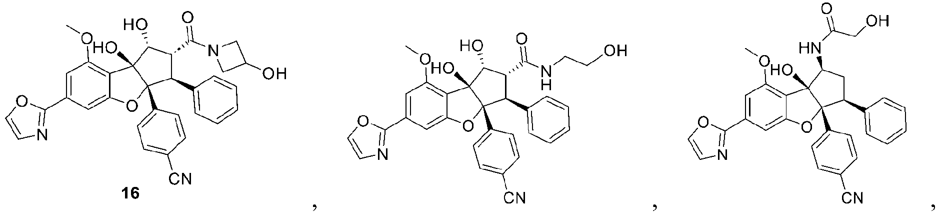

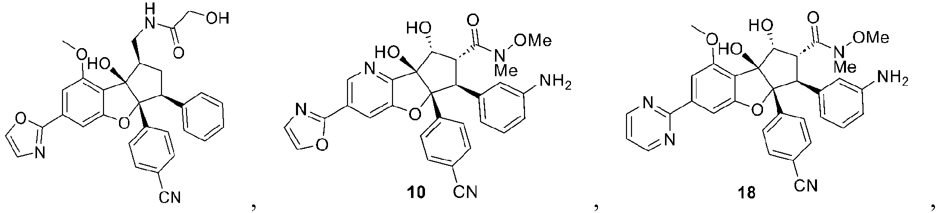

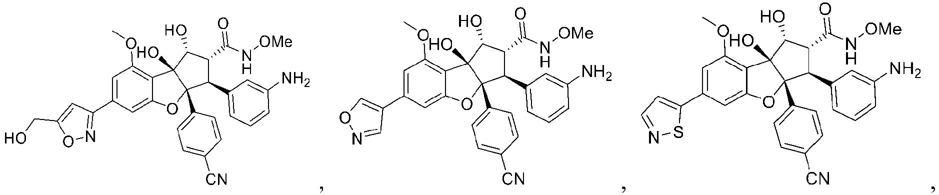

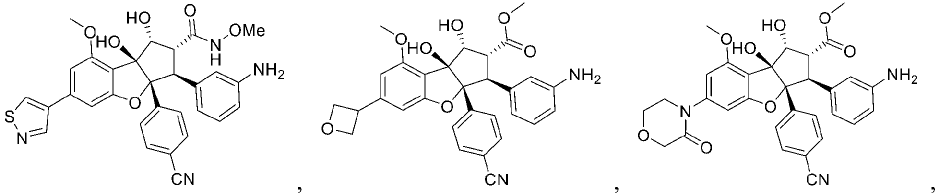

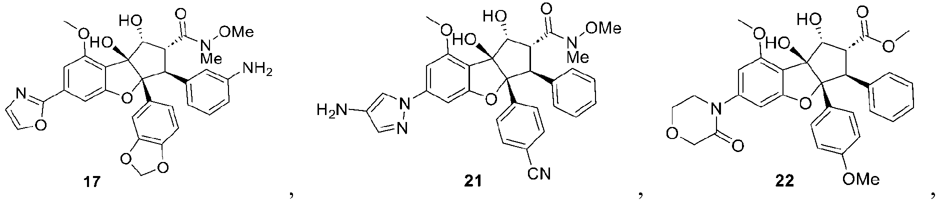

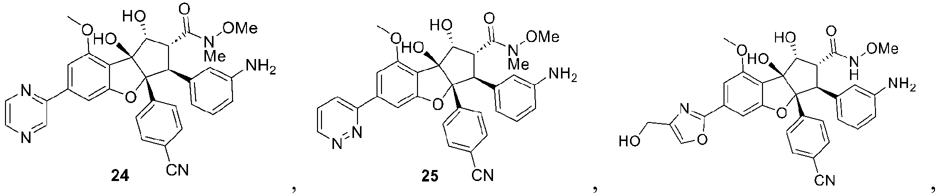

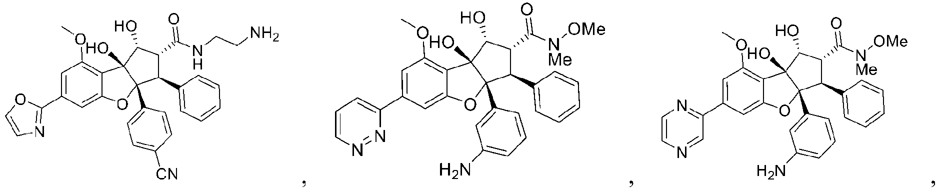

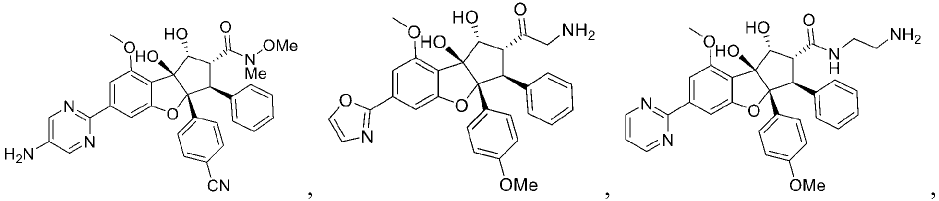

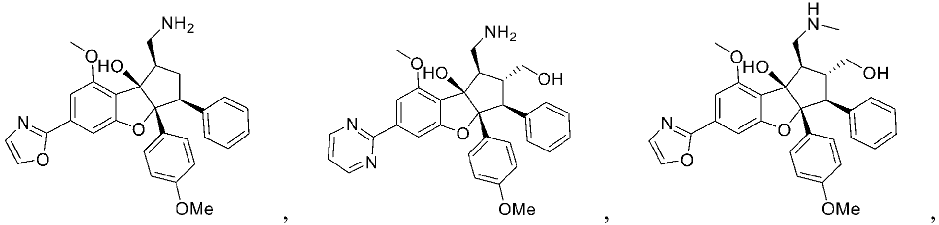

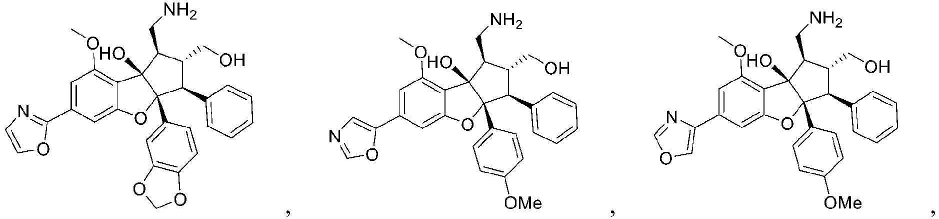

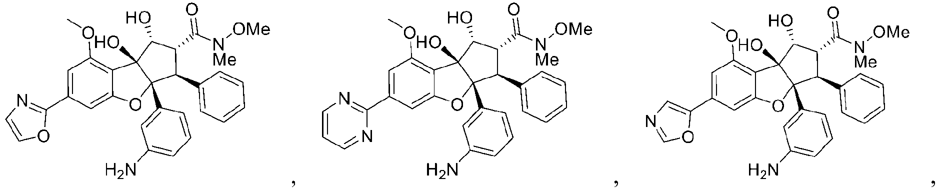

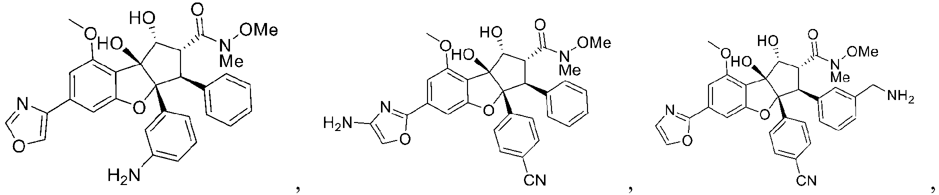

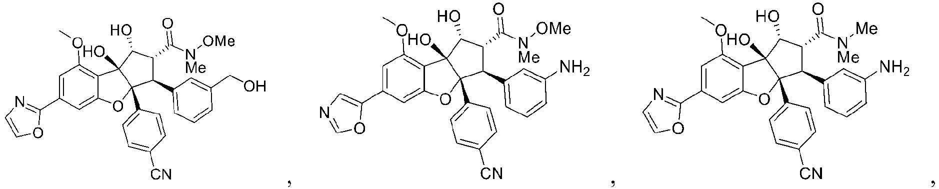

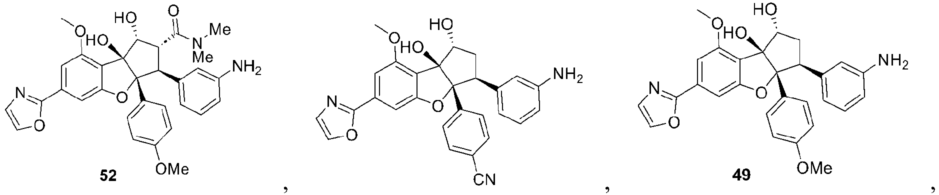

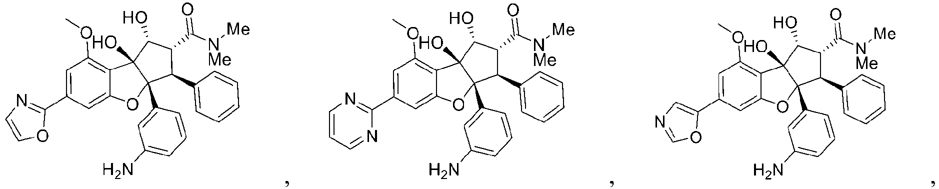

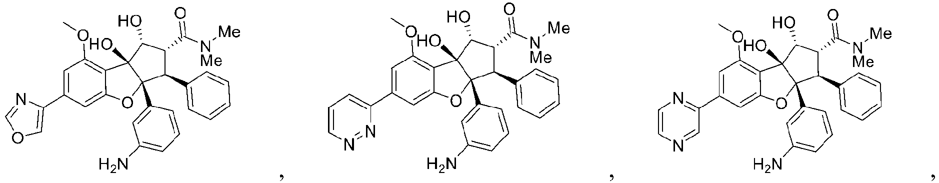

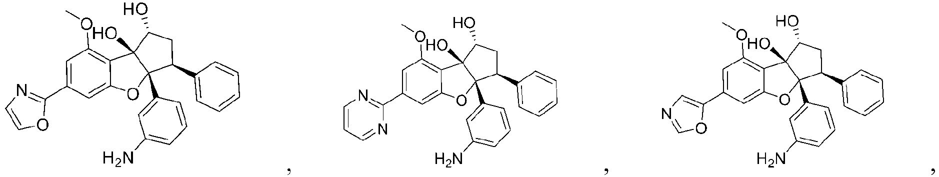

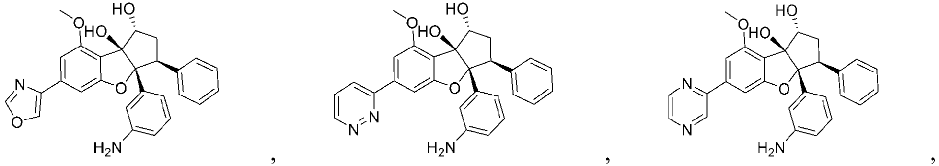

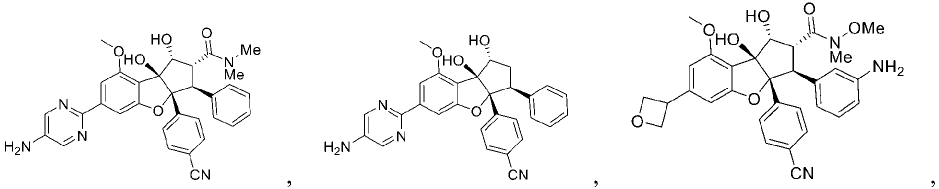

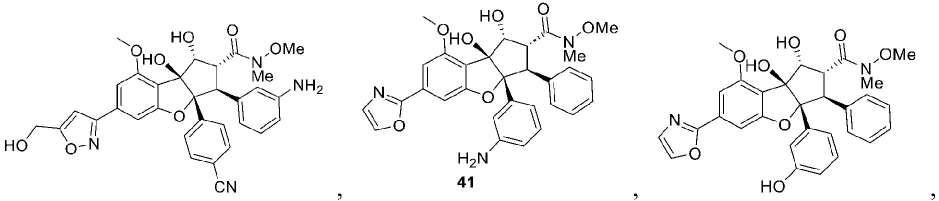

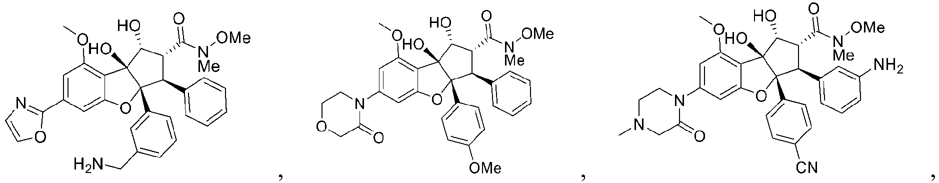

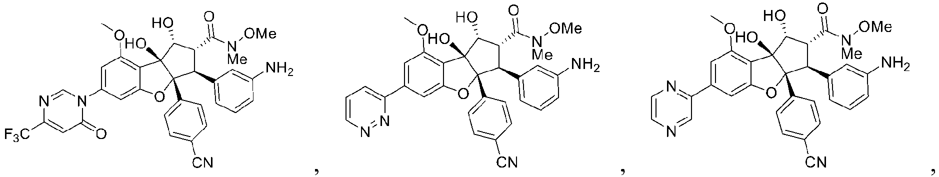

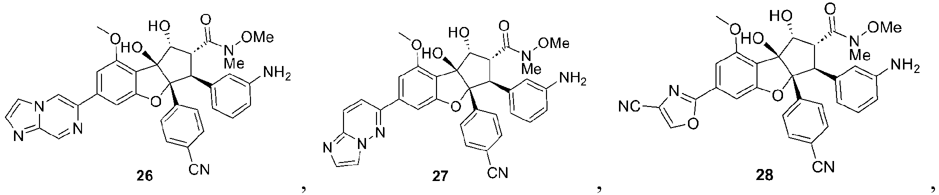

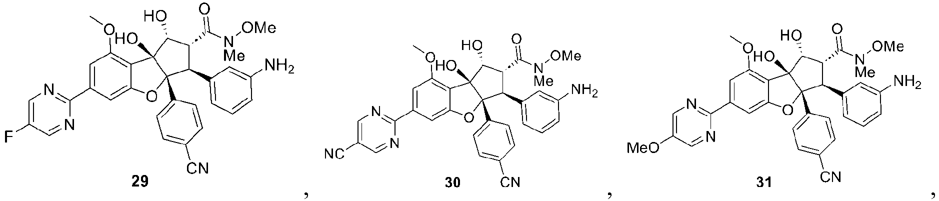

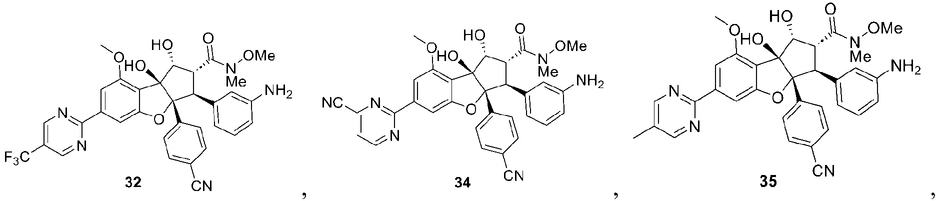

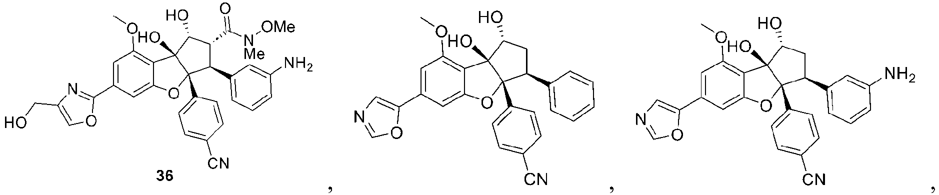

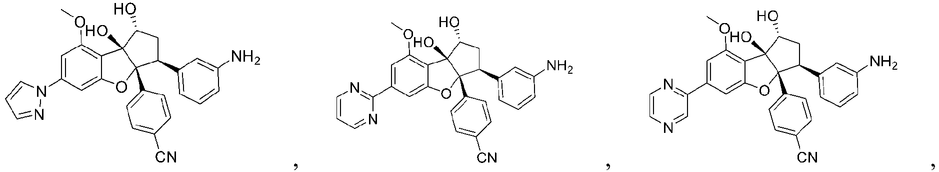

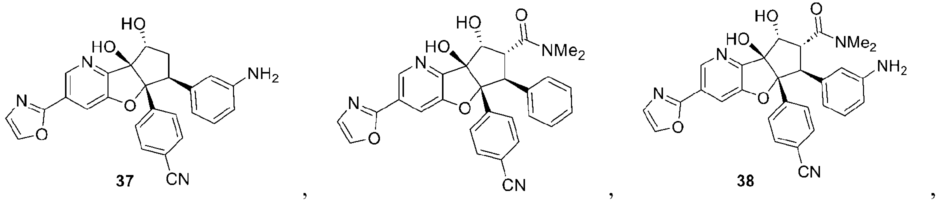

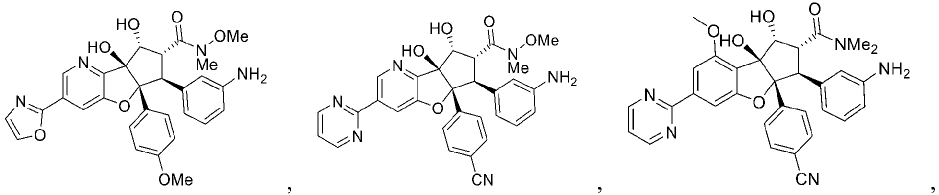

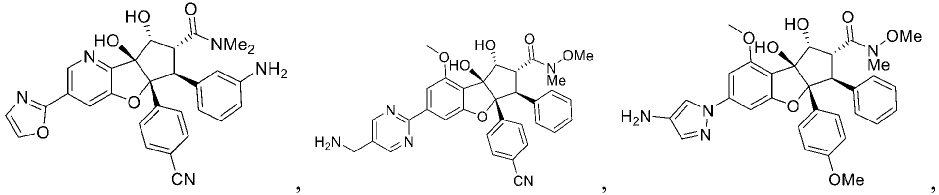

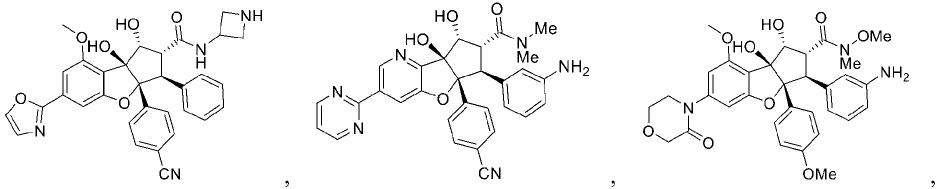

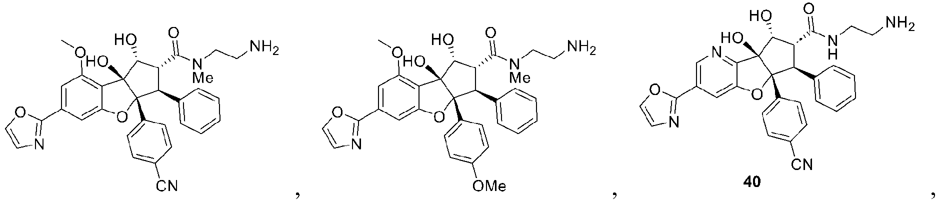

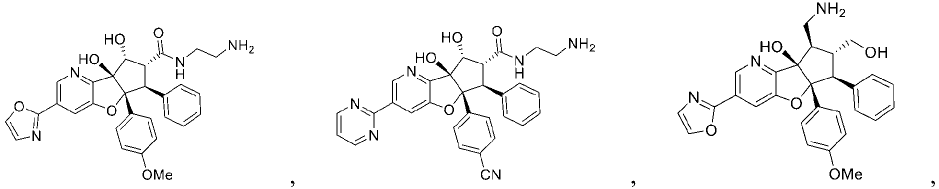

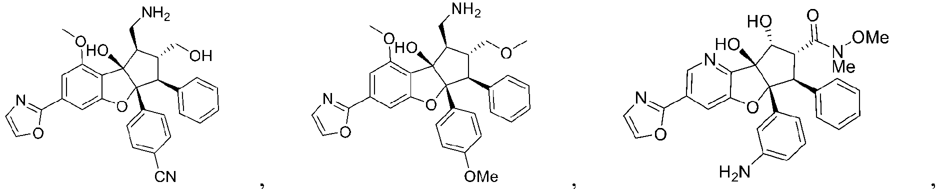

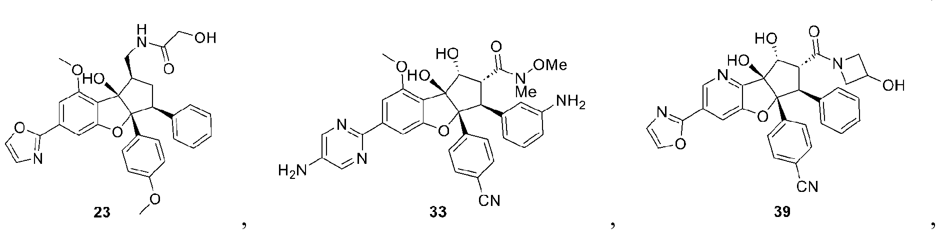

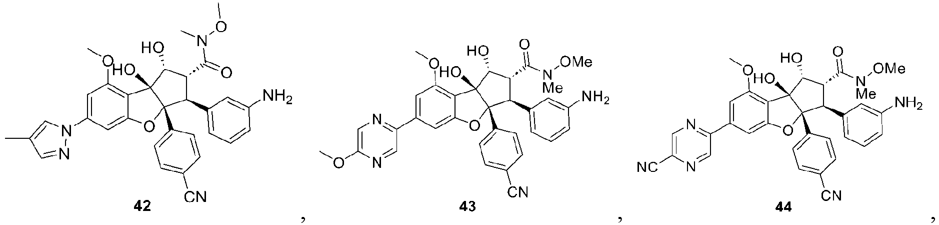

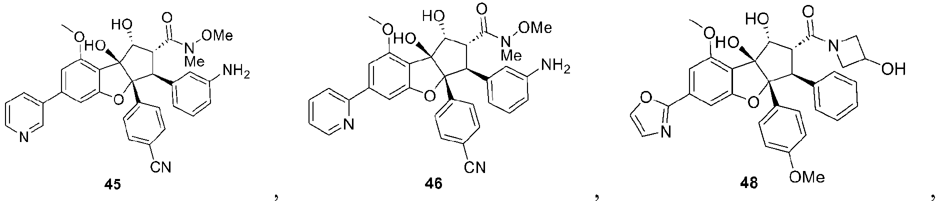

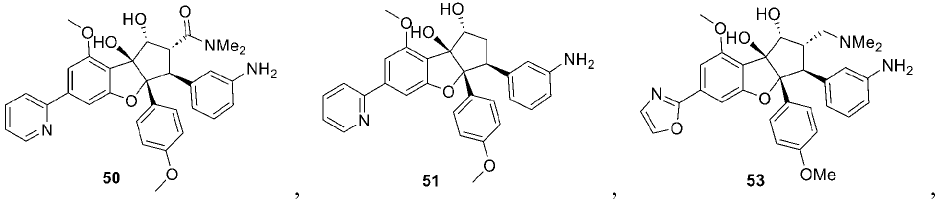

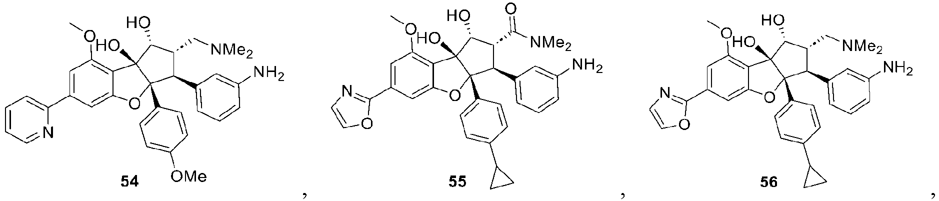

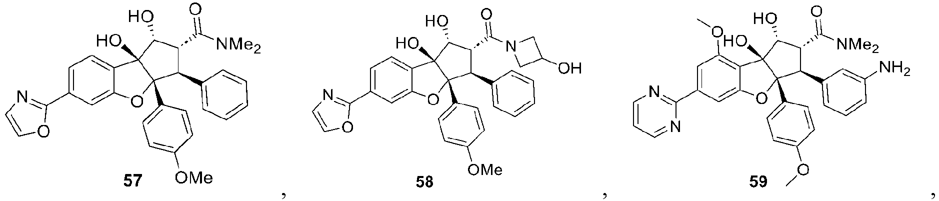

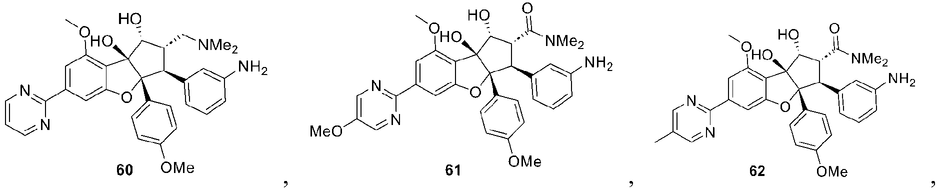

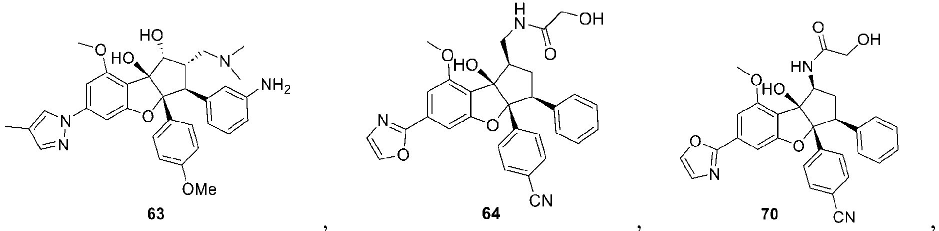

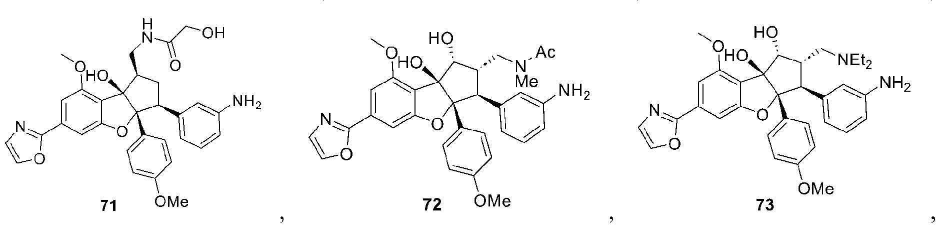

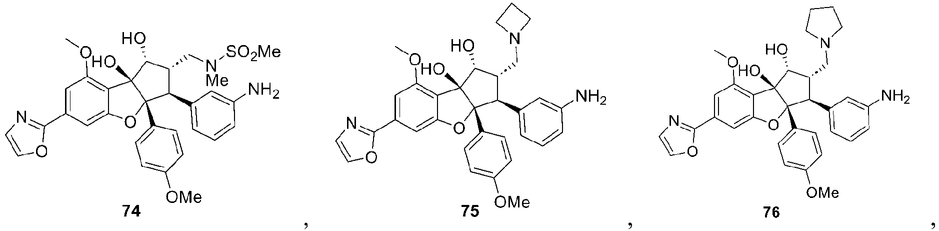

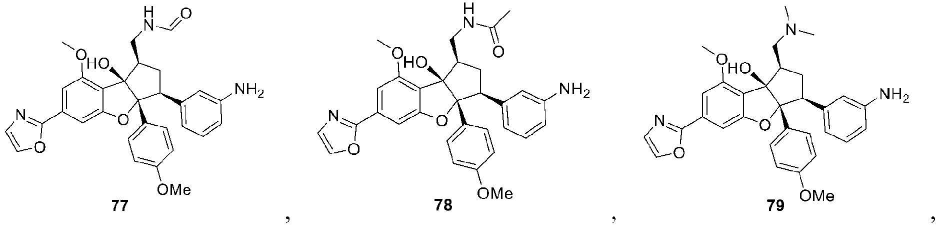

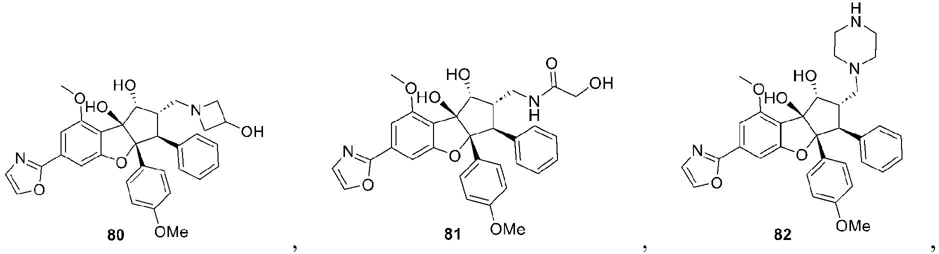

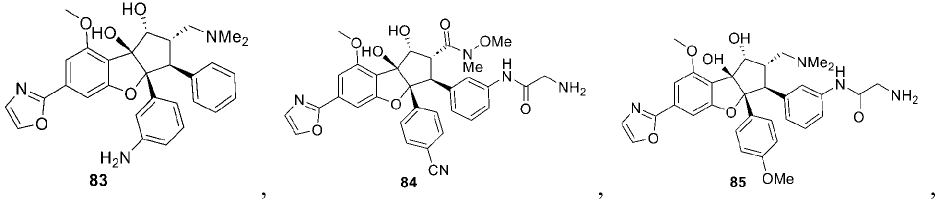

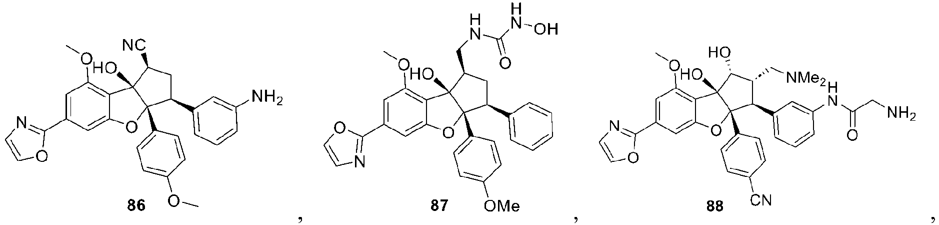

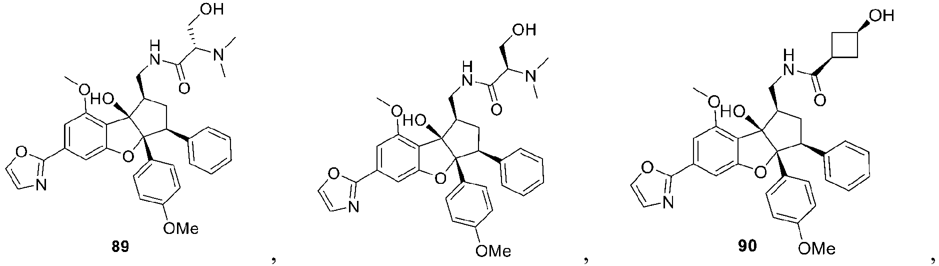

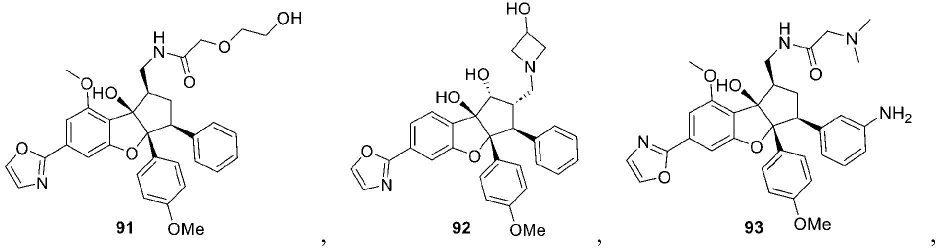

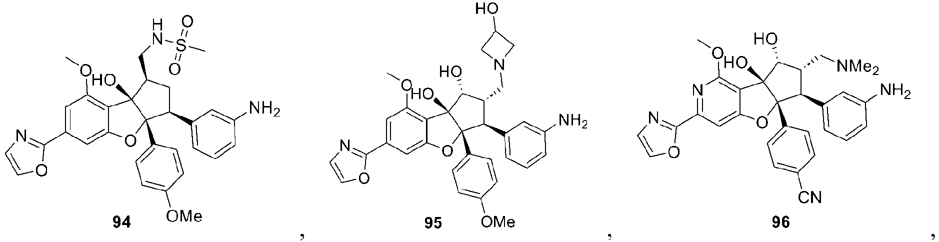

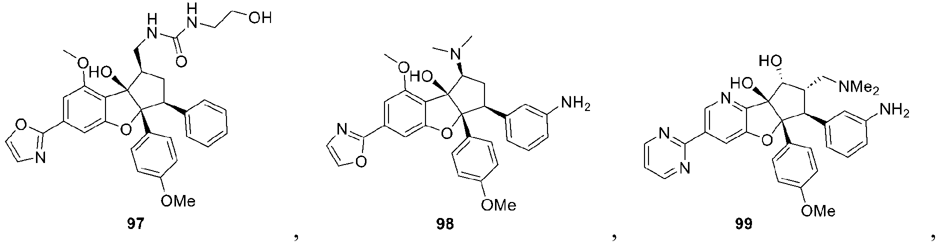

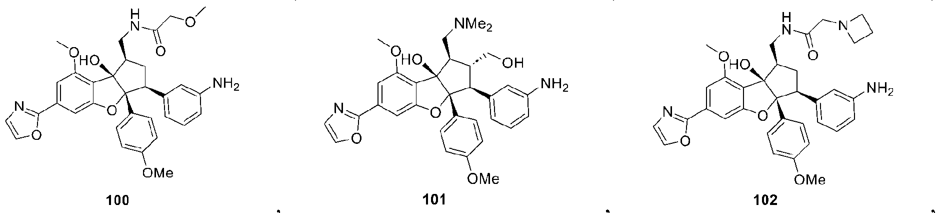

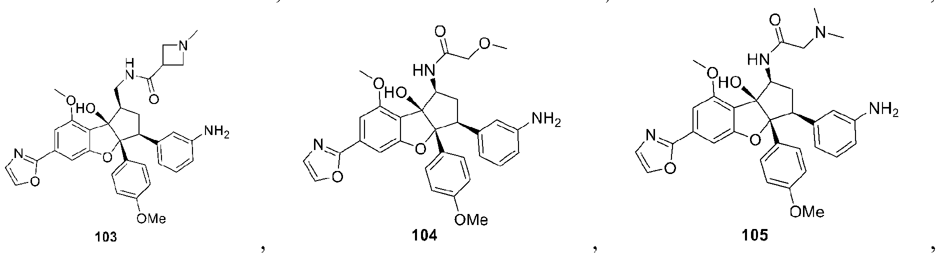

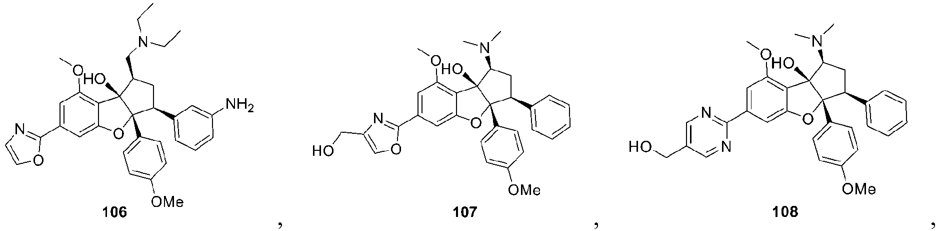

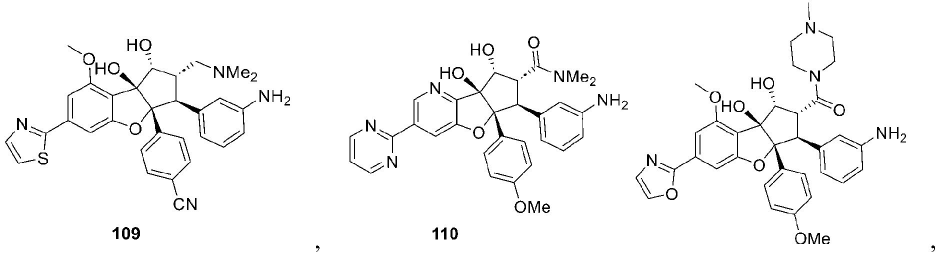

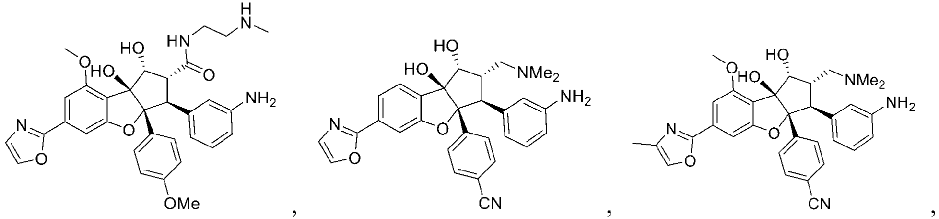

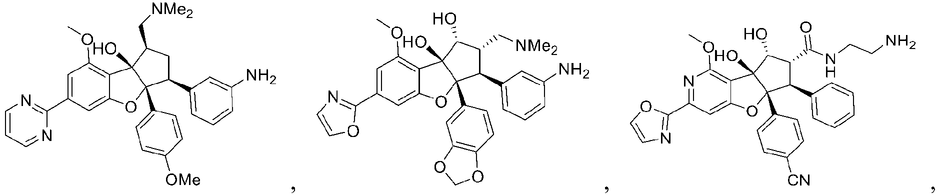

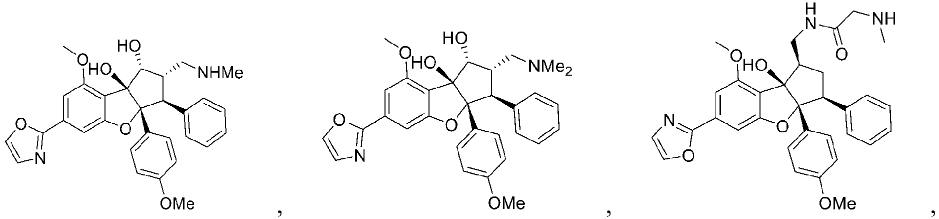

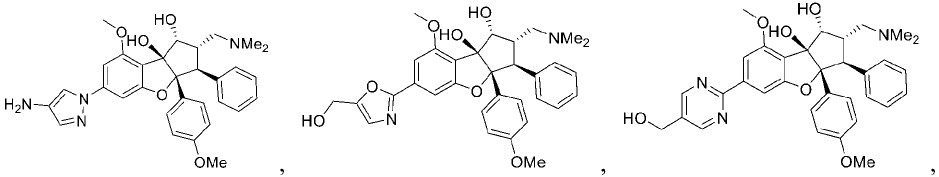

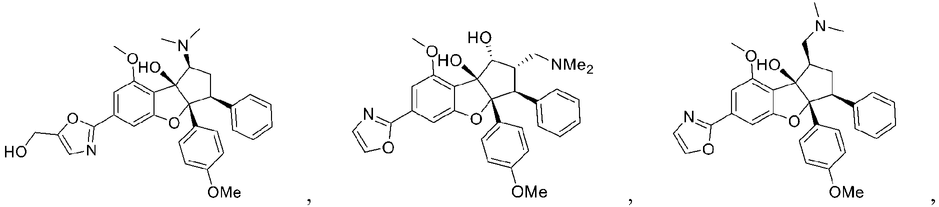

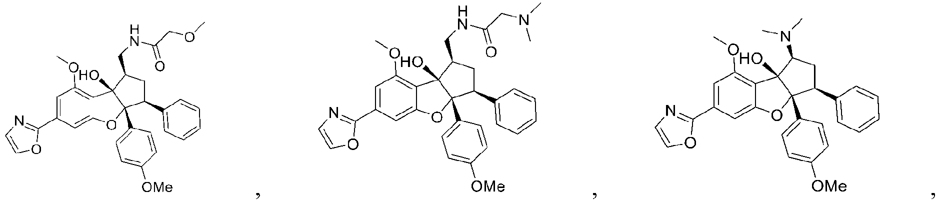

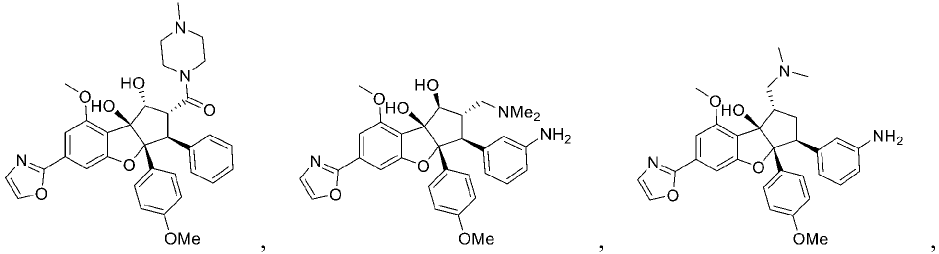

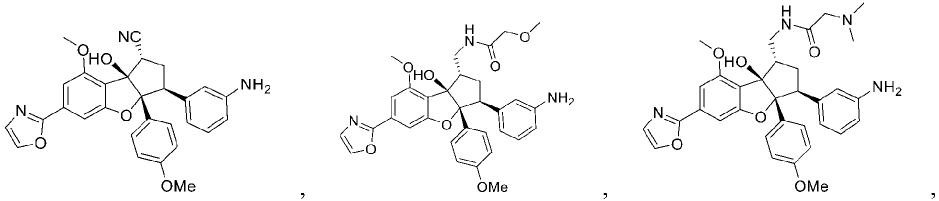

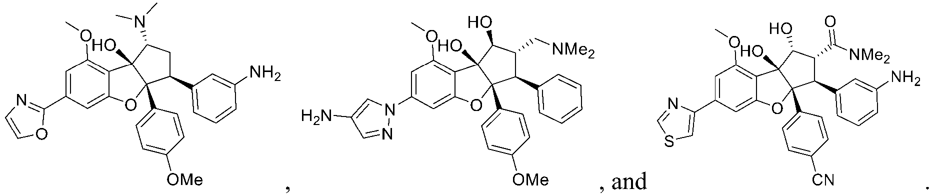

14. A pharmaceutical composition, comprising at least one of the compounds or the racemic mixtures, the enantiomers, the diastereoisomers, the pharmaceutically acceptable salts, or the isotopically labeled compounds thereof, or the mixtures of the aforementioned forms according to any one of claims 1-13, and one or more pharmaceutically acceptable carriers and/or excipients.

15. Use of the compound or the racemic mixture, the enantiomer, the diastereoisomer, the pharmaceutically acceptable salt, or the isotopically labeled compound thereof, or the mixture of the aforementioned forms according to any one of claims 1-13, or the pharmaceutical composition according to claim 14 in the preparation of a medicament for treating and/or preventing a disease or disorder or reducing the severity of the disease or disorder, wherein the disease or disorder is a tumor or cancer;
preferably, the tumor or cancer is selected from gastric cancer, colorectal cancer, breast cancer, ovarian cancer, and liver cancer.
